# EUROPEAN PATENT SPECIFICATION

(11) **EP 4 168 587 B1**
(45) Date of publication and mention of the grant of the patent: **21.10.2026**
(21) Application number: 21736665.7
(22) Date of filing: 17.06.2021
(51) Int. Cl.: C12Q 1/6886

(54) **METHODS FOR DETECTING AND PREDICTING CANCER AND/OR CIN3**
VERFAHREN ZUM NACHWEIS UND ZUR VORHERSAGE VON KREBS UND/ODER CIN3
PROCÉDÉS DE DÉTECTION ET DE PRÉDICTION D'UN CANCER ET/OU DE LA CIN3

(30) Priority: 17.06.2020 GB 202009224; 25.05.2021 GB 202107412
(43) Date of publication of application: 26.04.2023
(73) Proprietor: UCL Ventures Limited, London WC1E 6BT (GB)
(72) Inventor: WIDSCHWENDTER, Martin, London Greater London W1T 4TP (GB); BARRETT, James, London Greater London W1T 4TP (GB); JONES, Allison, London Greater London W1T 4TP (GB); EVANS, Iona, London Greater London W1T 4TP (GB)
(74) Representative: J A Kemp LLP
(86) International application number: PCT/GB2021/051536
(87) International publication number: WO 2021/255459

(56) References cited:
- WO-A1-2016/041010
- WO-A1-2016/109712
- WO-A1-2018/164577
- WO-A2-2013/033627
- WO-A2-2013/033627
- WO-A2-2020/115728
- FIRESTEIN GARY: "XX PR 01-SEP-2011. XX PA UNIV CALIFORNIA. XX QU DPT. XX PT Diagnosing, prognosing and treating rheumatoid arthritis or PT osteoarthritis comprises determining whether nucleic acid loci or PT genes in a test sample have methylation states indicative of PT rheumatoid arthritis or osteoarthritis XX SQ", 7 March 2013 (2013-03-07), pages 1 - 1, XP093326211, Retrieved from the Internet <URL:http://ibis.internal.epo.org/exam/dbfetch.jsp?id=GS_NUC_ALERT:WO2013033627.294280>
- MARKOWITZ SD: "BDC28869 standard; DNA; 476 BP. XX AC BDC28869; XX DT 25-AUG-2016 (first entry) XX DE Human genomic DNA, SEQ: 5589. XX KW adenocarcinoma; barretts esophagus; chemotherapy; cytostatic; KW diagnostic test; dna detection; dna methylation; ds; dysplasia; KW esophagus tumor; gastrointestinal-gen.; progno", 7 July 2016 (2016-07-07), pages 1 - 1, XP093326232, Retrieved from the Internet <URL:http://ibis.internal.epo.org/exam/dbfetch.jsp?id=GSN:BDC28869>
- SANJA A FARKAS ET AL: "Genome-wide DNA methylation assay reveals novel candidate biomarker genes in cervical cancer", EPIGENETICS, vol. 8, no. 11, 1 November 2013 (2013-11-01), pages 1213 - 1225, XP055203588, ISSN: 1559-2294, DOI: 10.4161/epi.26346
- RIJKERS M.: "Homo sapiens glycophorin C (Gerbich blood group) (GYPC) gene, GYPC*01_GX01.035 allele, complete cds", 12 December 2022 (2022-12-12), pages 1 - 2, XP093326269, Retrieved from the Internet <URL:http://ibis.internal.epo.org/exam/dbfetch.jsp?id=EM_STD:OP618340>
- DOUFEKAS KONSTANTINOS ET AL: "DNA Methylation Signatures in Vaginal Fluid Samples for Detection of Cervical and Endometrial Cancer :", INTERNATIONAL JOURNAL OF GYNECOLOGICAL CANCER, 1 June 2016 (2016-06-01), US, pages 1 - 6, XP055844119, ISSN: 1048-891X, Retrieved from the Internet <URL:http://dx.doi.org/10.1097/IGC.0000000000000739> DOI: 10.1097/IGC.0000000000000739
- BARTLETT THOMAS E. ET AL: "Epigenetic reprogramming of fallopian tube fimbriae in BRCA mutation carriers defines early ovarian cancer evolution", NATURE COMMUNICATIONS, vol. 7, no. 1, 1 September 2016 (2016-09-01), XP055844436, Retrieved from the Internet <URL:https://www.nature.com/articles/ncomms11620.pdf> DOI: 10.1038/ncomms11620
- VAN OSTADE XAVEER ET AL: "Candidate biomarkers in the cervical vaginal fluid for the (self-)diagnosis of cervical precancer", ARCHIVES OF GYNECOLOGY AND OBSTETRICS, SPRINGER VERLAG, BERLIN, DE, vol. 297, no. 2, 15 November 2017 (2017-11-15), pages 295 - 311, XP036391909, ISSN: 0932-0067, [retrieved on 20171115], DOI: 10.1007/S00404-017-4587-2

## Description

### FIELD OF THE INVENTION

The present invention relates to assays for predicting the presence, absence or development of endometrial cancer, by determining the methylation status of certain CpGs in a population of DNA molecules in a sample which has been taken from the individual, deriving an index value based on the methylation status of the certain CpGs, and predicting the presence, absence or development of cancer in the individual based on the cancer index value.

The project leading to this application has been funded by the European Commission's Horizon 2020 Research and Innovation Action, H2020 FORECEE under Grant Agreement No. 634570, the European Commission's Horizon 2020 European Research Council Executive Agency, H2020 BRCA-ERC under Grant Agreement No. 742432 as well as the charity, The Eve Appeal.

### BACKGROUND TO THE INVENTION

Endometrial cancer has become the most frequently occurring gynaecological cancer in developed countries. By 2030, it is expected that endometrial cancer will be the third most common cancer affecting women in the US after that of breast and thyroid. Approximately 20% of women with endometrial cancer present with high-risk and/or more advanced disease characteristics with an increased incidence of distant metastases and cancer-related death and hence in addition to surgery require adjuvant chemo- and radiotherapy which are associated with a high morbidity.

Therefore, a tool with which to diagnose earlier and predict specifically high-risk endometrial cancers is urgently required.

Imaging-based screening for endometrial cancers is not efficient. The performance characteristics of endometrial thickness and abnormalities for detection of endometrial cancer within one year of transvaginal ultrasound in a population-based nested case-control study of postmenopausal women showed a sensitivity of only 54.1% and a positive predictive value of 6.1%.

Recent evidence has shown that an assay (called PapSEEK) for mutations in 18 genes as well as an assay for aneuploidy in cervical brush samples is able to identify 81% of women presenting with endometrial cancer. However, in this study, the average age of cases and controls was 62 and 34 years, respectively. The consistent observation of a high allele frequency of pathogenic driver mutations in DNA from non-malignant normal endometrium with increasing age combined with the fact that the cases were almost twice as old as the controls makes it impossible to judge the true specificity of the PapSEEK test.

The inventors, along with other scientists, have shown in the past that DNA methylation in vaginal fluid or cervical smears may be able to identify women with endometrial cancer. All of these studies were relatively small and did not validate the findings in a large data set. In addition, none of these studies focused specifically on high risk endometrial cancers and, most importantly, none assessed cohort-based samples from women prior to the diagnosis of the disease.

Here, the inventors have developed and validated a DNA methylation signature in samples, particularly cervical smear samples, which is capable of both diagnosing and predicting the risk of developing cancer.

### SUMMARY OF THE INVENTION

In the center of the invention is an assay for assessing the presence, absence or development of endometrial cancer by means of the methylation marker in here that is represented with Sequence Identity Number 67, wherein the CpG is identified at nucleotide positions 61 to 62.

The current inventors set out to understand whether DNAme (DNA methylation) profiles may be used to detect the presence or absence of cancer, particularly endometrial and ovarian cancer. The inventors also set out to understand whether said DNAme profiles may be associated with the development of cancer, and therefore whether such profiles may be capable of functioning as surrogate markers for individual stratification purposes in connection with cancer.

In this regard the inventors have succeeded in developing assays involving "cancer index values" which are derived from and associated with DNAme profiles established from samples from tissue comprising epithelial cells from a given individual. The sample may particularly be derived from the cervix, the vagina, the buccal area, blood and/or urine. The sample is preferably a cervical liquid-based cytology sample, and more preferably a cervical smear sample. Subsequent DNAme profiles from a given individual, and which values can be used to stratify the individual in connection with endometrial cancer status. A preferred sample for use in any of the assays described and defined herein is a cervical liquid-based cytology sample. A particularly preferred sample for use in any of the assays described and defined herein is a cervical smear sample.

Accordingly, in contrast to prior art assays, the inventors have surprisingly determined that it is possible to derive "cancer index values" derived from and associated with DNAme profiles established from samples, wherein the samples are free of tumor-derived DNA. Thus the tissue(s) from which DNAme profiles of the present assays are established may act to provide surrogate markers for the presence, absence or development of cancer, wherein tumor cells, if present, or cells at risk of transforming into tumor cells, are located at an anatomical site distinct from the site from which the sample was taken.

The cancer index value is determined from data relating to the methylation status of one or more CpGs in a panel of CpGs as further defined and described herein. CpGs of the panel are methylation sites in DNA from cells derived from/obtained from samples comprising epithelial cells. The sample may particularly be derived from the cervix, the vagina, the buccal area, blood and/or urine. The sample is preferably a cervical liquid-based cytology sample, and more preferably a cervical smear sample.

For the purposes of the present invention, the cancer index value may be used interchangeably herein with "WID-EC-Index", "WID-Index", "cancer index", "index" or "index value" (WID = women's risk identification). Furthermore, any reference to a cancer index value in the context of the present invention, may be equally used for the assessment of the presence, absence or development of endometrial cancer and/or ovarian cancer in an individual.

Based on studies with patients known to be free of endometrial cancer, the inventors have established cancer index values, using specific panels of CpGs, which have been determined to be associated with/characteristic of endometrial tissue which is negative for endometrial cancer, i.e. normal endometrial tissue which is free of endometrial cancer. Based on studies with patients known to possess endometrial cancer, the inventors have established cancer index values which have been determined to be associated with/characteristic of endometrial tissue which is positive for endometrial cancer.

The inventors have further established that the same specific panels of CpGs that associate with endometrial tissue which is negative or positive for endometrial cancer may likewise be associated with ovarian tissue that is negative or positive for ovarian cancer and/or cervical tissue that is negative or positive for CIN3. Based on studies with patients known to be free of ovarian endometrial cancer, the inventors have established cancer index values, using specific panels of CpGs, which have been determined to be associated with/characteristic of ovarian tissue which is negative for ovarian cancer, i.e. normal ovarian tissue which is free of ovarian cancer, and/or of cervical tissue which is negative for CIN3. Based on studies with patients known to possess ovarian endometrial cancer, the inventors have established cancer index values which have been determined to be associated with/characteristic of ovarian tissue which is positive for ovarian cancer and or cervical tissue which is positive for CIN3.

Thus, the inventors have been able to establish cancer index values, using specific panels of CpGs, which can characterize an individual as having endometrial cancer or not having endometrial cancer, or having a high risk of endometrial cancer development.

By determining the methylation profile-based cancer index value from a sample derived from the individual, the individual may be seen to possess a cancer index value which correlates with those possessed by individuals which are known, via the inventor's studies described herein, to be cancer positive or negative. Such correlations have been determined with a high degree of statistical accuracy, particularly with respect to parameters relevant to biological assays such as receiver operating characteristics (ROC) sensitivity and specificity, as well as area under the curve (AUC). Accordingly, by determining the cancer index value from a sample from a given individual, the individual may be determined to possess endometrial and/or ovarian tissue which is positive for cancer, i.e. the individual is diagnosed as having endometrial and/or ovarian endometrial cancer, most preferably endometrial cancer. Conversely, by determining the cancer index value from a sample from a given individual, the individual may be determined to possess endometrial and/or ovarian tissue which is negative for cancer, i.e. the individual is diagnosed as not having endometrial and/or ovarian cancer, most preferably endometrial cancer.

Assessing the development of endometrial cancer in accordance with the assays of the invention may refer to assessing an increased or decreased likelihood of cancer development. Assessing of the development of endometrial cancer in accordance with the assays of the invention may refer to assessing progression or worsening of a preexisting cancer lesion in an individual. Assessment of the development of endometrial cancer in accordance with the assays of the invention may refer to predicting the likelihood of recurrence of cancer.

The observation that the cancer index value discussed herein correlates with the stage and severity of cancer in women indicates that the cancer index value can act as a surrogate marker for indicating the severity of cancer in an individual. These observations from the current inventors further establish that the cancer index value, as further described and defined herein, is dynamic and can change according to, at least, the stage and severity of the cancer. The cancer index value may therefore be used to monitor an individual's cancer status and risk of cancer development. Moreover, the cancer index value may be used to monitor the efficacy of cancer treatments being administered to an individual, including therapeutic treatments and preventative treatments.

Accordingly, in the context of the present invention, by determining the cancer index value from a sample from a given individual it is possible to assess the presence, absence or development of cancer in an individual, or in other words to stratify the individual for cancer. In the context of the present invention, stratification for endometrial cancer is the process of categorizing the individual as being a member of a group of individuals who possess a phenotype in connection with cancer, including the presence or absence of cancer in the individual, or the development of cancer, i.e. by having epithelial cells, particularly derived from the cervix, the vagina, the buccal area, blood and/or urine which is more characteristic of endometrial or ovarian tissue which is cancer positive than endometrial or ovarian tissue which is cancer negative, or which is more characteristic of cervical tissue which is CIN3 positive than cervical tissue which is CIN3 negative. The sample is preferably a cervical liquid-based cytology sample, and more preferably a cervical smear sample.

As explained herein, the assays of the invention are based on a cancer index value derived from a methylation profile from DNA originating from samples comprising epithelial cells. The sample may particularly be derived from the cervix, the vagina, the buccal area, blood and/or urine. The sample is preferably a cervical liquid-based cytology sample, and more preferably a cervical smear sample. Accordingly, the assays provide means for correlating samples derived from the cervix, the vagina, the buccal area, blood and/or urine-derived DNA methylation profile with a status connected with endometrial cancer ranging from the individual being cancer negative, to the individual being cancer positive, with high statistical accuracy. Because the assays of the invention provide a correlation between the methylation profile and the disease status, the skilled person will appreciate that as part of the stratification process and outcome, disease status is assigned on the basis of a likelihood. As such, the methods of the invention provide assays which are predictive of an individual's status with respect to cancer. The assays of the invention accordingly provide means for predicting the presence or absence of endometrial endometrial cancerin an individual. The assays of the invention accordingly also provide means for predicting the development of endometrial cancer in an individual. The assays of the invention can provide means for predicting the development of endometrial cancer in an individual since the inventors have demonstrated that specific cancer index values can define endometrial tissue which is cancer negative, whilst others can define endometrial tissue which is cancer positive, and since the specific cancer index values may be dynamic and thereby increased in association with known risk factors associated with endometrial cancer the values may be subject to change along a scale of endometrial cancerrisk.

Whilst disease status may be assigned on the basis of a likelihood, the inventors have demonstrated herein that correlations between DNA methylation profile and cancer status using cancer index values can be achieved with a very high degree of statistical accuracy using parameters relevant to biological assays, as described further herein. As such, the assays of the invention provide means for predicting the presence or absence of endometrial endometrial cancerin an individual and for predicting the development of cancer in an individual, and for stratifying an individual for cancer, and wherein the prediction/stratification can be defined to be statistically highly reliable and robust. This in turn means that the prediction/stratification can be made with a high level of confidence. The assays of the invention can be defined to be statistically accurate by means known in the art, as further described and defined herein. The assays of the invention can be defined according to parameters relating to their statistical specificity and sensitivity. These parameters define the likelihood of false positive and false negative test results. The lower the proportion of false positive and false negative test results the more statistically accurate the assay becomes. In this regard the inventors have established CpG panels, as described and defined further herein, wherein the methylation status of CpGs in the panel can be used to establish cancer index values such that the assays produce statistically accurate predictions of cancer status. Accordingly, the inventors have determined that the assays described herein may be defined according to statistical parameters such as percentage specificity and sensitivity and also by receiver operating characteristics (ROC) area under the curve (AUC). All such means are known in the art and are known to be defined measures of statistical accuracy for biological assays such as those described and defined herein.

Thus the methods of the invention provide assays which can be used, with a high degree of statistical accuracy, to predict the presence, absence or development of cancer. The methods of the invention provide assays which can be used, with a high degree of statistical accuracy, to stratify an individual with respect to cancer status. Accordingly, the methods of the invention provide useful information to individuals and their physicians concerning patient cancer status. This information may help inform actual therapeutic treatment measures if the presence of cancer is identified in the individual. The information may help to monitor the progress of therapeutic treatment measures in the individual by monitoring changes in the cancer index value over the course of a period of time. The information may help to monitor the progress of prophylactic or preventative treatment measures in the individual by monitoring changes in the cancer index value over the course of a period of time. As such the methods of the invention offer significant advantages in the personalized prevention and early detection as well as treatment and management of cancer in individuals.

Accordingly, the invention provides an assay for assessing the presence, absence or development of endometrial cancer in an individual, the assay comprising:
1. providing a sample which has been taken from the individual, the sample comprising a population of DNA molecules;
2. determining in the population of DNA molecules in the sample the methylation status of a panel of:
   i. one or more CpGs comprising the CpG identified in SEQ ID NO 67 wherein the CpG is comprising the CpG identified in SEQ ID NO 67 wherein the CpG isidentified at nucleotide positions 61 to 62
3. deriving a cancer index value based on the methylation status of the one or more CpGs in the panel; and
4. assessing the presence, absence or development of endometrial endometrial cancerin the individual based on the cancer index value;
wherein the assay is characterised as having an area under the curve (AUC) of 0.60 or more as determined by receiver operating characteristics (ROC).

The assay of the invention may be performed above and additionally wherein the panel of one or more CpGs comprises at least 50 CpGs selected from the CpGs identified at nucleotide positions 61 to 62 in SEQ ID NOs 1 to 500, preferably wherein the assay is characterised as having an AUC of at least 0.90.

The assay of the invention may be performed above and additionally wherein the panel of one or more CpGs comprises at least the CpGs identified in SEQ ID NOs 1 to 50 and identified at nucleotide positions 61 to 62, preferably wherein the assay is characterised as having an AUC of at least 0.95.

The assay of the invention may be performed above and additionally wherein the panel of one or more CpGs comprises at least 100 CpGs selected from the CpGs identified at nucleotide positions 61 to 62 in SEQ ID NOs 1 to 500, preferably wherein the assay is characterised as having an AUC of at least 0.93.

The assay of the invention may be performed above and additionally wherein the panel of one or more CpGs comprises at least the CpGs identified in SEQ ID NOs 1 to 100 and identified at nucleotide positions 61 to 62, preferably wherein the assay is characterised as having an AUC of at least 0.96.

The assay of the invention may be performed above and additionally wherein the panel of one or more CpGs comprises at least 150 CpGs selected from the CpGs identified at nucleotide positions 61 to 62 in SEQ ID NOs 1 to 500, preferably wherein the assay is characterised as having an AUC of at least 0.93.

The assay of the invention may be performed above and additionally the panel of one or more CpGs comprises at least the CpGs identified in SEQ ID NOs 1 to 150 and identified at nucleotide positions 61 to 62, preferably wherein the assay is characterised as having an AUC of at least 0.96.

The assay of the invention may be performed above and additionally wherein the panel of one or more CpGs comprises at least 200 CpGs selected from the CpGs identified at nucleotide positions 61 to 62 in SEQ ID NOs 1 to 500, preferably wherein the assay is characterised as having an AUC of at least 0.93.

The assay of the invention may be performed above and additionally wherein the panel of one or more CpGs comprises at least the CpGs identified in SEQ ID NOs 1 to 200 and identified at nucleotide positions 61 to 62, preferably wherein the assay is characterised as having an AUC of at least 0.96.

The assay of the invention may be performed above and additionally wherein the panel of one or more CpGs comprises at least the 500 CpGs identified at nucleotide positions 61 to 62 in SEQ ID NOs 1 to 500, and further wherein the assay is characterised as having an AUC of at least 0.97.

The assay of the invention may be performed above and additionally wherein the step of determining in the population of DNA molecules in the sample the methylation status of the one or more CpGs in the panel comprises determining a β value of each CpG.

The assay of the invention may be performed above and additionally wherein the step of deriving the cancer index value based on the methylation status of the one or more CpGs in the panel comprises:
1. providing a methylation β-value data set comprising the methylation β-values for each CpG in the panel;
2. providing a mathematical model capable of generating the cancer index from the methylation β-value data set; and
3. applying the mathematical model to the methylation β-value data set, thereby generating the cancer index.

The assay of the invention may be performed above and additionally wherein the cancer index value is a WID-EC-Index cancer index value , and wherein the mathematical model which is applied to the methylation β-value data set to generate the cancer index is an algorithm according to the following formula: $WID - EC - index = {\sum }_{i = 1}^{n} \frac{{w}_{i} {β}_{i} - μ}{σ}$ wherein:
1. *β*₁, ... , *βₙ* are methylation beta-values (between 0 and 1);
2. *w*₁, ... , *w*₅₀₀ are real valued coefficients;
3. µ and σ are real valued parameters used to scale the index; and
*4. n* refers to the number of CpGs in the panel of one or more CpGs; preferably wherein the cancer is endometrial cancer.

The assay of the invention may be performed above and additionally wherein when the cancer index value for the individual is about -0.201 or more, the individual is assessed as having endometrial cancer or as having a high risk of endometrial cancer development, or wherein when the cancer index value for the individual is less than about -0.201, the individual is assessed as not having endometrial cancer or as having a low risk of endometrial cancer development, preferably wherein:
1. the panel of one or more CpGs comprises at least 50 of the CpGs identified at nucleotide positions 61 to 62 in SEQ ID NOs 1 to 500, and wherein the sensitivity is at least 88% and the specificity is at least 76%;
2. the panel of one or more CpGs comprises at least the CpGs defined by SEQ ID NOs 1 to 50 and identified at nucleotide positions 61 to 62, and wherein the sensitivity is at least 90% and specificity is at least 80%;
3. the panel of one or more CpGs comprises at least the CpGs defined by SEQ ID NOs 1 to 100 and identified at nucleotide positions 61 to 62, and wherein the sensitivity is at least 92% and specificity is at least 79%; or
4. the panel of one or more CpGs comprises at least the CpGs defined by SEQ ID NOs 1 to 150 and identified at nucleotide positions 61 to 62, and wherein the sensitivity is at least 93% and specificity is at least 80%;
preferably wherein the assay comprises determining methylation β-values for each CpG in the panel of one or more CpGs, more preferably wherein the cancer is endometrial cancer.

The assay of the invention may be performed above and additionally wherein when the cancer index value for the individual is about 0.269 or more, the individual is assessed as having endometrial cancer, or as having a high risk of endometrial cancer development, or wherein when the cancer index value for the individual is less than about 0.269, the individual is assessed as not having endometrial cancer or as having a low risk of endometrial cancer development, preferably wherein:
1. the panel of one or more CpGs comprises at least 50 of the CpGs identified at nucleotide positions 61 to 62 in SEQ ID NOs 1 to 500, and wherein the sensitivity is at least 75% and the specificity is at least 94%;
2. the panel of one or more CpGs comprises at least the CpGs defined by SEQ ID NOs 1 to 50 and identified at nucleotide positions 61 to 62, and wherein the sensitivity is at least 73% and specificity is at least 98%;
3. the panel of one or more CpGs comprises at least the CpGs defined by SEQ ID NOs 1 to 100 and identified at nucleotide positions 61 to 62, and wherein the sensitivity is at least 75% and specificity is at least 98%; or
4. the panel of one or more CpGs comprises at least the CpGs defined by SEQ ID NOs 1 to 150 and identified at nucleotide positions 61 to 62, and wherein the sensitivity is at least 79% and specificity is at least 97%;
preferably wherein the assay comprises determining methylation β-values for each CpG in the panel of one or more CpGs, more preferably wherein the cancer is endometrial cancer.

The assay of the invention may be performed above and additionally wherein when the cancer index value for the individual is about 1.072 or more, the individual is assessed as having endometrial cancer or as having a high risk of endometrial cancer development, or wherein when the cancer index value for the individual is less than about 1.072, the individual is assessed as not having endometrial cancer or as having a low risk of endometrial cancer development, preferably wherein:
1. the panel of one or more CpGs comprises at least 50 of the CpGs identified at nucleotide positions 61 to 62 in SEQ ID NOs 1 to 500, and wherein the sensitivity is at least 58% and the specificity is at least 99%;
2. the panel of one or more CpGs comprises at least the CpGs defined by SEQ ID NOs 1 to 50 and identified at nucleotide positions 61 to 62, and wherein the sensitivity is at least 60% and specificity is 100%;
3. the panel of one or more CpGs comprises at least the CpGs defined by SEQ ID NOs 1 to 100 and identified at nucleotide positions 61 to 62, and wherein the sensitivity is at least 63% and specificity is 100%; or
4. the panel of one or more CpGs comprises at least the CpGs defined by SEQ ID NOs 1 to 150 and identified at nucleotide positions 61 to 62, and wherein the sensitivity is at least 64% and specificity is 100%;
preferably wherein the assay comprises determining methylation β-values for each CpG in the panel of one or more CpGs, more preferably wherein the cancer is endometrial cancer.
The assay of the invention may be performed above and additionally wherein when the cancer index value for the individual is:
1. less than about -0.660 the individual is assessed as not having endometrial cancer;
2. about -0.660 or more and less than about -0.430 the individual is assessed as having a low risk of endometrial cancer development;
3. about -0.430 or more and less than about -0.230 the individual is assessed as having a moderate risk of endometrial cancer development; or
4. about -0.230 or more the individual is assessed as having a high risk of endometrial cancer development;
preferably wherein the assay comprises determining methylation β-values for each CpG in the panel of one or more CpGs, more preferably wherein the cancer is endometrial cancer.

The assay of the invention may be performed above and additionally wherein the step of determining the methylation status of a panel of one or more CpGs comprises determining the methylation status of one or more CpGs denoted by CG identified in a panel of one or more DMRs defined by SEQ ID NOs 501 to 808, optionally wherein the panel of one or more CpGs comprises two or more CpGs denoted by CG identified in the panel of DMR(s), three or more CpGs denoted by CG identified in the panel of DMR(s), four or more CpGs denoted by CG identified in the panel of DMR(s), or all CpGs denoted by CG identified in the DMR(s) defined by SEQ ID NOs 501 to 808.

The assay of the invention may be performed above and additionally wherein the step of determining the methylation status of a panel of the one or more CpGs comprises determining the methylation status of five or more, six or more, seven or more, eight or more, or nine or more, or all of the CpGs denoted by CG within any one or more of the DMRs defined by SEQ ID NOs 501 to 808.

The assay of the invention may be performed above and additionally wherein the step of determining the methylation status of a panel of one or more CpGs comprises determining the methylation status of two or more, three or more, four or more, five or more, six or more, seven or more, eight or more, or nine or more, or all of the CpGs denoted by CG within:
1. any combination of two, three, four, five, six, seven, eight, or nine or more of DMRs 1 to 308;
2. any combination of ten, twenty, thirty, forty, fifty, sixty, seventy, eighty, or ninety or more of DMRs 1 to 308;
3. all 308 of DMRs 1 to 308;
4. one DMR defined by SEQ ID NO: 501, two DMRs defined by SEQ ID NOs: 501 to 502, three DMRs defined by SEQ ID NOs: 501 to 503, four DMRs defined by SEQ ID NOs: 501 to 504, five DMRs defined by SEQ ID NOs: 501 to 505, six DMRs defined by SEQ ID NOs: 501 to 506, seven DMRs defined by SEQ ID NOs: 501 to 507, eight DMRs defined by SEQ ID NOs: 501 to 508, or nine DMRs defined by SEQ ID NOs: 501 to 509; or
5. one DMR defined by SEQ ID NOs 922, 924, or 920, preferably within all of the DMRs defined by SEQ ID NOs 922, 924 and 920;
6. ten DMRs defined by SEQ ID NOs: 501 to 510, twenty DMRs defined by SEQ ID NOs: 501 to 520, thirty DMRs defined by SEQ ID NOs: 501 to 530, forty DMRs defined by SEQ ID NOs: 501 to 540, fifty DMRs defined by SEQ ID NOs: 501 to 550, sixty DMRs defined by SEQ ID NOs: 501 to 560, seventy DMRs defined by SEQ ID NOs: 501 to 570, eighty DMRs defined by SEQ ID NOs: 501 to 580, or ninety DMRs defined by SEQ ID NOs: 501 to 590; or
7. ten DMRs defined by SEQ ID NOs: 501 to 510, SEQ ID NOs: 511 to 520, SEQ ID NOs: 521 to 530, SEQ ID NOs: 531 to 540, SEQ ID NOs: 541 to 550, SEQ ID NOs: 551 to 560, SEQ ID NOs: 561 to 570, SEQ ID NOs: 571 to 580, SEQ ID NOs: 581 to 590, SEQ ID NOs: 591 to 600, SEQ ID NOs: 601 to 610, SEQ ID NOs: 611 to 620, SEQ ID NOs: 621 to 630, SEQ ID NOs: 631 to 640, SEQ ID NOs: 641 to 650, SEQ ID NOs: 651 to 660, SEQ ID NOs: 661 to 670, SEQ ID NOs: 671 to 680, SEQ ID NOs: 681 to 690, SEQ ID NOs: 691 to 700, SEQ ID NOs: 701 to 710, SEQ ID NOs: 711 to 720, SEQ ID NOs: 721 to 730, SEQ ID NOs: 731 to 740, SEQ ID NOs: 741 to 750; SEQ ID NOs: 751 to 760; SEQ ID NOs: 761 to 770; SEQ ID NOs: 771 to 780; SEQ ID NOs: 781 to 790; SEQ ID NOs: 791 to 800 or SEQ ID NOs: 801 to 808.

The assay of the invention may be performed above and additionally wherein the step of determining the methylation status of a panel of one or more CpGs comprises determining the methylation status of one or more CpGs within any one or more DMRs selected from the group of DMRs consisting of DMRs 1 to 308 as defined by SEQ ID NOs 501 to 808, including:
a. one or more CpGs within DMR 1 as defined by SEQ ID NO: 501 and denoted by CG, preferably wherein the assay is characterised as having an ROC AUC of at least 0.911, and more preferably wherein the panel of one or more CpGs comprises at least the CpGs denoted by [[CG]];
b. one or more CpGs within DMR 2 as defined by SEQ ID NO: 502 and denoted by CG, preferably wherein the assay is characterised as having an ROC AUC of at least 0.903, and more preferably wherein the panel of one or more CpGs comprises at least the CpGs denoted by [[CG]];
c. one or more CpGs within DMR 3 as defined by SEQ ID NO: 503 and denoted by CG, preferably wherein the assay is characterised as having an ROC AUC of at least 0.899, and more preferably wherein the panel of one or more CpGs comprises at least the CpGs denoted by [[CG]];
d. one or more CpGs within DMR 4 as defined by SEQ ID NO: 504 and denoted by CG, preferably wherein the assay is characterised as having an ROC AUC of at least 0.899, and more preferably wherein the panel of one or more CpGs comprises at least the CpGs denoted by [[CG]];
e. one or more CpGs within DMR 5 as defined by SEQ ID NO: 505 and denoted by CG, preferably wherein the assay is characterised as having an ROC AUC of at least 0.899, and more preferably wherein the panel of one or more CpGs comprises at least the CpGs denoted by [[CG]];
f. one or more CpGs within DMR 6 as defined by SEQ ID NO: 506 and denoted by CG, preferably wherein the assay is characterised as having an ROC AUC of at least 0.897, and more preferably wherein the panel of one or more CpGs comprises at least the CpGs denoted by [[CG]];
g. one or more CpGs within DMR 7 as defined by SEQ ID NO: 507 and denoted by CG, preferably wherein the assay is characterised as having an ROC AUC of at least 0.894, and more preferably wherein the panel of one or more CpGs comprises at least the CpGs denoted by [[CG]];
h. one or more CpGs within DMR 8 as defined by SEQ ID NO: 508 and denoted by CG, preferably wherein the assay is characterised as having an ROC AUC of at least 0.894, and more preferably wherein the panel of one or more CpGs comprises at least the CpGs denoted by [[CG]];
i. one or more CpGs within DMR 9 as defined by SEQ ID NO: 509 and denoted by CG, preferably wherein the assay is characterised as having an ROC AUC of at least 0.892, and more preferably wherein the panel of one or more CpGs comprises at least the CpGs denoted by [[CG]]; or
j. one or more CpGs within DMR 10 as defined by SEQ ID NO: 510 and denoted by CG, preferably wherein the assay is characterised as having an ROC AUC of at least 0.891, and more preferably wherein the panel of one or more CpGs comprises at least the CpGs denoted by [[CG]].

The assay of the invention may be performed above and additionally wherein:
1. when the cancer index for the individual is about 0.025 or more the individual is classified as having endometrial cancer or as having a high risk of endometrial cancer development; or
2. when the cancer index for the individual is less than about 0.025 the individual is classified as not having endometrial cancer;
preferably wherein the assay has a specificity of 95% or more, more preferably wherein the assay comprises determining mean β-values for each CpG in the panel of one or more CpGs.

The assay of the invention may be performed above and additionally wherein:
1. CpGs denoted by CG whose cancer index value is determined are located within at least DMR 1 defined by SEQ ID NO: 501, and wherein when the cancer index for the individual is about 0.209 or more the individual is classified as having endometrial cancer or as having a high risk of endometrial cancer development, and wherein the sensitivity of the assay is at least 70.00% and the specificity of the assay is at least 95.00%, preferably wherein the panel of one or more CpGs comprises at least three CpGs from DMR 1, and more preferably wherein the cancer index value is the mean β-value for the CpGs denoted by [[CG]] in SEQ ID NO: 501;
2. CpGs denoted by CG whose cancer index value is determined are located within at least DMR 1 defined by SEQ ID NO: 501, and wherein when the cancer index for the individual is less than about 0.209 the individual is classified as not having endometrial cancer or as having a low risk of endometrial cancer development, and wherein the sensitivity of the assay is at least 70.00% and the specificity of the assay is at least 95.00%, preferably wherein the panel of one or more CpGs comprises at least three CpGs from DMR 1, and more preferably wherein the cancer index value is the mean β-value for the CpGs denoted by [[CG]] in SEQ ID NO: 501;
3. CpGs denoted by CG whose cancer index value is determined are located within at least DMR 2 defined by SEQ ID NO: 502, and wherein when the cancer index for the individual is about 0.271 or more the individual is classified as having endometrial cancer or as having a high risk of endometrial cancer development, and wherein the sensitivity of the assay is at least 77.00% and the specificity of the assay is at least 95.00%, preferably wherein the panel of one or more CpGs comprises at least one CpG from DMR 2, and more preferably wherein the cancer index value is the mean β-value for the CpGs denoted by [[CG]] in SEQ ID NO: 502;
4. CpGs denoted by CG whose cancer index value is determined are located within at least DMR 2 defined by SEQ ID NO: 502, and wherein when the cancer index for the individual is less than about 0.271 the individual is classified as not having endometrial cancer or as having a low risk of endometrial cancer development, and wherein the sensitivity of the assay is at least 77.00% and the specificity of the assay is at least 95.00%, preferably wherein the panel of one or more CpGs comprises at least one CpGs from DMR 2, and more preferably wherein the cancer index value is the mean β-value for the CpGs denoted by [[CG]] in SEQ ID NO: 502;
5. CpGs denoted by CG whose cancer index value is determined are located within at least DMR 3 defined by SEQ ID NO: 503, and wherein when the cancer index for the individual is about 0.123 or more the individual is classified as having endometrial cancer or as having a high risk of endometrial cancer development, and wherein the sensitivity of the assay is at least 73.00% and the specificity of the assay is at least 95.00%, preferably wherein the panel of one or more CpGs comprises at least three CpGs from DMR 3, and more preferably wherein the cancer index value is the mean β-value for the CpGs denoted by [[CG]] in SEQ ID NO: 503;
6. CpGs denoted by CG whose cancer index value is determined are located within at least DMR 3 defined by SEQ ID NO: 503, and wherein when the cancer index for the individual is less than about 0.123 the individual is classified as not having endometrial cancer or as having a low risk of endometrial cancer development, and wherein the sensitivity of the assay is at least 73.00% and the specificity of the assay is at least 95.00%, preferably wherein the panel of one or more CpGs comprises at least three CpGs from DMR 3, and more preferably wherein the cancer index value is the mean β-value for the CpGs denoted by [[CG]] in SEQ ID NO: 503;
7. CpGs denoted by CG whose cancer index value is determined are located within at least DMR 4 defined by SEQ ID NO: 504, and wherein when the cancer index for the individual is about 0.123 or more the individual is classified as having endometrial cancer or as having a high risk of endometrial cancer development, and wherein the sensitivity of the assay is at least 73.00% and the specificity of the assay is at least 95.00%, preferably wherein the panel of one or more CpGs comprises at least three CpGs from DMR 4, and more preferably wherein the cancer index value is the mean β-value for the CpGs denoted by [[CG]] in SEQ ID NO: 504;
8. CpGs denoted by CG whose cancer index value is determined are located within at least DMR 4 defined by SEQ ID NO: 504, and wherein when the cancer index for the individual is less than about 0.123 the individual is classified as not having endometrial cancer or as having a low risk of endometrial cancer development, and wherein the sensitivity of the assay is at least 73.00% and the specificity of the assay is at least 95.00%, preferably wherein the panel of one or more CpGs comprises at least three CpGs from DMR 4, and more preferably wherein the cancer index value is the mean β-value for the CpGs denoted by [[CG]] in SEQ ID NO: 504;
9. CpGs denoted by CG whose cancer index value is determined are located within at least DMR 5 defined by SEQ ID NO: 505, and wherein when the cancer index for the individual is about 0.105 or more the individual is classified as having endometrial cancer or as having a high risk of endometrial cancer development, and wherein the sensitivity of the assay is at least 71.00% and the specificity of the assay is at least 95.00%, preferably wherein the panel of one or more CpGs comprises at least five CpGs from DMR 5, and more preferably wherein the cancer index value is the mean β-value for the CpGs denoted by [[CG]] in SEQ ID NO: 505; or
10. CpGs denoted by CG whose cancer index value is determined are located within at least DMR 5 defined by SEQ ID NO: 505, and wherein when the cancer index for the individual is less than about 0.105 the individual is classified as not having endometrial cancer or as having a low risk of endometrial cancer development, and wherein the sensitivity of the assay is at least 71.00% and the specificity of the assay is at least 95.00%, preferably wherein the panel of one or more CpGs comprises at least five CpGs from DMR 5, and more preferably wherein the cancer index value is the mean β-value for the CpGs denoted by [[CG]] in SEQ ID NO: 505.

The assay of the invention may be performed above and additionally wherein the step of determining the methylation status of the one or more CpGs in the panel further comprises or additionally comprises determining the methylation status of each CpG within one or more of the sequences identified by SEQ ID NOs 809 to 919.

The assay of the invention may be performed above and additionally wherein the step of determining the methylation status of the one or more CpGs in the panel comprises determining each CpG within:
a. SEQ ID NO 809 and/or SEQ ID NO 846 and/or SEQ ID NO 883 and wherein when the cancer index value is about 0.282 or more the individual is classified as having endometrial cancer or as having a high risk of endometrial cancer development, and wherein the sensitivity of the assay is at least 85.00%, the specificity of the assay is about 100.00%, and the AUC is about 1.00, preferably wherein the cancer index value is the percent methylated reference for sequence defined by SEQ ID NO 809 and/or SEQ ID NO 846 and/or SEQ ID NO 883;
b. SEQ ID NO 809 and/or SEQ ID NO 846 and/or SEQ ID NO 883 and wherein when the cancer index value is less than about 0.282 the individual is classified as not having endometrial cancer or as having a low risk of endometrial cancer development, and wherein the sensitivity of the assay is at least 85.00%, the specificity of the assay is about 100.00%, and the AUC is about 1.00, preferably wherein the cancer index value is the percent methylated reference for sequence defined by SEQ ID NO 809 and/or SEQ ID NO 846 and/or SEQ ID NO 883;
c. SEQ ID NO 810 and/or SEQ ID NO 847 and/or SEQ ID NO 884 and wherein when the cancer index value is about 0.212 or more the individual is classified as having endometrial cancer or as having a high risk of endometrial cancer development, and wherein the sensitivity of the assay is at least 55.00%, the specificity of the assay is about 100.00%, and the AUC is about 1.00, preferably wherein the cancer index value is the percent methylated reference for sequence defined by SEQ ID NO 810 and/or SEQ ID NO 847 and/or SEQ ID NO 883;
d. SEQ ID NO 810 and/or SEQ ID NO 847 and/or SEQ ID NO 884 and wherein when the cancer index value is less than about 0.212 the individual is classified as not having endometrial cancer or as having a low risk of endometrial cancer development, and wherein the sensitivity of the assay is at least 55.00%, the specificity of the assay is about 100.00%, and the AUC is about 1.00, preferably wherein the cancer index value is the percent methylated reference for sequence defined by SEQ ID NO 810 and/or SEQ ID NO 847 and/or SEQ ID NO 884;
e. SEQ ID NO 811 and/or SEQ ID NO 848 and/or SEQ ID NO 885 and wherein when the cancer index value is about 0.002 or more the individual is classified as having endometrial cancer or as having a high risk of endometrial cancer development, and wherein the sensitivity of the assay is at least 90.00%, the specificity of the assay is about 80.00%, and the AUC is about 0.98, preferably wherein the cancer index value is the percent methylated reference for sequence defined by SEQ ID NO 740 and/or SEQ ID NO 777 and/or SEQ ID NO 814;
f. SEQ ID NO 811 and/or SEQ ID NO 848 and/or SEQ ID NO 885 and wherein when the cancer index value is less than about 0.002 the individual is classified as not having endometrial cancer or as having a low risk of endometrial cancer development, and wherein the sensitivity of the assay is at least 90.00%, the specificity of the assay is about 80.00%, and the AUC is about 0.98, preferably wherein the cancer index value is the percent methylated reference for sequence defined by SEQ ID NO 811 and/or SEQ ID NO 848 and/or SEQ ID NO 885;
g. SEQ ID NO 812 and/or SEQ ID NO 849 and/or SEQ ID NO 886 and wherein when the cancer index value is about 0.026 or more the individual is classified as having endometrial cancer or as having a high risk of endometrial cancer development, and wherein the sensitivity of the assay is at least 85.00%, the specificity of the assay is about 90.00%, and the AUC is about 0.98, preferably wherein the cancer index value is the percent methylated reference for sequence defined by SEQ ID NO 812 and/or SEQ ID NO 849 and/or SEQ ID NO 886 ;
h. SEQ ID NO 812 and/or SEQ ID NO 849 and/or SEQ ID NO 886 and wherein when the cancer index value is less than about 0.026 the individual is classified as not having endometrial cancer or as having a low risk of endometrial cancer development, and wherein the sensitivity of the assay is at least 85.00%, the specificity of the assay is about 90.00%, and the AUC is about 0.98, preferably wherein the cancer index value is the percent methylated reference for sequence defined by SEQ ID NO 812 and/or SEQ ID NO 849 and/or SEQ ID NO 886;
i. SEQ ID NO 813 and/or SEQ ID NO 850 and/or SEQ ID NO 887 and wherein when the cancer index value is about 0.003 or more the individual is classified as having endometrial cancer or as having a high risk of endometrial cancer development, and wherein the sensitivity of the assay is at least 85.00%, the specificity of the assay is about 85.00%, and the AUC is about 0.97, preferably wherein the cancer index value is the percent methylated reference for sequence defined by SEQ ID NO 813 and/or SEQ ID NO 850 and/or SEQ ID NO 887 ; and/or
j. SEQ ID NO 813 and/or SEQ ID NO 850 and/or SEQ ID NO 887 and wherein when the cancer index value is less than about 0.003 the individual is classified as not having endometrial cancer or as having a low risk of endometrial cancer development, and wherein the sensitivity of the assay is at least 85.00%, the specificity of the assay is about 85.00%, and the AUC is about 0.97, preferably wherein the cancer index value is the percent methylated reference for sequence defined by SEQ ID NO 813 and/or SEQ ID NO 850 and/or SEQ ID NO 887.

The assay of the invention may be performed above and additionally wherein the step of determining the methylation status of the one or more CpGs in the panel further comprises or additionally comprises determining the methylation status of each CpG within one or more of the sequences identified by SEQ ID NOs 809, 846, 883, 811, 848, 885, 813, 850, and 887.

The assay of the invention may be performed above and additionally wherein the step of determining in the population of DNA molecules in the sample the methylation status of each CpG in the panel of one or more CpGs comprises:
1. performing a sequencing step to determine the sequence of each CpG;
2. hybridising DNA to an array comprising probes capable of discriminating between methylated and non-methylated forms of the CpGs and applying a detection system to the array so as to determine the methylation status of each CpG; and/or
3. performing a PCR step using methylation-specific primers, wherein the methylation status of the CpG is determined by the presence or absence of a PCR product.

The assay of the invention may be performed above and additionally wherein the step of determining the methylation status of each CpG in the panel of one or more CpGs comprises:
1. bisulphite converting the DNA; or
2. performing the steps of oxidising 5-methylcytosine bases (5mC) to 5-carboxylcytosine bases (5caC), preferably by ten-eleven translocation (TET), and/or oxidising 5-hydroxymethylcytosine bases (5hmC) to 5-carboxylcytosine bases (5caC), preferably by ten-eleven translocation (TET); followed by reducing 5-carboxylcytosine bases (5caC) to dihydrouracil bases (DHU), optionally with pyridine borane.

Disclosed but not claimed are the following numbered aspects.

Aspect 1) A method of treating or preventing cancer in an individual, the method comprising:
1. assessing the cancer status of the individual by assessing the presence, absence or development of cancer in the individual by performing the assay of any one of the assays of the invention;
2. administering one or more therapeutic or preventative treatments to the individual based on the assessment.

The method be performed above and additionally wherein the individual is assessed as not having endometrial cancer or as having a low risk of endometrial cancer development, and wherein the cancer index value is about -0.660 or more and less than about -0.430, and preferably wherein the assay comprises determining methylation β-values for each CpG in the panel of one or more CpGs, the individual is subjected to one or more treatments according to their cancer index value, the one or more treatments comprise:
1. a transvaginal ultrasound to assess endometrium and ovaries;
2. a repeat assay according to any one of the assays of the invention, preferably wherein the repeat assay is performed about two years after the previous assay.

The method may be performed above and additionally wherein the individual is assessed as having a moderate risk of having endometrial cancer or as having a moderate risk of endometrial cancer development, and wherein the cancer index value is about -0.430 or more and less than about -0.230, and preferably wherein the assay comprises determining methylation β-values for each CpG in the panel of one or more CpGs, the individual is subjected to one or more treatments according to their cancer index value, the one or more treatments comprise any of:
1. a transvaginal ultrasound to assess endometrium and ovaries;
2. intensified screening, preferably wherein the intensified screening comprises one or more of:
   i. a colposcopy;
   ii. a HPV test;
   iii. a cervical cytology test;
   iv. a test for CA125, preferably wherein the test is repeated six-monthly;
   v. a test for cell-free tumour DNA methylation in plasma/serum, preferably wherein the test is repeated annually;
   vi. a test for cell-free tumour DNA methylation in vaginal fluid, preferably wherein the test is repeated annually
   vii. a pelvic MRI scan, preferably wherein the individual being subjected to the pelvic MRI scan is post-menopausal, and preferably wherein the scan is repeated annually;
   viii. a repeat assay according to any one of the assays of the invention, preferably wherein the repeat assay is performed about one year after the previous assay
3. administration of one or more of progestogens, particularly wherein the progestogens are delivered locally or systemically, Aspirin, Metformin, aromatase-inhibitors, weight-loss regimen.

The method may be performed above and additionally wherein when the colposcopy, HPV and cytology tests are negative, the intensified screening further comprises a hysteroscopy and endocervical and endometrial biopsy.

The method may be performed above and additionally wherein when the transvaginal ultrasound and intensified screening are both negative:
1. the transvaginal ultrasound, the test for CA125, the test for cell-free tumour DNA methylation in plasma/serum, and the test for cell-free tumour DNA methylation in vaginal fluid is repeated about six months after the previous assay; and
2. the colposcopy, the HPV test, and the cervical cytology test, is repeated about one year after the previous assay.

The method may be performed above and additionally wherein the individual is assessed as having endometrial cancer or as having a high risk of endometrial cancer development, and wherein the cancer index value is about -0.230 or more, and preferably wherein the assay comprises determining methylation β-values for each CpG in the panel of one or more CpGs, the individual is subjected to one or more treatments according to their cancer index value, the one or more treatments comprise any of:
1. a transvaginal ultrasound to assess endometrium and ovaries;
2. intensified screening, preferably wherein the intensified screening comprises one or more of:
   i. a colposcopy;
   ii. a HPV test;
   iii. a cervical cytology test;
   iv. a test for CA125, preferably wherein the test is repeated six-monthly;
   v. a test for cell-free tumour DNA methylation in plasma/serum, preferably wherein the test is repeated annually;
   vi. a test for cell-free tumour DNA methylation in vaginal fluid, preferably wherein the test is repeated annually
   vii. a pelvic MRI scan, preferably wherein the individual being subjected to the pelvic MRI scan is post-menopausal, and preferably wherein the scan is repeated annually;
   viii. a repeat assay according to any one of the assays of the invention, preferably wherein the repeat assay is performed about one year after the previous assay;
3. administration of one or more of progestogens, particularly wherein the progestogens are delivered locally or systemically, Aspirin, Metformin, aromatase-inhibitors, weight-loss regimen;
4. a total hysterectomy and bilateral salpingo-oophorectomy.

The method may be performed above and additionally wherein when the colposcopy, HPV and cytology tests are negative, the intensified screening further comprises a hysteroscopy and endocervical and endometrial biopsy.

The method may be performed above and additionally wherein when the transvaginal ultrasound and intensified screening are both negative:
1. the transvaginal ultrasound, the test for CA125, the test for cell-free tumour DNA methylation in plasma/serum, the test for cell-free tumour DNA methylation in vaginal fluid, the colposcopy, the HPV test, and the cervical cytology test is repeated about six months after the previous assay; and
2. the pelvic MRI scan is repeated about one year after the previous assay.

The method may be performed above and additionally wherein the progestogens are delivered locally via an intrauterine device.

The method may be performed above and additionally wherein the one or more treatments that the individual is subjected to are repeated on a monthly, three monthly, six monthly, yearly or two yearly basis following an initial administration.

Aspect 2) A method of monitoring the cancer status of an individual according to the individual's cancer index value, the method comprising: (a) assessing the presence, absence or development of cancer in an individual by performing the assay according to any one of the assays of the invention at a first time point; (b) assessing the presence, absence or development of cancer in the individual by performing the assay according to any one of the assays of the invention at one or more further time points; and (c) monitoring any change in the cancer index value and/or cancer status and/or CIN3 status of the individual between time points.

The method may be performed above and additionally the further time points are monthly, three monthly, six monthly, yearly or two yearly basis following an initial assessment.

The method may be performed above and additionally wherein depending on the cancer index value and/or cancer status and/or CIN3 status of the individual, one or more treatments are administered to the individual according to any one of the methods of the invention, or when the cancer index value of the individual is less than about - 0.660 no treatment is administered to the individual.

The method may be performed above and additionally wherein an increase in the cancer index value indicates a negative response to the one or more treatments.

The method may be performed above and additionally wherein changes are made to the one or more treatments if a negative response is identified.

The method may be performed above and additionally wherein a decrease in the cancer index value indicates a positive response to the one or more treatments.

The method may be performed above and additionally wherein changes are made to the one or more treatments if a positive response is identified.

The assay of the invention may be performed above and additionally wherein the sample is obtained from a tissue comprising epithelial cells, preferably wherein the sample is not obtained from ovarian or endometrial tissue.

The assay of the invention may be performed above and additionally wherein the sample is obtained from:
1. cervical tissue;
2. vaginal tissue;
3. cervicovaginal tissue;
4. buccal tissue;
preferably wherein the sample is obtained from cervical tissue, most preferably wherein the sample is obtained from tissue from a cervical smear.

The assay of the invention may be performed above and additionally wherein the assay is for assessing the presence, absence or development of
1. an endometriod cancer, uterine carcinosarcoma, squamous cell carcinoma, small cell carcinoma, transitional carcinoma, serous carcinoma, clear-cell carcinoma, mucinous adenocarcinoma, undifferentiated carcinoma, dedifferentiated carcinoma or serous adenocarcinoma

Aspect 3) An array capable of discriminating between methylated and non-methylated forms of CpGs; the array comprising oligonucleotide probes specific for a methylated form of each CpG in a CpG panel and oligonucleotide probes specific for a non-methylated form of each CpG in the panel; wherein the panel consists of at least 50 CpGs selected from the CpGs identified in SEQ ID NOs 1 to 500 and identified at nucleotide positions 61 to 62, and identified in SEQ ID NOs 501 to 808 and denoted by CG.

The array may be performed above and additionally provided that the array is not an Infinium MethylationEPIC BeadChip array or an Infinium HumanMethylation450, and/or provided that the number of CpG-specific oligonucleotide probes of the array is 482,000 or less, 480,000 or less, 450,000 or less, 440,000 or less, 430,000 or less, 420,000 or less, 410,000 or less, or 400,000 or less.

The array may be performed above and additionally wherein the panel comprises any panel of CpGs defined in the assays of any one of the assays of the invention.

The array may be performed above and additionally further comprising one or more oligonucleotides comprising any set of CpGs defined in the assays of any one of the assays of the invention, wherein the one or more oligonucleotides are hybridized to corresponding oligonucleotide probes of the array.

Aspect 4) A hybridized array, wherein the array is obtainable by hybridizing to an array of the invention a group of oligonucleotides comprising any panel of CpGs defined in the assays of the invention.

Aspect 5) A process for making the hybridized array of the invention, comprising contacting an array of the invention with a group of oligonucleotides comprising any panel of CpGs defined in the assays of the invention.

### Brief description of the Figures

**Figure 1** shows the experimental design underpinning the discovery and validation of the WID-EC-index.
**Figure 2** shows ROC curve of the WID-EC-index in the internal validation set (Panel A). Box plot of the WID-EC index in the internal validation set (Panel B).
**Figure 3** shows WID-EC-index in endometrial cancer cases and healthy controls in the external validation dataset (Panel A). ROC curve in the external validation set (Panel C). WID-EC-index versus time-to-event in prospectively collected validation samples (Panel D). ROC curve corresponding to the prospective samples (Panel E).
**Figure 4** shows the WID-EC-index versus age in samples from the internal and external validation datasets (Panel A). Correlation with body mass index (BMI) in controls (Panel B). Menopause (Panel C). Parity (Panel D). Stage (Panel E). Grade (Panel F). Histology (Panel G). Panels A to D are based on controls from the internal and external validation datasets. Panels E to G are based on cases from the internal and external validation datasets.
**Figure 5** shows WID-EC-index versus the estimated proportion of tumor DNA (Panel A). The estimated proportion of tumor DNA in each cervical smear sample as estimated using the EpiDISH algorithm for controls (Panel B) and endometrial cancers (Panel C).

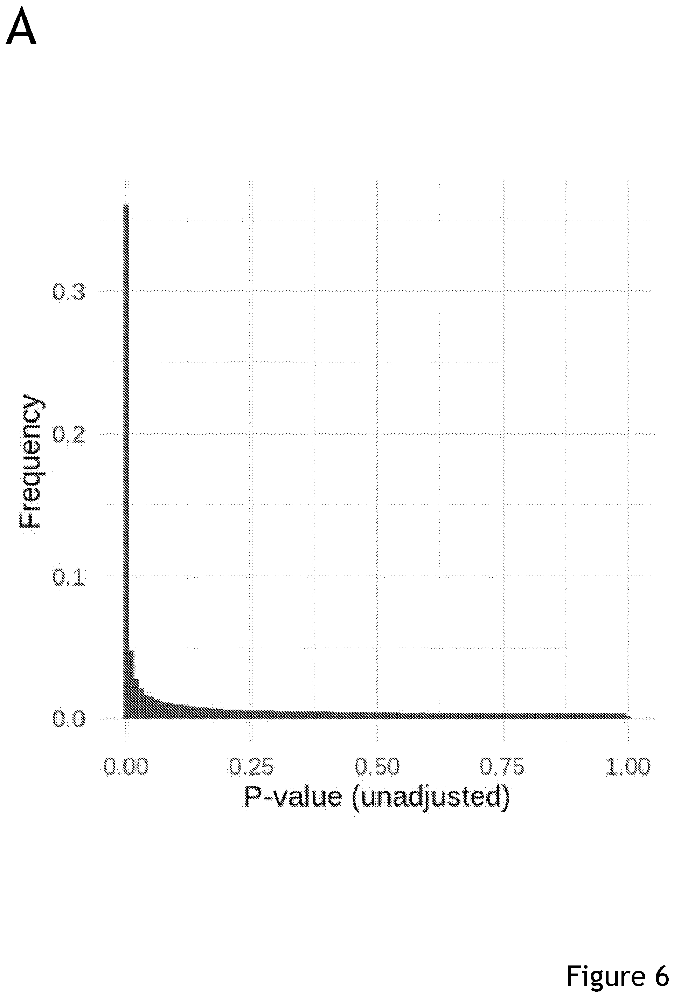

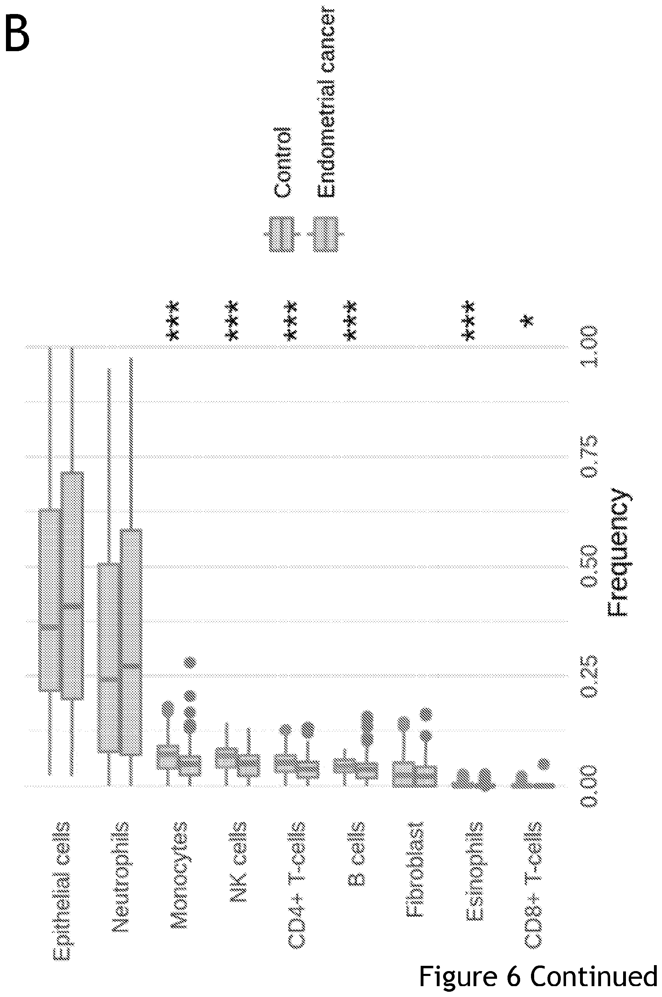

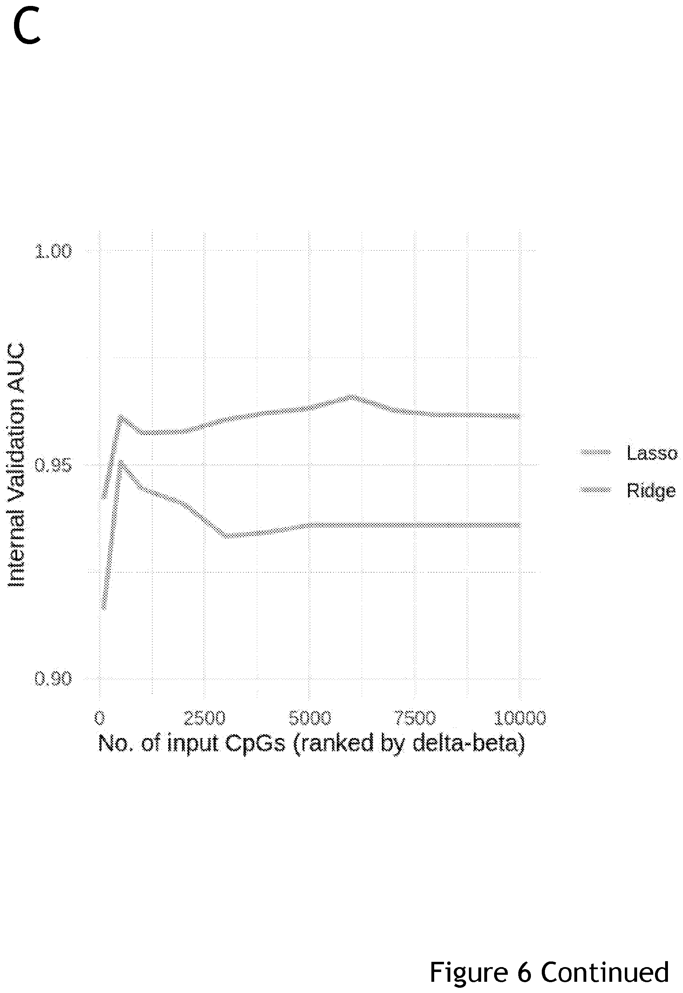

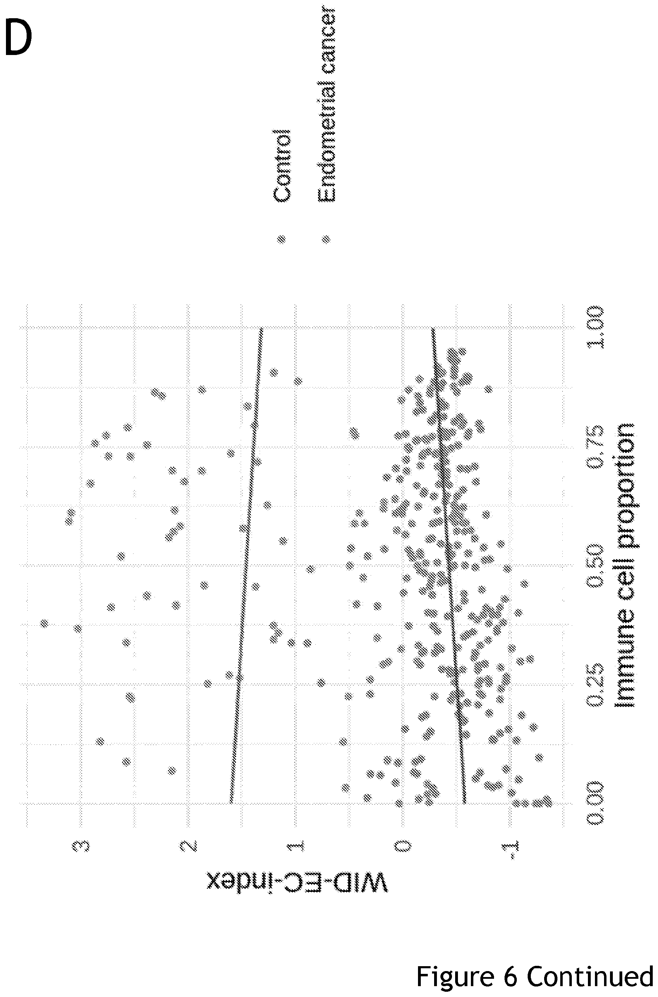

**Figure 6** shows the distribution of p-values obtained by comparing cases and controls at each CpG site and after controlling for immune cell proportion and age (Panel A). Distribution of different cell types in the discovery dataset inferred using the HEpiDISH algorithm (* p<0.05, ** p<0.01, *** p<0.001) (Panel B). Area under the reciever operating characteristic curve (AUROC) in the internal validation set as a function of the number of CpGs used to train the classifier (Panel C). Distribution of the WID-EC-index with respect to immune cell proportion in the internal validation set (Panel D).
**Figure 7** shows the distribution of different cell types in the external validation dataset inferred using the HEpiDISH algorithm (* p<0.05, ** p<0.01, *** p<0.001) (Panel A). Cell type distribution in the propspective validation dataset (Panel B). The mean delta-beta (difference between mean beta values in control samples from the proposective and discovery datasets) across different genomic regions (Panel C). Odds ratios corresonding to the genomic annotation of the 500 CpGs comprising the WID-EC-index (when compared to the 776,725 CpGs in the dataset; *** p<0.001) (Panel D).
**Figure 8** shows the distribution of the WID-EC-index in controls that volunteered from the general population and women that presented at hospitals for benign women-specific conditions (Discovery set) (Panel A). The WID-EC-index versus the number of days between sample collection and DNA extraction.

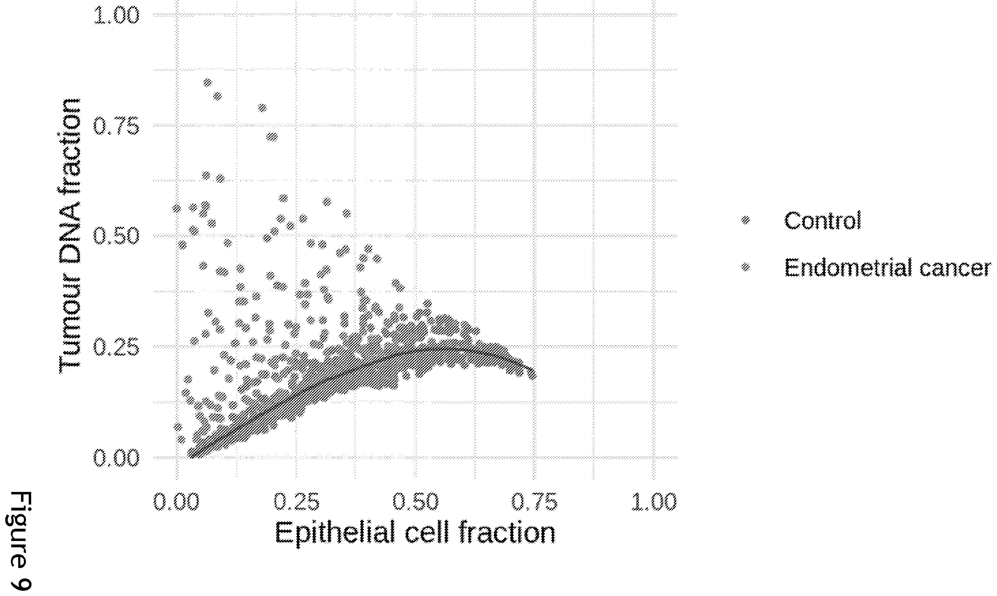

**Figure 9** shows the inferred tumour DNA proportion versus the inferred epithelial cell proportion in the discovery set as determined using the HEpiDISH algorithm.

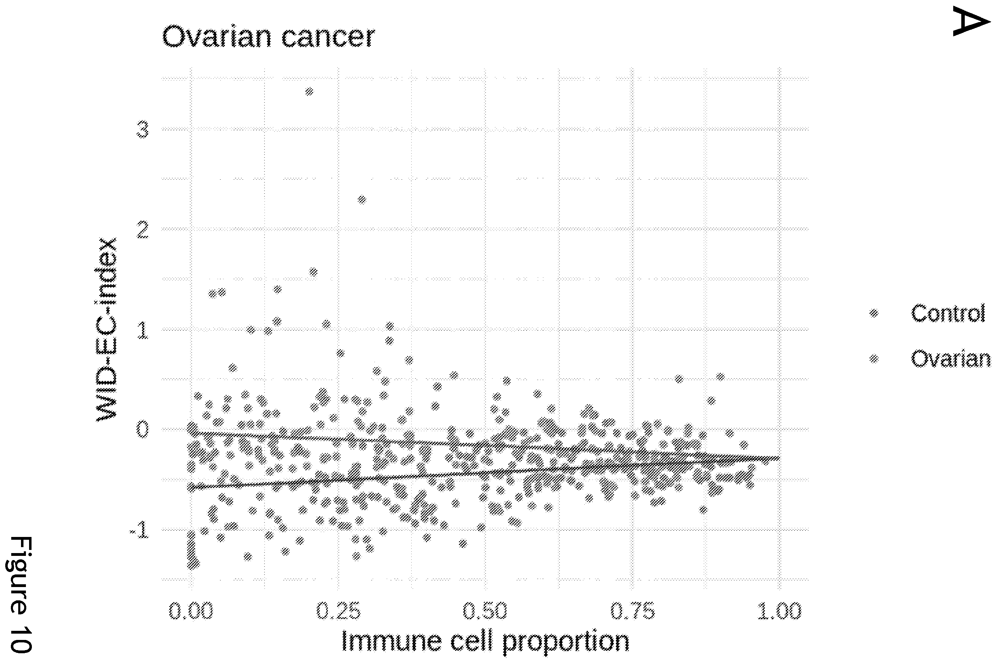

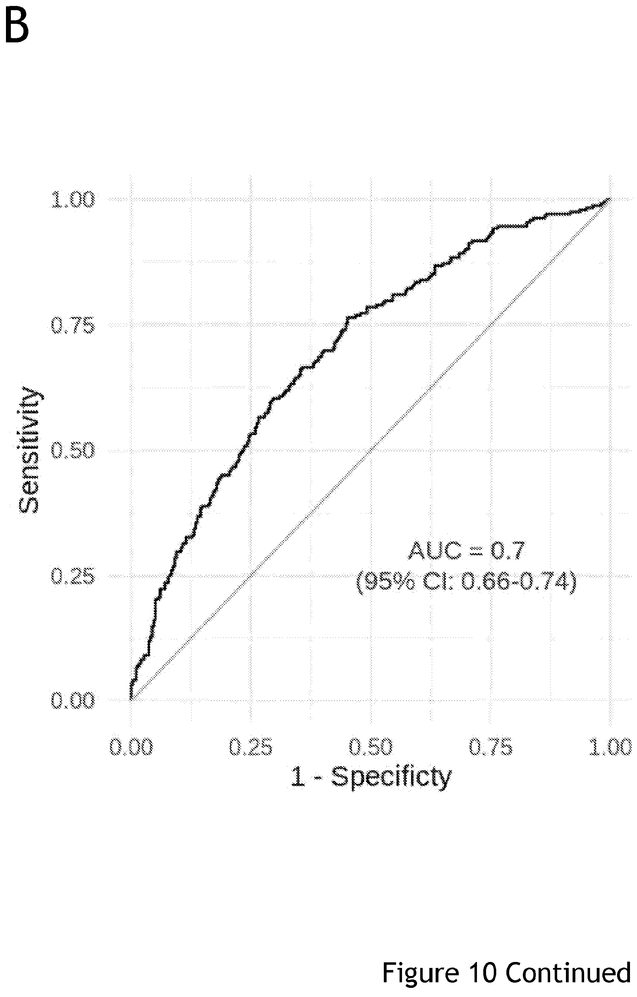

**Figure 10** shows the WID-EC-index versus immune cell proportion in the ovarian cancer validation dataset (Panel A) and the corresponding ROC curve (Panel B).

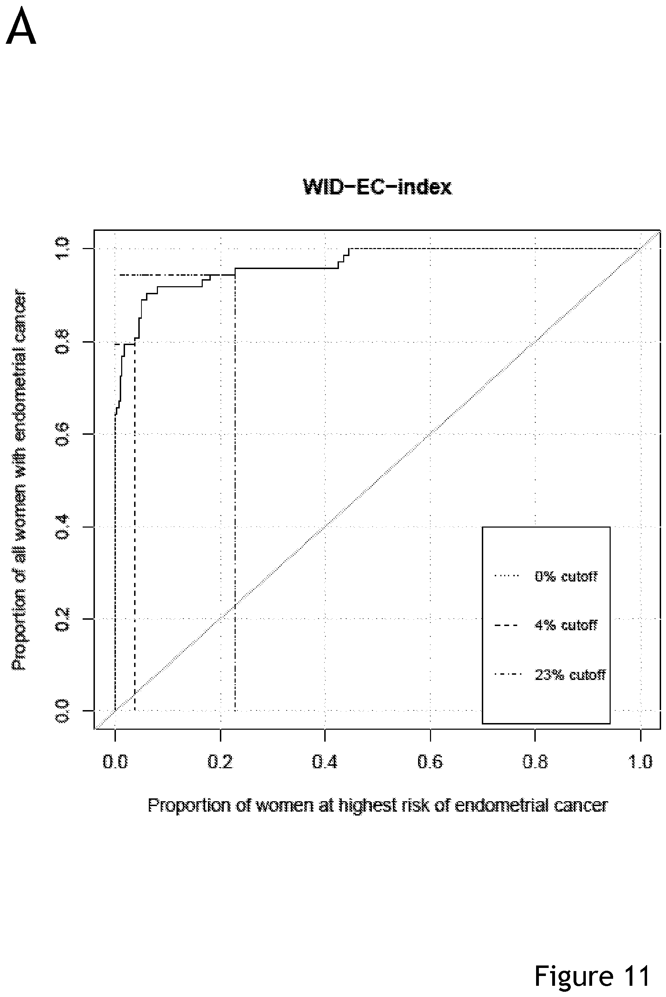

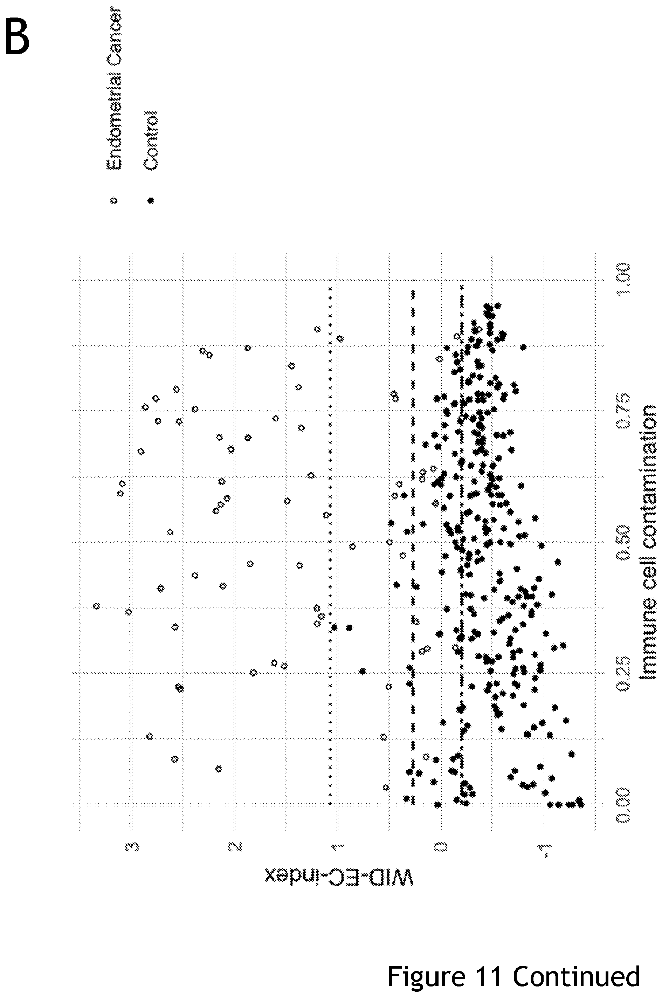

**Figure 11** shows cutpoints applied to the patient data, and consequent specificity and sensitivity for cancer status discrimination achieved when these cutpoints are applied.

### DETAILED DESCRIPTION OF THE INVENTION

### Identification of CpGs

The present inventors sought to identify CpG methylation-based assays capable of assessing the presence, absence or development of cancer in an individual. Any of the assays described herein for assessing the presence, absence or development of cancer in an individual are capable of being utilised for assessing the presence, absence or development of endometrial cancer. The present inventors compared CpG methylation levels in non-cancerous epithelial tissue, particularly cervical tissue, vaginal tissue or cervicovaginal tissue across groups of women that were either known to be both endometrial and ovarian cancer negative, and/or CIN3 negative, or known to be endometrial and/or ovarian cancer positive, and/or CIN3 positive. This led to the surprising establishment of a "cancer index", used interchangeable herein with "index", "index value", "WID-EC-Index" or "WID-Index" (WID = women's risk identification).

A CpG as defined herein refers to the CG dinucleotide motif identified in relation to each SEQ ID NO., wherein the CG dinucleotide of interest is denoted by CG. Thus by determining the methylation status of any panel of one or more CpGs defined by or identified in a given SEQ ID NO, it is meant that a determination is made as to the methylation status of the cytosine of the CG dinucleotide motif identified in square brackets in the panel of one or more CpGs in each sequence shown below, accepting that variations in the sequence upstream and downstream of any given CpG may exist due to sequencing errors or variation between individuals.

As set out in more detail in the Examples, the methylation status of sub-selections of the 500 CpGs, as identified in SEQ ID NOs 1 to 500, may be determined in order to assess an individual for the presence, absence or development of cancer with high sensitivity and specificity. The cancer is preferably endometrial cancer or ovarian cancer, most preferably endometrial cancer. A panel of one or more of the CpGs identified in SEQ ID NOs 1 to 500 may be utilised to derive a cancer index for an individual in accordance with the invention described herein.

The methylation status of a panel of one or more CpGs of the 500 CpGs defined according to SEQ ID NOs: 1 to 500 may be assessed by any suitable technique. As explained in more detail in the Examples below, one particular exemplary technique which the inventors have used is an array-based analysis technique coupled with beta value analysis. SEQ ID NOs 1 to 500 correspond to the sequences of commercial probes utilised in said array.

The inventors further identified 308 differentially methylated regions (DMRs) with relevance to cancer, particularly endometrial or ovarian cancer. The nucleotide sequences of the 308 DMRs are defined respectively by the nucleotide sequences of SEQ ID NO: 501 to 808 as set out in Table 1 below, accepting that variation in the nucleotide sequence of any given DMR may exist due to sequencing errors and/or variation between individuals. In each sequence shown below the cytosine of the CG dinucleotide motif identified in square brackets or double square brackets is a cytosine of a CpG which may be included in a panel of CpGs when performing the assays of the invention.

The inventors further defined 37 regions within a select number of the 308 DMRs with particular relevance to cancer and CIN3, particularly endometrial and ovarian cancer. The nucleotide sequence of the 37 regions are defined respectively by the nucleotide sequence of SEQ ID NOs: 809 to 919 as set out in Table 10 below, accepting that variation in the nucleotide sequence of any given DMR may exist due to sequencing errors and/or variation between individuals. When any one or more of the 37 regions are included in a panel of CpGs when performing the assays of the invention, the methylation status of every cytosine within a CG dinucleotide in the region is determined. The amplicon sequences generated by the 37 primer and probe reactions as set out Table 10 are described and defined by SEQ ID NOs 920 to 956 and in Table 12. In any of the assays described herein, the step of determining the methylation status of a panel of one or more CpGs may comprise determining the methylation status of one or more CpGs within any one or more the amplicons defined by SEQ ID NOs 920 to 956 and denoted by CG. More preferably, in any of the assays described herein, the step of determining the methylation status of a panel of one or more CpGs may comprise determining the methylation status of one or more CpGs within any one or more the amplicons defined by SEQ ID NOs 920, 922 and 924 and denoted by CG, yet more preferably all of the CpGs denoted by CG in the amplicons defined by SEQ ID NOs 920, 922 and 924.

**Table 10**

| **gene_name** | **Forward.Primer** | **SEQ ID NO of forward primer** | **Reverse.Primer** | **SEQ ID NO of reverse primer** | **Probe** | **SEQ ID NO of probe** |
|---|---|---|---|---|---|---|
| ZSCAN12 | AGAGCGGTCGCGGAAGT | 809 | AACCGAAAACGACCACGAAA | 846 | CGTTTCGCGCGGGTTGGTTTT | 883 |
| HCG4 | TCGATAGCGACGTTACGAGTTTT | 810 | TCCGACCCCTCCTACTCCAT | 847 | ATGGAGTCGCGGGCGTCGTG | 884 |
| GYPC | TTTGGTTAGTTTTCGCGGTTTT | 811 | CGACGAATACTCACCGAAACTAAA | 848 | TCGGGTTGACGTTTAGGAATGTGGTCG | 885 |
| RASSF1 | CGGGTTTCGATTTTAGCGTTT | 812 | CCATTACGCGACTCTCCTCAA | 849 | TAGGATTTAGATTGGGCGGCGGGAAG | 886 |
| GYPC | TGCGGGTCGCGGTTT | 813 | CCCAACAACACGACGTAACCT | 850 | TTTTTTTTACGGCGGGTATTTATCGAGGTTG | 887 |
| TRH | GATGTGTTCGCGTCGTGTTTT | 814 | CGATACACAAACGAACGCTATACTC | 851 | CGTCGGGTTTTCGGTTTGCGTTC | 888 |
| PTGER4 | AGGTTGCGTATCGTTAGTTATTATTATGTT | 815 | CACCGCCGAAATAATCACTAAAC | 852 | TTAATTCGTTCGTTTTTTTGAGTTTCGATCGG | 889 |
| FAIM2 | GAGGAGAGGGTCGGGTTTG | 816 | TTACCCCGAACTCGCGAAT | 853 | AAGATGTTGCGGAGGACGTTGCG | 890 |
| DMRTA2 | CGAGTTGTAGTTGTTTTACGGTATTGTC | 817 | TCGTAAACGAACCTCGAAAAAATAA | 854 | TTGGCGTTGGTCGTCGTTAACGGT | 891 |
| NA | GAGTTGGTGAGGTCGTTTAGTTAGG | 818 | GCCCGCTACGCTATACGAAA | 855 | TTGGTTAGCGTGCGATATAGGCGCG | 892 |
| TRABD | GTCGGGTTTTTTTGGAGGTTT | 819 | ACCCCCGACCTCACGAA | 856 | CGGACGATCGGAAGTGATTGAGCG | 893 |
| TCP11 | GAGGAGGAGGTGACGGTTTG | 820 | AAAACGTAAAAAAACCCCTTCCTT | 857 | TTTGGGTCGGCGTTTCGGGC | 894 |
| GATA3 | CGCGAGTATAGTCGAGGTTATGG | 821 | CCCGTTAAACACGACGAAATAAT | 858 | ACGGCGGATTAGTCGCGTTGGG | 895 |
| NA | GGCGGTGATGGTTTTGGA | 822 | AACGTACGACATAAACGCGAAA | 859 | CGCGCGGTCGTAGTGTGTCGTT | 896 |
| SPDYA | GTTGGTTAGCGATCGACGAGTA | 823 | CCGAACAACGACGATCAAAA | 860 | TCGTTTGAGGTTAGGAGCGTTGCGA | 897 |
| NA | GCGTAGCGGGCGGTATATT | 824 | CCCAACTATACGCATCGCTAACT | 861 | TAGAGTTATTATCGTTAGGAGGACGTATTCGGCG | 898 |
| KIAA1383 | AGTAGGTTGTAGGCGGAAGAGGTA | 825 | GCCGAACCGTAACCCGATA | 862 | TTTGTCGCGATCGAAGCGGATG | 899 |
| CDH4 | CGGCGGGAGTTGTAGGAATT | 826 | ACAACAACGCTACCCGAAAAA | 863 | TTTTTCGTGGTCGATCGGTTTCGTTG | 900 |
| EPHX3 | GGCGGGTTTAGCGGTTTC | 827 | CAAACGTACAAAAACGAACTAACGA | 864 | TTCGGCGTCGTTTTTTTGTTGGTTTAATAGG | 901 |
| NEFM | TTTTCGGTCGTCGTTTTTACG | 828 | CGCCGCAACCCATTTATAA | 865 | TTTTGGTTCGGGATTAGCGTCGCG | 902 |
| SORCS3 | CGTCGGGCGTAGTTTTTAATTATT | 829 | ACCCTTAACAAAAAAACGACTTCCT | 866 | ACGTTTGGGTTATTGTTTCGGTCGATGG | 903 |
| ZNF718 | CGTTTCGTCGTTGTTCGTATTTC | 830 | AAAAACGAATCCGCGACGTA | 867 | CGCGTTCGGAGTTTAGCGTAGTATAGAGGAGTC | 904 |
| TRH | CGGGATTCGTAGTCGGTAGGT | 831 | GACCCATCTAAAAAAAACTCGACAA | 868 | AGTTTGTAGCGTTGAGGATTTCGGGCG | 905 |
| DMRT1 | ACGCGTTTTCGGCGTTT | 832 | ACAACGACGACGCTTAAAATCC | 869 | ATCGGCGATGTAGCGTGGTCGG | 906 |
| MYO3A | TTTTTGCGTTGTTTCGATACGT | 833 | GTAACCAACGCCCCCGTAA | 870 | TTTGGGCGGGAAGTCGGGC | 907 |
| NA | TTCGGTTTTGCGGAGTAGTTG | 834 | ACTCGCTACGCCACGAAAAT | 871 | ATTCGAAGGGCGGCGCGG | 908 |
| FAM38B | GC6TTTTTTTTTAAGGTTTCGGTAT | 835 | CCTACGACCGACCCATTTAATATAA | 872 | TTTTGAAGAAGTCGTCGTCGTTGTCGG | 909 |
| NA | CGGAGGCGACGGAGATATTA | 836 | AATAACGACAAACGACAACAACAAA | 873 | CGAGAATTAGATGTTCGTCGTTCGCGTG | 910 |
| DMRTA2 | CGTTATGATACGTTGGCGTTTC | 837 | CAAAAACCACAAACGCTACTATCG | 874 | CGATGAGCGTGTATTTGGCGTATAGGTAGTTTT | 911 |
| LBX2 | CGGATTGGGAGTCGAGTATTG | 838 | TCGCGTCCCGAATTCATAA | 875 | CGGATAGTCGGATCGTTTTAGTTCGGTCG | 912 |
| NA | GCGTGGTGTCGTCGTTGTT | 839 | ACGCACAACTAAACCGTACAATAACT | 876 | CGGATTAGCGTTTTGGTTGTCGCG | 913 |
| NA | CGTATAGTTGGGTCGTGTAGTGGTT | 840 | ACTAACGTAATACCGCCGCTACTAA | 877 | CGCGGTAGTTAGAGCGTTGGTTCGC | 914 |
| NA | TTGCGTTAGGATTTTGTAGATTCG | 841 | CCGTACAACCCTAATATTACAAACGTAA | 878 | TCGCGGTCGGGTCGAGTGTTTT | 915 |
| GDF6 | GTTAGTCGGGTTGGAGTTTTGC | 842 | CCACTCGTATTAAAACCTCGAAACA | 879 | TTTCGGTTTTTTTGTTTTAGCGTGGTGTTTTC | 916 |
| GAD2 | GGAAGAGCGTTACGGTTTTAGAGT | 843 | ACGAAACCTACCCGCTAAATCC | 880 | TTTTTGGAGTTTCGAGGTTAGGTTCGGG | 917 |
| NA | TTATTAATTCGGTTGCGTAAATCG | 844 | CCGACAACTCACAAATAAAATAATCC | 881 | TTTGGTTGAGCGGTCGTTGTGCG | 918 |
| FOXE1 | GGGTAATTATTGGGCGTTTGATT | 845 | GCTTACGACGACGCAAAAAACT | 882 | TAACGCGGAGGATATGTTCGAGAGCG | 919 |

**Table 12**

| rxn_name | gene_name | Chromosome | Rxn.Start | Rxn.Finish | Amplicon.Location | Amplicon .Length | Amplicon SEQ ID NO | amplicon_sequence_5_to_3 |
|---|---|---|---|---|---|---|---|---|
| ML_cg25060829_ii | ZSCAN 12 | 6 | 28367535 | 28367599 | chr6:283675 35-28367599 | 65 | 920 | GGC[[CG]]GAAG[[CG]]GCCA[[CG]]GGGGGT CTAAGAACCAGCC[[CG]][[CG]][[CG]]GGG[[C G]]CACTTC[[CG]][[CG]]GC[[CG]]CTCT |
| ML_cg14443519_i | HCG4 | 6 | 29760319 | 29760385 | chr6:297603 19-29760385 | 67 | 921 | T[[CG]]ACAG[[CG]]A[[CG]]CCA[[CG]]AGTCC CAGGATGGAGC[[CG]][[CG]]GG[[CG]]C[[CG] ]TGGATGGAGCAGGAGGGGC[[CG]]GA |
| ML_cg15975865_i | GYPC | 2 | 127413797 | 127413902 | chr2:127413 797-127413902 | 106 | 922 | CCTGGCCAGTCCC[[CG]][[CG]]GTCTCTGCC[[ CG]]GGCTGA[[CG]]CCCAGGAATGTGGT[[CG ]]A[CG]AGAAGCCCCAACAGCA[CG]G[CG]TG GCCTCTCAGCCT[[CG]]GTGAGTACC[[CG]]C[[ CG]] |
| ML_cg27569446_i | RASSF1 | 3 | 50378305 | 50378379 | chr3:503783 05-50378379 | 75 | 923 | [[CG]]GGCCC[[CG]]ACTTCAG[[CG]]CCTCCCC CAGGATCCAGACTGGG[[CG]]G[[CG]]GGAA GGAGCTGAGGAGAGC[[CG]][[CG]]CAATGG |
| ML_cg15768103_ii | GYPC | 2 | 127413858 | 127413944 | chr2:127413 858-127413944 | 87 | 924 | CCCAACAGCA[[CG]]G[[CG]]TGGCCTCTCAG CCT[[CG]]GTGAGTACC[[CG]]C[[CG]]TGGGG AAGGGTCCTGGGGACCCACTGGAGGC[[CG]] [[CG]]GCC[[CG]]CA |
| ML_cg05835982_ii | TRH | 3 | 129693780 | 129693868 | chr3:129693 780-129693868 | 89 | 925 | [[CG]]GTGCACAGG[[CG]]GG[[CG]]CTGTGCT [CG]CACCCC[CG]GGTCCTCCC[CG]AG[[CG]] CAGGC[[CG]]GGGACC[[CG]]A[[CG]]GGAGG CA[[CG]]G[[CG]][[CG]]GGCACATC |
| ML_cg05971966_i | PTGER4 | 5 | 40681071 | 40681173 | chr5:406810 71-40681173 | 103 | 926 | AGGCTG[[CG]]CAC[[CG]]CCAGCCACTATCAT GTCCACTCC[CG]GGTCAATT[[CG]]TC[[CG]] CCTCCTTGAGCCC[[CG]]AC[[CG]]GCTGAACA GCCCAGTGACCATCC[[CG]]G[[CG]]GTG |
| ML_cg06947913_i | FAIM2 | 12 | 50297727 | 50297790 | chr12:50297 727-50297790 | 64 | 927 | GAGGAGAGGGC[[CG]]GGCCTGGGAAGATG CTG[[CG]]GAGGA[[CG]]CTG[[CG]]GATT[[CG ]][[CG]]AGCC[[CG]]GGGTAA |
| ML_cg16732616_ii | DMRTA2 | 1 | 50886745 | 50886828 | chr1:508867 45-50886828 | 84 | 928 | T[[CG]]TAGG[[CG]]GGCCT[[CG]]GGGGGAT GATGC[[CG]]TTGG[[CG]]G[[CG]]GCCAG[[C G]]CCAGCCCCT[CG]GCAGTGC[[CG]]TAGAG CAGCTGCAGCT[[CG]] |
| ML_cg22305167_i | N/A | 6 | 29521375 | 29521459 | chr6:295213 75-29521459 | 85 | 929 | GCC[[CG]]CTG[[CG]]CTATG[[CG]]GGGCT[C G]TCTCCC[[CG]][[CG]]CCTATGT[[CG]]CA[[C G]]CTGGCCAG[CG]CCTCCTGGCTAAG[[CG]] GCCTCACCAACTC |
| ML_cg05297854_i | N/A | 22 | 50623618 | 50623686 | chr22:50623 618-50623686 | 69 | 930 | GC[[CG]]GGTCCCCCTGGAGGCCTC[[CG]]GA[ [CG]]AT[[CG]]GAAGTGACTGAG[[CG]]TC[CG ]GGTC[[CG]]TGAGGC[[CG]]GGGGT |
| ML_cg13423262_i | TCP11 | 6 | 35108892 | 35108990 | chr6:351088 92-35108990 | 99 | 931 | GAAG[[CG]]TGGGAAGGCCCCTTCCTT[CG][C G]G[CG]AGGTGCCCCCCT[CG]CC[[CG]]AAA[ [CG]]C[[CG]]GCCCAGGCC[CG]CCTC[CG]GCT CCCAGGC[[CG]]TCACCTCCTCCTC |
| ML_cg15267232_i | GATA3 | 10 | 8097600 | 8097681 | chr10:80976 00-8097681 | 82 | 932 | [[CG]][[CG]]AGCACAGC[[CG]]AGGCCATGG AGGTGA[[CG]]G[[CG]]GACCAGC[[CG]][[CG] ]CTGGGTGAGCCACCACCACCC[[CG]]C[[CG]] TGCTCAA[[CG]]GG |
| ML_cg21632158_i | N/A | 6 | 29521337 | 29521423 | chr6:295213 37-29521423 | 87 | 933 | GG[[CG]]GTGATGGCTCTGGAC[[CG]][[CG]][[ CG]]GC[[CG]]CAGTGTGC[[CG]]CC[CG]CTG[ CG]CTATG[CG]GGGCT[CG]TCTCCC[[CG]][[C G]]CCTATGT[[CG]]CA[[CG]]CT |
| ML_cg14920003_i | SPDYA | 2 | 29033710 | 29033782 | chr2:290337 10-29033782 | 73 | 934 | GCTGGCTAG[[CG]]AC[[CG]]A[[CG]]AGCAAC [[CG]]TCTGAGGCCAGGAG[[CG]]CTG[[CG]]A [CG]GAGCCTTGAC[[CG]]C[[CG]]TTGCC[[CG] ]G |
| ML_cg21632158_ii | N/A | 6 | 29521288 | 29521385 | chr6:295212 88-29521385 | 98 | 935 | CCCAGCTGTG[[CG]]CAT[[CG]]CTGGCTCTGG GTTC[[CG]]C[[CG]]AATG[[CG]]TCCTCCTGG[ [CG]]GTGATGGCTCTGGAC[CG][CG][CG]GC[ CG]CAGTGTGC[[CG]]CC[[CG]]CTG[[CG]]C |
| ML_cg16430166_ii | MAP10 | 1 | 232941433 | 232941501 | chr1:232941 433-232941501 | 69 | 936 | GC[[CG]]AAC[[CG]]TGGCC[[CG]]GTGTCATC[ [CG]]CTT[[CG]]GT[[CG]][[CG]]GCAAGTCCTG CCTCTTC[[CG]]CCTGCAGCCTGCT |
| ML_cg16619425_i | CDH4 | 20 | 59828486 | 59828556 | chr20:59828 486-59828556 | 71 | 937 | [[CG]]G[[CG]]GGAGTTGCAGGAACCCCCTCT[ [CG]]TGGC[[CG]]AT[[CG]]GCCT[[CG]]CTGCC TTCTCC[[CG]]GGCAG[[CG]]CTGTTGT |
| ML_cg24520342_i | N/A | 19 | 15344240 | 15344316 | chr19:15344 240-15344316 | 77 | 938 | GG[[CG]]GGTCCAG[[CG]]GCCT[CG]GCC[[CG ]]G[[CG]]C[[CG]]TCCTCCTGCTGGCCCAACA GGCC[[CG]]CCAGCC[[CG]]CCCCTGTA[[CG]] TCTG |
| ML_cg07552803_i | NEFM | 8 | 24771188 | 24771260 | chr8:247711 88-24771260 | 73 | 939 | TCCT[[CG]]GC[[CG]]C[[CG]]CCTCCA[[CG]]G GG[CG]GGGCCCTGGCC[[CG]]GGACCAG[[C G]]C[[CG]][[CG]]GCTATAAATGGGCTG[[CG]] G[[CG]] |
| ML_cg01874697_i | SORCS3 | 10 | 106401479 | 106401574 | chr10:10640 1479-106401574 | 96 | 940 | [[CG]]C[[CG]]GG[[CG]]CAGCCCCCAATCACC CAGGAA[CG][CG]GGGA[[CG]]CCTGGGCCA CTGCTC[[CG]]GC[[CG]]ATGGTTCCAGAGGA AGC[[CG]]TCCCCTTGCTAAGGGT |
| ML_cg26894523_ii | ZNF718/ZN F595 | 4 | 107714 | 107802 | chr4:107714-107802 | 89 | 941 | AGGGG[[CG]]GGTC[[CG]][[CG]]G[[CG]]TG[C G]GGGCCCTGG[CG]GCTCCTCTGTGCTG[[CG ]]CTGGGCTC[[CG]]GG[[CG]][[CG]][CG]GGA TG[[CG]]GGCAG[[CG]]G[[CG]]GGA[[CG]] |
| ML_cg02700891_ii | TRH | 3 | 129693544 | 129693621 | chr3:129693 544-129693621 | 78 | 942 | GGCCCATCTGAAGAGGGCT[[CG]]GCAGG[[C G]]CC[[CG]]GGGTCCTCAG[[CG]]CTGCAGAC TCCTGACCTGC[[CG]]ACTG[[CG]]GATCC[[CG ]] |
| ML_cg07528692_ii | DMRT1 | 9 | 842623 | 842694 | chr9:842623-842694 | 72 | 943 | ACAG[[CG]]G[[CG]]G[[CG]]CTTGGGGTCCTG GGCTCC[[CG]]GCCA[[CG]]CTACAT[[CG]]C[[ CG]]GTCTGGGAG[[CG]]C[[CG]]GAGA[[CG]][ [CG]]T |
| ML_cg15843567_ii | MYO3A | 10 | 26223207 | 26223281 | chr10:26223 207-26223281 | 75 | 944 | GTAGCCAG[[CG]]CCCC[[CG]]TAAAGAAATA AGGAGG[CG]CC[[CG]]GCTTCC[[CG]]CCCAG GTA[[CG]]TGT[[CG]]GGACAG[[CG]]CAGGG G |
| ML_cg17759274_ii | N/A | 19 | 39260951 | 39261015 | chr19:39260 951-39261015 | 65 | 945 | GCT[[CG]]CTG[[CG]]CCA[[CG]]GAAATCC[[C G]][[CG]]C[[CG]]CCCTT[[CG]]AGTCCTCCCA GCTGCTC[[CG]]CAGAGC[[CG]]GG |
| ML_cg03602280_ii | PIEZO2 | 18 | 11148842 | 11148934 | chr18:11148 842-11148934 | 93 | 946 | CCTG[[CG]]GC[[CG]]GCCCATTTAGTGTGAAT CCTGGATC[[CG]]GCAG[[CG]]G[[CG]]G[[CG] ]GCTTCTTCAGGGGTAGCTGATGC[[CG]]GGG CCTTGGAGAGGGA[[CG]]C |
| ML_cg00370229_i | N/A | 6 | 29521559 | 29521637 | chr6:295215 59-29521637 | 79 | 947 | [[CG]]GAGG[[CG]]A[[CG]]GAGACACTAC[[C G]]AGAACCAGATGTT[[CG]]C[[CG]]CC[[CG]] [[CG]]TGGTCATCCTGCTGCTGC[[CG]]TTTGC [[CG]]TCATC |
| ML_cg25191628_i | DMRTA2 | 1 | 50886879 | 50886963 | chr1:508868 79-50886963 | 85 | 948 | [[CG]]CCATGACA[[CG]]CTGG[[CG]]CTC[[CG]][ [CG]]ATGAG[[CG]]TGCACTTGG[[CG]]CACA GGCAGTCCTTCCAG[[CG]]ACAGTAG[[CG]]T TTGTGGCCCTTG |
| ML_cg25040225_ii | LBX2 | 2 | 74726648 | 74726747 | chr2:747266 48-74726747 | 100 | 949 | T[[CG]][[CG]]TCC[[CG]]AGTTCATGGT[[CG]] GC[[CG]]GGCTGGGG[[CG]]GTC[[CG]]GCTGT C[[CG]]TTG[CG]CTAGGCTC[CG]CAAA[CG]C CTGGGCCCCAGTGCT[[CG]]GCTCCCAATC[[C G]] |
| ML_cg10491628_i | N/A | 6 | 29521219 | 29521300 | chr6:295212 19-29521300 | 82 | 950 | G[[CG]]TGGTGC[[CG]]C[[CG]]CTGCTGGCCA ACCTG[CG][[CG]]GACCAG[[CG]]CTCTGGCT GC[[CG]][[CG]]CAGCCACTGCA[[CG]]GCCCA GCTGTG[[CG]]C |
| ML_cg10491628_ii | N/A | 6 | 29521215 | 29521299 | chr6:295212 15-29521299 | 85 | 951 | ACTAG[[CG]]TGGTGC[[CG]]C[[CG]]CTGCTG GCCAACCTG[CG][[CG]]GACCAG[[CG]]CTCT GGCTGC[[CG]][[CG]]CAGCCACTGCA[[CG]]G CCCAGCTGTG[[CG]] |
| ML_cg10210594_i | N/A | 1 | 208132750 | 208132848 | chr1:208132 750-208132848 | 99 | 952 | CTG[[CG]]CCAGGACCCTGCAGACC[[CG]]GC[ [CG]][[CG]]GC[[CG]]GGC[[CG]]AGTGCCTCC[ CG]C[CG]GGGCCTTA[CG]CCCA[CG]GTCCA[[ CG]]CTTGCAACACCAGGGCTGTA[[CG]]G |
| ML_cg11073558_ii | GDF6 | 8 | 97171863 | 97171947 | chr8:971718 63-97171947 | 85 | 953 | CCACT[[CG]]TGTTGAGGCCT[[CG]]GAGCAG C[CG]GGAGCACCA[[CG]]CTGGAGCAAAAAA GC[[CG]]GAGGG[CG]CAGGGCTCCAGCC[[C G]]GCTGAC |
| ML_cg27206674_ii | GAD2 | 10 | 26507075 | 26507154 | chr10:26507 075-26507154 | 80 | 954 | G[[CG]]GGACCTGCC[[CG]]CTGGGTCCAAGC CCC[[CG]]AACCTGGCCT[[CG]]GAGCTCCAA GAGGGCTCTGAGGC[[CG]]TGG[[CG]]CTCTT CC |
| ML_cg21133153_i | N/A | 6 | 29521450 | 29521539 | chr6:295214 50-29521539 | 90 | 955 | TCACCAACT[[CG]]GTTG[[CG]]CAAAC[[CG]][ CG]CTCCTGGCTGAG[[CG]]GC[[CG]]CTGTG[[ CG]][CG]CCC[CG]CCTGCTGGACCACTTCATC TGTGAGCTGC[[CG]]G |
| ML_cg13791254_i | FOXE1 | 9 | 100616565 | 100616638 | chr9:100616 565-100616638 | 74 | 956 | GGGCAACTACTGGG[[CG]]CTTGACCCCAA[[ CG]][[CG]]GAGGACATGTT[[CG]]AGAG[[CG] ]GCAGCTTCCTG[[CG]]C[[CG]]C[[CG]]CAAGC |

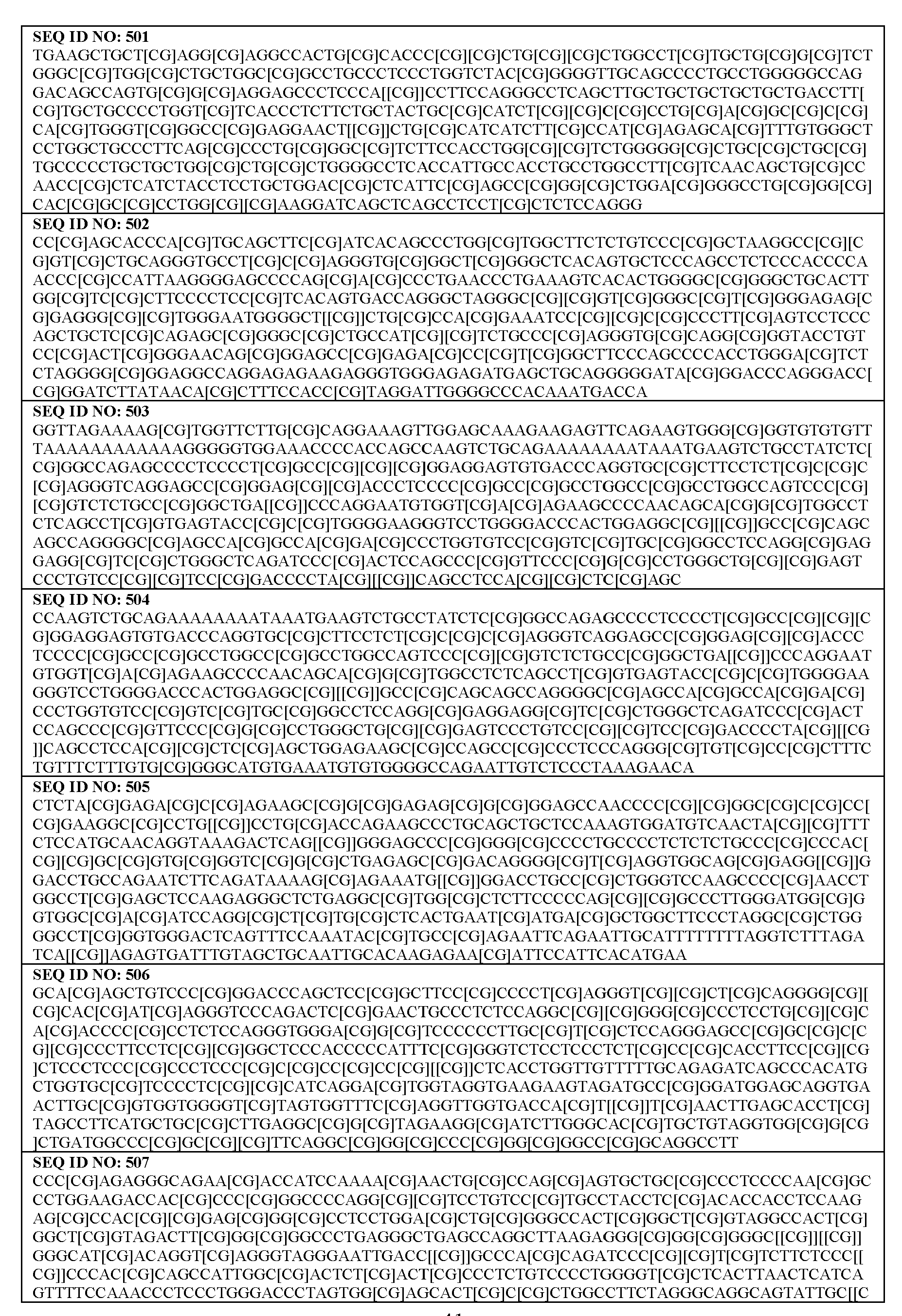

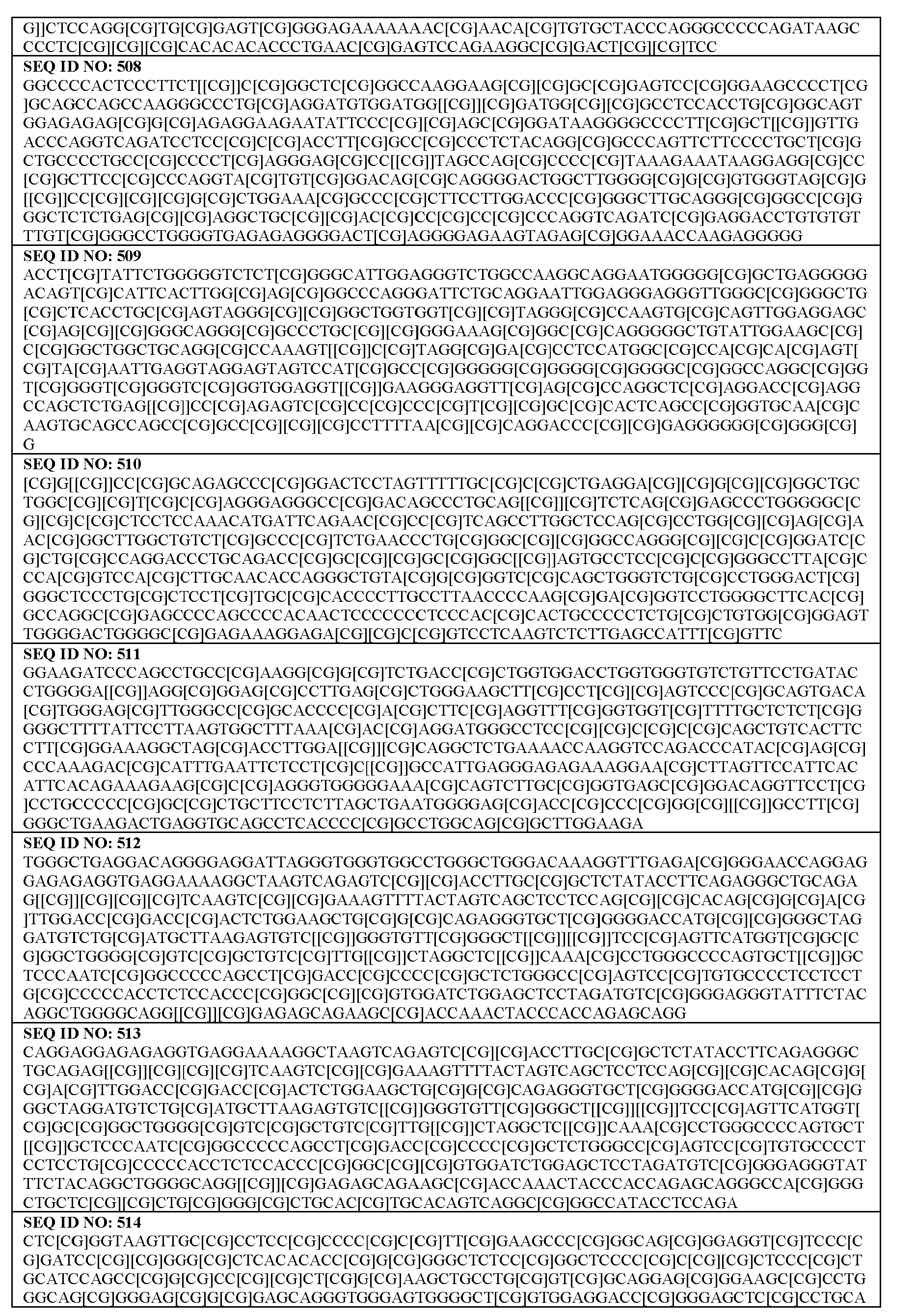

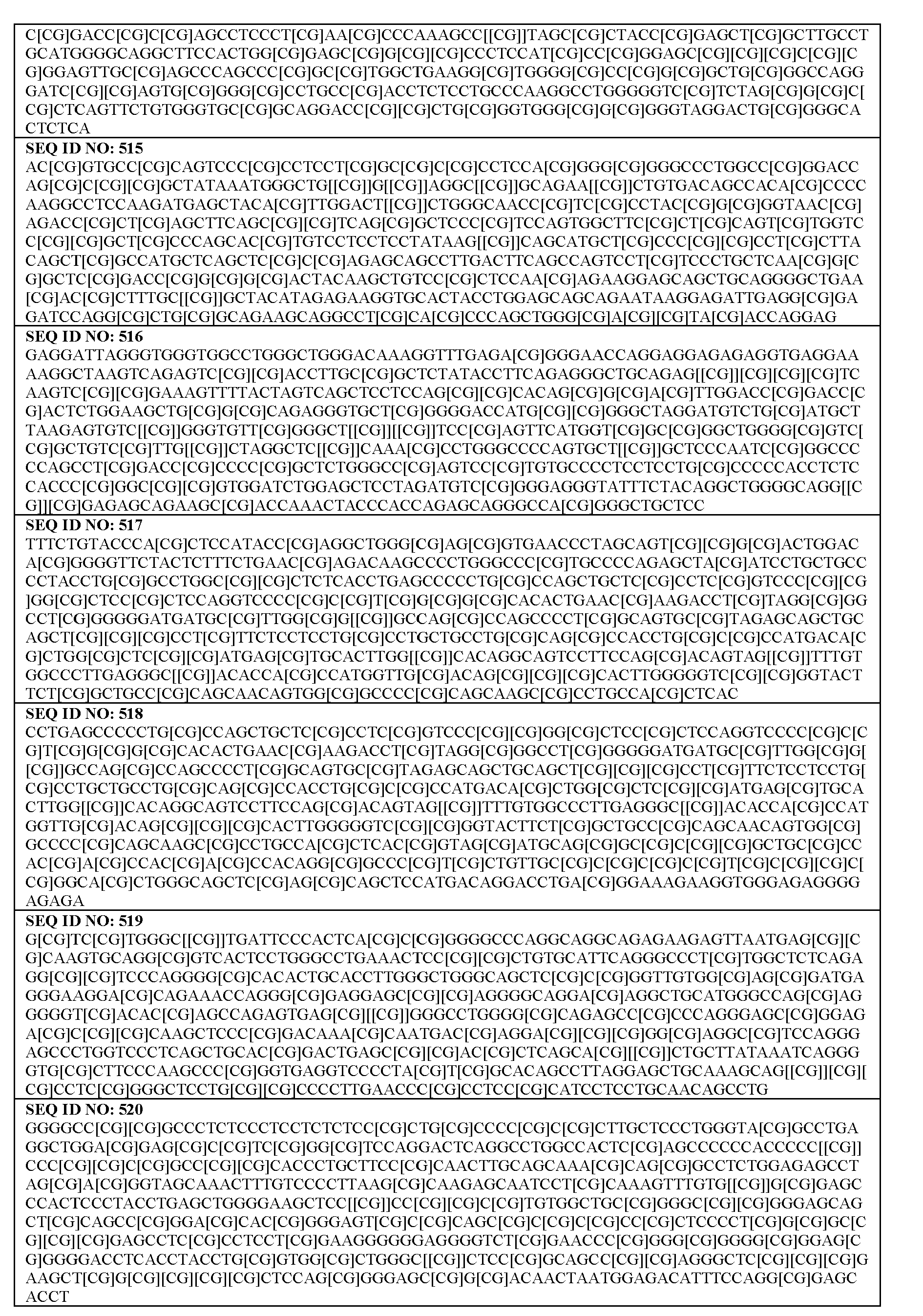

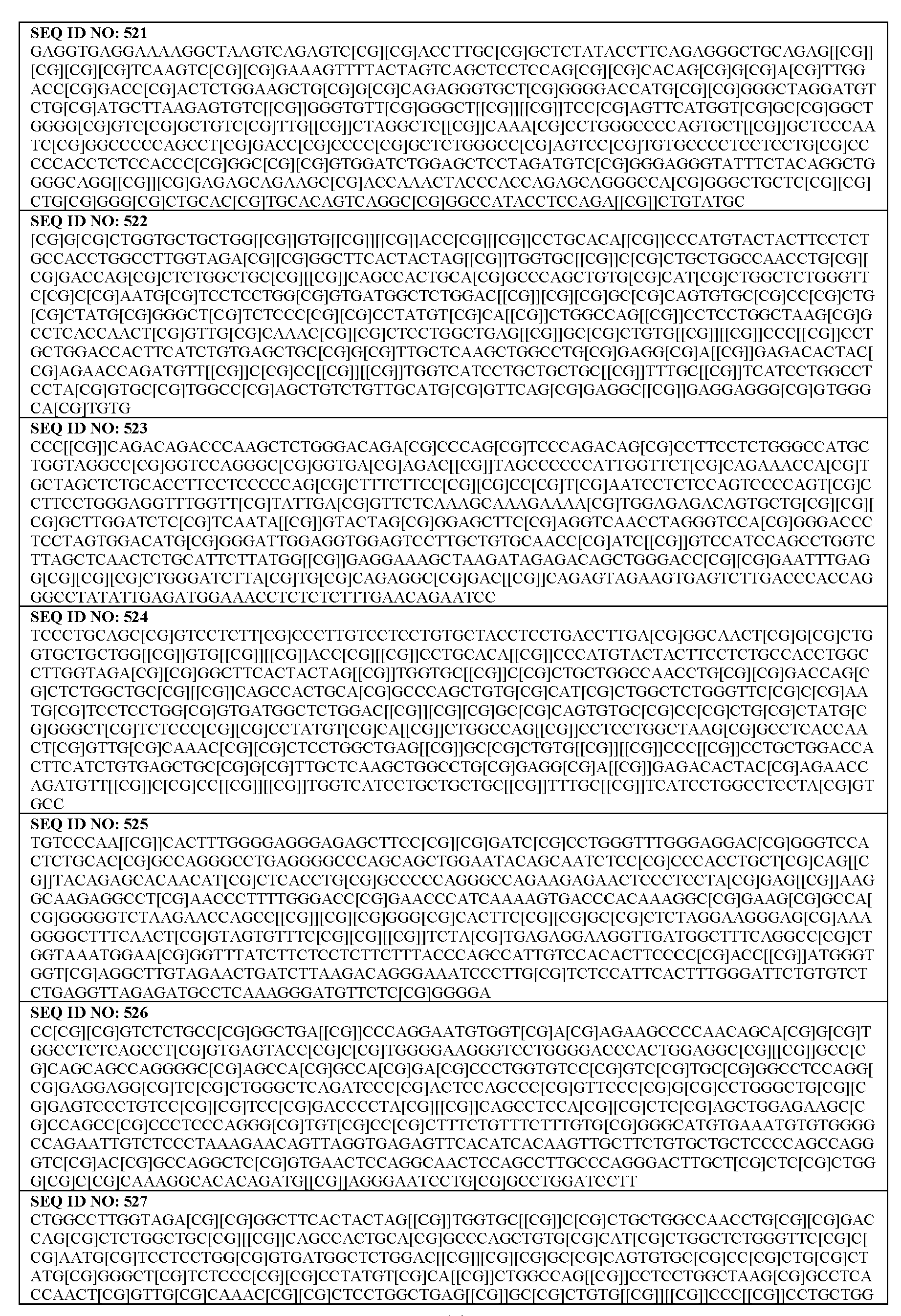

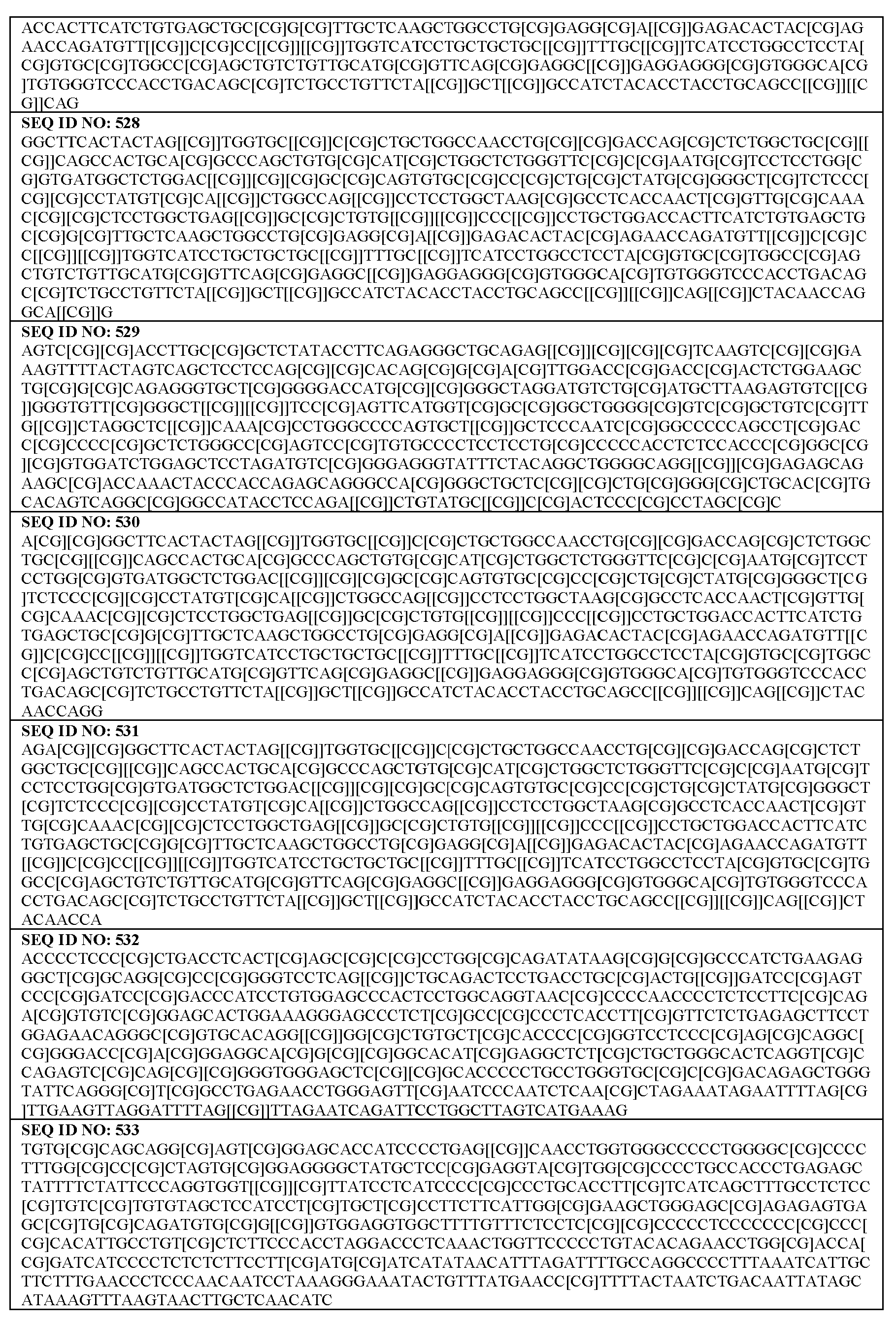

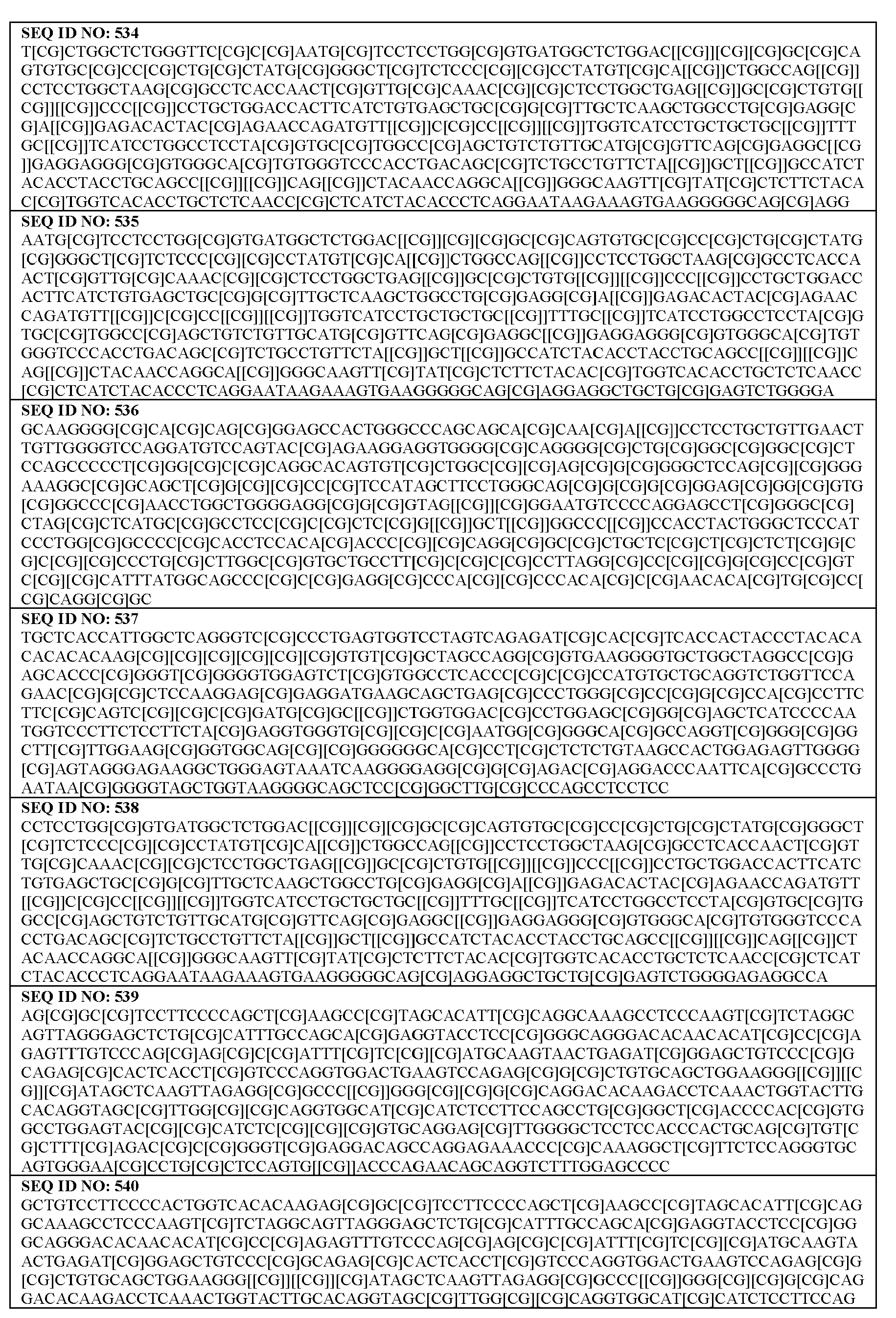

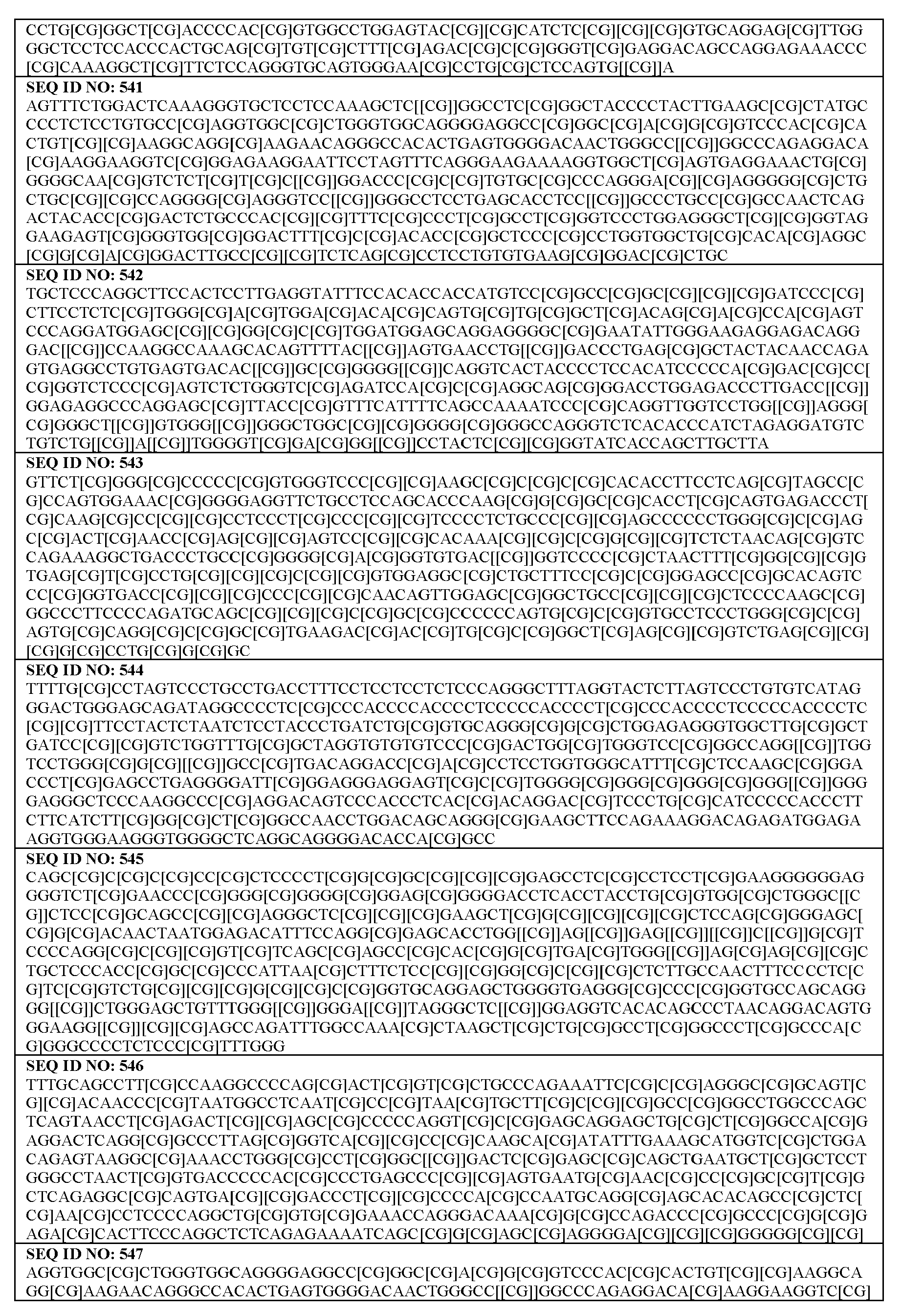

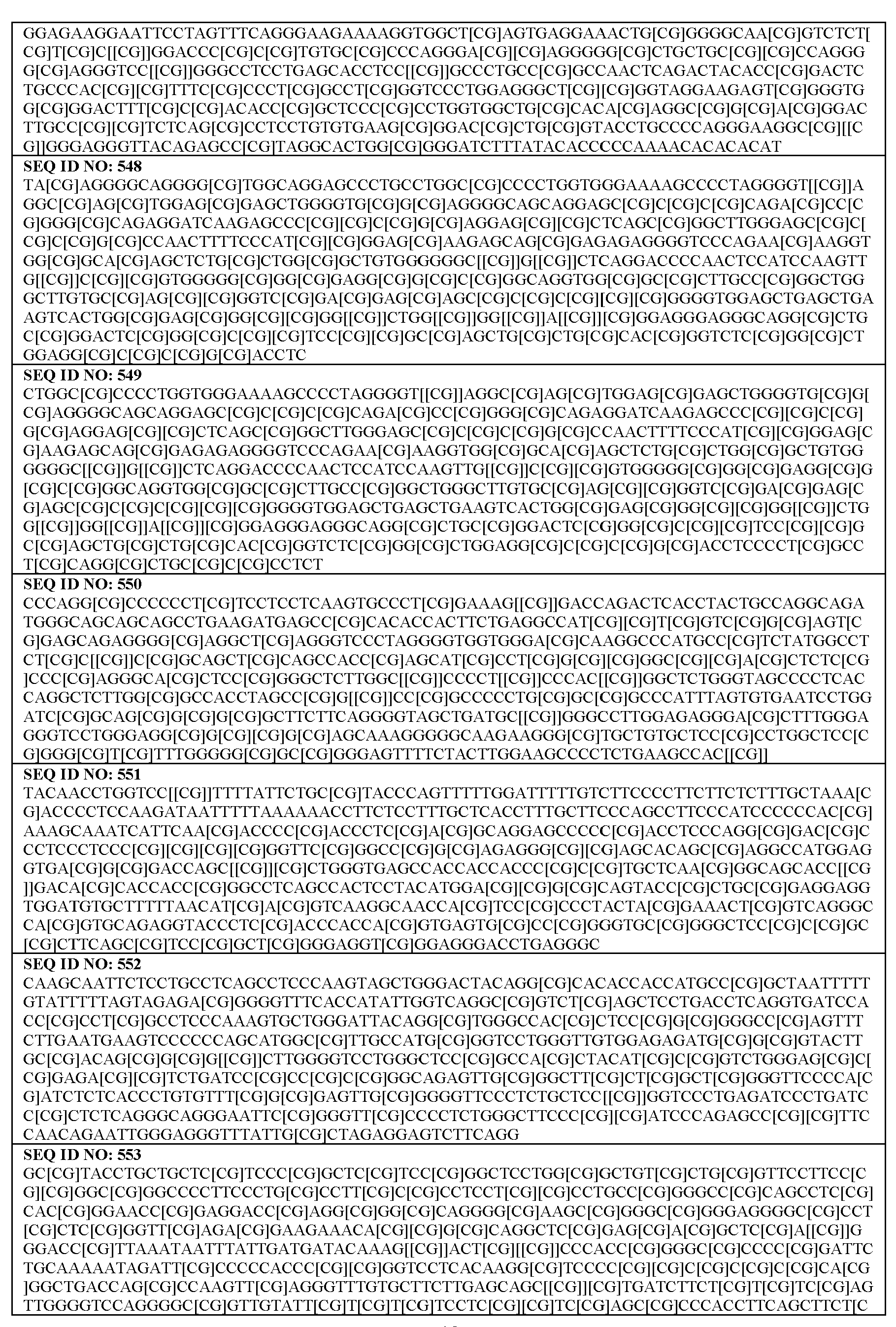

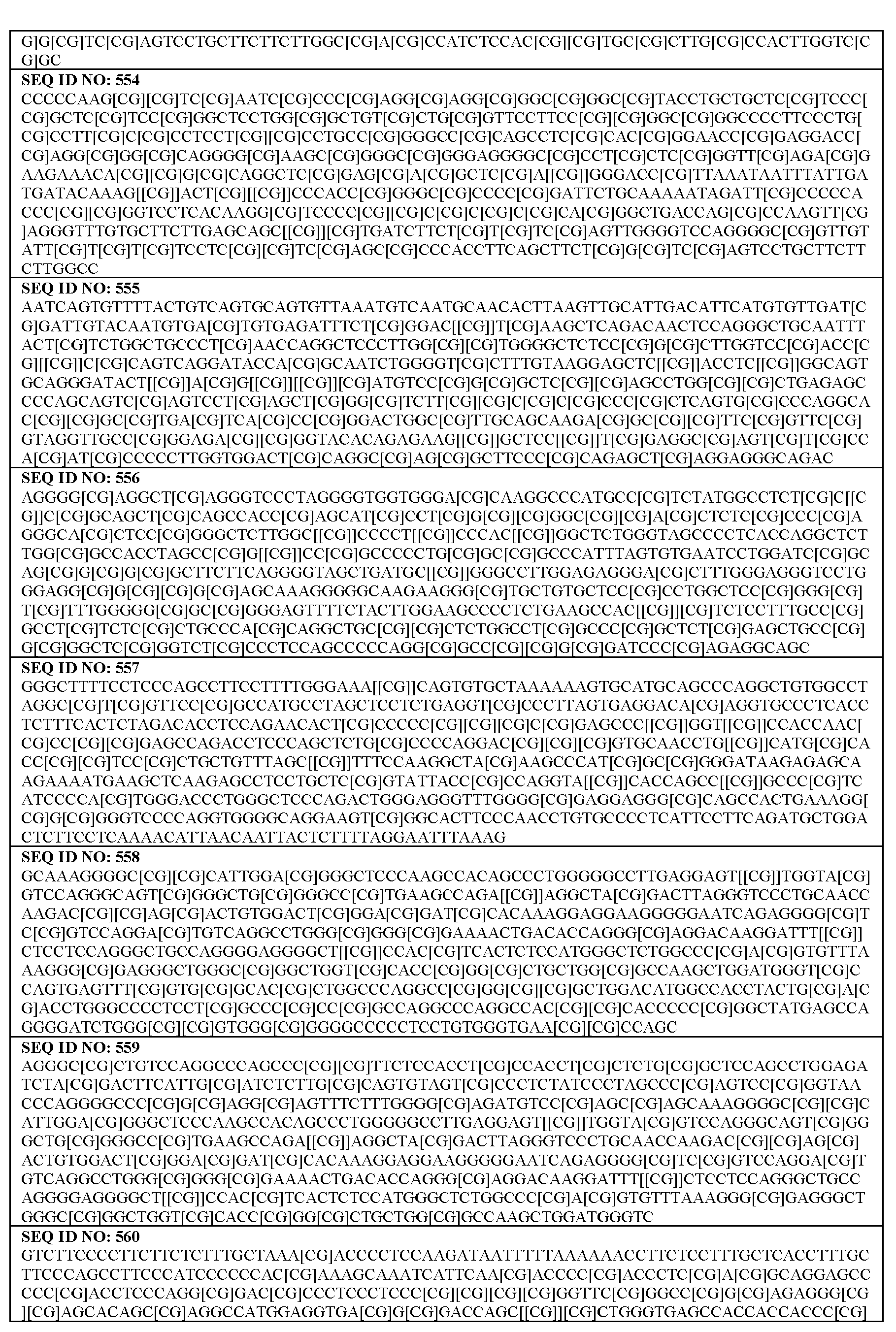

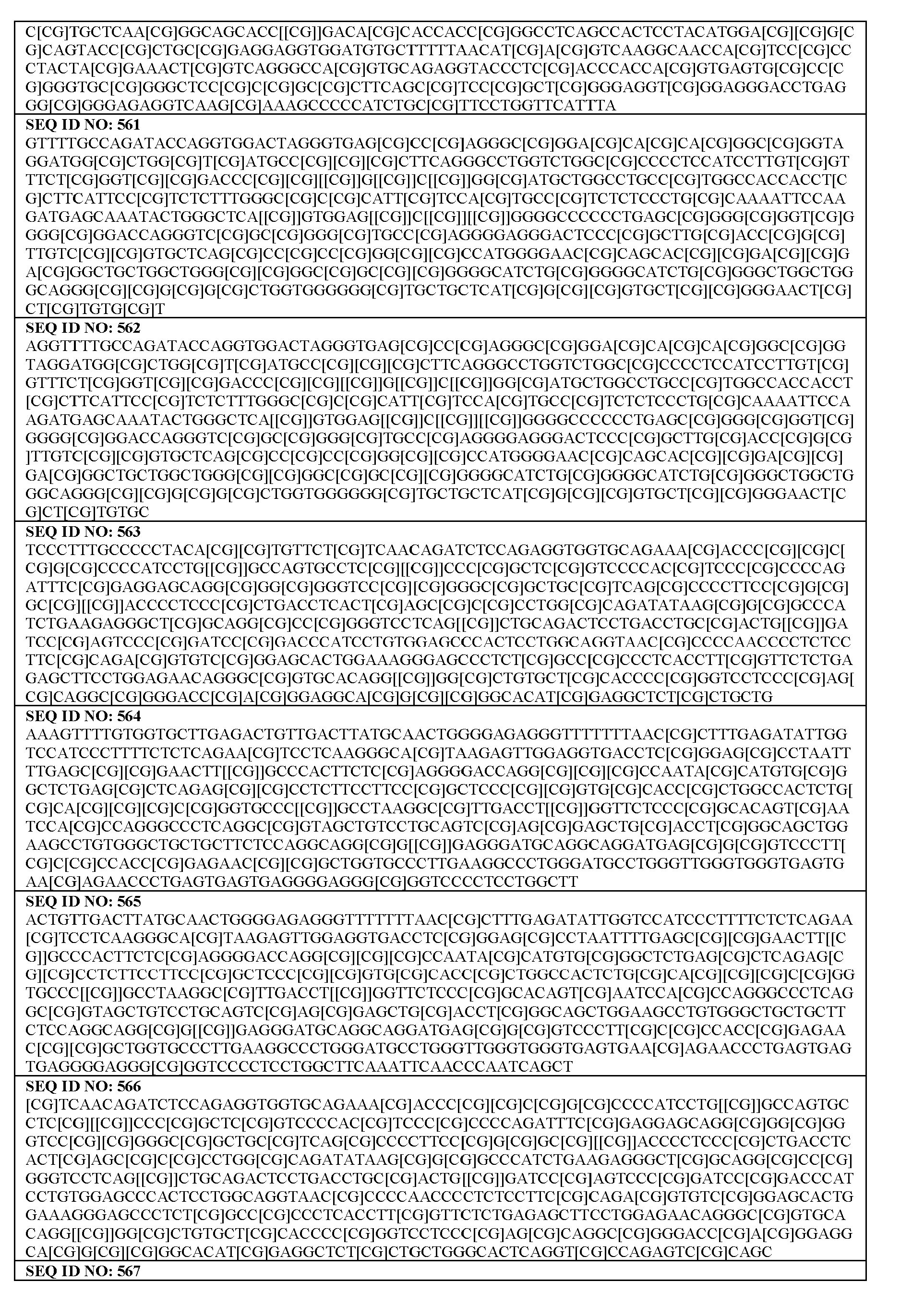

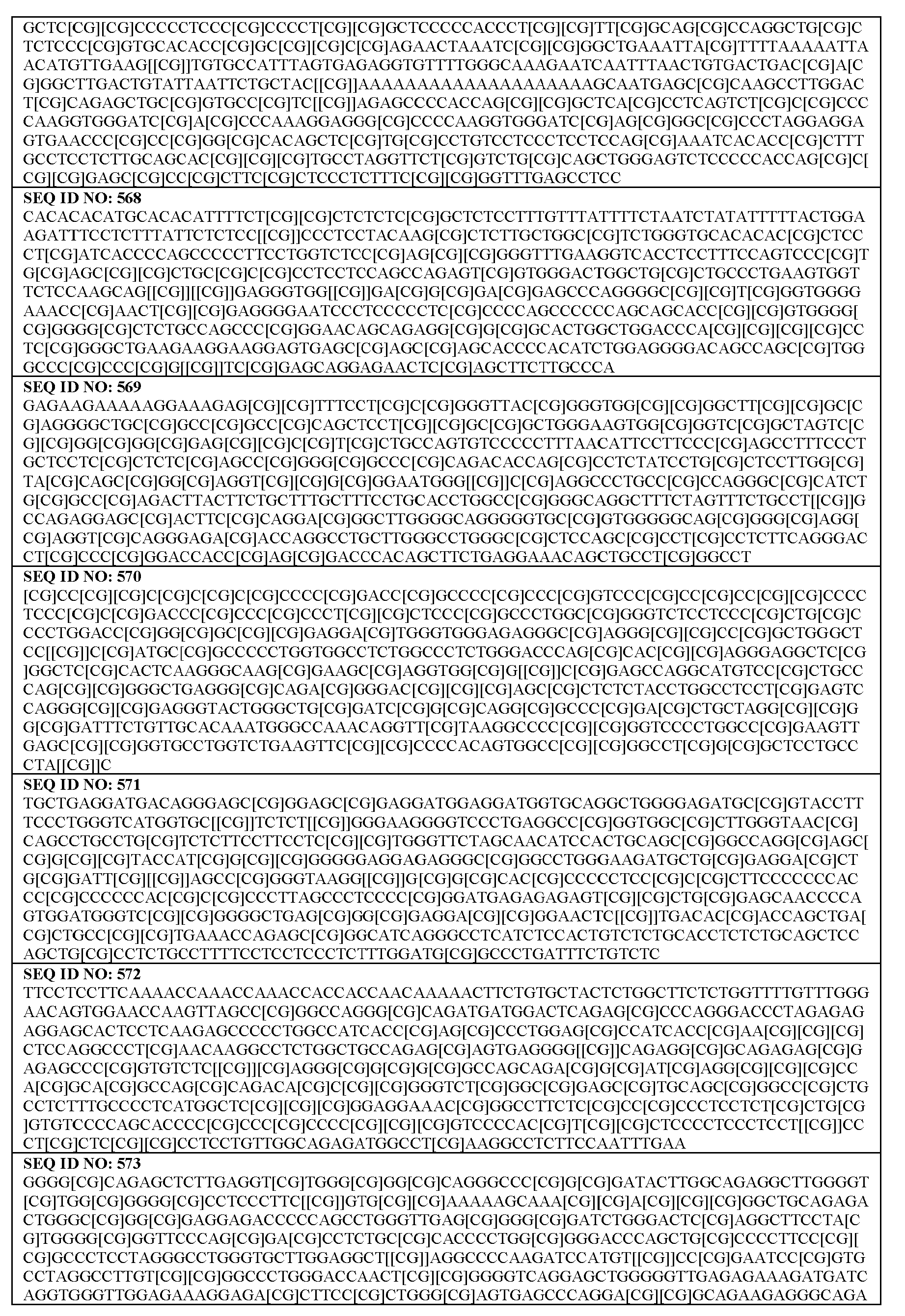

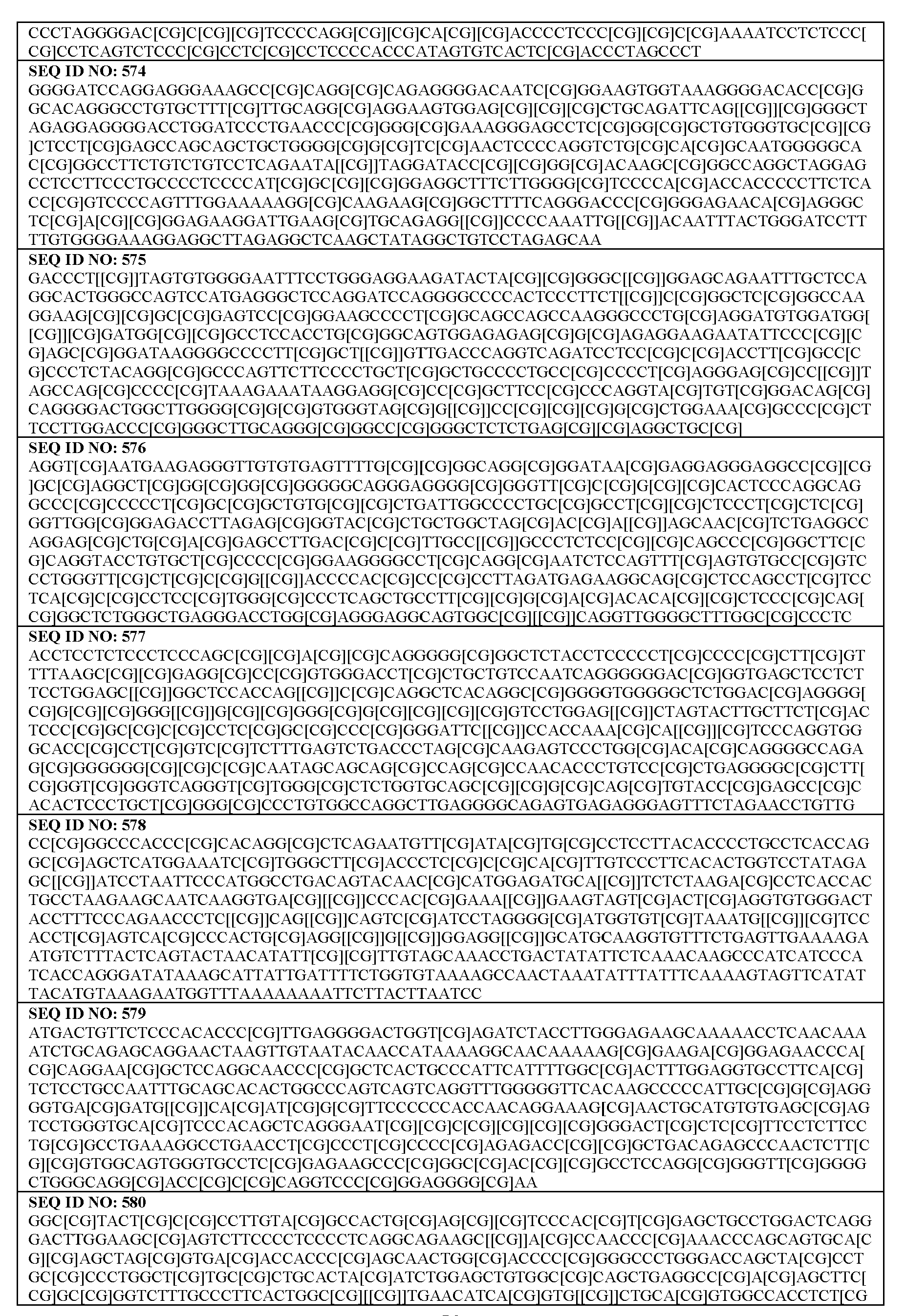

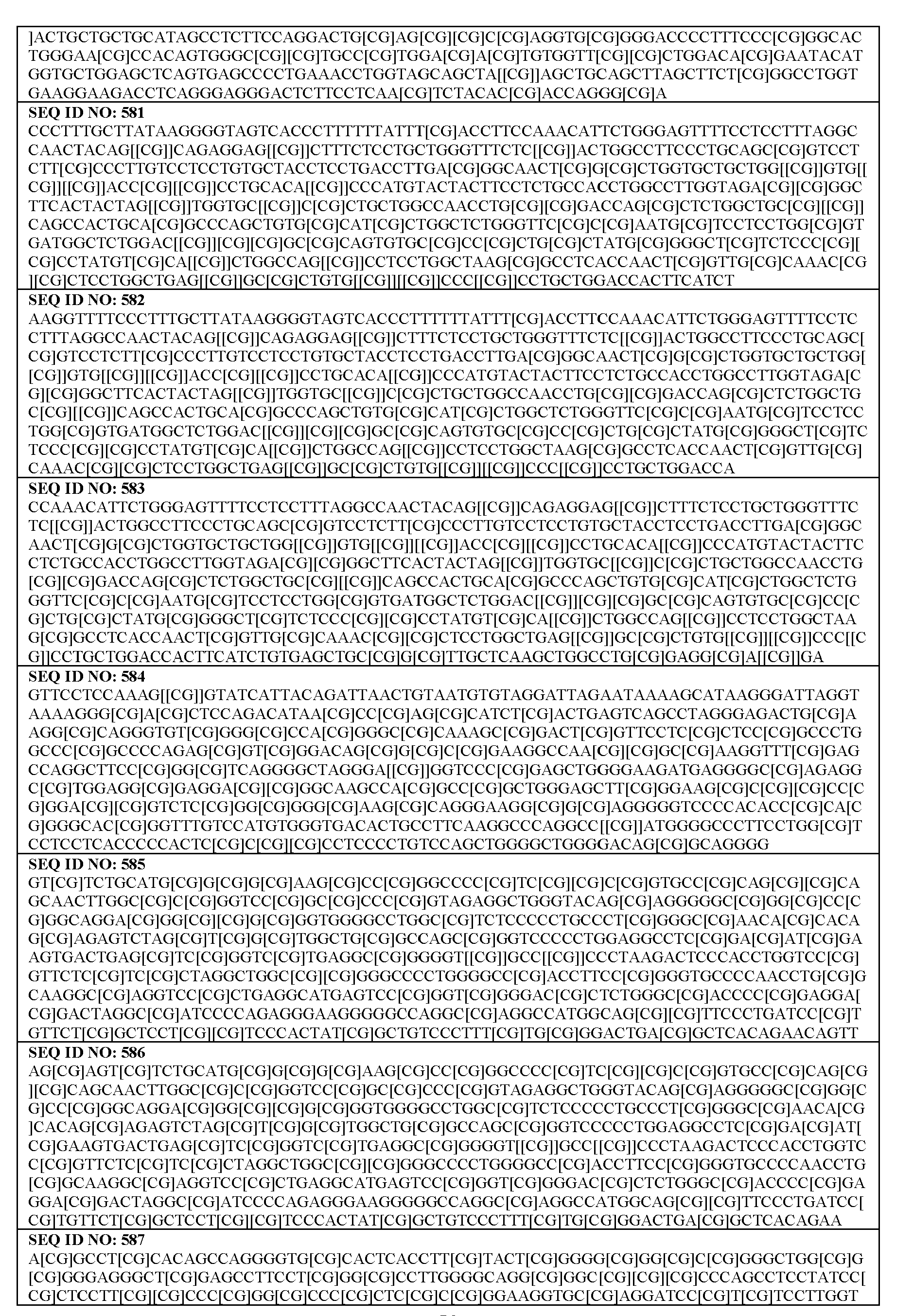

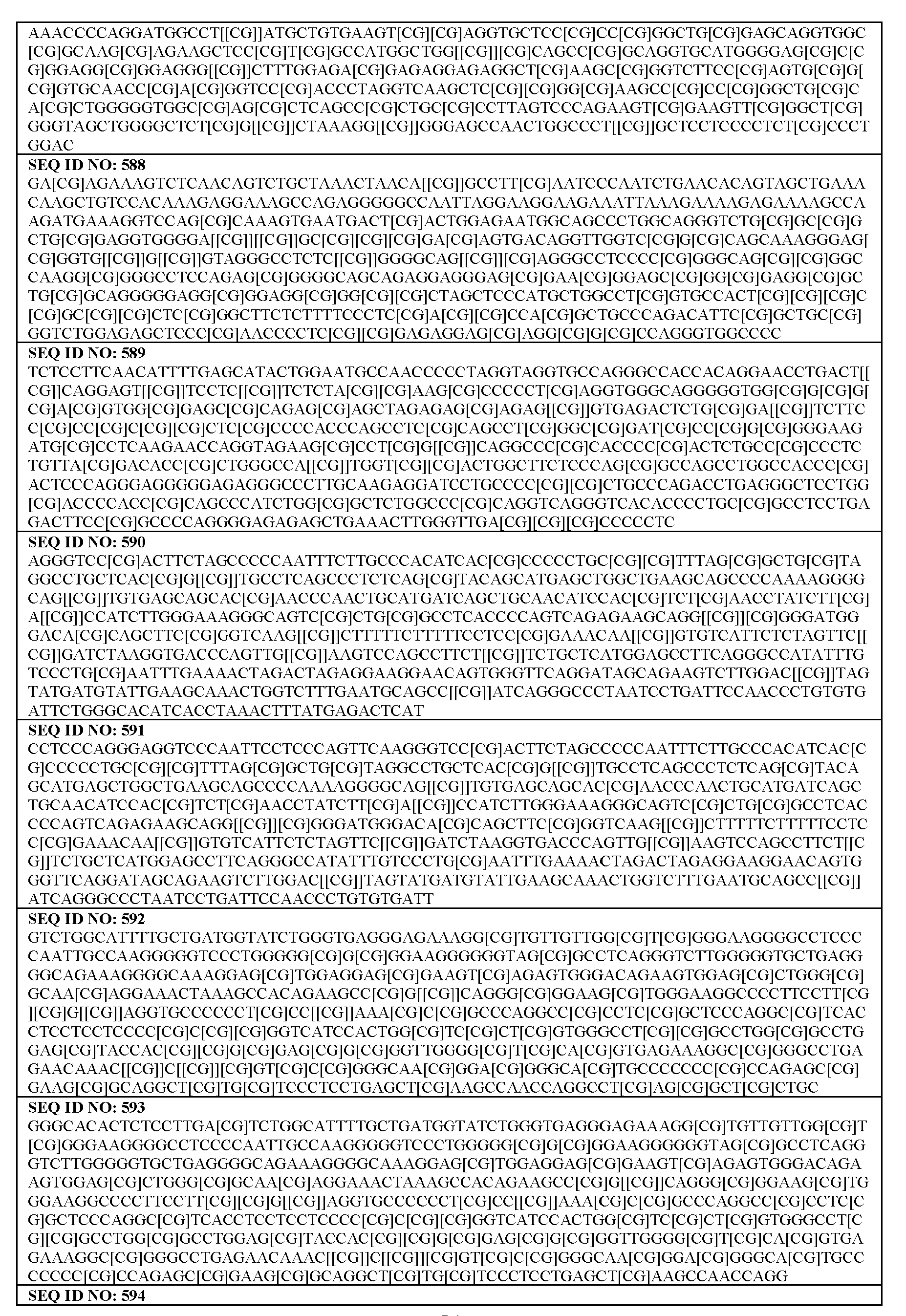

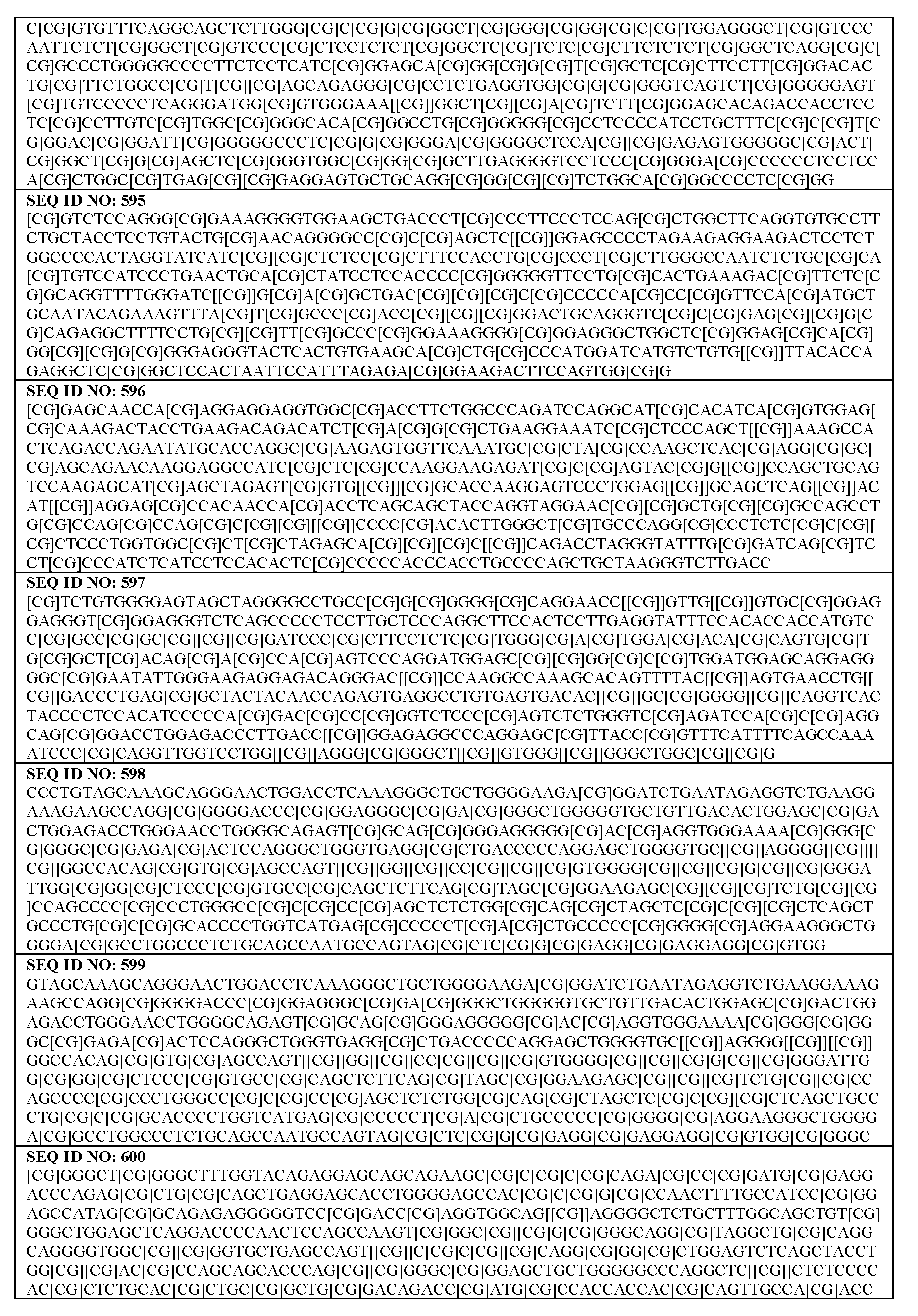

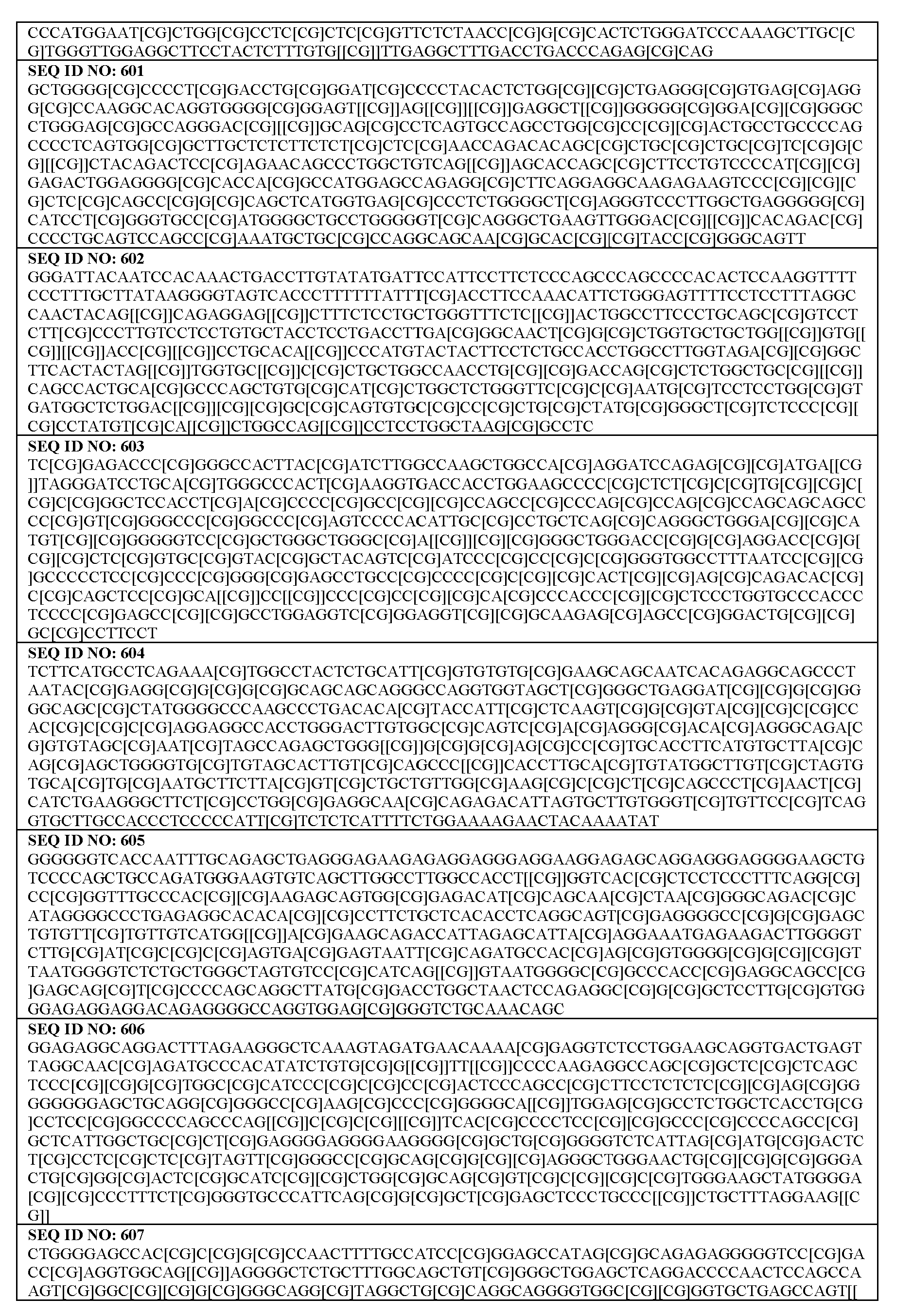

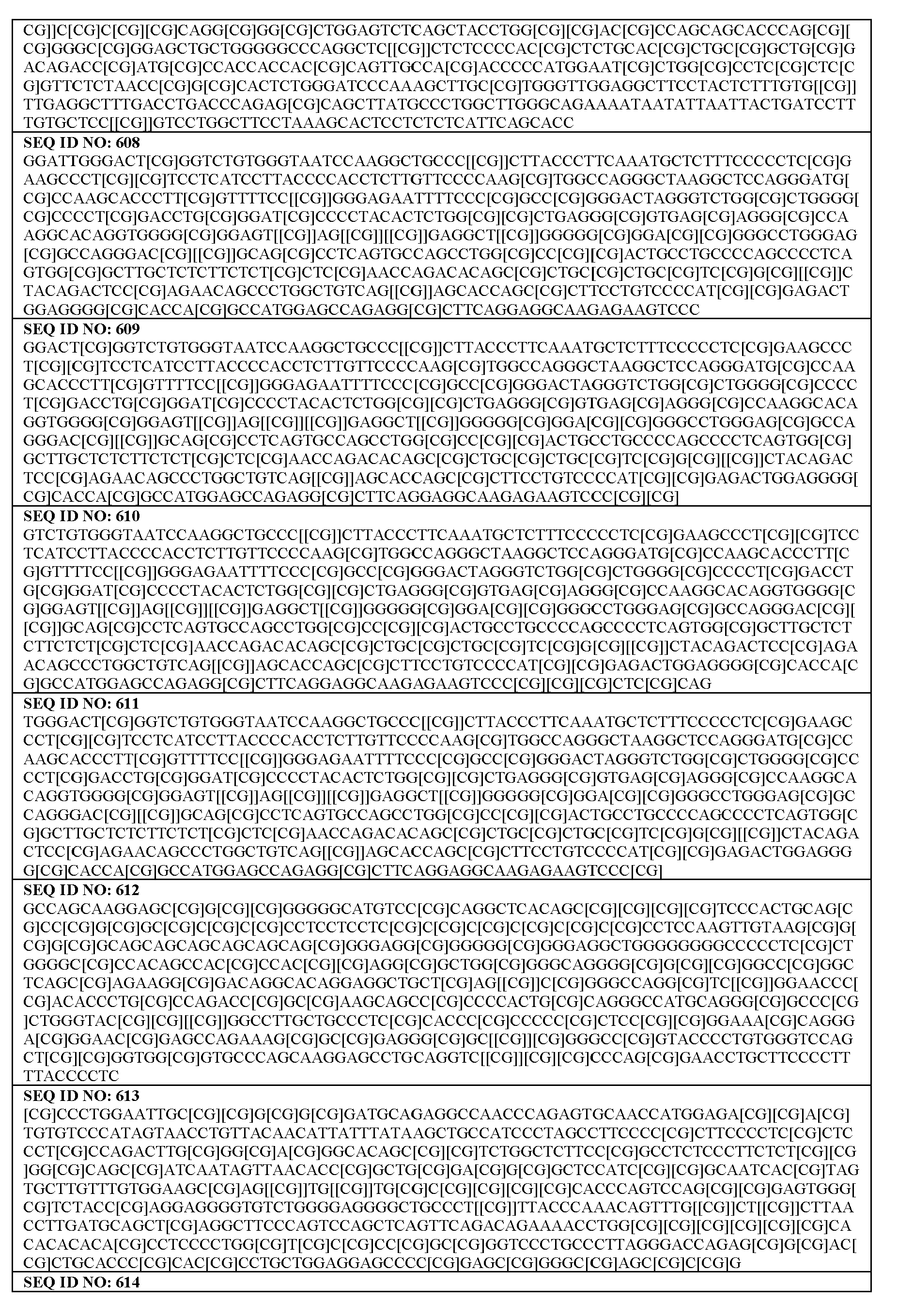

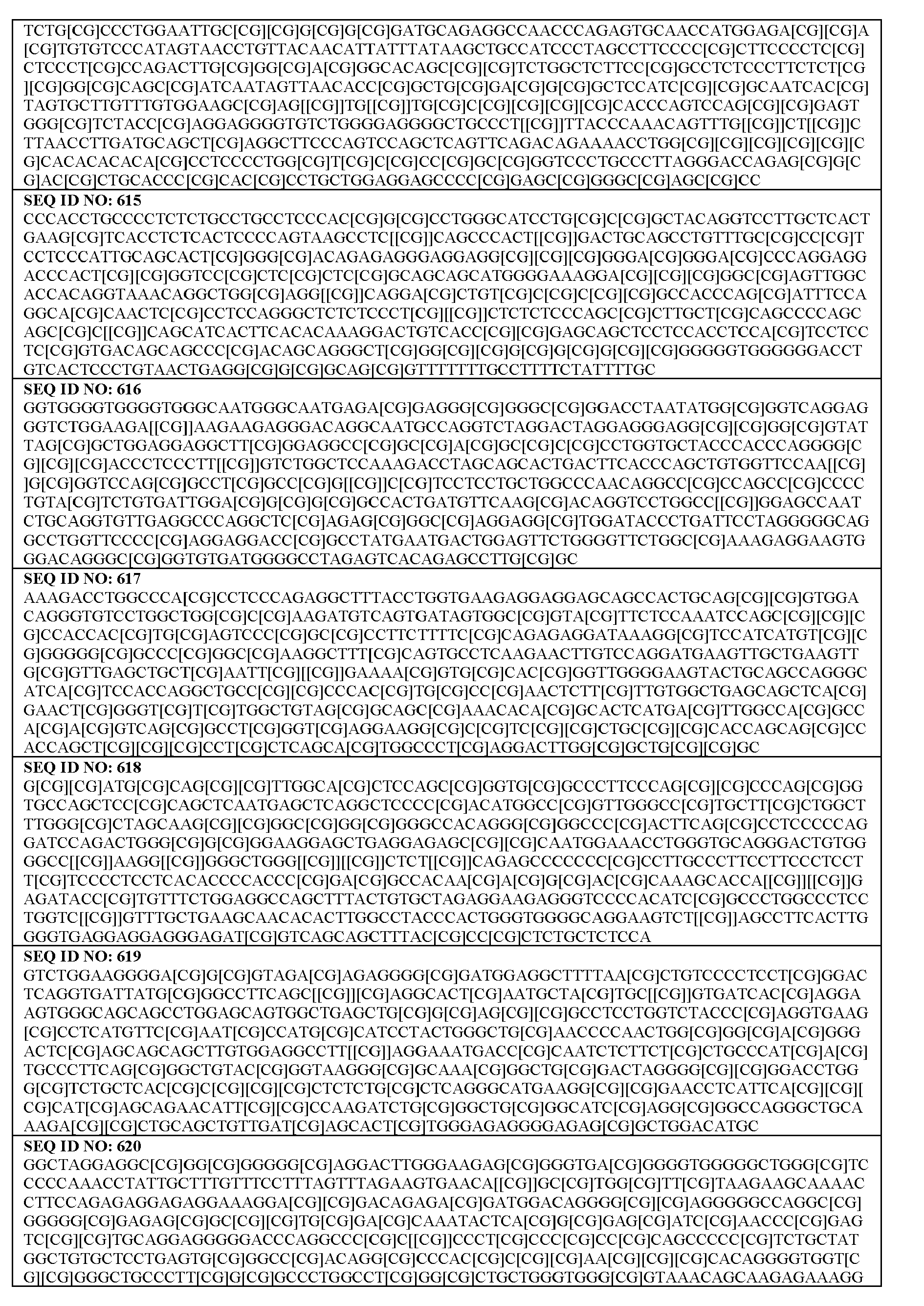

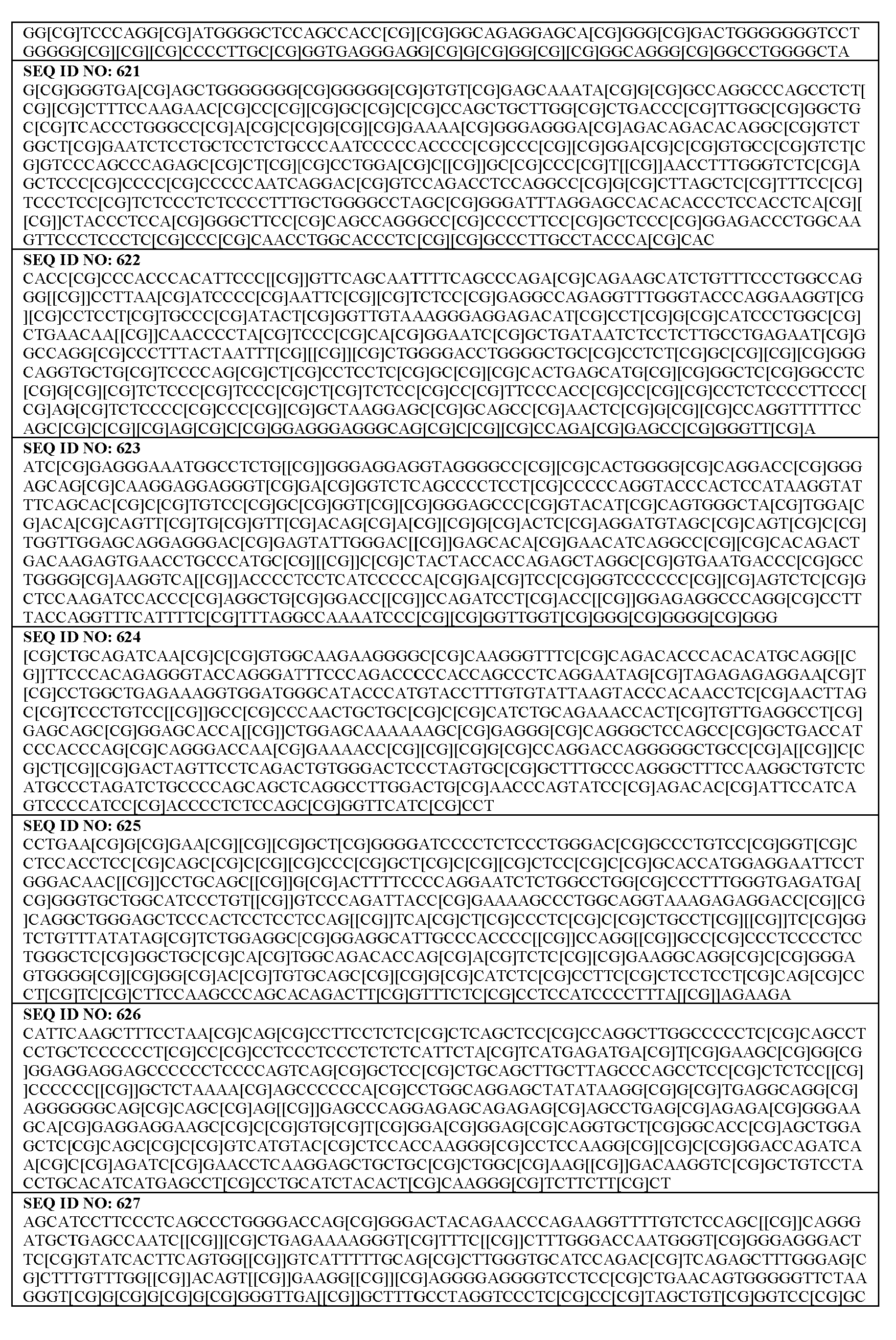

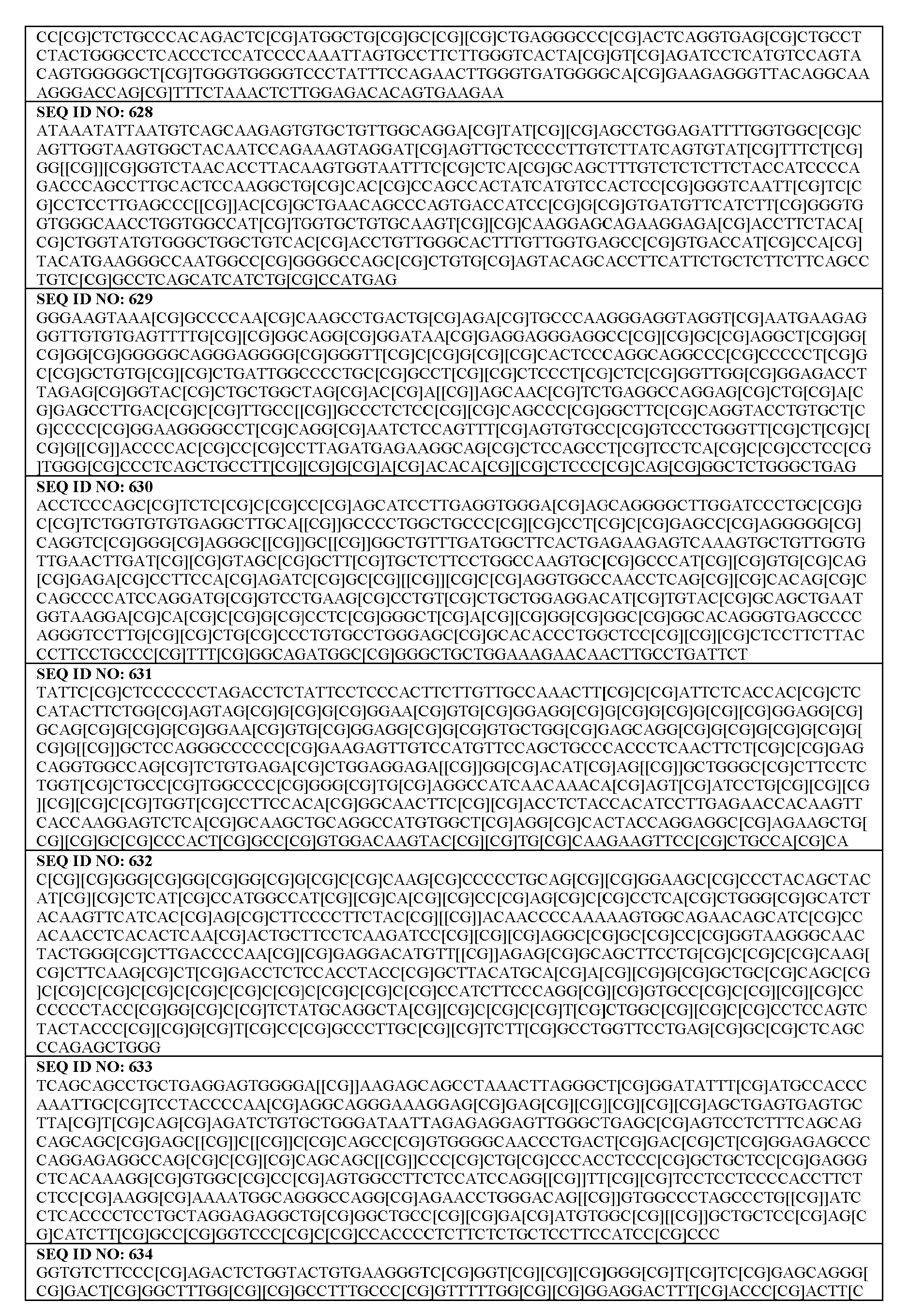

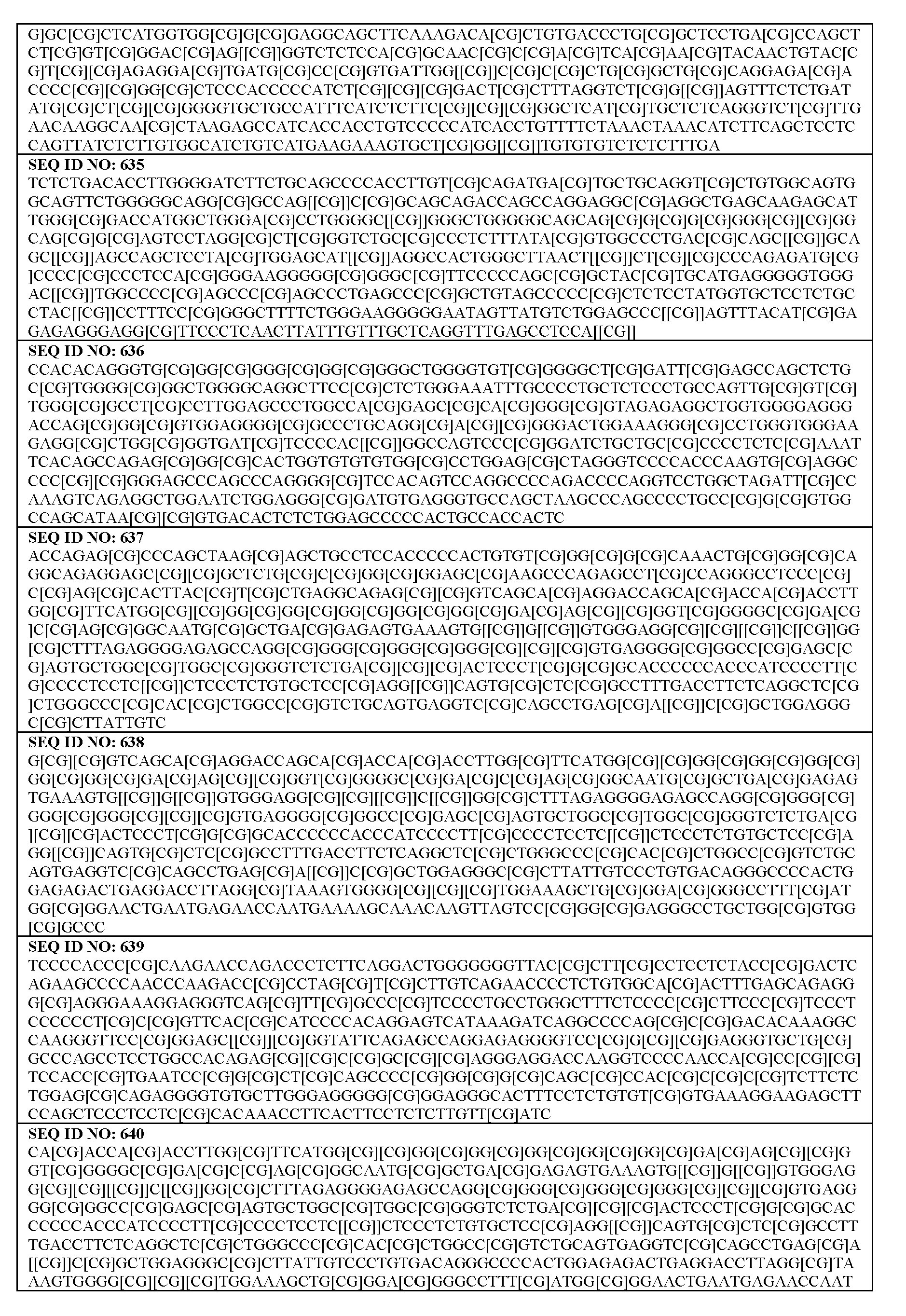

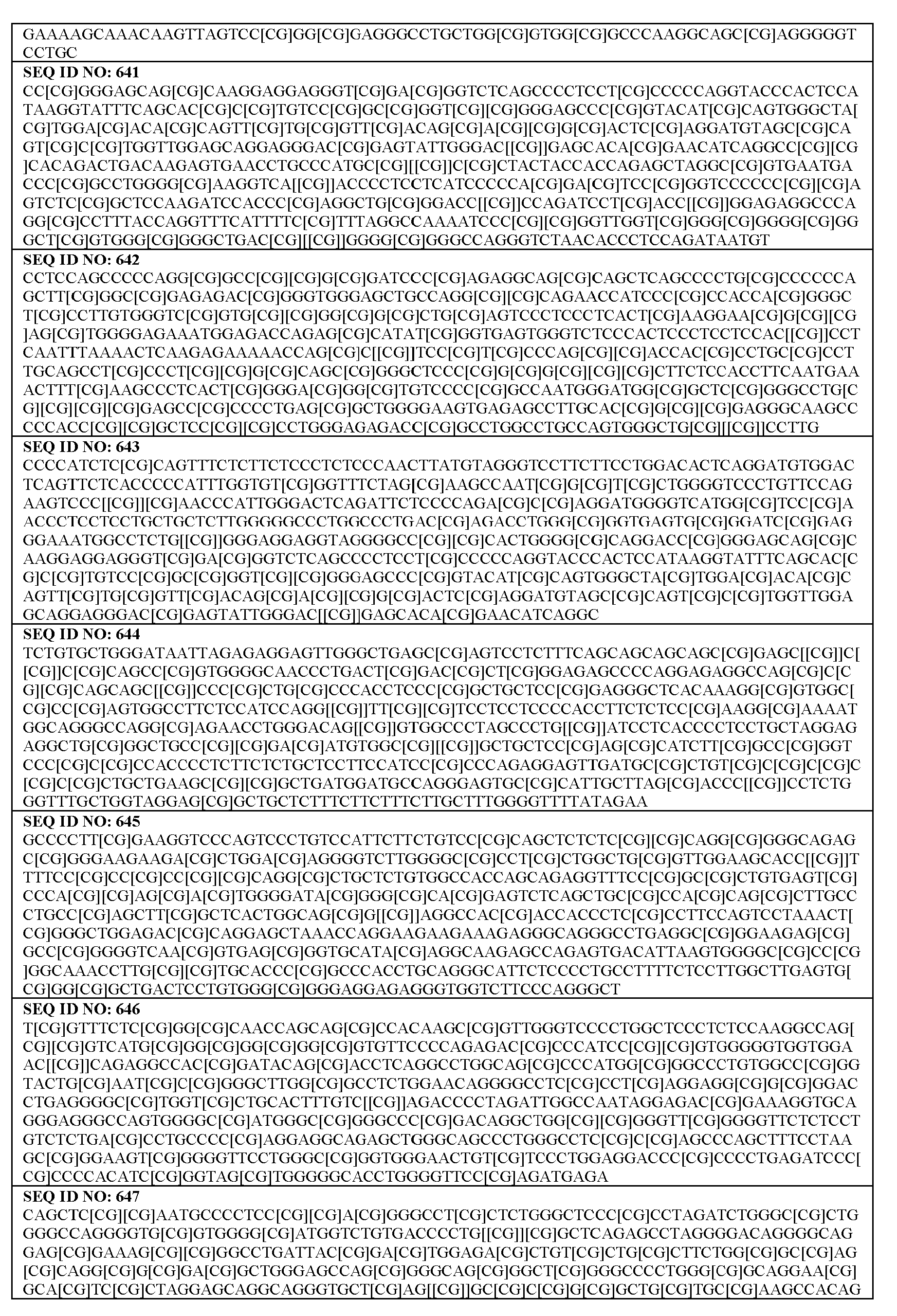

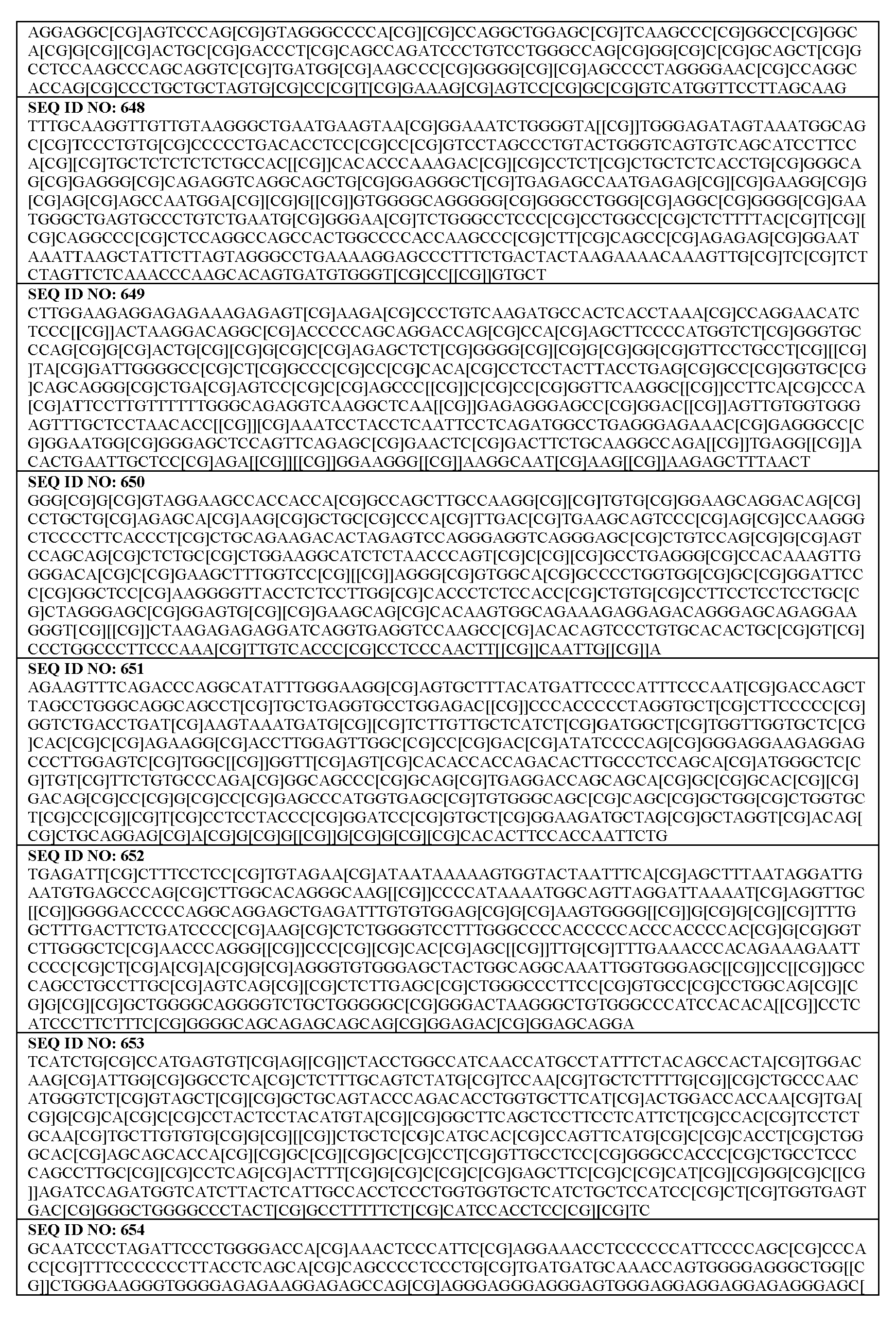

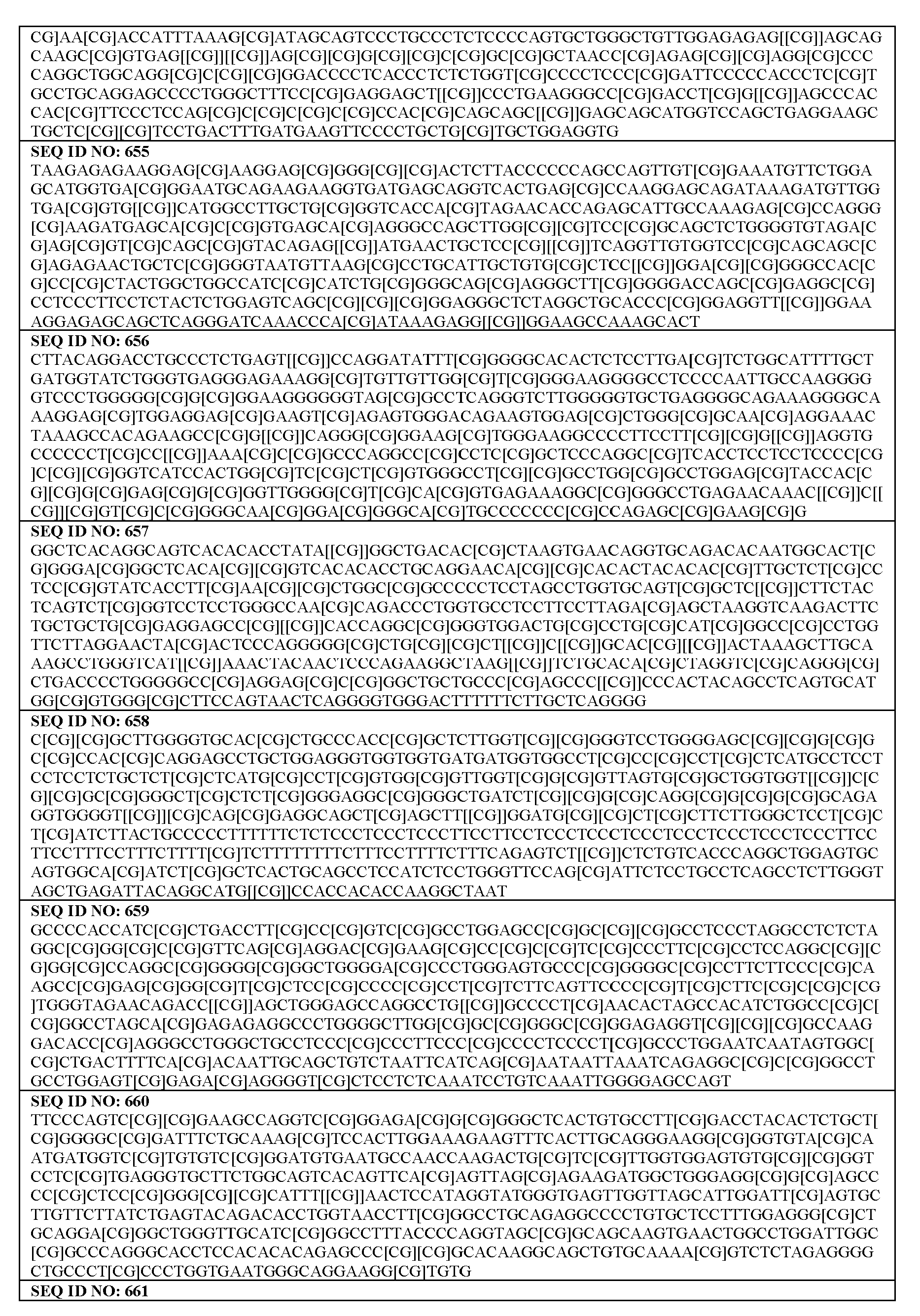

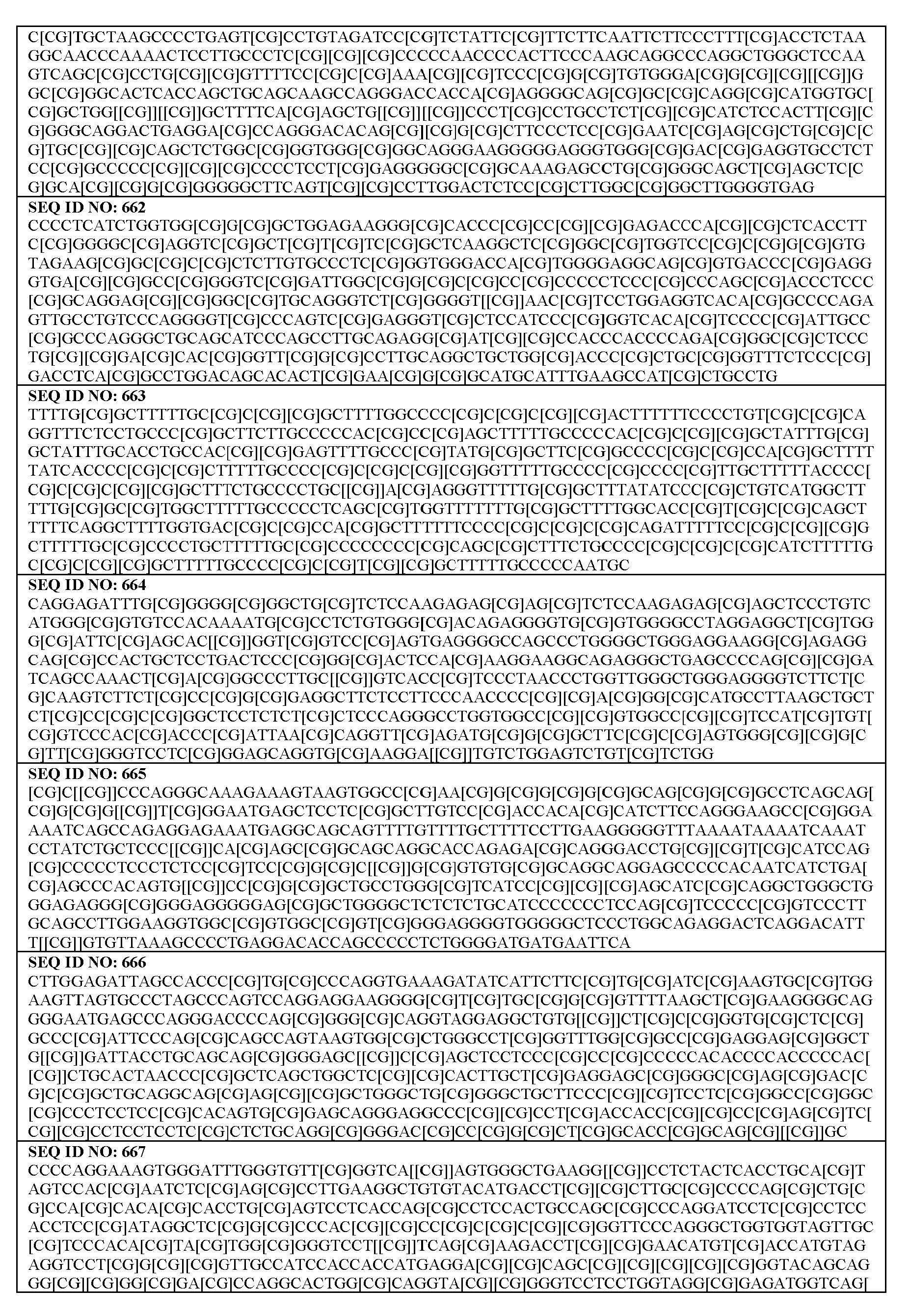

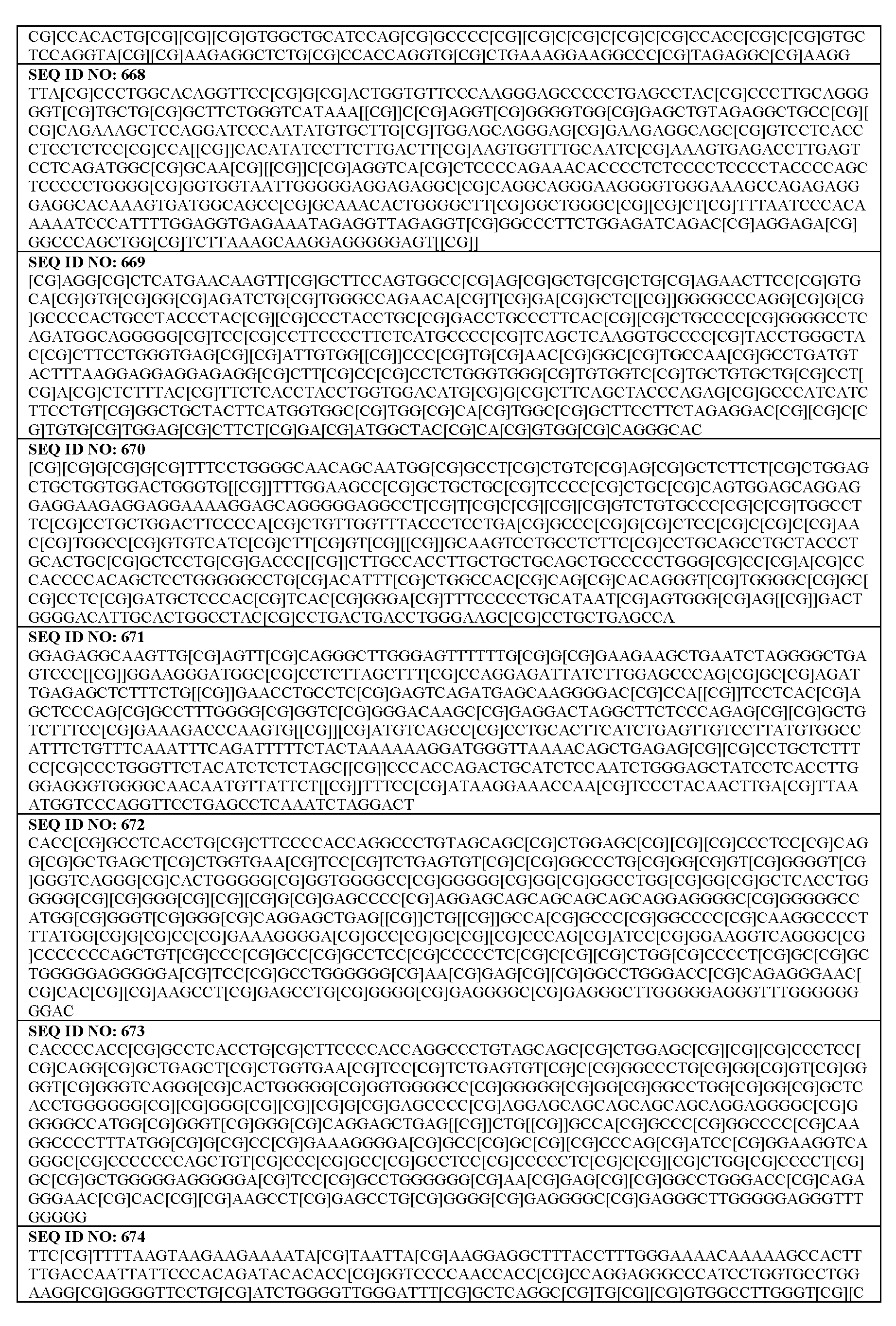

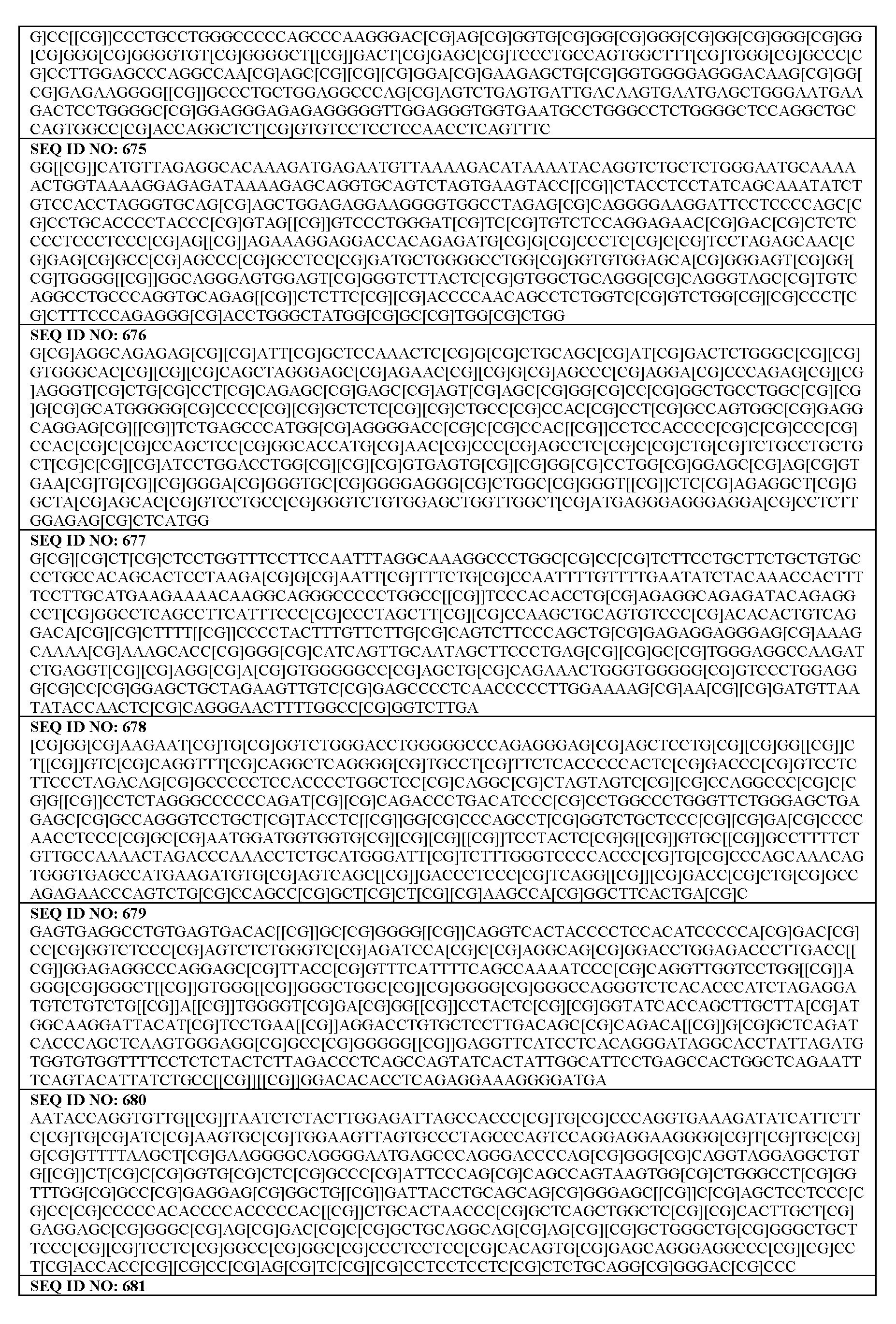

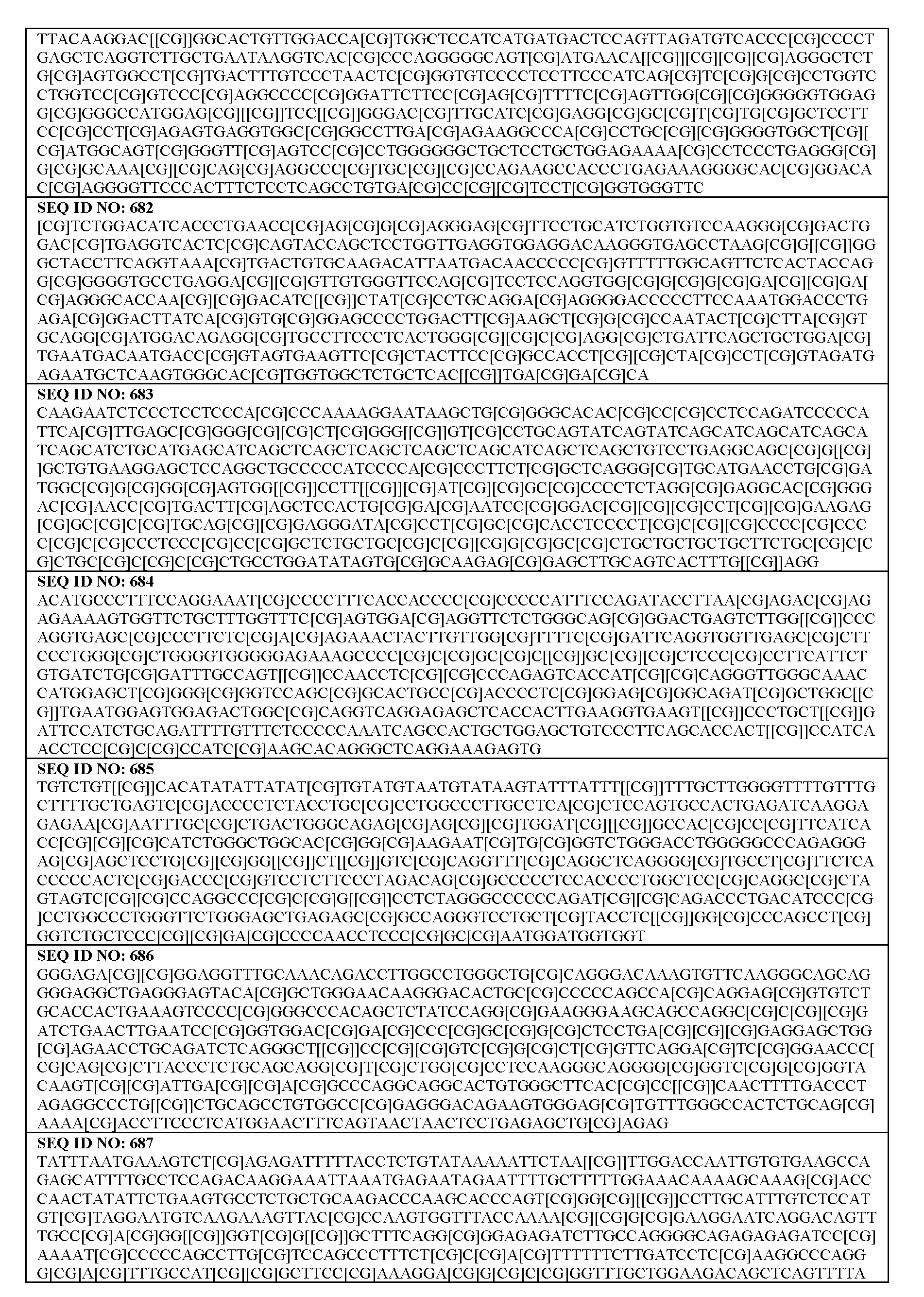

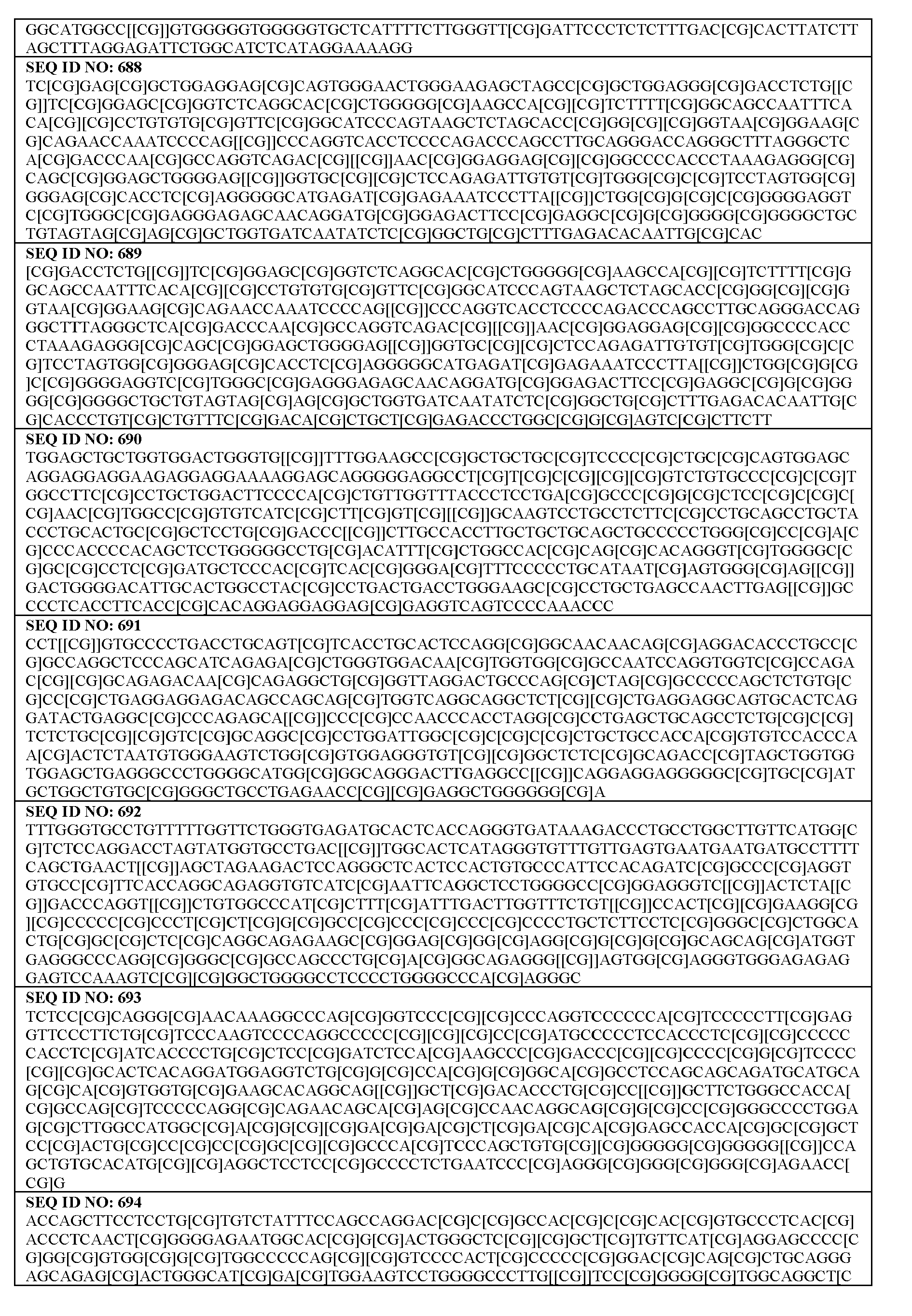

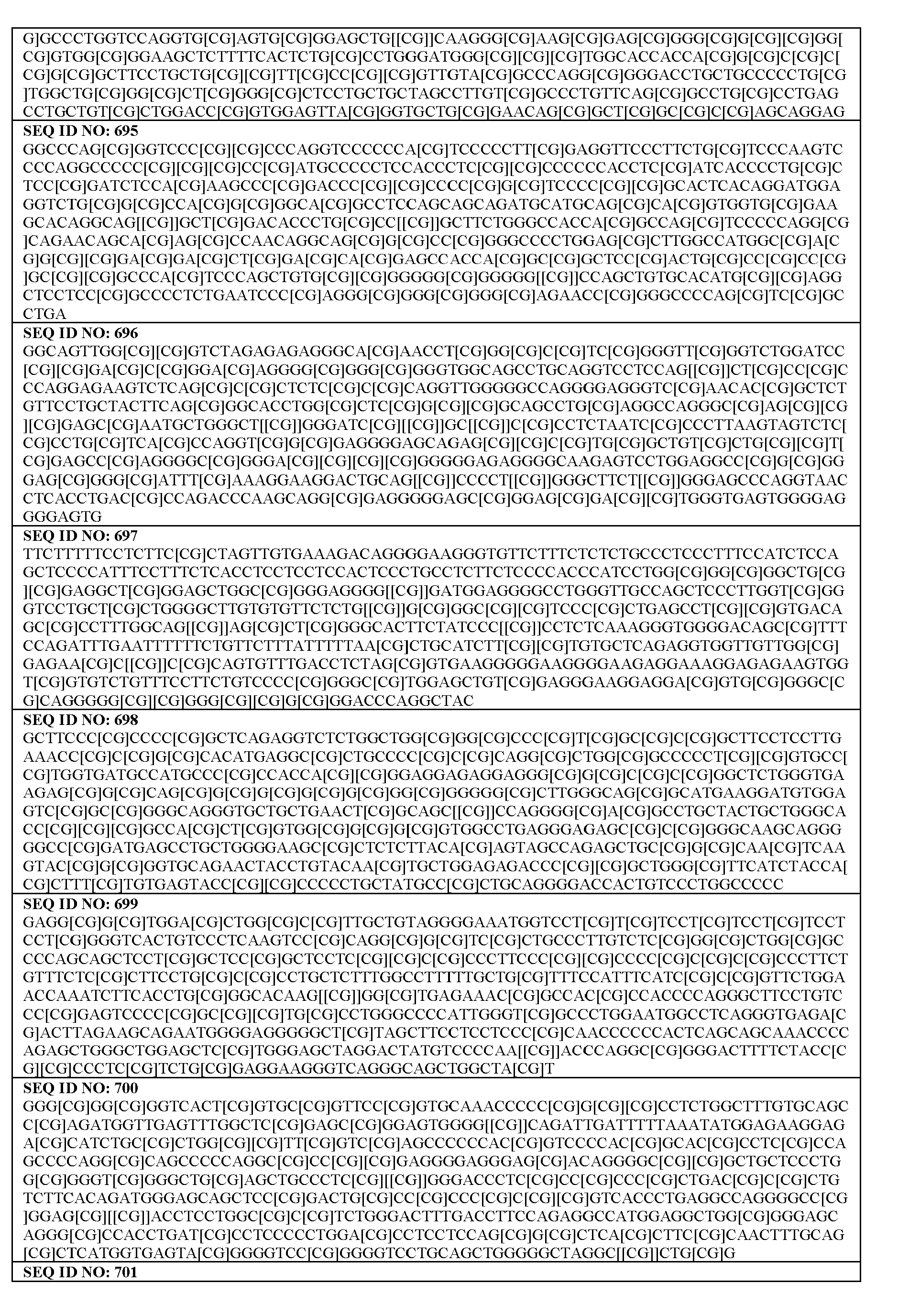

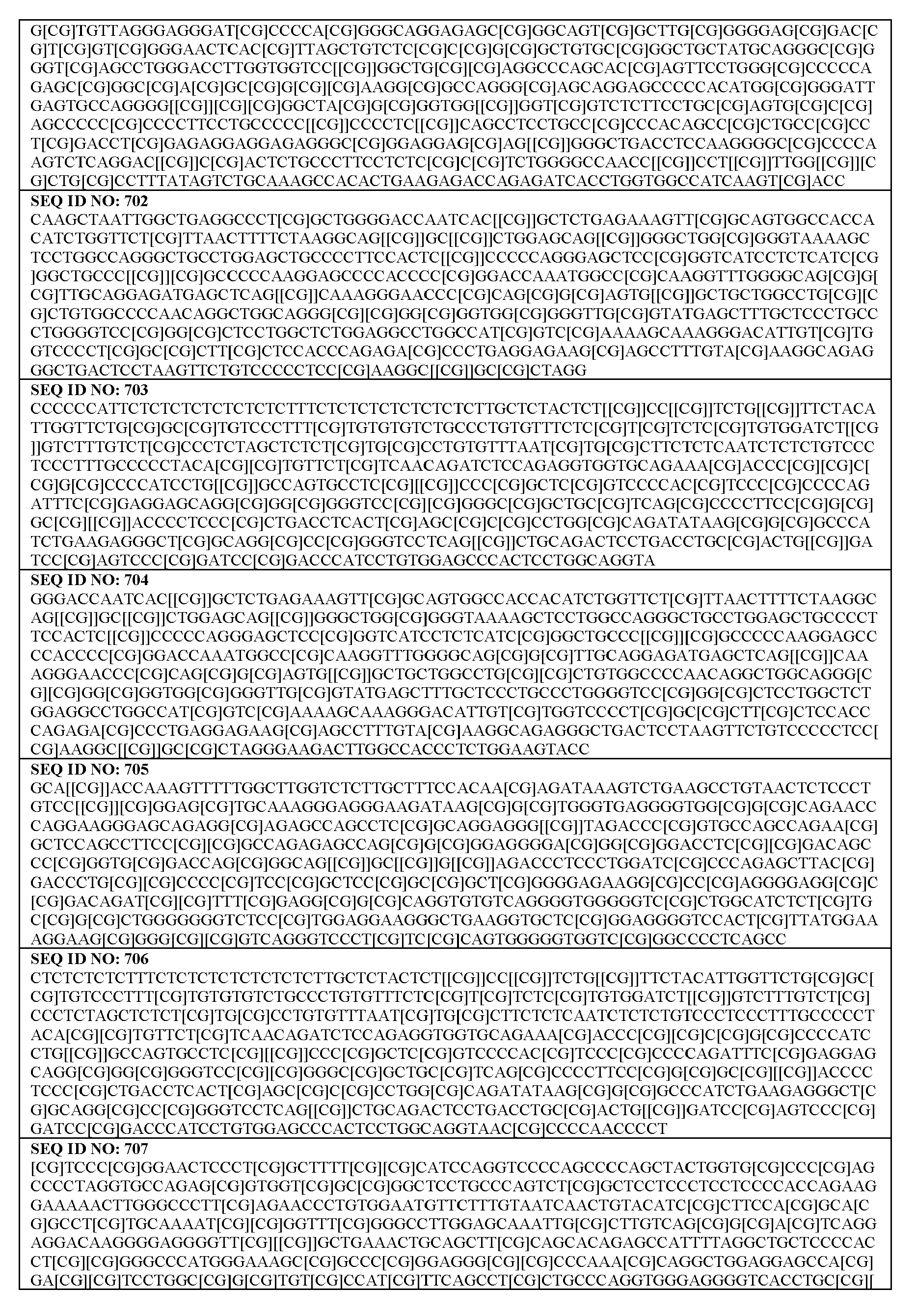

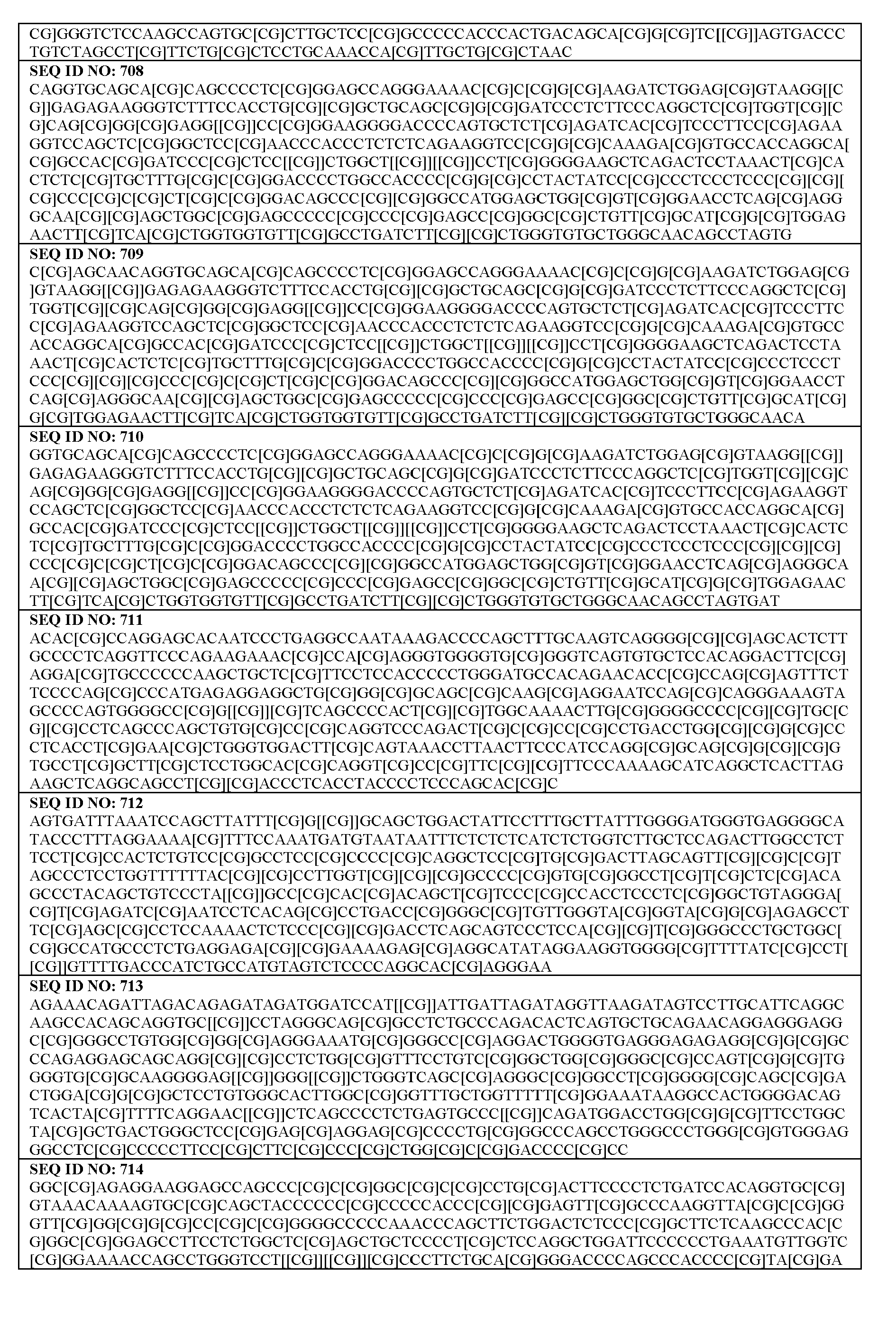

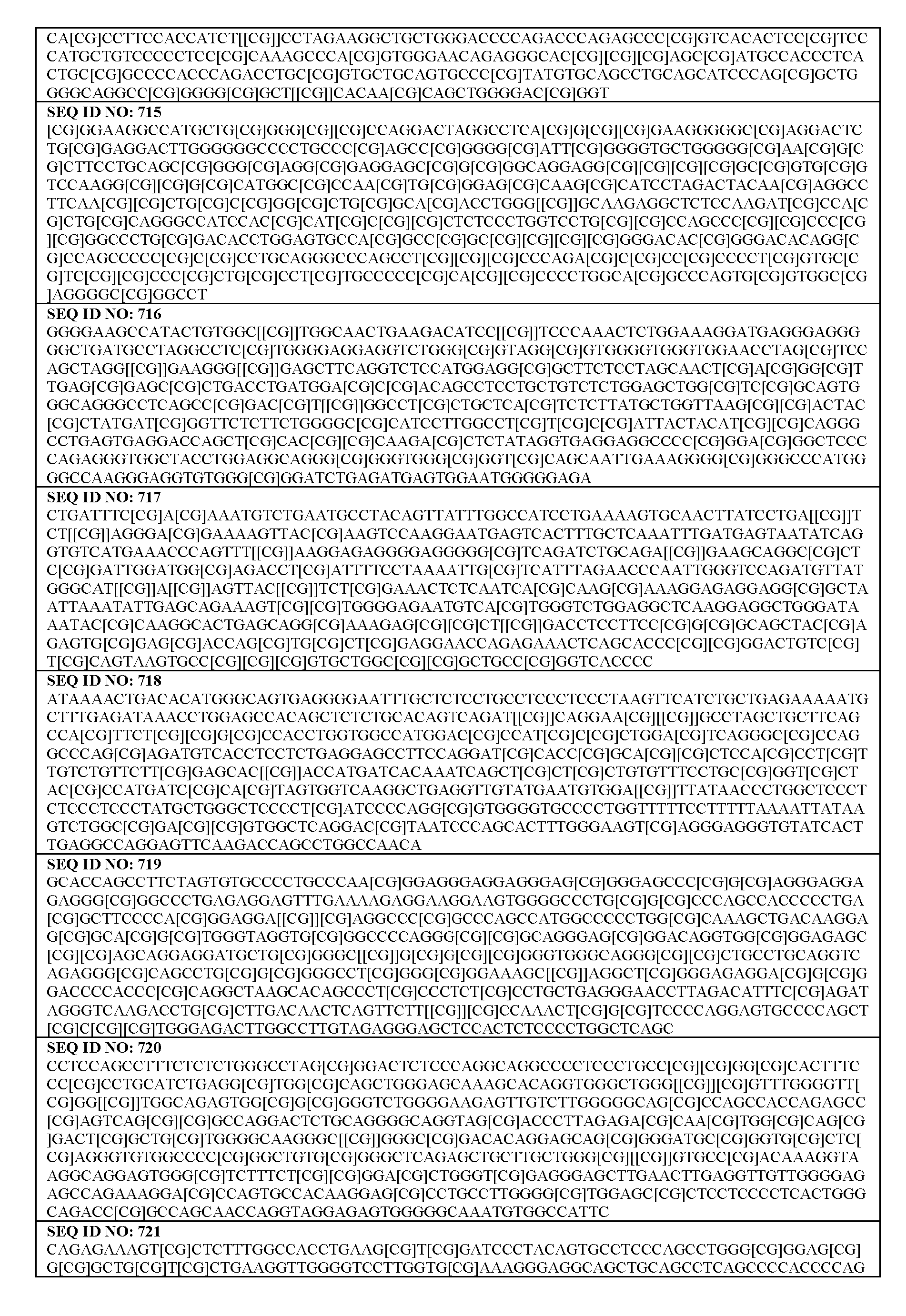

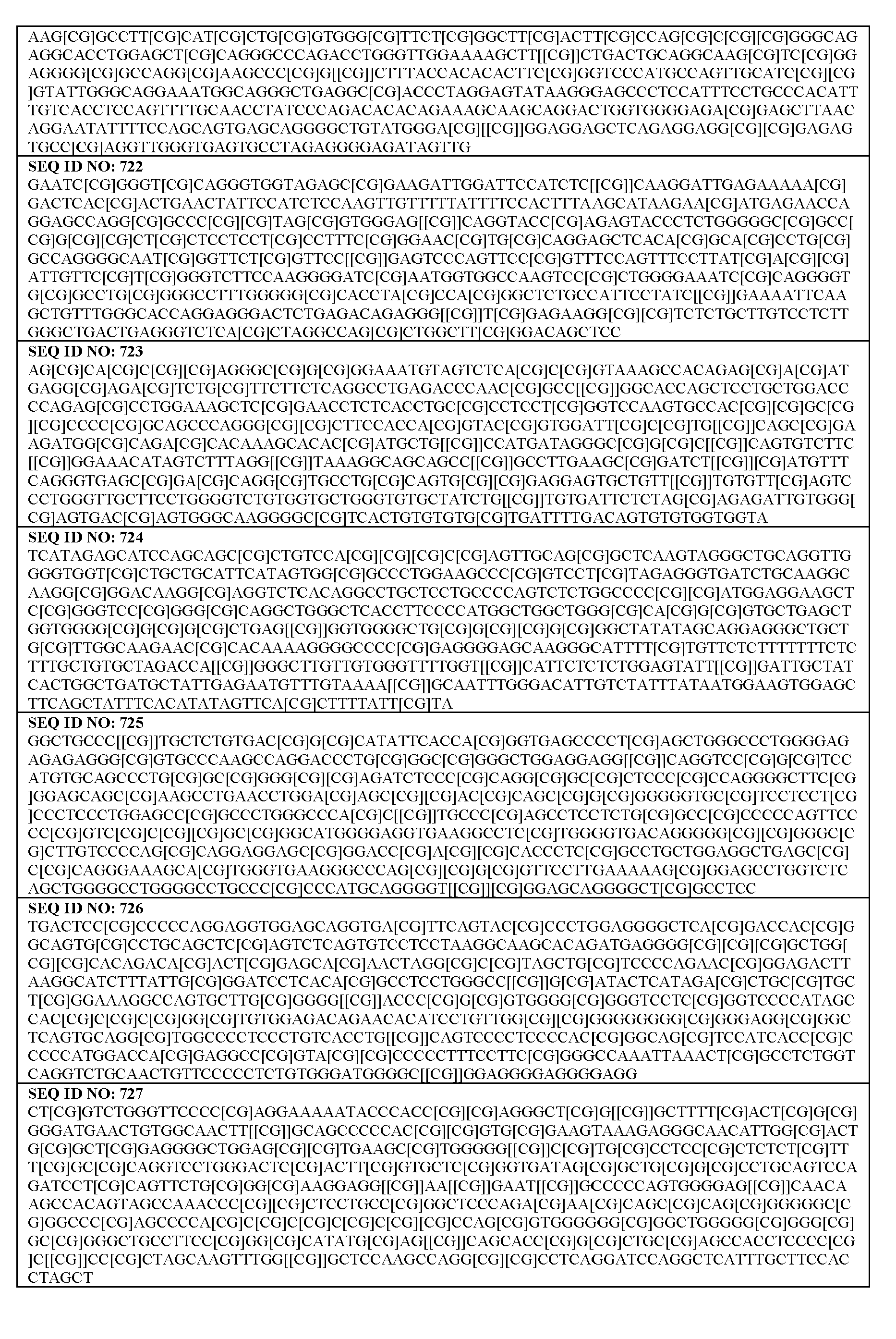

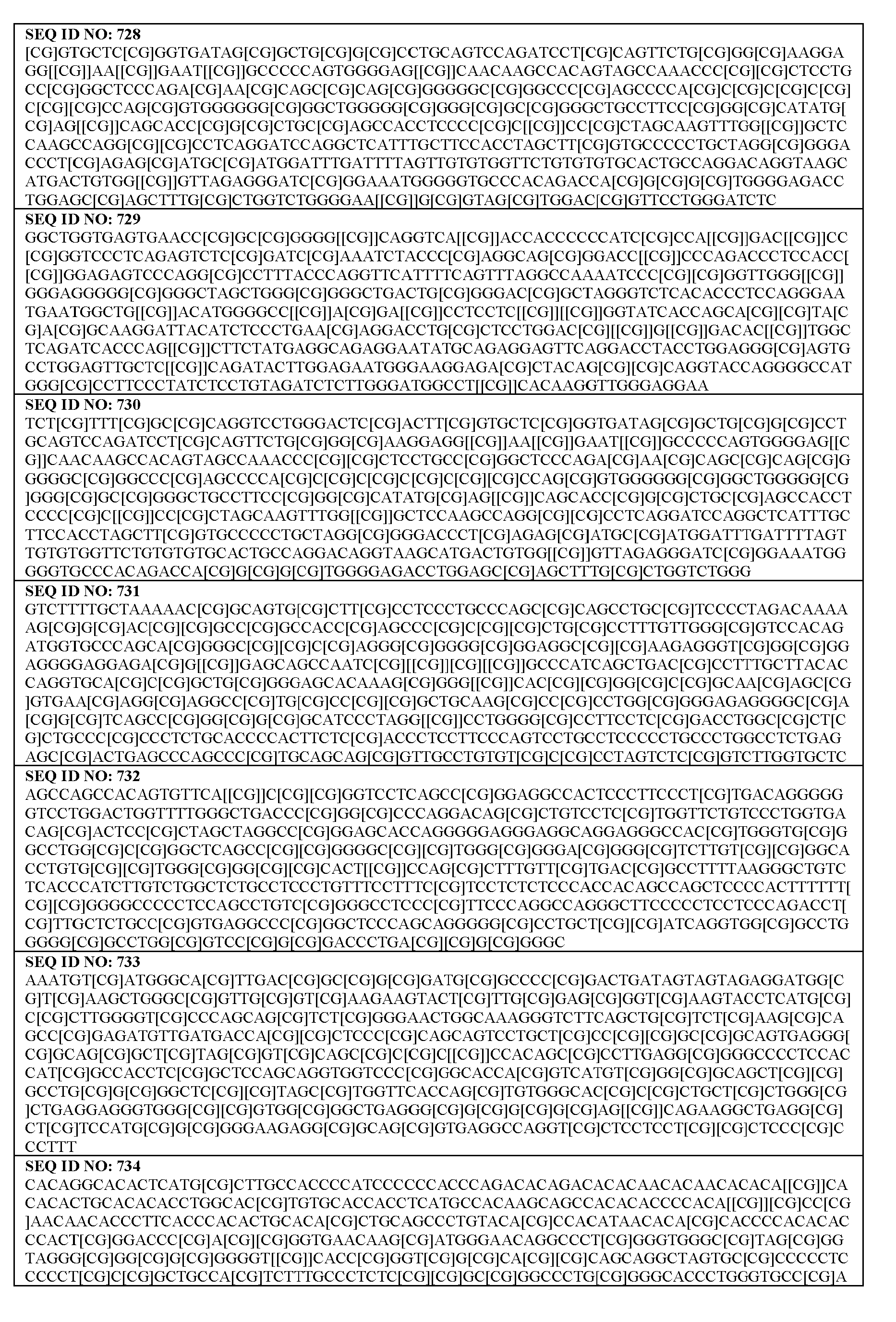

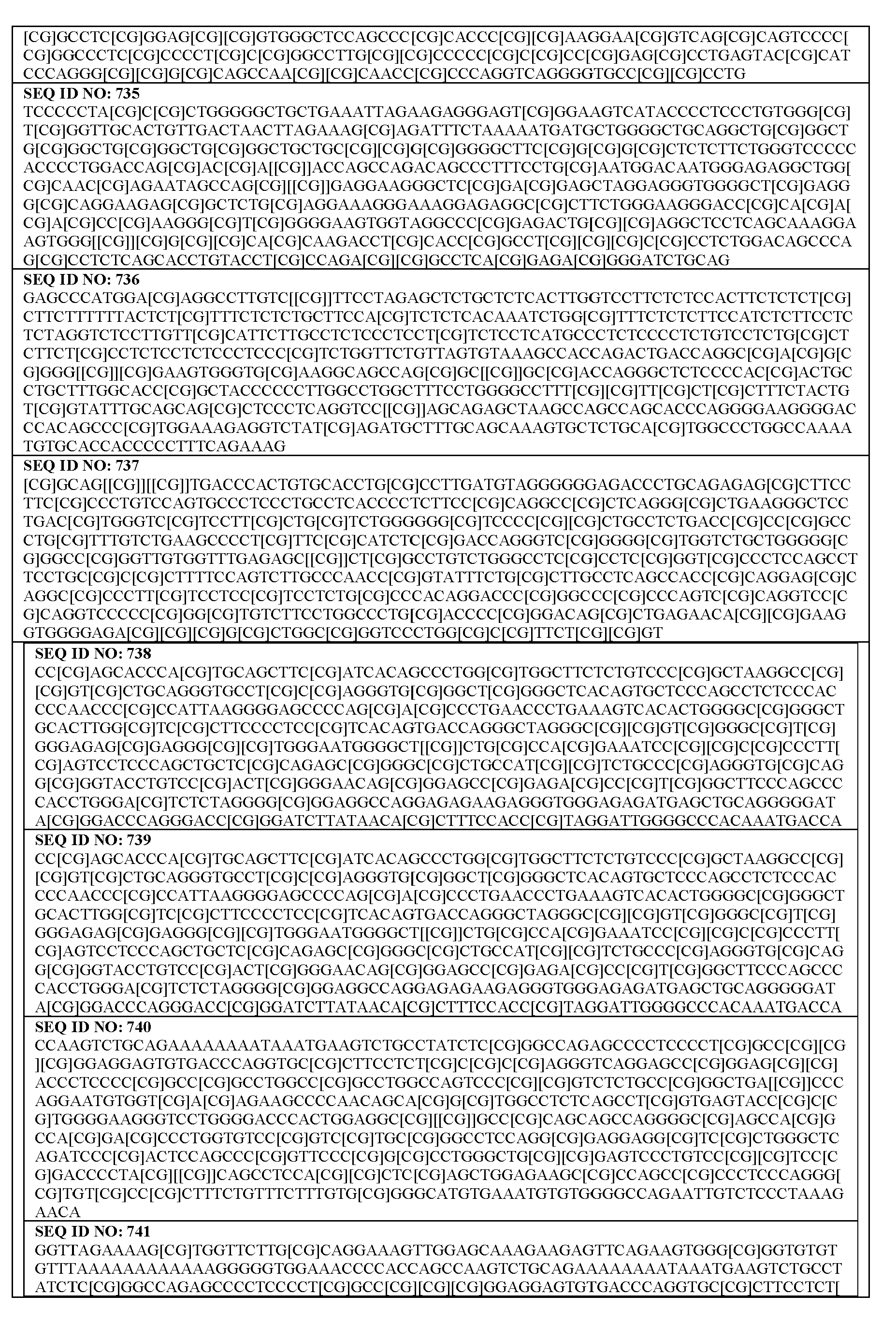

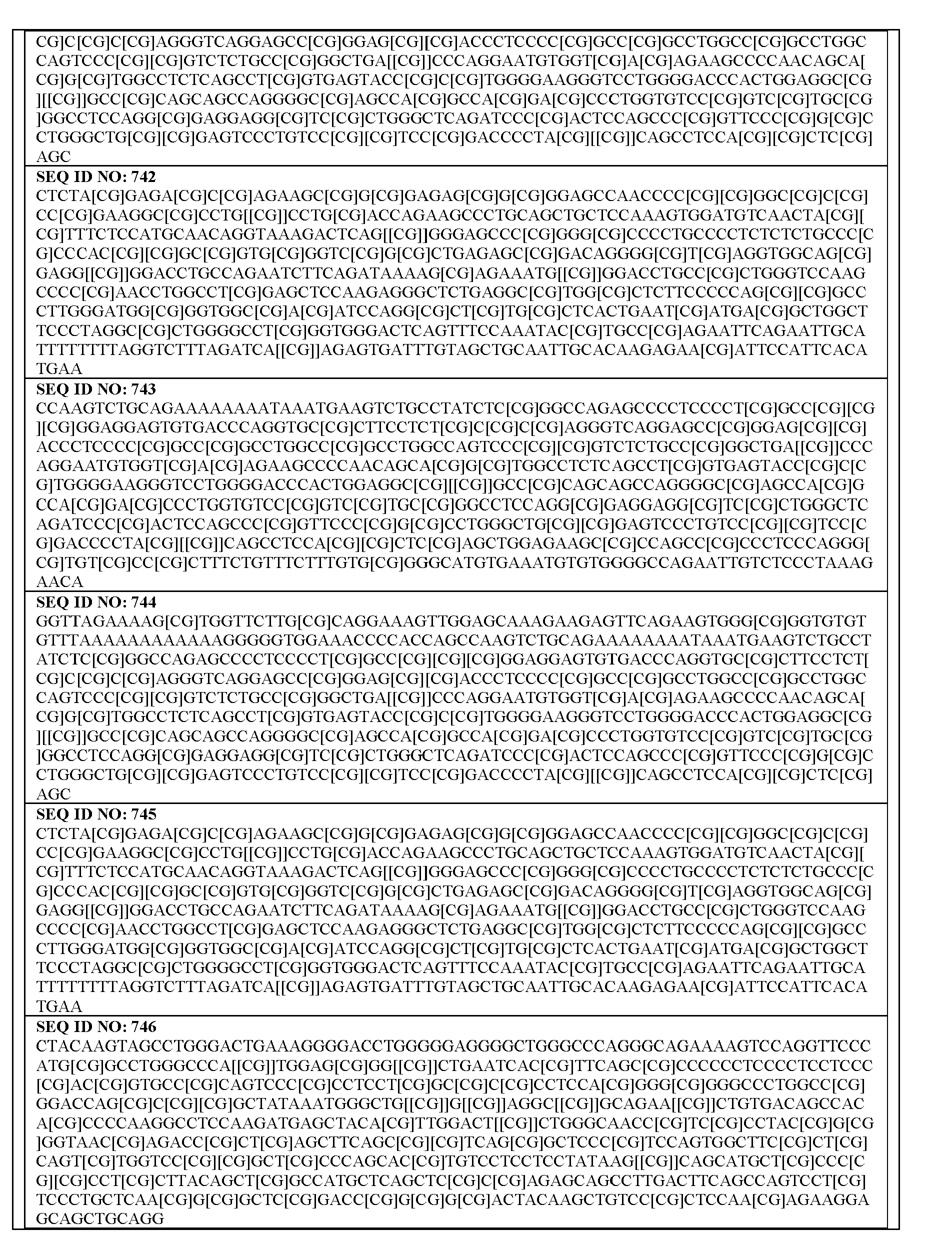

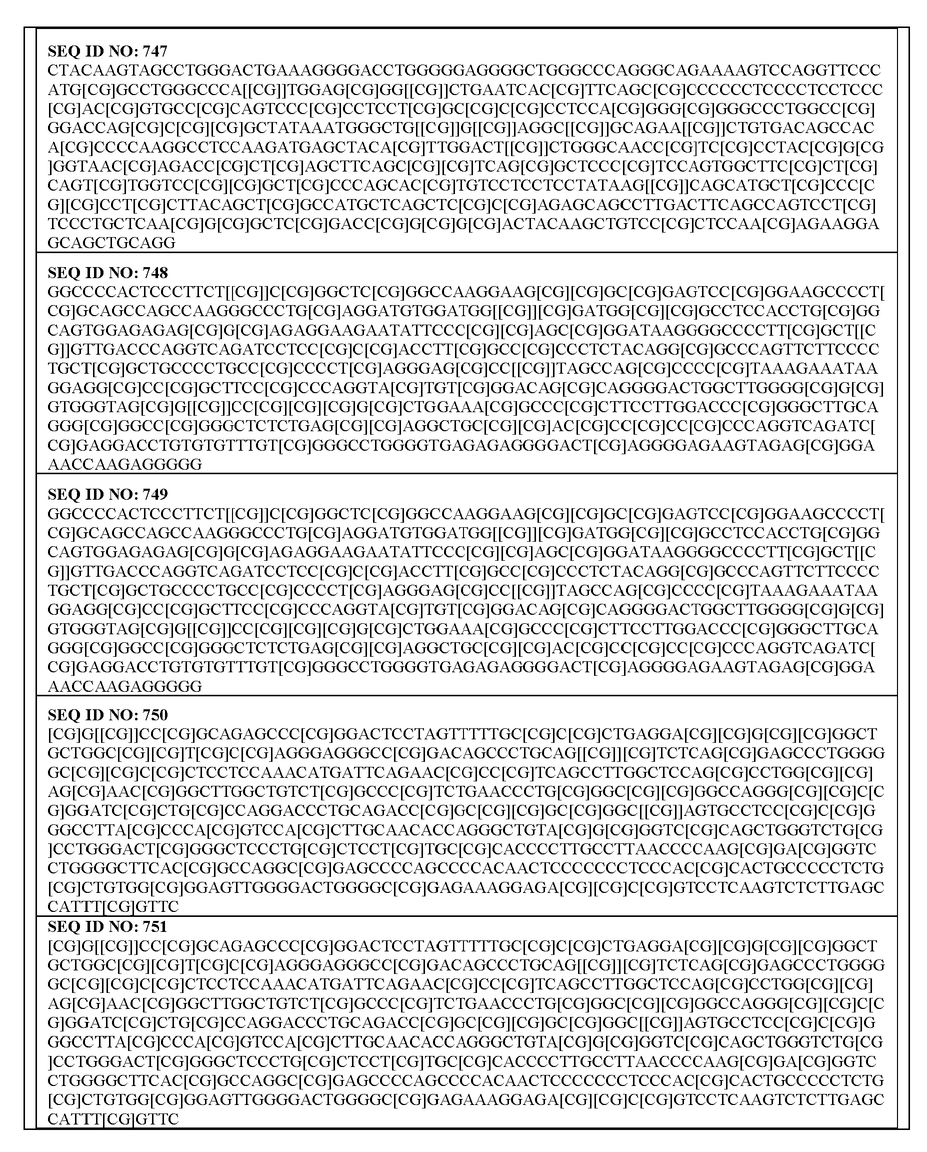

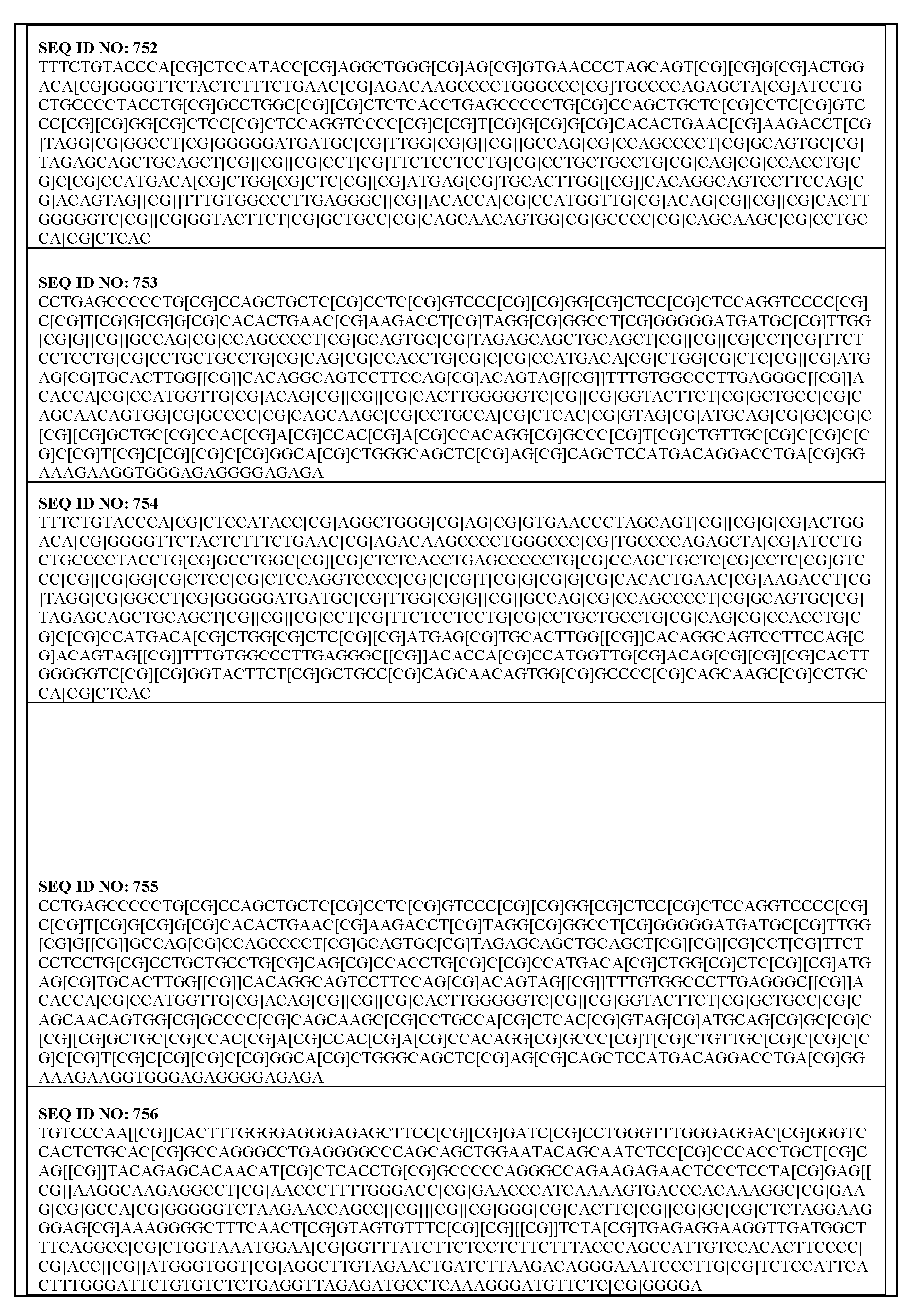

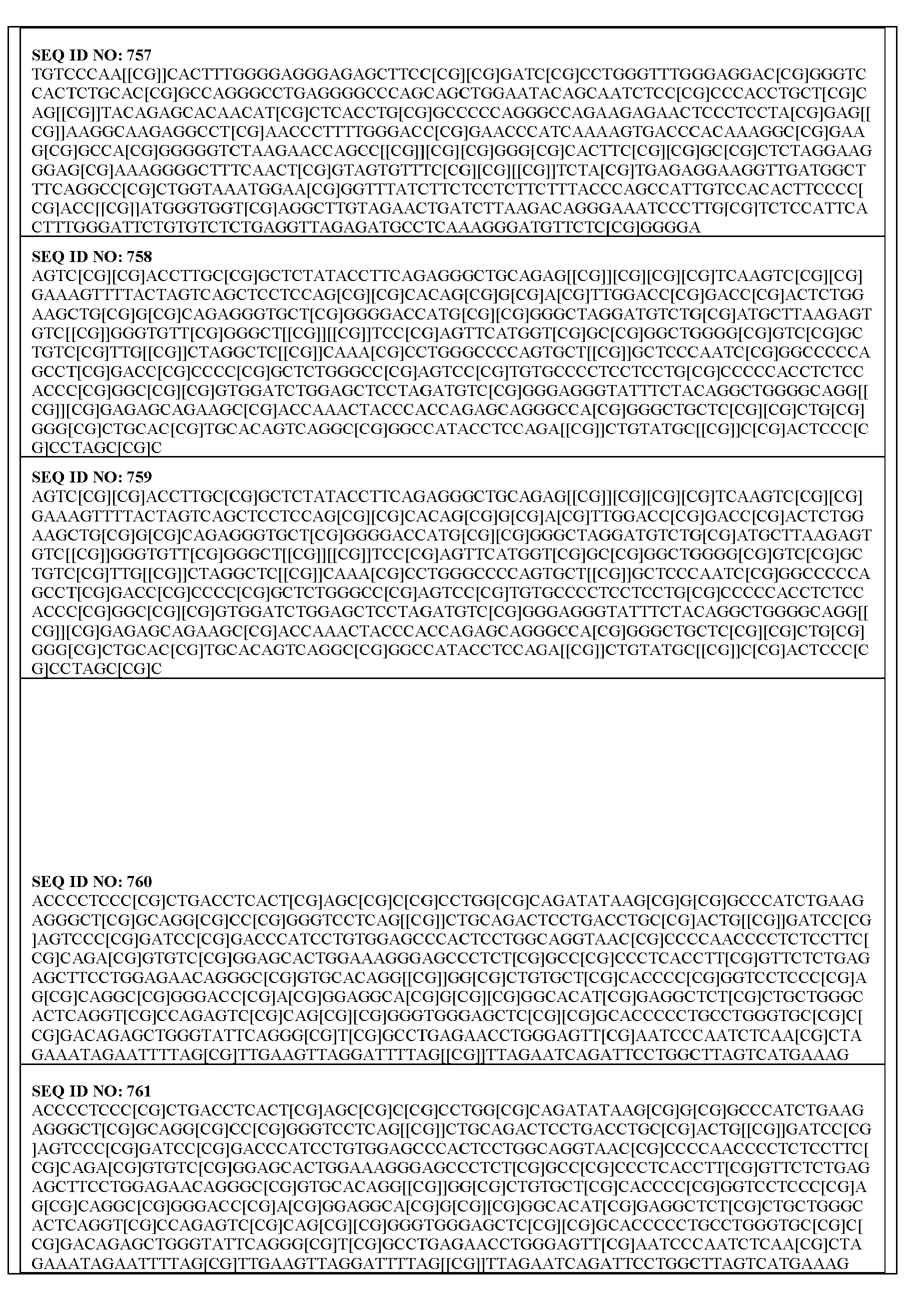

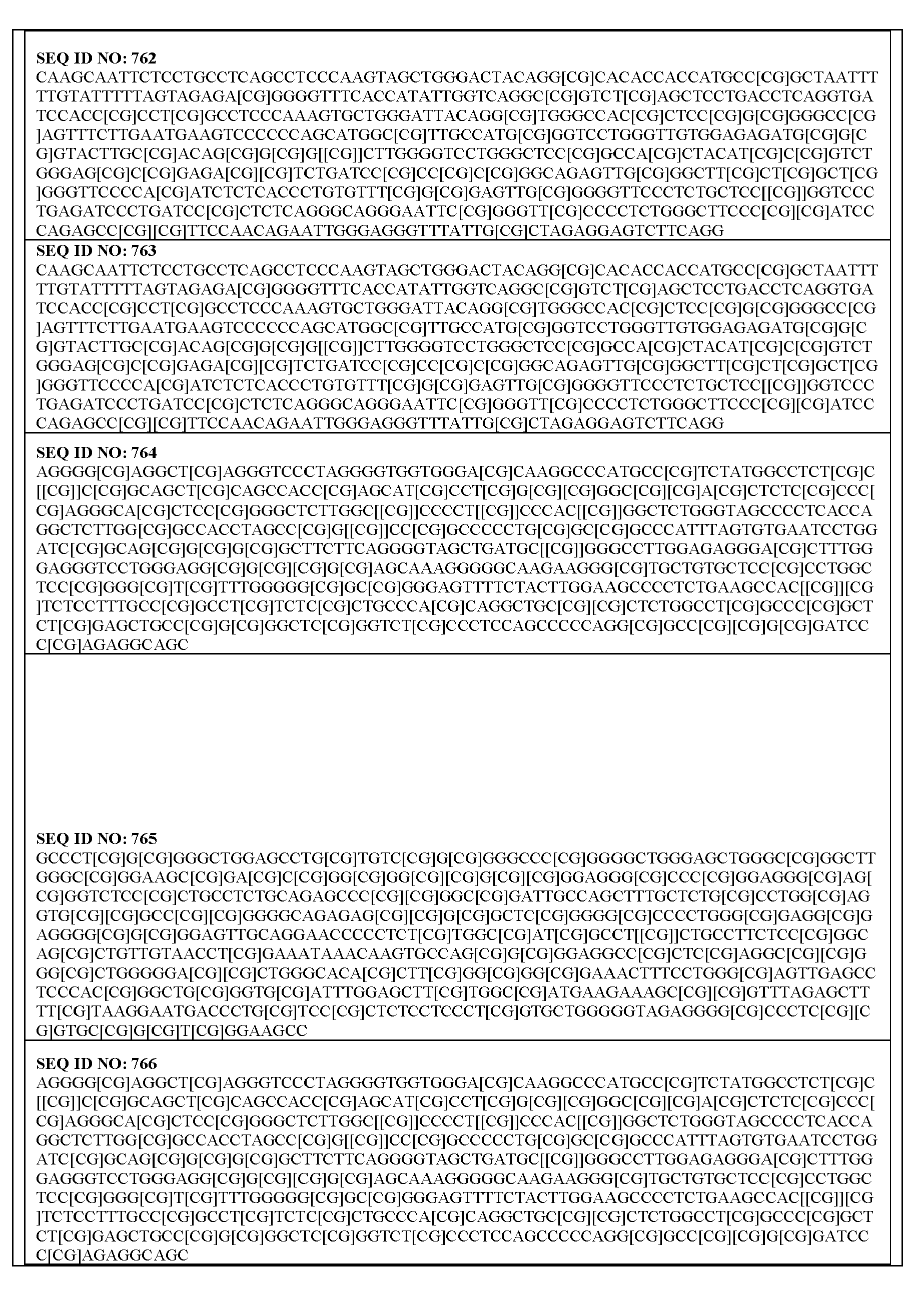

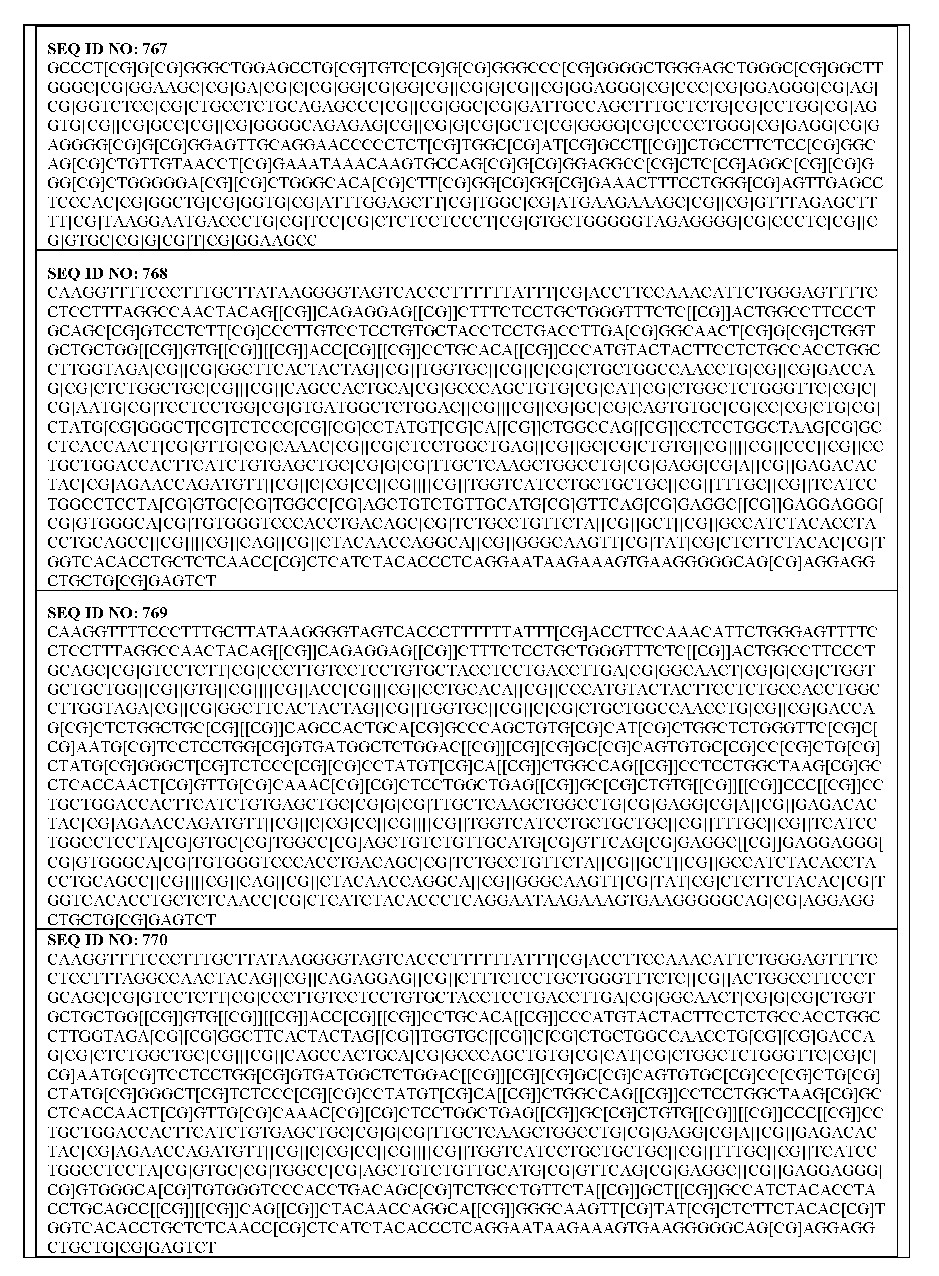

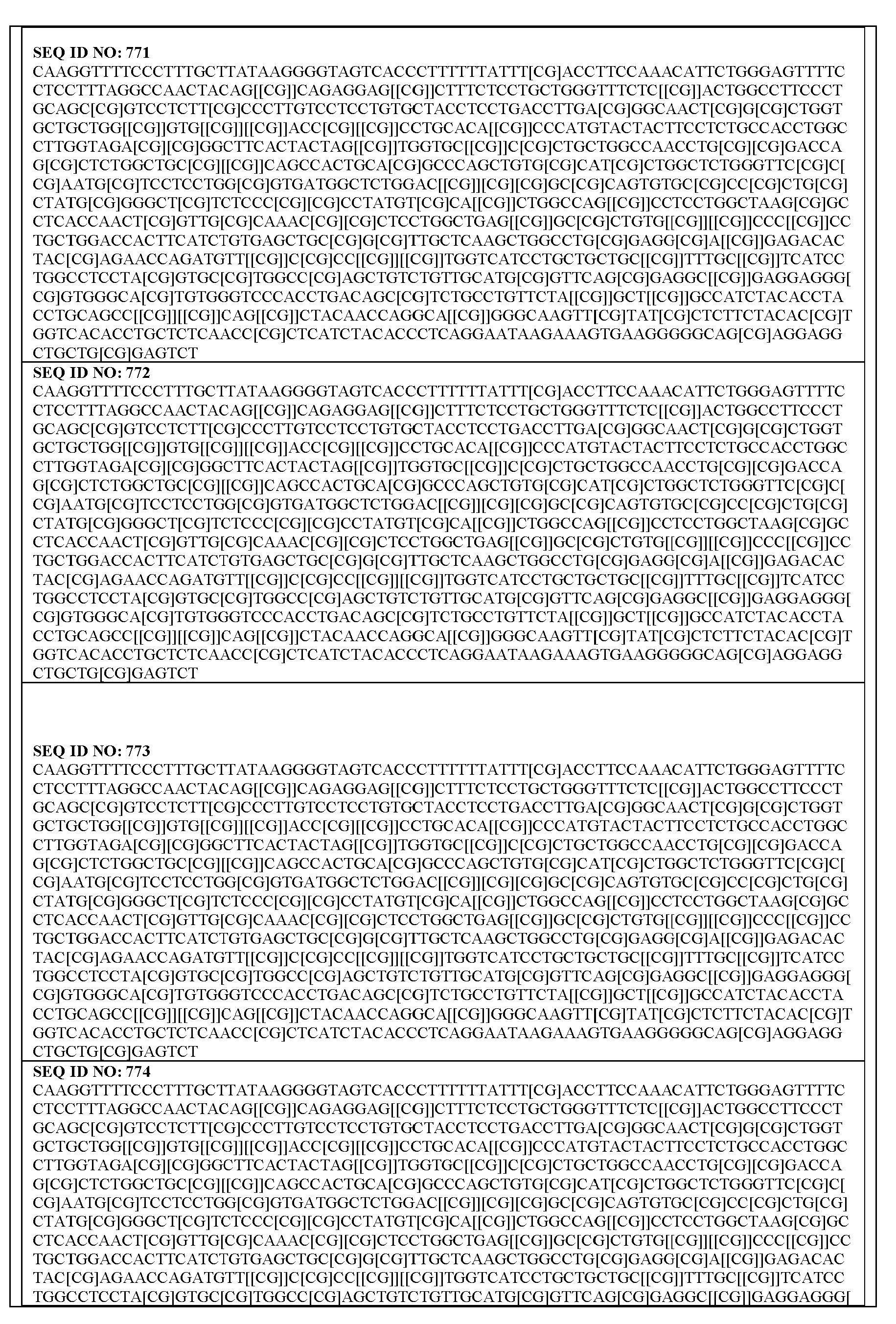

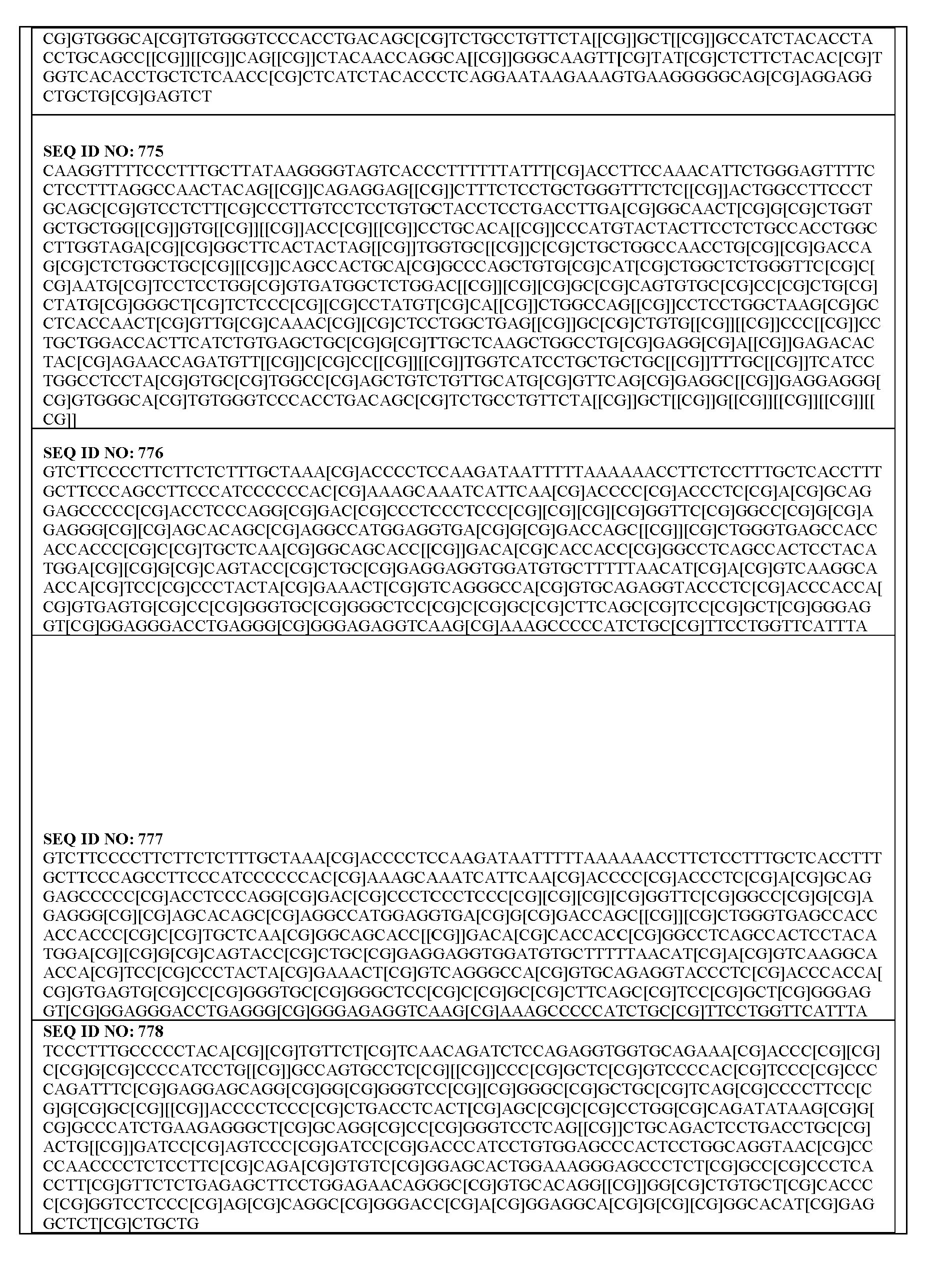

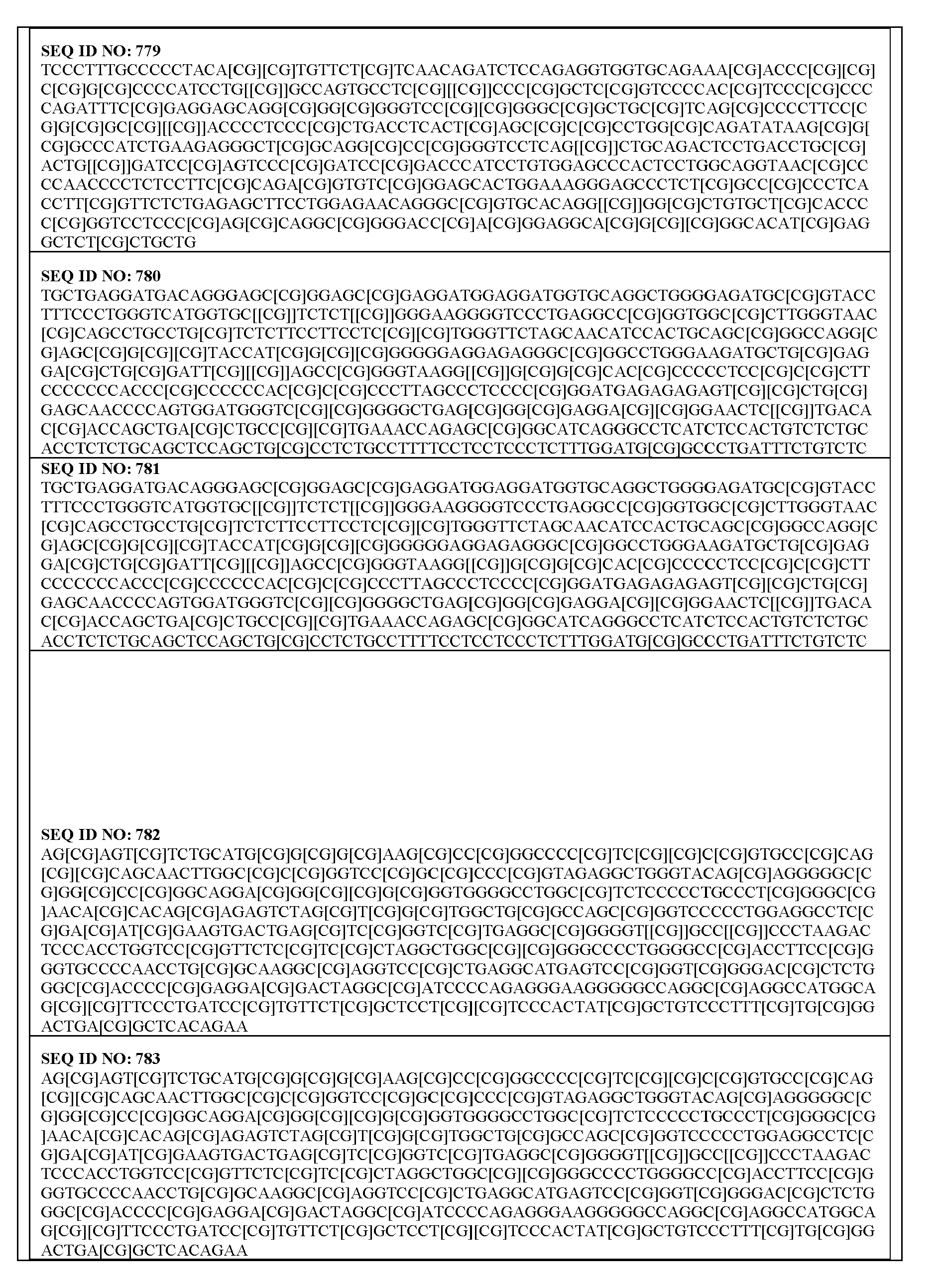

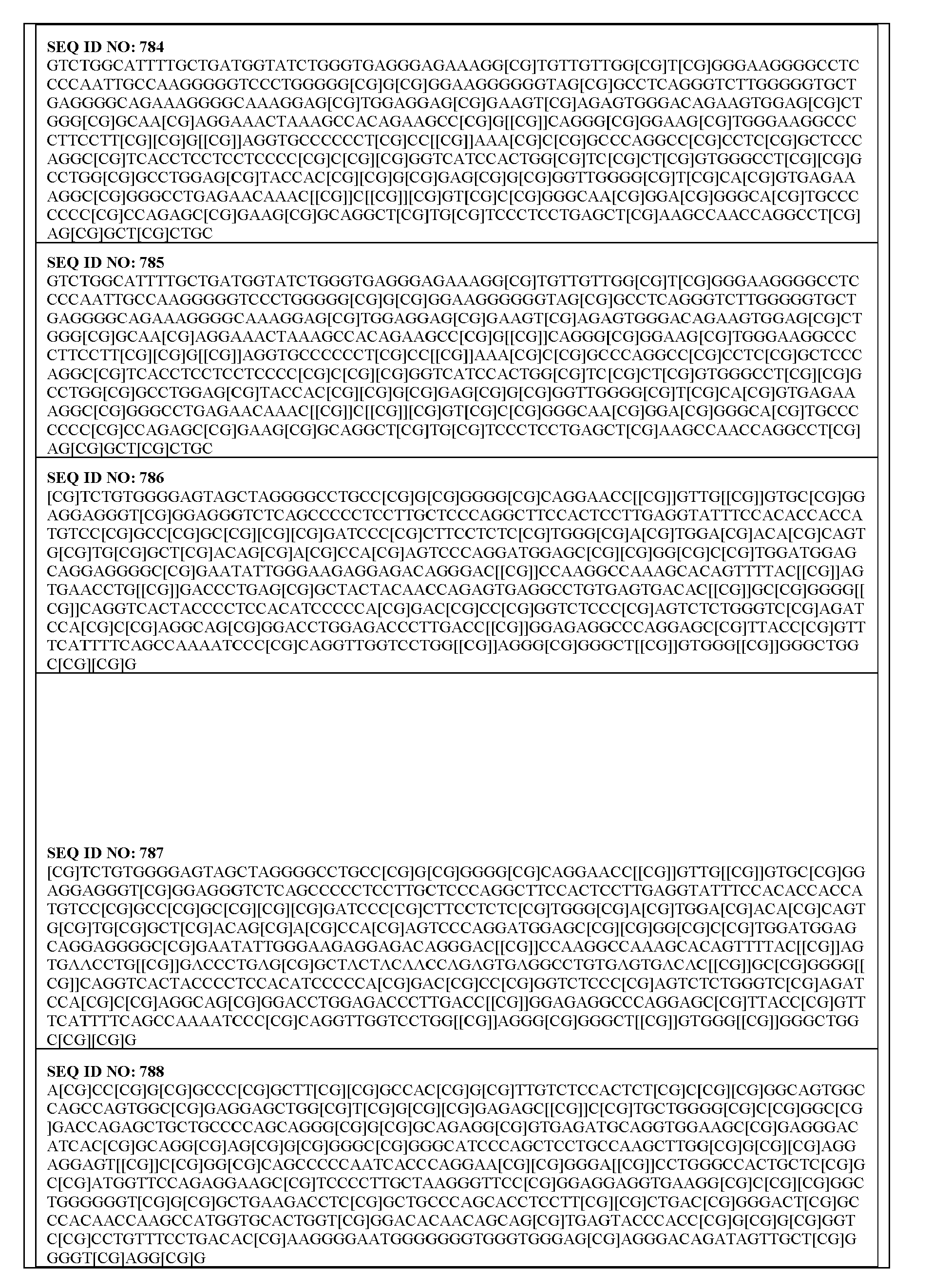

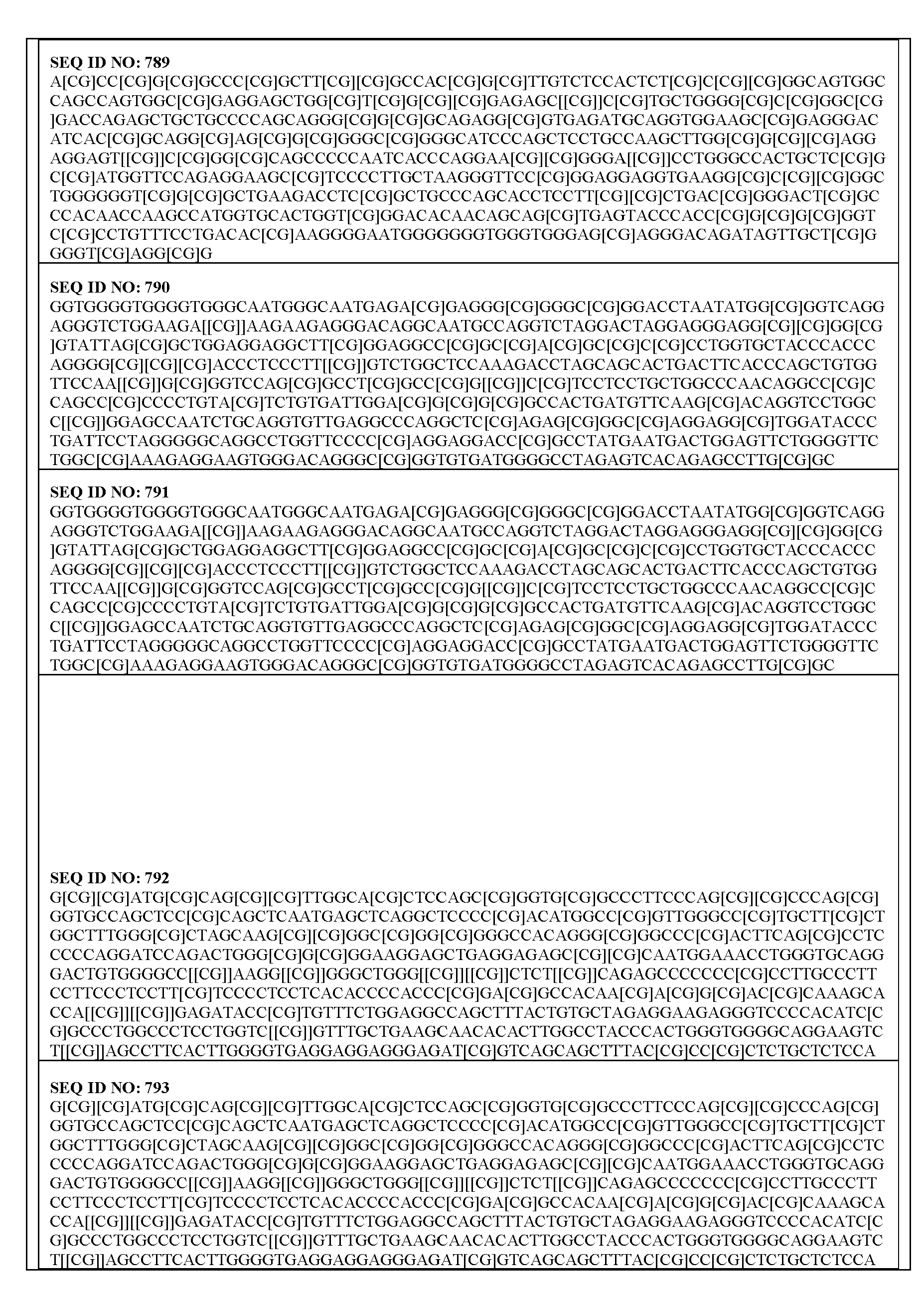

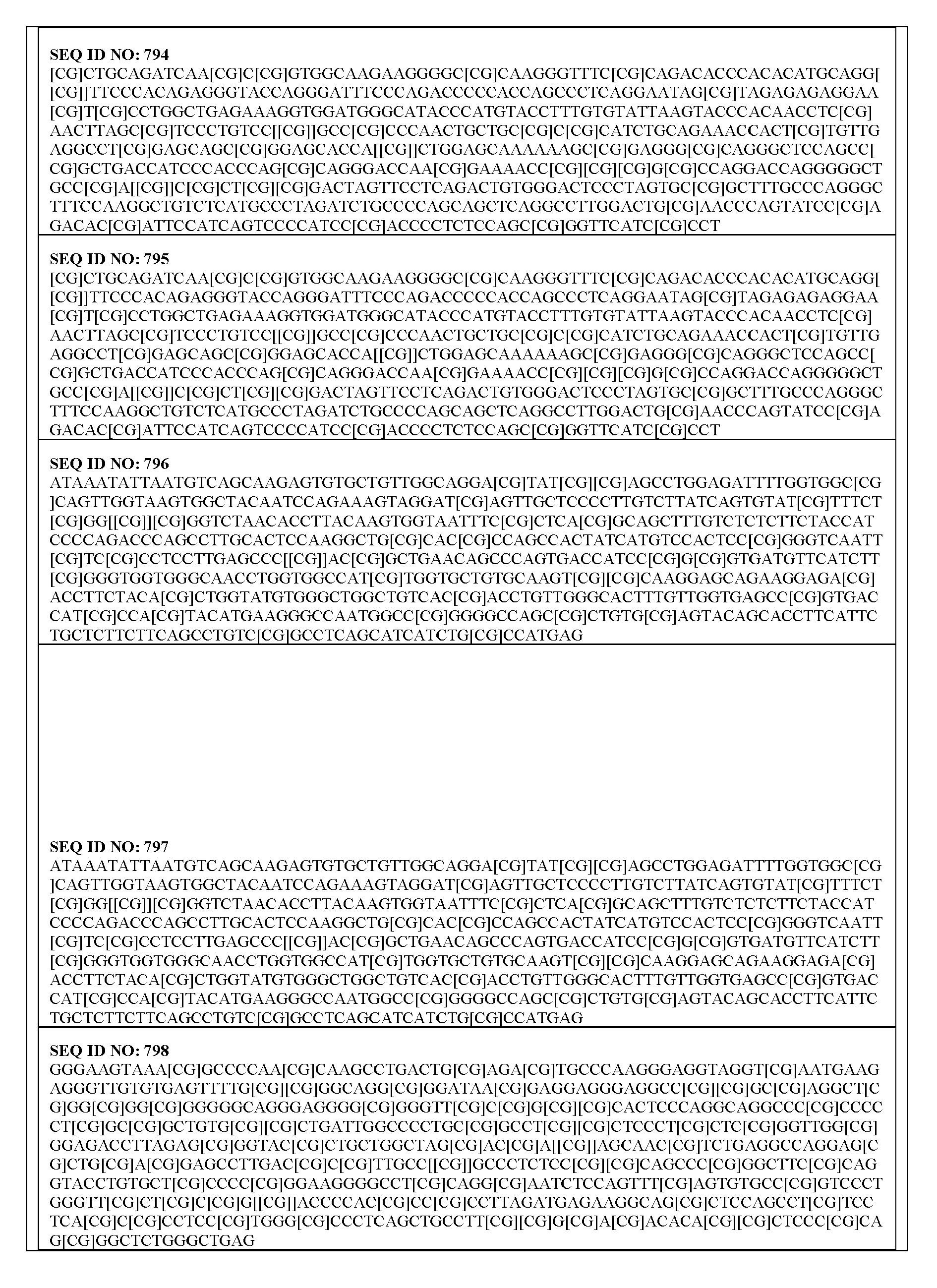

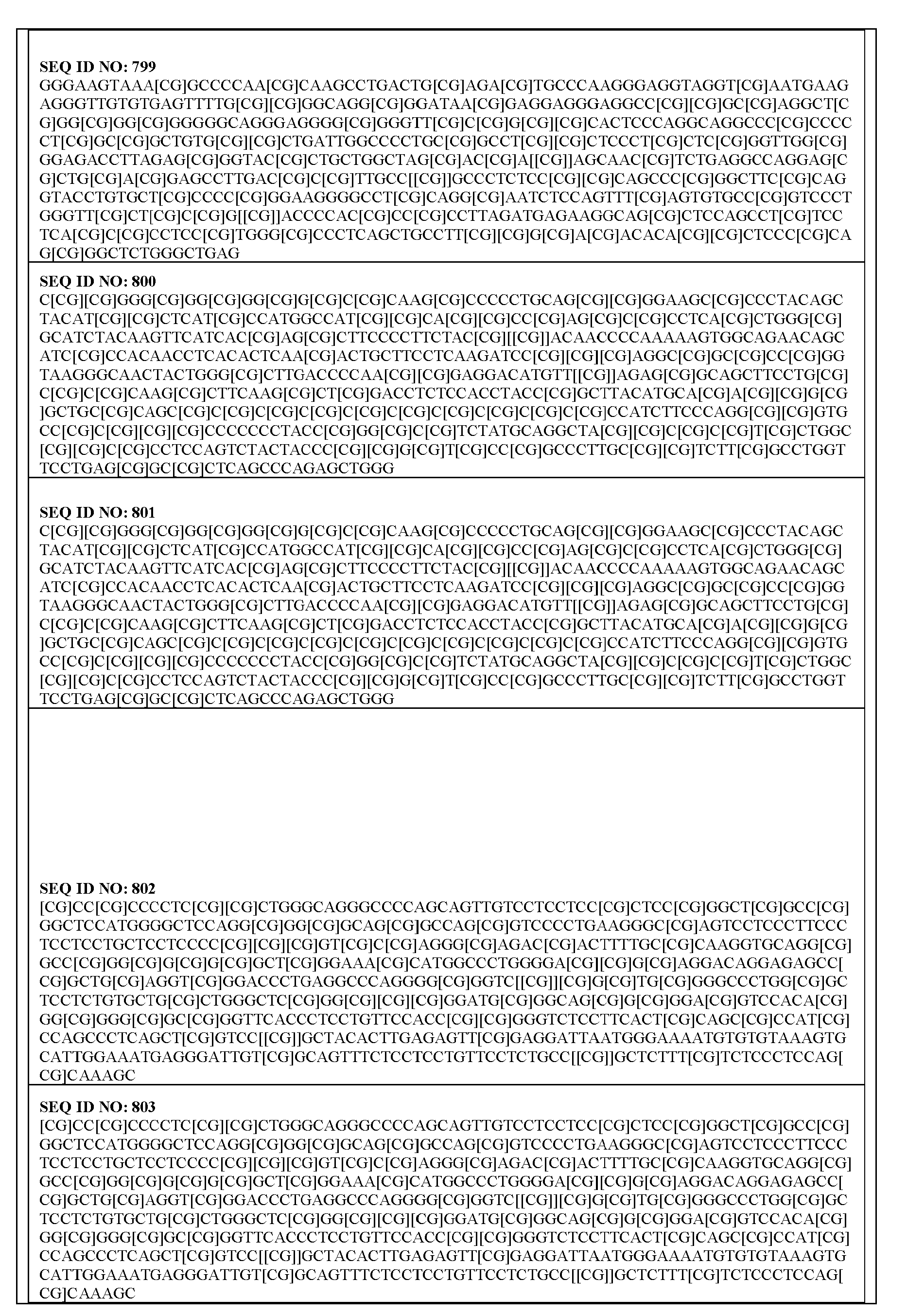

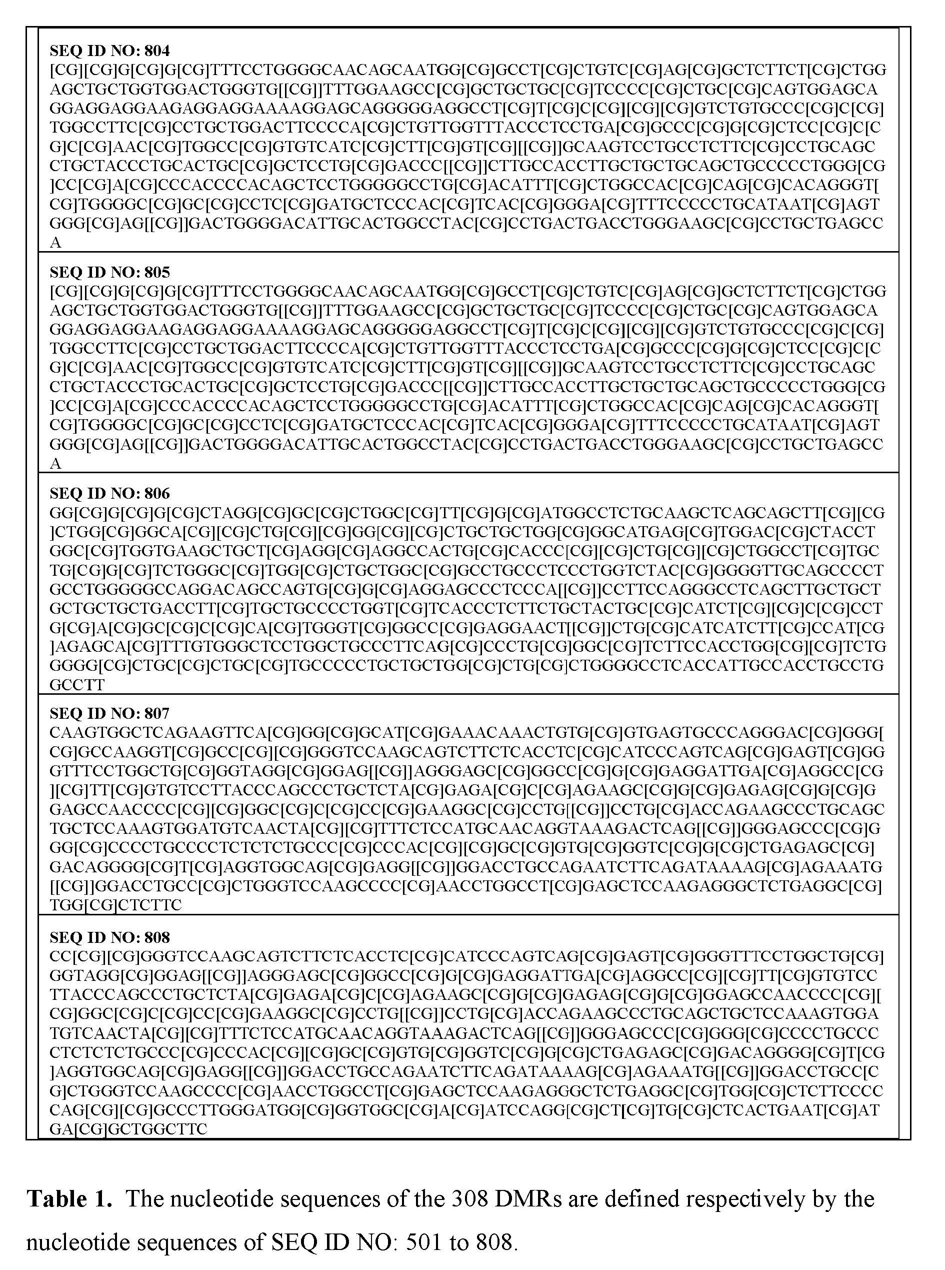

### Cancer-related CpGs for analysis

In any of the assays described herein, in a sample which has been taken from an individual, the sample comprises a population of DNA molecules.

The assay of the invention further comprises determining in the population of DNA molecules in the sample the methylation status of a panel of:
(i) one or more CpGs comprising the CpG identified in SEQ ID NO 67 wherein the CpG is identified at nucleotide positions 61 to 62

. A cancer index value is then derived based on the methylation status of the one or more CpGs in the panel, which is used to assess the presence, absence or development of cancer in the individual based on the cancer index value.

In any of the assays described herein, in DNA derived from cells in the sample the methylation status of each CpG in a panel of
(i) one or more CpGs comprising the CpG identified in SEQ ID NO 67 wherein the CpG is identified at nucleotide positions 61 to 62;
is determined.

In any of the assays described herein, in DNA derived from cells in the sample the methylation status of each CpG in a panel of one or more CpGs from a panel of CpGs identified at nucleotide positions 61 to 62 in SEQ ID NOs 1 to 500, is determined.

In any of the assays described herein, the panel of one or more CpGs may comprise at least 50 CpGs selected from the CpGs identified at nucleotide positions 61 to 62 in SEQ ID NOs 1 to 500, preferably wherein the assay is characterised as having a receiver operating characteristics (ROC) area under the curve (AUC) of at least 0.92. The panel of one or more CpGs may comprise at least the CpGs identified in SEQ ID NOs 1 to 50 and identified at nucleotide positions 61 to 62, preferably wherein the assay is characterised as having an ROC AUC of at least 0.95.

In any of the assays described herein, the panel of one or more CpGs may comprise at least 100 CpGs selected from the CpGs identified at nucleotide positions 61 to 62 in SEQ ID NOs 1 to 500, preferably wherein the assay is characterised as having a ROC AUC of at least 0.93. The panel of one or more CpGs may comprise at least the CpGs identified in SEQ ID NOs 1 to 100 and identified at nucleotide positions 61 to 62, preferably wherein the assay is characterised as having a ROC AUC of at least 0.96.

In any of the assays described herein, the panel of one or more CpGs may comprise at least 150 CpGs selected from the CpGs identified at nucleotide positions 61 to 62 in SEQ ID NOs 1 to 500, preferably wherein the assay is characterised as having a ROC AUC of at least 0.93. The panel of one or more CpGs may comprise at least the CpGs identified in SEQ ID NOs 1 to 150 and identified at nucleotide positions 61 to 62, preferably wherein the assay is characterised as having a ROC AUC of at least 0.96.

In any of the assays described herein, the panel of one or more CpGs may comprise at least 200 CpGs selected from the CpGs identified at nucleotide positions 61 to 62 in SEQ ID NOs 1 to 500, preferably wherein the assay is characterised as having an AUC of at least 0.93. The panel of one or more CpGs may comprise at least the CpGs identified in SEQ ID NOs 1 to 200 and identified at nucleotide positions 61 to 62, preferably wherein the assay is characterised as having a ROC AUC of at least 0.96.

In any of the assays described herein, the panel of one or more CpGs may comprise at least the 500 CpGs identified at nucleotide positions 61 to 62 in SEQ ID NOs 1 to 500, and further wherein the assay is characterised as having a ROC AUC of at least 0.97.

In any of the above-described assays, the assay may be characterised as having a ROC AUC of 0.60 or more, 0.61 or more, 0.62 or more, 0.63 or more, 0.64 or more, 0.65 or more, 0.66 or more, 0.67 or more, 0.68 or more, 0.69 or more, 0.70 or more, 0.71 or more, 0.72 or more, 0.73 or more, 0.74 or more, 0.75 or more, 0.76 or more, 0.77 or more, 0.78 or more, 0.79 or more, 0.80 or more, 0.81 or more, 0.82 or more, 0.83 or more, 0.84 or more, 0.85 or more, 0.86 or more, 0.87 or more, 0.88 or more, 0.89 or more or 0.90 or more.

In any of the described assays, the methylation status of the one or more CpGs in the panel is preferably determined by a β-value analysis, and the cancer is endometrial cancer.

In any of the assays described herein, the panel of one or more CpGs may comprise at least 50 CpGs selected from the CpGs identified at nucleotide positions 61 to 62 in SEQ ID NOs 1 to 500, optionally wherein:
1. the assay is characterised as having an ROC AUC (AUC) of at least 0.95, and more preferably wherein the panel of one or more CpGs comprises at least the CpGs identified in SEQ ID NOs 1 to 50 and identified at nucleotide positions 61 to 62;
2. the assay is characterised as having an AUC of at least 0.94, and more preferably wherein the panel of one or more CpGs comprises at least the CpGs identified in SEQ ID NOs 51 to 100 and identified at nucleotide positions 61 to 62;
3. the assay is characterised as having an ROC AUC (AUC) of at least 0.94, and more preferably wherein the panel of one or more CpGs comprises at least the CpGs identified in SEQ ID NOs 101 to 150 and identified at nucleotide positions 61 to 62;
4. the assay is characterised as having an ROC AUC (AUC) of at least 0.93, and more preferably wherein the panel of one or more CpGs comprises at least the CpGs identified in SEQ ID NOs 151 to 200 and identified at nucleotide positions 61 to 62;
5. the assay is characterised as having an ROC AUC (AUC) of at least 0.95, and more preferably wherein the panel of one or more CpGs comprises at least the CpGs identified in SEQ ID NOs 201 to 250 and identified at nucleotide positions 61 to 62;
6. the assay is characterised as having an ROC AUC (AUC) of at least 0.93, and more preferably wherein the panel of one or more CpGs comprises at least the CpGs identified in SEQ ID NOs 251 to 300 and identified at nucleotide positions 61 to 62;
7. the assay is characterised as having an ROC AUC (AUC) of at least 0.94, and more preferably wherein the panel of one or more CpGs comprises at least the CpGs identified in SEQ ID NOs 301 to 350 and identified at nucleotide positions 61 to 62;
8. the assay is characterised as having an ROC AUC (AUC) of at least 0.93, and more preferably wherein the panel of one or more CpGs comprises at least the CpGs identified in SEQ ID NOs 351 to 400 and identified at nucleotide positions 61 to 62;
9. the assay is characterised as having an ROC AUC (AUC) of at least 0.93, and more preferably wherein the panel of one or more CpGs comprises at least the CpGs identified in SEQ ID NOs 401 to 450 and identified at nucleotide positions 61 to 62; or
10. the assay is characterised as having an ROC AUC (AUC) of at least 0.92, and more preferably wherein the panel of one or more CpGs comprises at least the CpGs identified in SEQ ID NOs 451 to 500 and identified at nucleotide positions 61 to 62.

In any of the described assays, the methylation status of the one or more CpGs in the panel is preferably determined by a β-value analysis, and the cancer is endometrial cancer.

In any of the above-described assays, the assay may be characterised as having a ROC AUC of 0.60 or more, 0.61 or more, 0.62 or more, 0.63 or more, 0.64 or more, 0.65 or more, 0.66 or more, 0.67 or more, 0.68 or more, 0.69 or more, 0.70 or more, 0.71 or more, 0.72 or more, 0.73 or more, 0.74 or more, 0.75 or more, 0.76 or more, 0.77 or more, 0.78 or more, 0.79 or more, 0.80 or more, 0.81 or more, 0.82 or more, 0.83 or more, 0.84 or more, 0.85 or more, 0.86 or more, 0.87 or more, 0.88 or more, 0.89 or more or 0.90 or more.

In any of the assays described herein, the panel of one or more CpGs may comprise:
1. at least 50 CpGs selected from the CpGs identified at nucleotide positions 61 to 62 in SEQ ID NOs 1 to 500, preferably wherein the assay is characterised as having an AUC of at least 0.95, and more preferably wherein the panel of one or more CpGs comprises at least the CpGs identified in SEQ ID NOs 1 to 50 and identified at nucleotide positions 61 to 62;
2. at least 100 CpGs selected from the CpGs identified at nucleotide positions 61 to 62 in SEQ ID NOs 1 to 500, preferably wherein the assay is characterised as having an AUC of at least 0.96, and more preferably wherein the panel of one or more CpGs comprises at least the CpGs identified in SEQ ID NOs 1 to 100 and identified at nucleotide positions 61 to 62;
3. at least 150 CpGs selected from the CpGs identified at nucleotide positions 61 to 62 in SEQ ID NOs 1 to 500, preferably wherein the assay is characterised as having an AUC of at least 0.96, and more preferably wherein the panel of one or more CpGs comprises at least the CpGs identified in SEQ ID NOs 1 to 150 and identified at nucleotide positions 61 to 62;
4. at least 200 CpGs selected from the CpGs identified at nucleotide positions 61 to 62 in SEQ ID NOs 1 to 500, preferably wherein the assay is characterised as having an AUC of at least 0.96, and more preferably wherein the panel of one or more CpGs comprises at least the CpGs identified in SEQ ID NOs 1 to 200 and identified at nucleotide positions 61 to 62;
5. at least 250 CpGs selected from the CpGs identified at nucleotide positions 61 to 62 in SEQ ID NOs 1 to 500, preferably wherein the assay is characterised as having an AUC of at least 0.96, and more preferably wherein the panel of one or more CpGs comprises at least the CpGs identified in SEQ ID NOs 1 to 250 and identified at nucleotide positions 61 to 62;
6. at least 300 CpGs selected from the CpGs identified at nucleotide positions 61 to 62 in SEQ ID NOs 1 to 500, preferably wherein the assay is characterised as having an AUC of at least 0.96, and more preferably wherein the panel of one or more CpGs comprises at least the CpGs identified in SEQ ID NOs 1 to 300 and identified at nucleotide positions 61 to 62;
7. at least 350 CpGs selected from the CpGs identified at nucleotide positions 61 to 62 in SEQ ID NOs 1 to 500, preferably wherein the assay is characterised as having an AUC of at least 0.96, and more preferably wherein the panel of one or more CpGs comprises at least the CpGs identified in SEQ ID NOs 1 to 350 and identified at nucleotide positions 61 to 62;
8. at least 400 CpGs selected from the CpGs identified at nucleotide positions 61 to 62 in SEQ ID NOs 1 to 500, preferably wherein the assay is characterised as having an AUC of at least 0.96, and more preferably wherein the panel of one or more CpGs comprises at least the CpGs identified in SEQ ID NOs 1 to 400 and identified at nucleotide positions 61 to 62;
9. at least 450 CpGs selected from the CpGs identified at nucleotide positions 61 to 62 in SEQ ID NOs 1 to 500, preferably wherein the assay is characterised as having an AUC of at least 0.97, and more preferably wherein the panel of one or more CpGs comprises at least the CpGs identified in SEQ ID NOs 1 to 450 and identified at nucleotide positions 61 to 62; or
10. at least 500 CpGs selected from the CpGs identified at nucleotide positions 61 to 62 in SEQ ID NOs 1 to 500, preferably wherein the assay is characterised as having an AUC of at least 0.97, and more preferably wherein the panel of one or more CpGs comprises at least the CpGs identified at nucleotide positions 61 to 62 in SEQ ID NOs 1 to 500.

In any of the described assays, the methylation status of the one or more CpGs in the panel is preferably determined by a β-value analysis, and the cancer is endometrial cancer.

In any of the above-described assays, the assay may be characterised as having a ROC AUC of 0.60 or more, 0.61 or more, 0.62 or more, 0.63 or more, 0.64 or more, 0.65 or more, 0.66 or more, 0.67 or more, 0.68 or more, 0.69 or more, 0.70 or more, 0.71 or more, 0.72 or more, 0.73 or more, 0.74 or more, 0.75 or more, 0.76 or more, 0.77 or more, 0.78 or more, 0.79 or more, 0.80 or more, 0.81 or more, 0.82 or more, 0.83 or more, 0.84 or more, 0.85 or more, 0.86 or more, 0.87 or more, 0.88 or more, 0.89 or more or 0.90 or more.

In any of the assays described herein, the panel of one or more CpGs may comprise:
1. at least 50 CpGs selected from the CpGs identified at nucleotide positions 61 to 62 in SEQ ID NOs 1 to 500, preferably wherein the assay is characterised as having an AUC of at least 0.92, and more preferably wherein the panel of one or more CpGs comprises at least the CpGs identified in SEQ ID NOs 451 to 500 and identified at nucleotide positions 61 to 62;
2. at least 100 CpGs selected from the CpGs identified at nucleotide positions 61 to 62 in SEQ ID NOs 1 to 500, preferably wherein the assay is characterised as having an AUC of at least 0.93, and more preferably wherein the panel of one or more CpGs comprises at least the CpGs identified in SEQ ID NOs 401 to 500 and identified at nucleotide positions 61 to 62;
3. at least 150 CpGs selected from the CpGs identified at nucleotide positions 61 to 62 in SEQ ID NOs 1 to 500, preferably wherein the assay is characterised as having an AUC of at least 0.93, and more preferably wherein the panel of one or more CpGs comprises at least the CpGs identified in SEQ ID NOs 351 to 500 and identified at nucleotide positions 61 to 62;
4. at least 200 CpGs selected from the CpGs identified at nucleotide positions 61 to 62 in SEQ ID NOs 1 to 500, preferably wherein the assay is characterised as having an AUC of at least 0.93, and more preferably wherein the panel of one or more CpGs comprises at least the CpGs identified in SEQ ID NOs 301 to 500 and identified at nucleotide positions 61 to 62;
5. at least 250 CpGs selected from the CpGs identified at nucleotide positions 61 to 62 in SEQ ID NOs 1 to 500, preferably wherein the assay is characterised as having an AUC of at least 0.93, and more preferably wherein the panel of one or more CpGs comprises at least the CpGs identified in SEQ ID NOs 251 to 500 and identified at nucleotide positions 61 to 62;
6. at least 300 CpGs selected from the CpGs identified at nucleotide positions 61 to 62 in SEQ ID NOs 1 to 500, preferably wherein the assay is characterised as having an AUC of at least 0.94, and more preferably wherein the panel of one or more CpGs comprises at least the CpGs identified in SEQ ID NOs 201 to 500 and identified at nucleotide positions 61 to 62;
7. at least 350 CpGs selected from the CpGs identified at nucleotide positions 61 to 62 in SEQ ID NOs 1 to 500, preferably wherein the assay is characterised as having an AUC of at least 0.94, and more preferably wherein the panel of one or more CpGs comprises at least the CpGs identified in SEQ ID NOs 151 to 500 and identified at nucleotide positions 61 to 62;
8. at least 400 CpGs selected from the CpGs identified at nucleotide positions 61 to 62 in SEQ ID NOs 1 to 500, preferably wherein the assay is characterised as having an AUC of at least 0.94, and more preferably wherein the panel of one or more CpGs comprises at least the CpGs identified in SEQ ID NOs 101 to 500 and identified at nucleotide positions 61 to 62;
9. at least 450 CpGs selected from the CpGs identified at nucleotide positions 61 to 62 in SEQ ID NOs 1 to 500, preferably wherein the assay is characterised as having an AUC of at least 0.95, and more preferably wherein the panel of one or more CpGs comprises at least the CpGs identified in SEQ ID NOs 51 to 500 and identified at nucleotide positions 61 to 62; or
10. at least 500 CpGs selected from the CpGs identified at nucleotide positions 61 to 62 in SEQ ID NOs 1 to 500, preferably wherein the assay is characterised as having an AUC of at least 0.97, and more preferably wherein the panel of one or more CpGs comprises at least the CpGs identified at nucleotide positions 61 to 62 in SEQ ID NOs 1 to 500.

In any of the described assays, the methylation status of the one or more CpGs in the panel is preferably determined by a β-value analysis, and the cancer is endometrial cancer.

In any of the above-described assays, the assay may be characterised as having a ROC AUC of 0.60 or more, 0.61 or more, 0.62 or more, 0.63 or more, 0.64 or more, 0.65 or more, 0.66 or more, 0.67 or more, 0.68 or more, 0.69 or more, 0.70 or more, 0.71 or more, 0.72 or more, 0.73 or more, 0.74 or more, 0.75 or more, 0.76 or more, 0.77 or more, 0.78 or more, 0.79 or more, 0.80 or more, 0.81 or more, 0.82 or more, 0.83 or more, 0.84 or more, 0.85 or more, 0.86 or more, 0.87 or more, 0.88 or more, 0.89 or more or 0.90 or more.

In any of the assays described herein, the step of determining the methylation status of the one or more CpGs in the panel may comprise further determining the methylation status of one or more CpGs selected from within a panel of one or more Differentially Methylated Regions (DMRs) defined by SEQ ID NOs 501 to 808, wherein selected CpGs in each DMR are denoted by CG. The nucleotide sequences of the 308 DMRs are defined respectively by the nucleotide sequences of SEQ ID NO: 501 to 808 as set out in Table 1, accepting that variation in the nucleotide sequence of any given DMR may exist due to sequencing errors and/or variation between individuals. In each sequence shown below the cytosine of the CG dinucleotide motifs identified in square brackets and double square brackets is a cytosine of a CpG which may be included in a panel of CpGs when performing the assays of the invention.

In any of the assays described herein, the step of determining the methylation status of the one or more CpGs in the panel may comprise determining the methylation status of one or more CpGs denoted by CG within any one or more DMRs or within any combination of two or more DMRs defined by SEQ ID NOs 501 to 808, wherein selected CpGs in each DMR are denoted by CG. The DMRs are selected from the group consisting of DMRs 1 to 308 (SEQ ID NOs 501 to 808; as set out in Table 1).

The step of determining the methylation status of a panel of one or more CpGs may comprise determining a cancer index value of one or more of the CpGs denoted by CG within any one of the DMRs 1 to 308, or within any combination of two or more DMRs of 1 to 308.

The step of determining the methylation status of a panel of one or more CpGs may comprise determining a cancer index value of two or more, three or more, four or more, five or more, six or more, seven or more, eight or more, or nine or more of the CpGs denoted by CG within any one of the DMRs 1 to 308, optionally within any combination of two or more DMRs of 1 to 308.

The panel of one or more CpGs may comprise two or more CpGs of the DMR(s), three or more CpGs of the DMR(s), four or more CpGs of the DMR(s) or all CpGs of the DMR(s).

The step of determining the methylation status of a panel of one or more CpGs may comprise determining a cancer index value of least two or more, three or more, four or more, five or more, six or more, seven or more, eight or more, or nine or more of the CpGs denoted by CG within any one of the DMRs 1 to 308, or within:
a. any combination of two, three, four, five, six, seven, eight, or nine or more of DMRs 1 to 308;
b. any combination of ten, twenty, thirty, forty, fifty, sixty, seventy, eighty, or ninety or more of DMRs 1 to 308;
c. all 308 of DMRs 1 to 308;
d. one DMR defined by SEQ ID NO: 501, two DMRs defined by SEQ ID NOs: 501 to 502, three DMRs defined by SEQ ID NOs: 501 to 503, four DMRs defined by SEQ ID NOs: 501 to 504, five DMRs defined by SEQ ID NOs: 501 to 505, six DMRs defined by SEQ ID NOs: 501 to 506, seven DMRs defined by SEQ ID NOs: 501 to 507, eight DMRs defined by SEQ ID NOs: 501 to 508, or nine DMRs defined by SEQ ID NOs: 501 to 509; or
e. ten DMRs defined by SEQ ID NOs: 501 to 510, twenty DMRs defined by SEQ ID NOs: 501 to 520, thirty DMRs defined by SEQ ID NOs: 501 to 530, forty DMRs defined by SEQ ID NOs: 501 to 540, fifty DMRs defined by SEQ ID NOs: 501 to 550, sixty DMRs defined by SEQ ID NOs: 501 to 560, seventy DMRs defined by SEQ ID NOs: 501 to 570, eighty DMRs defined by SEQ ID NOs: 501 to 580, or ninety DMRs defined by SEQ ID NOs: 501 to 590; or
f. ten DMRs defined by SEQ ID NOs: 501 to 510, SEQ ID NOs: 511 to 520, SEQ ID NOs: 521 to 530, SEQ ID NOs: 531 to 540, SEQ ID NOs: 541 to 550, SEQ ID NOs: 551 to 560, SEQ ID NOs: 561 to 570, SEQ ID NOs: 571 to 580, SEQ ID NOs: 581 to 590, SEQ ID NOs: 591 to 600, SEQ ID NOs: 601 to 610, SEQ ID NOs: 611 to 620, SEQ ID NOs: 621 to 630, SEQ ID NOs: 631 to 640, SEQ ID NOs: 641 to 650, SEQ ID NOs: 651 to 660, SEQ ID NOs: 661 to 670, SEQ ID NOs: 671 to 680, SEQ ID NOs: 681 to 690, SEQ ID NOs: 691 to 700, SEQ ID NOs: 701 to 710, SEQ ID NOs: 711 to 720, SEQ ID NOs: 721 to 730, SEQ ID NOs: 731 to 740, SEQ ID NOs: 741 to 750; SEQ ID NOs: 751 to 760; SEQ ID NOs: 761 to 770; SEQ ID NOs: 771 to 780; SEQ ID NOs: 781 to 790; SEQ ID NOs: 791 to 800 or SEQ ID NOs: 801 to 808.

In any of the assays described herein, the step of determining the methylation status of a panel of one or more CpGs may comprise determining the methylation status of one or more CpGs within any one or more DMRs selected from the group of DMRs consisting of DMRs 1 to 308 as defined by SEQ ID NOs 501 to 808, including:
1. one or more CpGs within DMR 1 as defined by SEQ ID NO: 501 and denoted by CG, preferably wherein the assay is characterised as having an ROC AUC of at least 0.911, and more preferably wherein the panel of one or more CpGs comprises at least the CpGs denoted by [[CG]];
2. one or more CpGs within DMR 2 as defined by SEQ ID NO: 502 and denoted by CG, preferably wherein the assay is characterised as having an ROC AUC of at least 0.903, and more preferably wherein the panel of one or more CpGs comprises at least the CpGs denoted by [[CG]];
3. one or more CpGs within DMR 3 as defined by SEQ ID NO: 503 and denoted by CG, preferably wherein the assay is characterised as having an ROC AUC of at least 0.899, and more preferably wherein the panel of one or more CpGs comprises at least the CpGs denoted by [[CG]];
4. one or more CpGs within DMR 4 as defined by SEQ ID NO: 504 and denoted by CG, preferably wherein the assay is characterised as having an ROC AUC of at least 0.899, and more preferably wherein the panel of one or more CpGs comprises at least the CpGs denoted by [[CG]];
5. one or more CpGs within DMR 5 as defined by SEQ ID NO: 505 and denoted by CG, preferably wherein the assay is characterised as having an ROC AUC of at least 0.899, and more preferably wherein the panel of one or more CpGs comprises at least the CpGs denoted by [[CG]];
6. one or more CpGs within DMR 6 as defined by SEQ ID NO: 506 and denoted by CG, preferably wherein the assay is characterised as having an ROC AUC of at least 0.897, and more preferably wherein the panel of one or more CpGs comprises at least the CpGs denoted by [[CG]];
7. one or more CpGs within DMR 7 as defined by SEQ ID NO: 507 and denoted by CG, preferably wherein the assay is characterised as having an ROC AUC of at least 0.894, and more preferably wherein the panel of one or more CpGs comprises at least the CpGs denoted by [[CG]];
8. one or more CpGs within DMR 8 as defined by SEQ ID NO: 508 and denoted by CG, preferably wherein the assay is characterised as having an ROC AUC of at least 0.894, and more preferably wherein the panel of one or more CpGs comprises at least the CpGs denoted by [[CG]];
9. one or more CpGs within DMR 9 as defined by SEQ ID NO: 509 and denoted by CG, preferably wherein the assay is characterised as having an ROC AUC of at least 0.892, and more preferably wherein the panel of one or more CpGs comprises at least the CpGs denoted by [[CG]];
10. one or more CpGs within DMR 10 as defined by SEQ ID NO: 510 and denoted by CG, preferably wherein the assay is characterised as having an ROC AUC of at least 0.891, and more preferably wherein the panel of one or more CpGs comprises at least the CpGs denoted by [[CG]];
11. one or more CpGs within DMR 11 as defined by SEQ ID NO: 511 and denoted by CG, preferably wherein the assay is characterised as having an ROC AUC of at least 0.89, and more preferably wherein the panel of one or more CpGs comprises at least the CpGs denoted by [[CG]];
12. one or more CpGs within DMR 12 as defined by SEQ ID NO: 512 and denoted by CG, preferably wherein the assay is characterised as having an ROC AUC of at least 0.888, and more preferably wherein the panel of one or more CpGs comprises at least the CpGs denoted by [[CG]];
13. one or more CpGs within DMR 13 as defined by SEQ ID NO: 513 and denoted by CG, preferably wherein the assay is characterised as having an ROC AUC of at least 0.888, and more preferably wherein the panel of one or more CpGs comprises at least the CpGs denoted by [[CG]];
14. one or more CpGs within DMR 14 as defined by SEQ ID NO: 514 and denoted by CG, preferably wherein the assay is characterised as having an ROC AUC of at least 0.888, and more preferably wherein the panel of one or more CpGs comprises at least the CpGs denoted by [[CG]];
15. one or more CpGs within DMR 15 as defined by SEQ ID NO: 515 and denoted by CG, preferably wherein the assay is characterised as having an ROC AUC of at least 0.888, and more preferably wherein the panel of one or more CpGs comprises at least the CpGs denoted by [[CG]];
16. one or more CpGs within DMR 16 as defined by SEQ ID NO: 516 and denoted by CG, preferably wherein the assay is characterised as having an ROC AUC of at least 0.888, and more preferably wherein the panel of one or more CpGs comprises at least the CpGs denoted by [[CG]];
17. one or more CpGs within DMR 17 as defined by SEQ ID NO: 517 and denoted by CG, preferably wherein the assay is characterised as having an ROC AUC of at least 0.886, and more preferably wherein the panel of one or more CpGs comprises at least the CpGs denoted by [[CG]];
18. one or more CpGs within DMR 18 as defined by SEQ ID NO: 518 and denoted by CG, preferably wherein the assay is characterised as having an ROC AUC of at least 0.886, and more preferably wherein the panel of one or more CpGs comprises at least the CpGs denoted by [[CG]];
19. one or more CpGs within DMR 19 as defined by SEQ ID NO: 519 and denoted by CG, preferably wherein the assay is characterised as having an ROC AUC of at least 0.885, and more preferably wherein the panel of one or more CpGs comprises at least the CpGs denoted by [[CG]];
20. one or more CpGs within DMR 20 as defined by SEQ ID NO: 520 and denoted by CG, preferably wherein the assay is characterised as having an ROC AUC of at least 0.884, and more preferably wherein the panel of one or more CpGs comprises at least the CpGs denoted by [[CG]];
21. one or more CpGs within DMR 21 as defined by SEQ ID NO: 521 and denoted by CG, preferably wherein the assay is characterised as having an ROC AUC of at least 0.882, and more preferably wherein the panel of one or more CpGs comprises at least the CpGs denoted by [[CG]];
22. one or more CpGs within DMR 22 as defined by SEQ ID NO: 522 and denoted by CG, preferably wherein the assay is characterised as having an ROC AUC of at least 0.881, and more preferably wherein the panel of one or more CpGs comprises at least the CpGs denoted by [[CG]];
23. one or more CpGs within DMR 23 as defined by SEQ ID NO: 523 and denoted by CG, preferably wherein the assay is characterised as having an ROC AUC of at least 0.88, and more preferably wherein the panel of one or more CpGs comprises at least the CpGs denoted by [[CG]];
24. one or more CpGs within DMR 24 as defined by SEQ ID NO: 524 and denoted by CG, preferably wherein the assay is characterised as having an ROC AUC of at least 0.879, and more preferably wherein the panel of one or more CpGs comprises at least the CpGs denoted by [[CG]];
25. one or more CpGs within DMR 25 as defined by SEQ ID NO: 525 and denoted by CG, preferably wherein the assay is characterised as having an ROC AUC of at least 0.878, and more preferably wherein the panel of one or more CpGs comprises at least the CpGs denoted by [[CG]];
26. one or more CpGs within DMR 26 as defined by SEQ ID NO: 526 and denoted by CG, preferably wherein the assay is characterised as having an ROC AUC of at least 0.878, and more preferably wherein the panel of one or more CpGs comprises at least the CpGs denoted by [[CG]];
27. one or more CpGs within DMR 27 as defined by SEQ ID NO: 527 and denoted by CG, preferably wherein the assay is characterised as having an ROC AUC of at least 0.877, and more preferably wherein the panel of one or more CpGs comprises at least the CpGs denoted by [[CG]];
28. one or more CpGs within DMR 28 as defined by SEQ ID NO: 528 and denoted by CG, preferably wherein the assay is characterised as having an ROC AUC of at least 0.875, and more preferably wherein the panel of one or more CpGs comprises at least the CpGs denoted by [[CG]];
29. one or more CpGs within DMR 29 as defined by SEQ ID NO: 529 and denoted by CG, preferably wherein the assay is characterised as having an ROC AUC of at least 0.875, and more preferably wherein the panel of one or more CpGs comprises at least the CpGs denoted by [[CG]];
30. one or more CpGs within DMR 30 as defined by SEQ ID NO: 530 and denoted by CG, preferably wherein the assay is characterised as having an ROC AUC of at least 0.874, and more preferably wherein the panel of one or more CpGs comprises at least the CpGs denoted by [[CG]];
31. one or more CpGs within DMR 31 as defined by SEQ ID NO: 531 and denoted by CG, preferably wherein the assay is characterised as having an ROC AUC of at least 0.874, and more preferably wherein the panel of one or more CpGs comprises at least the CpGs denoted by [[CG]];
32. one or more CpGs within DMR 32 as defined by SEQ ID NO: 532 and denoted by CG, preferably wherein the assay is characterised as having an ROC AUC of at least 0.874, and more preferably wherein the panel of one or more CpGs comprises at least the CpGs denoted by [[CG]];
33. one or more CpGs within DMR 33 as defined by SEQ ID NO: 533 and denoted by CG, preferably wherein the assay is characterised as having an ROC AUC of at least 0.874, and more preferably wherein the panel of one or more CpGs comprises at least the CpGs denoted by [[CG]];
34. one or more CpGs within DMR 34 as defined by SEQ ID NO: 534 and denoted by CG, preferably wherein the assay is characterised as having an ROC AUC of at least 0.873, and more preferably wherein the panel of one or more CpGs comprises at least the CpGs denoted by [[CG]];
35. one or more CpGs within DMR 35 as defined by SEQ ID NO: 535 and denoted by CG, preferably wherein the assay is characterised as having an ROC AUC of at least 0.873, and more preferably wherein the panel of one or more CpGs comprises at least the CpGs denoted by [[CG]];
36. one or more CpGs within DMR 36 as defined by SEQ ID NO: 536 and denoted by CG, preferably wherein the assay is characterised as having an ROC AUC of at least 0.873, and more preferably wherein the panel of one or more CpGs comprises at least the CpGs denoted by [[CG]];
37. one or more CpGs within DMR 37 as defined by SEQ ID NO: 537 and denoted by CG, preferably wherein the assay is characterised as having an ROC AUC of at least 0.873, and more preferably wherein the panel of one or more CpGs comprises at least the CpGs denoted by [[CG]];
38. one or more CpGs within DMR 38 as defined by SEQ ID NO: 538 and denoted by CG, preferably wherein the assay is characterised as having an ROC AUC of at least 0.873, and more preferably wherein the panel of one or more CpGs comprises at least the CpGs denoted by [[CG]];
39. one or more CpGs within DMR 39 as defined by SEQ ID NO: 539 and denoted by CG, preferably wherein the assay is characterised as having an ROC AUC of at least 0.872, and more preferably wherein the panel of one or more CpGs comprises at least the CpGs denoted by [[CG]];
40. one or more CpGs within DMR 40 as defined by SEQ ID NO: 540 and denoted by CG, preferably wherein the assay is characterised as having an ROC AUC of at least 0.872, and more preferably wherein the panel of one or more CpGs comprises at least the CpGs denoted by [[CG]];
41. one or more CpGs within DMR 41 as defined by SEQ ID NO: 541 and denoted by CG, preferably wherein the assay is characterised as having an ROC AUC of at least 0.871, and more preferably wherein the panel of one or more CpGs comprises at least the CpGs denoted by [[CG]];
42. one or more CpGs within DMR 42 as defined by SEQ ID NO: 542 and denoted by CG, preferably wherein the assay is characterised as having an ROC AUC of at least 0.871, and more preferably wherein the panel of one or more CpGs comprises at least the CpGs denoted by [[CG]];
43. one or more CpGs within DMR 43 as defined by SEQ ID NO: 543 and denoted by CG, preferably wherein the assay is characterised as having an ROC AUC of at least 0.87, and more preferably wherein the panel of one or more CpGs comprises at least the CpGs denoted by [[CG]];
44. one or more CpGs within DMR 44 as defined by SEQ ID NO: 544 and denoted by CG, preferably wherein the assay is characterised as having an ROC AUC of at least 0.87, and more preferably wherein the panel of one or more CpGs comprises at least the CpGs denoted by [[CG]];
45. one or more CpGs within DMR 45 as defined by SEQ ID NO: 545 and denoted by CG, preferably wherein the assay is characterised as having an ROC AUC of at least 0.869, and more preferably wherein the panel of one or more CpGs comprises at least the CpGs denoted by [[CG]];
46. one or more CpGs within DMR 46 as defined by SEQ ID NO: 546 and denoted by CG, preferably wherein the assay is characterised as having an ROC AUC of at least 0.869, and more preferably wherein the panel of one or more CpGs comprises at least the CpGs denoted by [[CG]];
47. one or more CpGs within DMR 47 as defined by SEQ ID NO: 547 and denoted by CG, preferably wherein the assay is characterised as having an ROC AUC of at least 0.869, and more preferably wherein the panel of one or more CpGs comprises at least the CpGs denoted by [[CG]];
48. one or more CpGs within DMR 48 as defined by SEQ ID NO: 548 and denoted by CG, preferably wherein the assay is characterised as having an ROC AUC of at least 0.867, and more preferably wherein the panel of one or more CpGs comprises at least the CpGs denoted by [[CG]];
49. one or more CpGs within DMR 49 as defined by SEQ ID NO: 549 and denoted by CG, preferably wherein the assay is characterised as having an ROC AUC of at least 0.867, and more preferably wherein the panel of one or more CpGs comprises at least the CpGs denoted by [[CG]];
50. one or more CpGs within DMR 50 as defined by SEQ ID NO: 550 and denoted by CG, preferably wherein the assay is characterised as having an ROC AUC of at least 0.867, and more preferably wherein the panel of one or more CpGs comprises at least the CpGs denoted by [[CG]];
51. one or more CpGs within DMR 551 as defined by SEQ ID NO: 551 and denoted by CG, preferably wherein the assay is characterised as having an ROC AUC of at least 0.867, and more preferably wherein the panel of one or more CpGs comprises at least the CpGs denoted by [[CG]];
52. one or more CpGs within DMR 52 as defined by SEQ ID NO: 552 and denoted by CG, preferably wherein the assay is characterised as having an ROC AUC of at least 0.865, and more preferably wherein the panel of one or more CpGs comprises at least the CpGs denoted by [[CG]];
53. one or more CpGs within DMR 53 as defined by SEQ ID NO: 553 and denoted by CG, preferably wherein the assay is characterised as having an ROC AUC of at least 0.864, and more preferably wherein the panel of one or more CpGs comprises at least the CpGs denoted by [[CG]];
54. one or more CpGs within DMR 54 as defined by SEQ ID NO: 554 and denoted by CG, preferably wherein the assay is characterised as having an ROC AUC of at least 0.864, and more preferably wherein the panel of one or more CpGs comprises at least the CpGs denoted by [[CG]];
55. one or more CpGs within DMR 55 as defined by SEQ ID NO: 555 and denoted by CG, preferably wherein the assay is characterised as having an ROC AUC of at least 0.863, and more preferably wherein the panel of one or more CpGs comprises at least the CpGs denoted by [[CG]];
56. one or more CpGs within DMR 56 as defined by SEQ ID NO: 556 and denoted by CG, preferably wherein the assay is characterised as having an ROC AUC of at least 0.863, and more preferably wherein the panel of one or more CpGs comprises at least the CpGs denoted by [[CG]];
57. one or more CpGs within DMR 57 as defined by SEQ ID NO: 557 and denoted by CG, preferably wherein the assay is characterised as having an ROC AUC of at least 0.863, and more preferably wherein the panel of one or more CpGs comprises at least the CpGs denoted by [[CG]];
58. one or more CpGs within DMR 58 as defined by SEQ ID NO: 558 and denoted by CG, preferably wherein the assay is characterised as having an ROC AUC of at least 0.862, and more preferably wherein the panel of one or more CpGs comprises at least the CpGs denoted by [[CG]];
59. one or more CpGs within DMR 59 as defined by SEQ ID NO: 559 and denoted by CG, preferably wherein the assay is characterised as having an ROC AUC of at least 0.862, and more preferably wherein the panel of one or more CpGs comprises at least the CpGs denoted by [[CG]];
60. one or more CpGs within DMR 60 as defined by SEQ ID NO: 560 and denoted by CG, preferably wherein the assay is characterised as having an ROC AUC of at least 0.861, and more preferably wherein the panel of one or more CpGs comprises at least the CpGs denoted by [[CG]];
61. one or more CpGs within DMR 61 as defined by SEQ ID NO: 561 and denoted by CG, preferably wherein the assay is characterised as having an ROC AUC of at least 0.861, and more preferably wherein the panel of one or more CpGs comprises at least the CpGs denoted by [[CG]];
62. one or more CpGs within DMR 62 as defined by SEQ ID NO: 562 and denoted by CG, preferably wherein the assay is characterised as having an ROC AUC of at least 0.861, and more preferably wherein the panel of one or more CpGs comprises at least the CpGs denoted by [[CG]];
63. one or more CpGs within DMR 63 as defined by SEQ ID NO: 563 and denoted by CG, preferably wherein the assay is characterised as having an ROC AUC of at least 0.86, and more preferably wherein the panel of one or more CpGs comprises at least the CpGs denoted by [[CG]];
64. one or more CpGs within DMR 64 as defined by SEQ ID NO: 564 and denoted by CG, preferably wherein the assay is characterised as having an ROC AUC of at least 0.86, and more preferably wherein the panel of one or more CpGs comprises at least the CpGs denoted by [[CG]];
65. one or more CpGs within DMR 65 as defined by SEQ ID NO: 565 and denoted by CG, preferably wherein the assay is characterised as having an ROC AUC of at least 0.86, and more preferably wherein the panel of one or more CpGs comprises at least the CpGs denoted by [[CG]];
66. one or more CpGs within DMR 66 as defined by SEQ ID NO: 566 and denoted by CG, preferably wherein the assay is characterised as having an ROC AUC of at least 0.86, and more preferably wherein the panel of one or more CpGs comprises at least the CpGs denoted by [[CG]];
67. one or more CpGs within DMR 67 as defined by SEQ ID NO: 567 and denoted by CG, preferably wherein the assay is characterised as having an ROC AUC of at least 0.859, and more preferably wherein the panel of one or more CpGs comprises at least the CpGs denoted by [[CG]];
68. one or more CpGs within DMR 68 as defined by SEQ ID NO: 568 and denoted by CG, preferably wherein the assay is characterised as having an ROC AUC of at least 0.859, and more preferably wherein the panel of one or more CpGs comprises at least the CpGs denoted by [[CG]];
69. one or more CpGs within DMR 69 as defined by SEQ ID NO: 569 and denoted by CG, preferably wherein the assay is characterised as having an ROC AUC of at least 0.859, and more preferably wherein the panel of one or more CpGs comprises at least the CpGs denoted by [[CG]];
70. one or more CpGs within DMR 70 as defined by SEQ ID NO: 570 and denoted by CG, preferably wherein the assay is characterised as having an ROC AUC of at least 0.859, and more preferably wherein the panel of one or more CpGs comprises at least the CpGs denoted by [[CG]];
71. one or more CpGs within DMR 71 as defined by SEQ ID NO: 571 and denoted by CG, preferably wherein the assay is characterised as having an ROC AUC of at least 0.859, and more preferably wherein the panel of one or more CpGs comprises at least the CpGs denoted by [[CG]];
72. one or more CpGs within DMR 72 as defined by SEQ ID NO: 572 and denoted by CG, preferably wherein the assay is characterised as having an ROC AUC of at least 0.858, and more preferably wherein the panel of one or more CpGs comprises at least the CpGs denoted by [[CG]];
73. one or more CpGs within DMR 73 as defined by SEQ ID NO: 573 and denoted by CG, preferably wherein the assay is characterised as having an ROC AUC of at least 0.857, and more preferably wherein the panel of one or more CpGs comprises at least the CpGs denoted by [[CG]];
74. one or more CpGs within DMR 74 as defined by SEQ ID NO: 574 and denoted by CG, preferably wherein the assay is characterised as having an ROC AUC of at least 0.857, and more preferably wherein the panel of one or more CpGs comprises at least the CpGs denoted by [[CG]];
75. one or more CpGs within DMR 75 as defined by SEQ ID NO: 575 and denoted by CG, preferably wherein the assay is characterised as having an ROC AUC of at least 0.856, and more preferably wherein the panel of one or more CpGs comprises at least the CpGs denoted by [[CG]];
76. one or more CpGs within DMR 76 as defined by SEQ ID NO: 576 and denoted by CG, preferably wherein the assay is characterised as having an ROC AUC of at least 0.856, and more preferably wherein the panel of one or more CpGs comprises at least the CpGs denoted by [[CG]];
77. one or more CpGs within DMR 77 as defined by SEQ ID NO: 577 and denoted by CG, preferably wherein the assay is characterised as having an ROC AUC of at least 0.856, and more preferably wherein the panel of one or more CpGs comprises at least the CpGs denoted by [[CG]];
78. one or more CpGs within DMR 78 as defined by SEQ ID NO: 578 and denoted by CG, preferably wherein the assay is characterised as having an ROC AUC of at least 0.855, and more preferably wherein the panel of one or more CpGs comprises at least the CpGs denoted by [[CG]];
79. one or more CpGs within DMR 79 as defined by SEQ ID NO: 579 and denoted by CG, preferably wherein the assay is characterised as having an ROC AUC of at least 0.855, and more preferably wherein the panel of one or more CpGs comprises at least the CpGs denoted by [[CG]];
80. one or more CpGs within DMR 80 as defined by SEQ ID NO: 580 and denoted by CG, preferably wherein the assay is characterised as having an ROC AUC of at least 0.855, and more preferably wherein the panel of one or more CpGs comprises at least the CpGs denoted by [[CG]];
81. one or more CpGs within DMR 81 as defined by SEQ ID NO: 581 and denoted by CG, preferably wherein the assay is characterised as having an ROC AUC of at least 0.854, and more preferably wherein the panel of one or more CpGs comprises at least the CpGs denoted by [[CG]];
82. one or more CpGs within DMR 82 as defined by SEQ ID NO: 582 and denoted by CG, preferably wherein the assay is characterised as having an ROC AUC of at least 0.854, and more preferably wherein the panel of one or more CpGs comprises at least the CpGs denoted by [[CG]];
83. one or more CpGs within DMR 83 as defined by SEQ ID NO: 583 and denoted by CG, preferably wherein the assay is characterised as having an ROC AUC of at least 0.854, and more preferably wherein the panel of one or more CpGs comprises at least the CpGs denoted by [[CG]];
84. one or more CpGs within DMR 84 as defined by SEQ ID NO: 584 and denoted by CG, preferably wherein the assay is characterised as having an ROC AUC of at least 0.854, and more preferably wherein the panel of one or more CpGs comprises at least the CpGs denoted by [[CG]];
85. one or more CpGs within DMR 85 as defined by SEQ ID NO: 585 and denoted by CG, preferably wherein the assay is characterised as having an ROC AUC of at least 0.853, and more preferably wherein the panel of one or more CpGs comprises at least the CpGs denoted by [[CG]];
86. one or more CpGs within DMR 86 as defined by SEQ ID NO: 586 and denoted by CG, preferably wherein the assay is characterised as having an ROC AUC of at least 0.853, and more preferably wherein the panel of one or more CpGs comprises at least the CpGs denoted by [[CG]];
87. one or more CpGs within DMR 87 as defined by SEQ ID NO: 587 and denoted by CG, preferably wherein the assay is characterised as having an ROC AUC of at least 0.853, and more preferably wherein the panel of one or more CpGs comprises at least the CpGs denoted by [[CG]];
88. one or more CpGs within DMR 88 as defined by SEQ ID NO: 588 and denoted by CG, preferably wherein the assay is characterised as having an ROC AUC of at least 0.853, and more preferably wherein the panel of one or more CpGs comprises at least the CpGs denoted by [[CG]];
89. one or more CpGs within DMR 89 as defined by SEQ ID NO: 589 and denoted by CG, preferably wherein the assay is characterised as having an ROC AUC of at least 0.852, and more preferably wherein the panel of one or more CpGs comprises at least the CpGs denoted by [[CG]];
90. one or more CpGs within DMR 90 as defined by SEQ ID NO: 590 and denoted by CG, preferably wherein the assay is characterised as having an ROC AUC of at least 0.852, and more preferably wherein the panel of one or more CpGs comprises at least the CpGs denoted by [[CG]];
91. one or more CpGs within DMR 91 as defined by SEQ ID NO: 591 and denoted by CG, preferably wherein the assay is characterised as having an ROC AUC of at least 0.852, and more preferably wherein the panel of one or more CpGs comprises at least the CpGs denoted by [[CG]];
92. one or more CpGs within DMR 92 as defined by SEQ ID NO: 592 and denoted by CG, preferably wherein the assay is characterised as having an ROC AUC of at least 0.851, and more preferably wherein the panel of one or more CpGs comprises at least the CpGs denoted by [[CG]];
93. one or more CpGs within DMR 93 as defined by SEQ ID NO: 593 and denoted by CG, preferably wherein the assay is characterised as having an ROC AUC of at least 0.851, and more preferably wherein the panel of one or more CpGs comprises at least the CpGs denoted by [[CG]];
94. one or more CpGs within DMR 94 as defined by SEQ ID NO: 594 and denoted by CG, preferably wherein the assay is characterised as having an ROC AUC of at least 0.851, and more preferably wherein the panel of one or more CpGs comprises at least the CpGs denoted by [[CG]];
95. one or more CpGs within DMR 95 as defined by SEQ ID NO: 595 and denoted by CG, preferably wherein the assay is characterised as having an ROC AUC of at least 0.851, and more preferably wherein the panel of one or more CpGs comprises at least the CpGs denoted by [[CG]];
96. one or more CpGs within DMR 96 as defined by SEQ ID NO: 596 and denoted by CG, preferably wherein the assay is characterised as having an ROC AUC of at least 0.85, and more preferably wherein the panel of one or more CpGs comprises at least the CpGs denoted by [[CG]];
97. one or more CpGs within DMR 97 as defined by SEQ ID NO: 597 and denoted by CG, preferably wherein the assay is characterised as having an ROC AUC of at least 0.849, and more preferably wherein the panel of one or more CpGs comprises at least the CpGs denoted by [[CG]];
98. one or more CpGs within DMR 98 as defined by SEQ ID NO: 598 and denoted by CG, preferably wherein the assay is characterised as having an ROC AUC of at least 0.849, and more preferably wherein the panel of one or more CpGs comprises at least the CpGs denoted by [[CG]];
99. one or more CpGs within DMR 99 as defined by SEQ ID NO: 599 and denoted by CG, preferably wherein the assay is characterised as having an ROC AUC of at least 0.849, and more preferably wherein the panel of one or more CpGs comprises at least the CpGs denoted by [[CG]];
100. one or more CpGs within DMR 100 as defined by SEQ ID NO: 600 and denoted by CG, preferably wherein the assay is characterised as having an ROC AUC of at least 0.849, and more preferably wherein the panel of one or more CpGs comprises at least the CpGs denoted by [[CG]];
101. one or more CpGs within DMR 101 as defined by SEQ ID NO: 601 and denoted by CG, preferably wherein the assay is characterised as having an ROC AUC of at least 0.849, and more preferably wherein the panel of one or more CpGs comprises at least the CpGs denoted by [[CG]];
102. one or more CpGs within DMR 102 as defined by SEQ ID NO: 602 and denoted by CG, preferably wherein the assay is characterised as having an ROC AUC of at least 0.848, and more preferably wherein the panel of one or more CpGs comprises at least the CpGs denoted by [[CG]];
103. one or more CpGs within DMR 103 as defined by SEQ ID NO: 603 and denoted by CG, preferably wherein the assay is characterised as having an ROC AUC of at least 0.847, and more preferably wherein the panel of one or more CpGs comprises at least the CpGs denoted by [[CG]];
104. one or more CpGs within DMR 104 as defined by SEQ ID NO: 604 and denoted by CG, preferably wherein the assay is characterised as having an ROC AUC of at least 0.847, and more preferably wherein the panel of one or more CpGs comprises at least the CpGs denoted by [[CG]];
105. one or more CpGs within DMR 105 as defined by SEQ ID NO: 605 and denoted by CG, preferably wherein the assay is characterised as having an ROC AUC of at least 0.847, and more preferably wherein the panel of one or more CpGs comprises at least the CpGs denoted by [[CG]];
106. one or more CpGs within DMR 106 as defined by SEQ ID NO: 606 and denoted by CG, preferably wherein the assay is characterised as having an ROC AUC of at least 0.847, and more preferably wherein the panel of one or more CpGs comprises at least the CpGs denoted by [[CG]];
107. one or more CpGs within DMR 107 as defined by SEQ ID NO: 607 and denoted by CG, preferably wherein the assay is characterised as having an ROC AUC of at least 0.846, and more preferably wherein the panel of one or more CpGs comprises at least the CpGs denoted by [[CG]];
108. one or more CpGs within DMR 108 as defined by SEQ ID NO: 608 and denoted by CG, preferably wherein the assay is characterised as having an ROC AUC of at least 0.845, and more preferably wherein the panel of one or more CpGs comprises at least the CpGs denoted by [[CG]];
109. one or more CpGs within DMR 109 as defined by SEQ ID NO: 609 and denoted by CG, preferably wherein the assay is characterised as having an ROC AUC of at least 0.845, and more preferably wherein the panel of one or more CpGs comprises at least the CpGs denoted by [[CG]];
110. one or more CpGs within DMR 110 as defined by SEQ ID NO: 610 and denoted by CG, preferably wherein the assay is characterised as having an ROC AUC of at least 0.845, and more preferably wherein the panel of one or more CpGs comprises at least the CpGs denoted by [[CG]];
111. one or more CpGs within DMR 111 as defined by SEQ ID NO: 611 and denoted by CG, preferably wherein the assay is characterised as having an ROC AUC of at least 0.845, and more preferably wherein the panel of one or more CpGs comprises at least the CpGs denoted by [[CG]];
112. one or more CpGs within DMR 112 as defined by SEQ ID NO: 612 and denoted by CG, preferably wherein the assay is characterised as having an ROC AUC of at least 0.844, and more preferably wherein the panel of one or more CpGs comprises at least the CpGs denoted by [[CG]];
113. one or more CpGs within DMR 113 as defined by SEQ ID NO: 613 and denoted by CG, preferably wherein the assay is characterised as having an ROC AUC of at least 0.843, and more preferably wherein the panel of one or more CpGs comprises at least the CpGs denoted by [[CG]];
114. one or more CpGs within DMR 114 as defined by SEQ ID NO: 614 and denoted by CG, preferably wherein the assay is characterised as having an ROC AUC of at least 0.843, and more preferably wherein the panel of one or more CpGs comprises at least the CpGs denoted by [[CG]];
115. one or more CpGs within DMR 115 as defined by SEQ ID NO: 615 and denoted by CG, preferably wherein the assay is characterised as having an ROC AUC of at least 0.842, and more preferably wherein the panel of one or more CpGs comprises at least the CpGs denoted by [[CG]];
116. one or more CpGs within DMR 116 as defined by SEQ ID NO: 616 and denoted by CG, preferably wherein the assay is characterised as having an ROC AUC of at least 0.842, and more preferably wherein the panel of one or more CpGs comprises at least the CpGs denoted by [[CG]];
117. one or more CpGs within DMR 117 as defined by SEQ ID NO: 617 and denoted by CG, preferably wherein the assay is characterised as having an ROC AUC of at least 0.841, and more preferably wherein the panel of one or more CpGs comprises at least the CpGs denoted by [[CG]];
118. one or more CpGs within DMR 118 as defined by SEQ ID NO: 618 and denoted by CG, preferably wherein the assay is characterised as having an ROC AUC of at least 0.841, and more preferably wherein the panel of one or more CpGs comprises at least the CpGs denoted by [[CG]];
119. one or more CpGs within DMR 119 as defined by SEQ ID NO: 619 and denoted by CG, preferably wherein the assay is characterised as having an ROC AUC of at least 0.841, and more preferably wherein the panel of one or more CpGs comprises at least the CpGs denoted by [[CG]];
120. one or more CpGs within DMR 120 as defined by SEQ ID NO: 620 and denoted by CG, preferably wherein the assay is characterised as having an ROC AUC of at least 0.84, and more preferably wherein the panel of one or more CpGs comprises at least the CpGs denoted by [[CG]];
121. one or more CpGs within DMR 121 as defined by SEQ ID NO: 621 and denoted by CG, preferably wherein the assay is characterised as having an ROC AUC of at least 0.839, and more preferably wherein the panel of one or more CpGs comprises at least the CpGs denoted by [[CG]];
122. one or more CpGs within DMR 122 as defined by SEQ ID NO: 622 and denoted by CG, preferably wherein the assay is characterised as having an ROC AUC of at least 0.839, and more preferably wherein the panel of one or more CpGs comprises at least the CpGs denoted by [[CG]];
123. one or more CpGs within DMR 123 as defined by SEQ ID NO: 623 and denoted by CG, preferably wherein the assay is characterised as having an ROC AUC of at least 0.838, and more preferably wherein the panel of one or more CpGs comprises at least the CpGs denoted by [[CG]];
124. one or more CpGs within DMR 124 as defined by SEQ ID NO: 624 and denoted by CG, preferably wherein the assay is characterised as having an ROC AUC of at least 0.838, and more preferably wherein the panel of one or more CpGs comprises at least the CpGs denoted by [[CG]];
125. one or more CpGs within DMR 125 as defined by SEQ ID NO: 625 and denoted by CG, preferably wherein the assay is characterised as having an ROC AUC of at least 0.838, and more preferably wherein the panel of one or more CpGs comprises at least the CpGs denoted by [[CG]];
126. one or more CpGs within DMR 126 as defined by SEQ ID NO: 626 and denoted by CG, preferably wherein the assay is characterised as having an ROC AUC of at least 0.835, and more preferably wherein the panel of one or more CpGs comprises at least the CpGs denoted by [[CG]];
127. one or more CpGs within DMR 127 as defined by SEQ ID NO: 627 and denoted by CG, preferably wherein the assay is characterised as having an ROC AUC of at least 0.835, and more preferably wherein the panel of one or more CpGs comprises at least the CpGs denoted by [[CG]];
128. one or more CpGs within DMR 128 as defined by SEQ ID NO: 628 and denoted by CG, preferably wherein the assay is characterised as having an ROC AUC of at least 0.833, and more preferably wherein the panel of one or more CpGs comprises at least the CpGs denoted by [[CG]];
129. one or more CpGs within DMR 129 as defined by SEQ ID NO: 629 and denoted by CG, preferably wherein the assay is characterised as having an ROC AUC of at least 0.832, and more preferably wherein the panel of one or more CpGs comprises at least the CpGs denoted by [[CG]];
130. one or more CpGs within DMR 130 as defined by SEQ ID NO: 630 and denoted by CG, preferably wherein the assay is characterised as having an ROC AUC of at least 0.832, and more preferably wherein the panel of one or more CpGs comprises at least the CpGs denoted by [[CG]];
131. one or more CpGs within DMR 131 as defined by SEQ ID NO: 631 and denoted by CG, preferably wherein the assay is characterised as having an ROC AUC of at least 0.832, and more preferably wherein the panel of one or more CpGs comprises at least the CpGs denoted by [[CG]];
132. one or more CpGs within DMR 132 as defined by SEQ ID NO: 632 and denoted by CG, preferably wherein the assay is characterised as having an ROC AUC of at least 0.831, and more preferably wherein the panel of one or more CpGs comprises at least the CpGs denoted by [[CG]];
133. one or more CpGs within DMR 133 as defined by SEQ ID NO: 633 and denoted by CG, preferably wherein the assay is characterised as having an ROC AUC of at least 0.831, and more preferably wherein the panel of one or more CpGs comprises at least the CpGs denoted by [[CG]];
134. one or more CpGs within DMR 134 as defined by SEQ ID NO: 634 and denoted by CG, preferably wherein the assay is characterised as having an ROC AUC of at least 0.83, and more preferably wherein the panel of one or more CpGs comprises at least the CpGs denoted by [[CG]];
135. one or more CpGs within DMR 135 as defined by SEQ ID NO: 635 and denoted by CG, preferably wherein the assay is characterised as having an ROC AUC of at least 0.83, and more preferably wherein the panel of one or more CpGs comprises at least the CpGs denoted by [[CG]];
136. one or more CpGs within DMR 136 as defined by SEQ ID NO: 636 and denoted by CG, preferably wherein the assay is characterised as having an ROC AUC of at least 0.83, and more preferably wherein the panel of one or more CpGs comprises at least the CpGs denoted by [[CG]];
137. one or more CpGs within DMR 137 as defined by SEQ ID NO: 637 and denoted by CG, preferably wherein the assay is characterised as having an ROC AUC of at least 0.829, and more preferably wherein the panel of one or more CpGs comprises at least the CpGs denoted by [[CG]];
138. one or more CpGs within DMR 138 as defined by SEQ ID NO: 638 and denoted by CG, preferably wherein the assay is characterised as having an ROC AUC of at least 0.829, and more preferably wherein the panel of one or more CpGs comprises at least the CpGs denoted by [[CG]];
139. one or more CpGs within DMR 139 as defined by SEQ ID NO: 639 and denoted by CG, preferably wherein the assay is characterised as having an ROC AUC of at least 0.829, and more preferably wherein the panel of one or more CpGs comprises at least the CpGs denoted by [[CG]];
140. one or more CpGs within DMR 140 as defined by SEQ ID NO: 640 and denoted by CG, preferably wherein the assay is characterised as having an ROC AUC of at least 0.829, and more preferably wherein the panel of one or more CpGs comprises at least the CpGs denoted by [[CG]];
141. one or more CpGs within DMR 141 as defined by SEQ ID NO: 641 and denoted by CG, preferably wherein the assay is characterised as having an ROC AUC of at least 0.828, and more preferably wherein the panel of one or more CpGs comprises at least the CpGs denoted by [[CG]];
142. one or more CpGs within DMR 142 as defined by SEQ ID NO: 642 and denoted by CG, preferably wherein the assay is characterised as having an ROC AUC of at least 0.828, and more preferably wherein the panel of one or more CpGs comprises at least the CpGs denoted by [[CG]];
143. one or more CpGs within DMR 143 as defined by SEQ ID NO: 643 and denoted by CG, preferably wherein the assay is characterised as having an ROC AUC of at least 0.828, and more preferably wherein the panel of one or more CpGs comprises at least the CpGs denoted by [[CG]];
144. one or more CpGs within DMR 144 as defined by SEQ ID NO: 644 and denoted by CG, preferably wherein the assay is characterised as having an ROC AUC of at least 0.828, and more preferably wherein the panel of one or more CpGs comprises at least the CpGs denoted by [[CG]];
145. one or more CpGs within DMR 145 as defined by SEQ ID NO: 645 and denoted by CG, preferably wherein the assay is characterised as having an ROC AUC of at least 0.828, and more preferably wherein the panel of one or more CpGs comprises at least the CpGs denoted by [[CG]];
146. one or more CpGs within DMR 146 as defined by SEQ ID NO: 646 and denoted by CG, preferably wherein the assay is characterised as having an ROC AUC of at least 0.825, and more preferably wherein the panel of one or more CpGs comprises at least the CpGs denoted by [[CG]];
147. one or more CpGs within DMR 147 as defined by SEQ ID NO: 647 and denoted by CG, preferably wherein the assay is characterised as having an ROC AUC of at least 0.825, and more preferably wherein the panel of one or more CpGs comprises at least the CpGs denoted by [[CG]];
148. one or more CpGs within DMR 148 as defined by SEQ ID NO: 648 and denoted by CG, preferably wherein the assay is characterised as having an ROC AUC of at least 0.824, and more preferably wherein the panel of one or more CpGs comprises at least the CpGs denoted by [[CG]];
149. one or more CpGs within DMR 149 as defined by SEQ ID NO: 649 and denoted by CG, preferably wherein the assay is characterised as having an ROC AUC of at least 0.824, and more preferably wherein the panel of one or more CpGs comprises at least the CpGs denoted by [[CG]];
150. one or more CpGs within DMR 150 as defined by SEQ ID NO: 650 and denoted by CG, preferably wherein the assay is characterised as having an ROC AUC of at least 0.824, and more preferably wherein the panel of one or more CpGs comprises at least the CpGs denoted by [[CG]];
151. one or more CpGs within DMR 151 as defined by SEQ ID NO: 651 and denoted by CG, preferably wherein the assay is characterised as having an ROC AUC of at least 0.824, and more preferably wherein the panel of one or more CpGs comprises at least the CpGs denoted by [[CG]];
152. one or more CpGs within DMR 152 as defined by SEQ ID NO: 652 and denoted by CG, preferably wherein the assay is characterised as having an ROC AUC of at least 0.823, and more preferably wherein the panel of one or more CpGs comprises at least the CpGs denoted by [[CG]];
153. one or more CpGs within DMR 153 as defined by SEQ ID NO: 653 and denoted by CG, preferably wherein the assay is characterised as having an ROC AUC of at least 0.82, and more preferably wherein the panel of one or more CpGs comprises at least the CpGs denoted by [[CG]];
154. one or more CpGs within DMR 154 as defined by SEQ ID NO: 654 and denoted by CG, preferably wherein the assay is characterised as having an ROC AUC of at least 0.82, and more preferably wherein the panel of one or more CpGs comprises at least the CpGs denoted by [[CG]];
155. one or more CpGs within DMR 155 as defined by SEQ ID NO: 655 and denoted by CG, preferably wherein the assay is characterised as having an ROC AUC of at least 0.819, and more preferably wherein the panel of one or more CpGs comprises at least the CpGs denoted by [[CG]];
156. one or more CpGs within DMR 156 as defined by SEQ ID NO: 656 and denoted by CG, preferably wherein the assay is characterised as having an ROC AUC of at least 0.816, and more preferably wherein the panel of one or more CpGs comprises at least the CpGs denoted by [[CG]];
157. one or more CpGs within DMR 157 as defined by SEQ ID NO: 657 and denoted by CG, preferably wherein the assay is characterised as having an ROC AUC of at least 0.816, and more preferably wherein the panel of one or more CpGs comprises at least the CpGs denoted by [[CG]];
158. one or more CpGs within DMR 158 as defined by SEQ ID NO: 658 and denoted by CG, preferably wherein the assay is characterised as having an ROC AUC of at least 0.814, and more preferably wherein the panel of one or more CpGs comprises at least the CpGs denoted by [[CG]];
159. one or more CpGs within DMR 159 as defined by SEQ ID NO: 659 and denoted by CG, preferably wherein the assay is characterised as having an ROC AUC of at least 0.814, and more preferably wherein the panel of one or more CpGs comprises at least the CpGs denoted by [[CG]];
160. one or more CpGs within DMR 160 as defined by SEQ ID NO: 660 and denoted by CG, preferably wherein the assay is characterised as having an ROC AUC of at least 0.814, and more preferably wherein the panel of one or more CpGs comprises at least the CpGs denoted by [[CG]];
161. one or more CpGs within DMR 161 as defined by SEQ ID NO: 661 and denoted by CG, preferably wherein the assay is characterised as having an ROC AUC of at least 0.813, and more preferably wherein the panel of one or more CpGs comprises at least the CpGs denoted by [[CG]];
162. one or more CpGs within DMR 162 as defined by SEQ ID NO: 662 and denoted by CG, preferably wherein the assay is characterised as having an ROC AUC of at least 0.812, and more preferably wherein the panel of one or more CpGs comprises at least the CpGs denoted by [[CG]];
163. one or more CpGs within DMR 163 as defined by SEQ ID NO: 663 and denoted by CG, preferably wherein the assay is characterised as having an ROC AUC of at least 0.811, and more preferably wherein the panel of one or more CpGs comprises at least the CpGs denoted by [[CG]];
164. one or more CpGs within DMR 164 as defined by SEQ ID NO: 664 and denoted by CG, preferably wherein the assay is characterised as having an ROC AUC of at least 0.811, and more preferably wherein the panel of one or more CpGs comprises at least the CpGs denoted by [[CG]];
165. one or more CpGs within DMR 165 as defined by SEQ ID NO: 665 and denoted by CG, preferably wherein the assay is characterised as having an ROC AUC of at least 0.81, and more preferably wherein the panel of one or more CpGs comprises at least the CpGs denoted by [[CG]];
166. one or more CpGs within DMR 166 as defined by SEQ ID NO: 666 and denoted by CG, preferably wherein the assay is characterised as having an ROC AUC of at least 0.809, and more preferably wherein the panel of one or more CpGs comprises at least the CpGs denoted by [[CG]];
167. one or more CpGs within DMR 167 as defined by SEQ ID NO: 667 and denoted by CG, preferably wherein the assay is characterised as having an ROC AUC of at least 0.808, and more preferably wherein the panel of one or more CpGs comprises at least the CpGs denoted by [[CG]];
168. one or more CpGs within DMR 168 as defined by SEQ ID NO: 668 and denoted by CG, preferably wherein the assay is characterised as having an ROC AUC of at least 0.807, and more preferably wherein the panel of one or more CpGs comprises at least the CpGs denoted by [[CG]];
169. one or more CpGs within DMR 169 as defined by SEQ ID NO: 669 and denoted by CG, preferably wherein the assay is characterised as having an ROC AUC of at least 0.807, and more preferably wherein the panel of one or more CpGs comprises at least the CpGs denoted by [[CG]];
170. one or more CpGs within DMR 170 as defined by SEQ ID NO: 670 and denoted by CG, preferably wherein the assay is characterised as having an ROC AUC of at least 0.806, and more preferably wherein the panel of one or more CpGs comprises at least the CpGs denoted by [[CG]];
171. one or more CpGs within DMR 171 as defined by SEQ ID NO: 671 and denoted by CG, preferably wherein the assay is characterised as having an ROC AUC of at least 0.806, and more preferably wherein the panel of one or more CpGs comprises at least the CpGs denoted by [[CG]];
172. one or more CpGs within DMR 172 as defined by SEQ ID NO: 672 and denoted by CG, preferably wherein the assay is characterised as having an ROC AUC of at least 0.805, and more preferably wherein the panel of one or more CpGs comprises at least the CpGs denoted by [[CG]];
173. one or more CpGs within DMR 173 as defined by SEQ ID NO: 673 and denoted by CG, preferably wherein the assay is characterised as having an ROC AUC of at least 0.805, and more preferably wherein the panel of one or more CpGs comprises at least the CpGs denoted by [[CG]];
174. one or more CpGs within DMR 174 as defined by SEQ ID NO: 674 and denoted by CG, preferably wherein the assay is characterised as having an ROC AUC of at least 0.803, and more preferably wherein the panel of one or more CpGs comprises at least the CpGs denoted by [[CG]];
175. one or more CpGs within DMR 175 as defined by SEQ ID NO: 675 and denoted by CG, preferably wherein the assay is characterised as having an ROC AUC of at least 0.803, and more preferably wherein the panel of one or more CpGs comprises at least the CpGs denoted by [[CG]];
176. one or more CpGs within DMR 176 as defined by SEQ ID NO: 676 and denoted by CG, preferably wherein the assay is characterised as having an ROC AUC of at least 0.801, and more preferably wherein the panel of one or more CpGs comprises at least the CpGs denoted by [[CG]];
177. one or more CpGs within DMR 177 as defined by SEQ ID NO: 677 and denoted by CG, preferably wherein the assay is characterised as having an ROC AUC of at least 0.799, and more preferably wherein the panel of one or more CpGs comprises at least the CpGs denoted by [[CG]];
178. one or more CpGs within DMR 178 as defined by SEQ ID NO: 678 and denoted by CG, preferably wherein the assay is characterised as having an ROC AUC of at least 0.799, and more preferably wherein the panel of one or more CpGs comprises at least the CpGs denoted by [[CG]];
179. one or more CpGs within DMR 179 as defined by SEQ ID NO: 679 and denoted by CG, preferably wherein the assay is characterised as having an ROC AUC of at least 0.799, and more preferably wherein the panel of one or more CpGs comprises at least the CpGs denoted by [[CG]];
180. one or more CpGs within DMR 180 as defined by SEQ ID NO: 680 and denoted by CG, preferably wherein the assay is characterised as having an ROC AUC of at least 0.799, and more preferably wherein the panel of one or more CpGs comprises at least the CpGs denoted by [[CG]];
181. one or more CpGs within DMR 181 as defined by SEQ ID NO: 681 and denoted by CG, preferably wherein the assay is characterised as having an ROC AUC of at least 0.798, and more preferably wherein the panel of one or more CpGs comprises at least the CpGs denoted by [[CG]];
182. one or more CpGs within DMR 182 as defined by SEQ ID NO: 682 and denoted by CG, preferably wherein the assay is characterised as having an ROC AUC of at least 0.797, and more preferably wherein the panel of one or more CpGs comprises at least the CpGs denoted by [[CG]];
183. one or more CpGs within DMR 183 as defined by SEQ ID NO: 683 and denoted by CG, preferably wherein the assay is characterised as having an ROC AUC of at least 0.796, and more preferably wherein the panel of one or more CpGs comprises at least the CpGs denoted by [[CG]];
184. one or more CpGs within DMR 184 as defined by SEQ ID NO: 684 and denoted by CG, preferably wherein the assay is characterised as having an ROC AUC of at least 0.793, and more preferably wherein the panel of one or more CpGs comprises at least the CpGs denoted by [[CG]];
185. one or more CpGs within DMR 185 as defined by SEQ ID NO: 685 and denoted by CG, preferably wherein the assay is characterised as having an ROC AUC of at least 0.792, and more preferably wherein the panel of one or more CpGs comprises at least the CpGs denoted by [[CG]];
186. one or more CpGs within DMR 186 as defined by SEQ ID NO: 686 and denoted by CG, preferably wherein the assay is characterised as having an ROC AUC of at least 0.792, and more preferably wherein the panel of one or more CpGs comprises at least the CpGs denoted by [[CG]];
187. one or more CpGs within DMR 187 as defined by SEQ ID NO: 687 and denoted by CG, preferably wherein the assay is characterised as having an ROC AUC of at least 0.791, and more preferably wherein the panel of one or more CpGs comprises at least the CpGs denoted by [[CG]];
188. one or more CpGs within DMR 188 as defined by SEQ ID NO: 688 and denoted by CG, preferably wherein the assay is characterised as having an ROC AUC of at least 0.79, and more preferably wherein the panel of one or more CpGs comprises at least the CpGs denoted by [[CG]];
189. one or more CpGs within DMR 189 as defined by SEQ ID NO: 689 and denoted by CG, preferably wherein the assay is characterised as having an ROC AUC of at least 0.79, and more preferably wherein the panel of one or more CpGs comprises at least the CpGs denoted by [[CG]];
190. one or more CpGs within DMR 190 as defined by SEQ ID NO: 690 and denoted by CG, preferably wherein the assay is characterised as having an ROC AUC of at least 0.789, and more preferably wherein the panel of one or more CpGs comprises at least the CpGs denoted by [[CG]];
191. one or more CpGs within DMR 191 as defined by SEQ ID NO: 691 and denoted by CG, preferably wherein the assay is characterised as having an ROC AUC of at least 0.788, and more preferably wherein the panel of one or more CpGs comprises at least the CpGs denoted by [[CG]];
192. one or more CpGs within DMR 192 as defined by SEQ ID NO: 692 and denoted by CG, preferably wherein the assay is characterised as having an ROC AUC of at least 0.788, and more preferably wherein the panel of one or more CpGs comprises at least the CpGs denoted by [[CG]];
193. one or more CpGs within DMR 193 as defined by SEQ ID NO: 693 and denoted by CG, preferably wherein the assay is characterised as having an ROC AUC of at least 0.787, and more preferably wherein the panel of one or more CpGs comprises at least the CpGs denoted by [[CG]];
194. one or more CpGs within DMR 194 as defined by SEQ ID NO: 694 and denoted by CG, preferably wherein the assay is characterised as having an ROC AUC of at least 0.787, and more preferably wherein the panel of one or more CpGs comprises at least the CpGs denoted by [[CG]];
195. one or more CpGs within DMR 195 as defined by SEQ ID NO: 695 and denoted by CG, preferably wherein the assay is characterised as having an ROC AUC of at least 0.787, and more preferably wherein the panel of one or more CpGs comprises at least the CpGs denoted by [[CG]];
196. one or more CpGs within DMR 196 as defined by SEQ ID NO: 696 and denoted by CG, preferably wherein the assay is characterised as having an ROC AUC of at least 0.784, and more preferably wherein the panel of one or more CpGs comprises at least the CpGs denoted by [[CG]];
197. one or more CpGs within DMR 197 as defined by SEQ ID NO: 697 and denoted by CG, preferably wherein the assay is characterised as having an ROC AUC of at least 0.784, and more preferably wherein the panel of one or more CpGs comprises at least the CpGs denoted by [[CG]];
198. one or more CpGs within DMR 198 as defined by SEQ ID NO: 698 and denoted by CG, preferably wherein the assay is characterised as having an ROC AUC of at least 0.783, and more preferably wherein the panel of one or more CpGs comprises at least the CpGs denoted by [[CG]];
199. one or more CpGs within DMR 199 as defined by SEQ ID NO: 699 and denoted by CG, preferably wherein the assay is characterised as having an ROC AUC of at least 0.781, and more preferably wherein the panel of one or more CpGs comprises at least the CpGs denoted by [[CG]];
200. one or more CpGs within DMR 200 as defined by SEQ ID NO: 700 and denoted by CG, preferably wherein the assay is characterised as having an ROC AUC of at least 0.78, and more preferably wherein the panel of one or more CpGs comprises at least the CpGs denoted by [[CG]];
201. one or more CpGs within DMR 201 as defined by SEQ ID NO: 701 and denoted by CG, preferably wherein the assay is characterised as having an ROC AUC of at least 0.777, and more preferably wherein the panel of one or more CpGs comprises at least the CpGs denoted by [[CG]];
202. one or more CpGs within DMR 202 as defined by SEQ ID NO: 702 and denoted by CG, preferably wherein the assay is characterised as having an ROC AUC of at least 0.777, and more preferably wherein the panel of one or more CpGs comprises at least the CpGs denoted by [[CG]];
203. one or more CpGs within DMR 203 as defined by SEQ ID NO: 703 and denoted by CG, preferably wherein the assay is characterised as having an ROC AUC of at least 0.777, and more preferably wherein the panel of one or more CpGs comprises at least the CpGs denoted by [[CG]];
204. one or more CpGs within DMR 204 as defined by SEQ ID NO: 704 and denoted by CG, preferably wherein the assay is characterised as having an ROC AUC of at least 0.777, and more preferably wherein the panel of one or more CpGs comprises at least the CpGs denoted by [[CG]];
205. one or more CpGs within DMR 205 as defined by SEQ ID NO: 705 and denoted by CG, preferably wherein the assay is characterised as having an ROC AUC of at least 0.777, and more preferably wherein the panel of one or more CpGs comprises at least the CpGs denoted by [[CG]];
206. one or more CpGs within DMR 206 as defined by SEQ ID NO: 706 and denoted by CG, preferably wherein the assay is characterised as having an ROC AUC of at least 0.777, and more preferably wherein the panel of one or more CpGs comprises at least the CpGs denoted by [[CG]];
207. one or more CpGs within DMR 207 as defined by SEQ ID NO: 707 and denoted by CG, preferably wherein the assay is characterised as having an ROC AUC of at least 0.777, and more preferably wherein the panel of one or more CpGs comprises at least the CpGs denoted by [[CG]];
208. one or more CpGs within DMR 208 as defined by SEQ ID NO: 708 and denoted by CG, preferably wherein the assay is characterised as having an ROC AUC of at least 0.776, and more preferably wherein the panel of one or more CpGs comprises at least the CpGs denoted by [[CG]];
209. one or more CpGs within DMR 209 as defined by SEQ ID NO: 209 and denoted by CG, preferably wherein the assay is characterised as having an ROC AUC of at least 0.776, and more preferably wherein the panel of one or more CpGs comprises at least the CpGs denoted by [[CG]];
210. one or more CpGs within DMR 210 as defined by SEQ ID NO: 710 and denoted by CG, preferably wherein the assay is characterised as having an ROC AUC of at least 0.776, and more preferably wherein the panel of one or more CpGs comprises at least the CpGs denoted by [[CG]];
211. one or more CpGs within DMR 211 as defined by SEQ ID NO: 711 and denoted by CG, preferably wherein the assay is characterised as having an ROC AUC of at least 0.775, and more preferably wherein the panel of one or more CpGs comprises at least the CpGs denoted by [[CG]];
212. one or more CpGs within DMR 212 as defined by SEQ ID NO: 712 and denoted by CG, preferably wherein the assay is characterised as having an ROC AUC of at least 0.775, and more preferably wherein the panel of one or more CpGs comprises at least the CpGs denoted by [[CG]];
213. one or more CpGs within DMR 213 as defined by SEQ ID NO: 713 and denoted by CG, preferably wherein the assay is characterised as having an ROC AUC of at least 0.775, and more preferably wherein the panel of one or more CpGs comprises at least the CpGs denoted by [[CG]];
214. one or more CpGs within DMR 214 as defined by SEQ ID NO: 714 and denoted by CG, preferably wherein the assay is characterised as having an ROC AUC of at least 0.771, and more preferably wherein the panel of one or more CpGs comprises at least the CpGs denoted by [[CG]];
215. one or more CpGs within DMR 215 as defined by SEQ ID NO: 715 and denoted by CG, preferably wherein the assay is characterised as having an ROC AUC of at least 0.769, and more preferably wherein the panel of one or more CpGs comprises at least the CpGs denoted by [[CG]];
216. one or more CpGs within DMR 216 as defined by SEQ ID NO: 716 and denoted by CG, preferably wherein the assay is characterised as having an ROC AUC of at least 0.767, and more preferably wherein the panel of one or more CpGs comprises at least the CpGs denoted by [[CG]];
217. one or more CpGs within DMR 217 as defined by SEQ ID NO: 717 and denoted by CG, preferably wherein the assay is characterised as having an ROC AUC of at least 0.762, and more preferably wherein the panel of one or more CpGs comprises at least the CpGs denoted by [[CG]];
218. one or more CpGs within DMR 218 as defined by SEQ ID NO: 718 and denoted by CG, preferably wherein the assay is characterised as having an ROC AUC of at least 0.76, and more preferably wherein the panel of one or more CpGs comprises at least the CpGs denoted by [[CG]];
219. one or more CpGs within DMR 219 as defined by SEQ ID NO: 719 and denoted by CG, preferably wherein the assay is characterised as having an ROC AUC of at least 0.758, and more preferably wherein the panel of one or more CpGs comprises at least the CpGs denoted by [[CG]];
220. one or more CpGs within DMR 220 as defined by SEQ ID NO: 720 and denoted by CG, preferably wherein the assay is characterised as having an ROC AUC of at least 0.757, and more preferably wherein the panel of one or more CpGs comprises at least the CpGs denoted by [[CG]];
221. one or more CpGs within DMR 221 as defined by SEQ ID NO: 721 and denoted by CG, preferably wherein the assay is characterised as having an ROC AUC of at least 0.751, and more preferably wherein the panel of one or more CpGs comprises at least the CpGs denoted by [[CG]];
222. one or more CpGs within DMR 222 as defined by SEQ ID NO: 722 and denoted by CG, preferably wherein the assay is characterised as having an ROC AUC of at least 0.742, and more preferably wherein the panel of one or more CpGs comprises at least the CpGs denoted by [[CG]];
223. one or more CpGs within DMR 223 as defined by SEQ ID NO: 723 and denoted by CG, preferably wherein the assay is characterised as having an ROC AUC of at least 0.738, and more preferably wherein the panel of one or more CpGs comprises at least the CpGs denoted by [[CG]];
224. one or more CpGs within DMR 224 as defined by SEQ ID NO: 724 and denoted by CG, preferably wherein the assay is characterised as having an ROC AUC of at least 0.73, and more preferably wherein the panel of one or more CpGs comprises at least the CpGs denoted by [[CG]];
225. one or more CpGs within DMR 225 as defined by SEQ ID NO: 725 and denoted by CG, preferably wherein the assay is characterised as having an ROC AUC of at least 0.726, and more preferably wherein the panel of one or more CpGs comprises at least the CpGs denoted by [[CG]];
226. one or more CpGs within DMR 226 as defined by SEQ ID NO: 726 and denoted by CG, preferably wherein the assay is characterised as having an ROC AUC of at least 0.726, and more preferably wherein the panel of one or more CpGs comprises at least the CpGs denoted by [[CG]];
227. one or more CpGs within DMR 227 as defined by SEQ ID NO: 727 and denoted by CG, preferably wherein the assay is characterised as having an ROC AUC of at least 0.72, and more preferably wherein the panel of one or more CpGs comprises at least the CpGs denoted by [[CG]];
228. one or more CpGs within DMR 228 as defined by SEQ ID NO: 728 and denoted by CG, preferably wherein the assay is characterised as having an ROC AUC of at least 0.715, and more preferably wherein the panel of one or more CpGs comprises at least the CpGs denoted by [[CG]];
229. one or more CpGs within DMR 229 as defined by SEQ ID NO: 729 and denoted by CG, preferably wherein the assay is characterised as having an ROC AUC of at least 0.713, and more preferably wherein the panel of one or more CpGs comprises at least the CpGs denoted by [[CG]];
230. one or more CpGs within DMR 230 as defined by SEQ ID NO: 730 and denoted by CG, preferably wherein the assay is characterised as having an ROC AUC of at least 0.713, and more preferably wherein the panel of one or more CpGs comprises at least the CpGs denoted by [[CG]];
231. one or more CpGs within DMR 231 as defined by SEQ ID NO: 731 and denoted by CG, preferably wherein the assay is characterised as having an ROC AUC of at least 0.708, and more preferably wherein the panel of one or more CpGs comprises at least the CpGs denoted by [[CG]];
232. one or more CpGs within DMR 232 as defined by SEQ ID NO: 732 and denoted by CG, preferably wherein the assay is characterised as having an ROC AUC of at least 0.707, and more preferably wherein the panel of one or more CpGs comprises at least the CpGs denoted by [[CG]];
233. one or more CpGs within DMR 233 as defined by SEQ ID NO: 733 and denoted by CG, preferably wherein the assay is characterised as having an ROC AUC of at least 0.704, and more preferably wherein the panel of one or more CpGs comprises at least the CpGs denoted by [[CG]];
234. one or more CpGs within DMR 234 as defined by SEQ ID NO: 734 and denoted by CG, preferably wherein the assay is characterised as having an ROC AUC of at least 0.697, and more preferably wherein the panel of one or more CpGs comprises at least the CpGs denoted by [[CG]];
235. one or more CpGs within DMR 235 as defined by SEQ ID NO: 735 and denoted by CG, preferably wherein the assay is characterised as having an ROC AUC of at least 0.697, and more preferably wherein the panel of one or more CpGs comprises at least the CpGs denoted by [[CG]];
236. one or more CpGs within DMR 236 as defined by SEQ ID NO: 736 and denoted by CG, preferably wherein the assay is characterised as having an ROC AUC of at least 0.693, and more preferably wherein the panel of one or more CpGs comprises at least the CpGs denoted by [[CG]];
237. one or more CpGs within DMR 237 as defined by SEQ ID NO: 737 and denoted by CG, preferably wherein the assay is characterised as having an ROC AUC of at least 0.638, and more preferably wherein the panel of one or more CpGs comprises at least the CpGs denoted by [[CG]];
238. one or more CpGs within DMR 238 as defined by SEQ ID NO: 738 and denoted by CG, preferably wherein the assay is characterised as having an ROC AUC of at least 0.903, and more preferably wherein the panel of one or more CpGs comprises at least the CpGs denoted by [[CG]];
239. one or more CpGs within DMR 239 as defined by SEQ ID NO: 739 and denoted by CG, preferably wherein the assay is characterised as having an ROC AUC of at least 0.903, and more preferably wherein the panel of one or more CpGs comprises at least the CpGs denoted by [[CG]];
240. one or more CpGs within DMR 240 as defined by SEQ ID NO: 740 and denoted by CG, preferably wherein the assay is characterised as having an ROC AUC of at least 0.899, and more preferably wherein the panel of one or more CpGs comprises at least the CpGs denoted by [[CG]];
241. one or more CpGs within DMR 241 as defined by SEQ ID NO: 741 and denoted by CG, preferably wherein the assay is characterised as having an ROC AUC of at least 0.899, and more preferably wherein the panel of one or more CpGs comprises at least the CpGs denoted by [[CG]];
242. one or more CpGs within DMR 242 as defined by SEQ ID NO: 742 and denoted by CG, preferably wherein the assay is characterised as having an ROC AUC of at least 0.899, and more preferably wherein the panel of one or more CpGs comprises at least the CpGs denoted by [[CG]];
243. one or more CpGs within DMR 243 as defined by SEQ ID NO: 743 and denoted by CG, preferably wherein the assay is characterised as having an ROC AUC of at least 0.899, and more preferably wherein the panel of one or more CpGs comprises at least the CpGs denoted by [[CG]];
244. one or more CpGs within DMR 244 as defined by SEQ ID NO: 744 and denoted by CG, preferably wherein the assay is characterised as having an ROC AUC of at least 0.899, and more preferably wherein the panel of one or more CpGs comprises at least the CpGs denoted by [[CG]];
245. one or more CpGs within DMR 245 as defined by SEQ ID NO: 745 and denoted by CG, preferably wherein the assay is characterised as having an ROC AUC of at least 0.899, and more preferably wherein the panel of one or more CpGs comprises at least the CpGs denoted by [[CG]];
246. one or more CpGs within DMR 246 as defined by SEQ ID NO: 746 and denoted by CG, preferably wherein the assay is characterised as having an ROC AUC of at least 0.895, and more preferably wherein the panel of one or more CpGs comprises at least the CpGs denoted by [[CG]];
247. one or more CpGs within DMR 247 as defined by SEQ ID NO: 747 and denoted by CG, preferably wherein the assay is characterised as having an ROC AUC of at least 0.895, and more preferably wherein the panel of one or more CpGs comprises at least the CpGs denoted by [[CG]];
248. one or more CpGs within DMR 248 as defined by SEQ ID NO: 748 and denoted by CG, preferably wherein the assay is characterised as having an ROC AUC of at least 0.893, and more preferably wherein the panel of one or more CpGs comprises at least the CpGs denoted by [[CG]];
249. one or more CpGs within DMR 249 as defined by SEQ ID NO: 749 and denoted by CG, preferably wherein the assay is characterised as having an ROC AUC of at least 0.893, and more preferably wherein the panel of one or more CpGs comprises at least the CpGs denoted by [[CG]];
250. one or more CpGs within DMR 250 as defined by SEQ ID NO: 750 and denoted by CG, preferably wherein the assay is characterised as having an ROC AUC of at least 0.891, and more preferably wherein the panel of one or more CpGs comprises at least the CpGs denoted by [[CG]];
251. one or more CpGs within DMR 251 as defined by SEQ ID NO: 751 and denoted by CG, preferably wherein the assay is characterised as having an ROC AUC of at least 0.891, and more preferably wherein the panel of one or more CpGs comprises at least the CpGs denoted by [[CG]];
252. one or more CpGs within DMR 252 as defined by SEQ ID NO: 752 and denoted by CG, preferably wherein the assay is characterised as having an ROC AUC of at least 0.886, and more preferably wherein the panel of one or more CpGs comprises at least the CpGs denoted by [[CG]];
253. one or more CpGs within DMR 253 as defined by SEQ ID NO: 753 and denoted by CG, preferably wherein the assay is characterised as having an ROC AUC of at least 0.886, and more preferably wherein the panel of one or more CpGs comprises at least the CpGs denoted by [[CG]];
254. one or more CpGs within DMR 254 as defined by SEQ ID NO: 754 and denoted by CG, preferably wherein the assay is characterised as having an ROC AUC of at least 0.886, and more preferably wherein the panel of one or more CpGs comprises at least the CpGs denoted by [[CG]];
255. one or more CpGs within DMR 255 as defined by SEQ ID NO: 755 and denoted by CG, preferably wherein the assay is characterised as having an ROC AUC of at least 0.886, and more preferably wherein the panel of one or more CpGs comprises at least the CpGs denoted by [[CG]];
256. one or more CpGs within DMR 256 as defined by SEQ ID NO: 756 and denoted by CG, preferably wherein the assay is characterised as having an ROC AUC of at least 0.878, and more preferably wherein the panel of one or more CpGs comprises at least the CpGs denoted by [[CG]];
257. one or more CpGs within DMR 257 as defined by SEQ ID NO: 757 and denoted by CG, preferably wherein the assay is characterised as having an ROC AUC of at least 0.878, and more preferably wherein the panel of one or more CpGs comprises at least the CpGs denoted by [[CG]];
258. one or more CpGs within DMR 258 as defined by SEQ ID NO: 758 and denoted by CG, preferably wherein the assay is characterised as having an ROC AUC of at least 0.875, and more preferably wherein the panel of one or more CpGs comprises at least the CpGs denoted by [[CG]];
259. one or more CpGs within DMR 259 as defined by SEQ ID NO: 759 and denoted by CG, preferably wherein the assay is characterised as having an ROC AUC of at least 0.875, and more preferably wherein the panel of one or more CpGs comprises at least the CpGs denoted by [[CG]];
260. one or more CpGs within DMR 260 as defined by SEQ ID NO: 760 and denoted by CG, preferably wherein the assay is characterised as having an ROC AUC of at least 0.874, and more preferably wherein the panel of one or more CpGs comprises at least the CpGs denoted by [[CG]];
261. one or more CpGs within DMR 261 as defined by SEQ ID NO: 761 and denoted by CG, preferably wherein the assay is characterised as having an ROC AUC of at least 0.874, and more preferably wherein the panel of one or more CpGs comprises at least the CpGs denoted by [[CG]];
262. one or more CpGs within DMR 262 as defined by SEQ ID NO: 762 and denoted by CG, preferably wherein the assay is characterised as having an ROC AUC of at least 0.865, and more preferably wherein the panel of one or more CpGs comprises at least the CpGs denoted by [[CG]];
263. one or more CpGs within DMR 263 as defined by SEQ ID NO: 763 and denoted by CG, preferably wherein the assay is characterised as having an ROC AUC of at least 0.865, and more preferably wherein the panel of one or more CpGs comprises at least the CpGs denoted by [[CG]];
264. one or more CpGs within DMR 264 as defined by SEQ ID NO: 764 and denoted by CG, preferably wherein the assay is characterised as having an ROC AUC of at least 0.863, and more preferably wherein the panel of one or more CpGs comprises at least the CpGs denoted by [[CG]];
265. one or more CpGs within DMR 265 as defined by SEQ ID NO: 765 and denoted by CG, preferably wherein the assay is characterised as having an ROC AUC of at least 0.863, and more preferably wherein the panel of one or more CpGs comprises at least the CpGs denoted by [[CG]];
266. one or more CpGs within DMR 267 as defined by SEQ ID NO: 767 and denoted by CG, preferably wherein the assay is characterised as having an ROC AUC of at least 0.863, and more preferably wherein the panel of one or more CpGs comprises at least the CpGs denoted by [[CG]];
267. one or more CpGs within DMR 268 as defined by SEQ ID NO: 768 and denoted by CG, preferably wherein the assay is characterised as having an ROC AUC of at least 0.862, and more preferably wherein the panel of one or more CpGs comprises at least the CpGs denoted by [[CG]];
268. one or more CpGs within DMR 269 as defined by SEQ ID NO: 769 and denoted by CG, preferably wherein the assay is characterised as having an ROC AUC of at least 0.862, and more preferably wherein the panel of one or more CpGs comprises at least the CpGs denoted by [[CG]];
269. one or more CpGs within DMR 270 as defined by SEQ ID NO: 770 and denoted by CG, preferably wherein the assay is characterised as having an ROC AUC of at least 0.862, and more preferably wherein the panel of one or more CpGs comprises at least the CpGs denoted by [[CG]];
270. one or more CpGs within DMR 271 as defined by SEQ ID NO: 771 and denoted by CG, preferably wherein the assay is characterised as having an ROC AUC of at least 0.862, and more preferably wherein the panel of one or more CpGs comprises at least the CpGs denoted by [[CG]];
271. one or more CpGs within DMR 272 as defined by SEQ ID NO: 772 and denoted by CG, preferably wherein the assay is characterised as having an ROC AUC of at least 0.862, and more preferably wherein the panel of one or more CpGs comprises at least the CpGs denoted by [[CG]];
272. one or more CpGs within DMR 273 as defined by SEQ ID NO: 773 and denoted by CG, preferably wherein the assay is characterised as having an ROC AUC of at least 0.862, and more preferably wherein the panel of one or more CpGs comprises at least the CpGs denoted by [[CG]];
273. one or more CpGs within DMR 274 as defined by SEQ ID NO: 774 and denoted by CG, preferably wherein the assay is characterised as having an ROC AUC of at least 0.862, and more preferably wherein the panel of one or more CpGs comprises at least the CpGs denoted by [[CG]];
274. one or more CpGs within DMR 275 as defined by SEQ ID NO: 775 and denoted by CG, preferably wherein the assay is characterised as having an ROC AUC of at least 0.862, and more preferably wherein the panel of one or more CpGs comprises at least the CpGs denoted by [[CG]];
275. one or more CpGs within DMR 276 as defined by SEQ ID NO: 776 and denoted by CG, preferably wherein the assay is characterised as having an ROC AUC of at least 0.861, and more preferably wherein the panel of one or more CpGs comprises at least the CpGs denoted by [[CG]];
276. one or more CpGs within DMR 277 as defined by SEQ ID NO: 777 and denoted by CG, preferably wherein the assay is characterised as having an ROC AUC of at least 0.861, and more preferably wherein the panel of one or more CpGs comprises at least the CpGs denoted by [[CG]];
277. one or more CpGs within DMR 278 as defined by SEQ ID NO: 778 and denoted by CG, preferably wherein the assay is characterised as having an ROC AUC of at least 0.860, and more preferably wherein the panel of one or more CpGs comprises at least the CpGs denoted by [[CG]];
278. one or more CpGs within DMR 279 as defined by SEQ ID NO: 779 and denoted by CG, preferably wherein the assay is characterised as having an ROC AUC of at least 0.860, and more preferably wherein the panel of one or more CpGs comprises at least the CpGs denoted by [[CG]];
279. one or more CpGs within DMR 280 as defined by SEQ ID NO: 780 and denoted by CG, preferably wherein the assay is characterised as having an ROC AUC of at least 0.859, and more preferably wherein the panel of one or more CpGs comprises at least the CpGs denoted by [[CG]];
280. one or more CpGs within DMR 281 as defined by SEQ ID NO: 781 and denoted by CG, preferably wherein the assay is characterised as having an ROC AUC of at least 0.859, and more preferably wherein the panel of one or more CpGs comprises at least the CpGs denoted by [[CG]];
281. one or more CpGs within DMR 282 as defined by SEQ ID NO: 782 and denoted by CG, preferably wherein the assay is characterised as having an ROC AUC of at least 0.853, and more preferably wherein the panel of one or more CpGs comprises at least the CpGs denoted by [[CG]];
282. one or more CpGs within DMR 283 as defined by SEQ ID NO: 783 and denoted by CG, preferably wherein the assay is characterised as having an ROC AUC of at least 0.853, and more preferably wherein the panel of one or more CpGs comprises at least the CpGs denoted by [[CG]];
283. one or more CpGs within DMR 284 as defined by SEQ ID NO: 784 and denoted by CG, preferably wherein the assay is characterised as having an ROC AUC of at least 0.851, and more preferably wherein the panel of one or more CpGs comprises at least the CpGs denoted by [[CG]];
284. one or more CpGs within DMR 285 as defined by SEQ ID NO: 785 and denoted by CG, preferably wherein the assay is characterised as having an ROC AUC of at least 0.851, and more preferably wherein the panel of one or more CpGs comprises at least the CpGs denoted by [[CG]];
285. one or more CpGs within DMR 286 as defined by SEQ ID NO: 786 and denoted by CG, preferably wherein the assay is characterised as having an ROC AUC of at least 0.849, and more preferably wherein the panel of one or more CpGs comprises at least the CpGs denoted by [[CG]];
286. one or more CpGs within DMR 287 as defined by SEQ ID NO: 787 and denoted by CG, preferably wherein the assay is characterised as having an ROC AUC of at least 0.849, and more preferably wherein the panel of one or more CpGs comprises at least the CpGs denoted by [[CG]];
287. one or more CpGs within DMR 288 as defined by SEQ ID NO: 788 and denoted by CG, preferably wherein the assay is characterised as having an ROC AUC of at least 0.846, and more preferably wherein the panel of one or more CpGs comprises at least the CpGs denoted by [[CG]];
288. one or more CpGs within DMR 289 as defined by SEQ ID NO: 789 and denoted by CG, preferably wherein the assay is characterised as having an ROC AUC of at least 0.846, and more preferably wherein the panel of one or more CpGs comprises at least the CpGs denoted by [[CG]];
289. one or more CpGs within DMR 290 as defined by SEQ ID NO: 790 and denoted by CG, preferably wherein the assay is characterised as having an ROC AUC of at least 0.842, and more preferably wherein the panel of one or more CpGs comprises at least the CpGs denoted by [[CG]];
290. one or more CpGs within DMR 291 as defined by SEQ ID NO: 791 and denoted by CG, preferably wherein the assay is characterised as having an ROC AUC of at least 0.842, and more preferably wherein the panel of one or more CpGs comprises at least the CpGs denoted by [[CG]];
291. one or more CpGs within DMR 292 as defined by SEQ ID NO: 792 and denoted by CG, preferably wherein the assay is characterised as having an ROC AUC of at least 0.841, and more preferably wherein the panel of one or more CpGs comprises at least the CpGs denoted by [[CG]];
292. one or more CpGs within DMR 293 as defined by SEQ ID NO: 793 and denoted by CG, preferably wherein the assay is characterised as having an ROC AUC of at least 0.841, and more preferably wherein the panel of one or more CpGs comprises at least the CpGs denoted by [[CG]];
293. one or more CpGs within DMR 294 as defined by SEQ ID NO: 794 and denoted by CG, preferably wherein the assay is characterised as having an ROC AUC of at least 0.838, and more preferably wherein the panel of one or more CpGs comprises at least the CpGs denoted by [[CG]];
294. one or more CpGs within DMR 295 as defined by SEQ ID NO: 795 and denoted by CG, preferably wherein the assay is characterised as having an ROC AUC of at least 0.838, and more preferably wherein the panel of one or more CpGs comprises at least the CpGs denoted by [[CG]];
295. one or more CpGs within DMR 296 as defined by SEQ ID NO: 796 and denoted by CG, preferably wherein the assay is characterised as having an ROC AUC of at least 0.833, and more preferably wherein the panel of one or more CpGs comprises at least the CpGs denoted by [[CG]];
296. one or more CpGs within DMR 297 as defined by SEQ ID NO: 797 and denoted by CG, preferably wherein the assay is characterised as having an ROC AUC of at least 0.833, and more preferably wherein the panel of one or more CpGs comprises at least the CpGs denoted by [[CG]];
297. one or more CpGs within DMR 298 as defined by SEQ ID NO: 798 and denoted by CG, preferably wherein the assay is characterised as having an ROC AUC of at least 0.832, and more preferably wherein the panel of one or more CpGs comprises at least the CpGs denoted by [[CG]];
298. one or more CpGs within DMR 299 as defined by SEQ ID NO: 799 and denoted by CG, preferably wherein the assay is characterised as having an ROC AUC of at least 0.832, and more preferably wherein the panel of one or more CpGs comprises at least the CpGs denoted by [[CG]];
299. one or more CpGs within DMR 300 as defined by SEQ ID NO: 800 and denoted by CG, preferably wherein the assay is characterised as having an ROC AUC of at least 0.831, and more preferably wherein the panel of one or more CpGs comprises at least the CpGs denoted by [[CG]];
300. one or more CpGs within DMR 301 as defined by SEQ ID NO: 801 and denoted by CG, preferably wherein the assay is characterised as having an ROC AUC of at least 0.831, and more preferably wherein the panel of one or more CpGs comprises at least the CpGs denoted by [[CG]];
301. one or more CpGs within DMR 302 as defined by SEQ ID NO: 802 and denoted by CG, preferably wherein the assay is characterised as having an ROC AUC of at least 0.826, and more preferably wherein the panel of one or more CpGs comprises at least the CpGs denoted by [[CG]];
302. one or more CpGs within DMR 303 as defined by SEQ ID NO: 803 and denoted by CG, preferably wherein the assay is characterised as having an ROC AUC of at least 0.826, and more preferably wherein the panel of one or more CpGs comprises at least the CpGs denoted by [[CG]];
303. one or more CpGs within DMR 304 as defined by SEQ ID NO: 804 and denoted by CG, preferably wherein the assay is characterised as having an ROC AUC of at least 0.806, and more preferably wherein the panel of one or more CpGs comprises at least the CpGs denoted by [[CG]];
304. one or more CpGs within DMR 305 as defined by SEQ ID NO: 805 and denoted by CG, preferably wherein the assay is characterised as having an ROC AUC of at least 0.806, and more preferably wherein the panel of one or more CpGs comprises at least the CpGs denoted by [[CG]];
305. one or more CpGs within DMR 306 as defined by SEQ ID NO: 806 and denoted by CG, preferably wherein the assay is characterised as having an ROC AUC of at least 0.911, and more preferably wherein the panel of one or more CpGs comprises at least the CpGs denoted by [[CG]];
306. one or more CpGs within DMR 307 as defined by SEQ ID NO: 807 and denoted by CG, preferably wherein the assay is characterised as having an ROC AUC of at least 0.905, and more preferably wherein the panel of one or more CpGs comprises at least the CpGs denoted by [[CG]];
307. one or more CpGs within DMR 308 as defined by SEQ ID NO: 808 and denoted by CG, preferably wherein the assay is characterised as having an ROC AUC of at least 0.905, and more preferably wherein the panel of one or more CpGs comprises at least the CpGs denoted by [[CG]],
preferably wherein the methylation status of the one or more CpGs in the panel is preferably determined by a β-value analysis, and the cancer is endometrial cancer.

In any of the assays described herein, the step of determining the methylation status of a panel of one or more CpGs comprises determining the methylation status of two or more, three or more, four or more, five or more, six or more, seven or more, eight or more, or nine or more, or all of the CpGs denoted by CG within any combination of:
i. one or more DMRs defined by SEQ ID NOs: 525, 756 and 757, preferably within all of SEQ ID NOs: 525, 756 and 757;
ii. one or more DMRs defined by SEQ ID NOs: 503, 504, 526, 740, 741, 743 and 744, preferably within all of SEQ ID NOs: 503, 504, 526, 740, 741, 743 and 744; and
iii. one or more DMRs defined by SEQ ID NOs: 525, 756, 757, 503, 504, 526, 740, 741, 743 and 744, preferably within all of SEQ ID NOs: 525, 756, 757, 503, 504, 526, 740, 741, 743 and 744.
In any one of the assays described herein, the step of determining the methylation status of the one or more CpGs in the panel may comprise or may additionally comprise determining the methylation status of each CpG within one or more of the sequences identified by SEQ ID NOs 809 to 919.

In any one of the assays described herein, the step of determining the methylation status of the one or more CpGs in the panel may preferably comprise or may preferably additionally comprise determining the methylation status of each CpG within one or more of the sequences identified by SEQ ID NOs 809, 746, 883, 811, 848, 885, 813, 850, and 887. More preferably, in any one of the assays described herein, the step of determining the methylation status of the one or more CpGs in the panel may comprise or may additionally comprise determining the methylation status of each CpG within all of the sequences identified by SEQ ID NOs 809, 746, 883, 811, 848, 885, 813, 850, and 887.

The invention also provides a variety of assays, each comprising any 1, 2, 3, 4, 5, 6, 7, 8, 9, 10 or more (or any range derivable therein) of a variety of steps and in no particular order, including methods of the following: measuring in a sample; analyzing a sample; assessing a sample; evaluating a sample; measuring nucleic acids in a sample; assessing nucleic acids in a sample; detecting nucleic acids in a sample; measuring methylation in nucleic acids in a sample; analyzing nucleic acids in a sample; assessing nucleic acids in a sample; measuring methylation at one or more CpG dinucleotides in a sample; detecting methylation at one or more CpG dinucleotides in a sample; assaying methylation at one or more CpG dinucleotides in a sample; assessing methylation at one or more CpG dinucleotides in a sample; measuring a methylation status in a sample; assaying a methylation status in a sample; detecting methylation status in a sample; determining methylation status in a sample; identifying methylation status in a sample; measuring one or more DNA methylation markers in a sample; assessing one or more DNA methylation markers in a sample; detecting one or more DNA methylation markers in a sample; measuring the presence of methylation at one or more markers in a sample; detecting the presence of methylation at one or more markers in a sample; assessing the presence of methylation at one or more markers in a sample; assaying the presence of one of more markers in a sample; measuring one or more DNA methylation markers in a sample but excluding the measuring of one or more other DNA methylation markers in the sample; assessing one or more DNA methylation markers in a sample but excluding the assessing of one or more other DNA methylation markers in the sample; analyzing one or more DNA methylation markers in a sample but excluding the analyzing of one or more other DNA methylation markers in the sample; detecting one or more DNA methylation markers in a sample but excluding the detecting of one or more other DNA methylation markers in the sample; measuring methylation status in nucleic acids from a sample from tissue from an individual other than tissue from the individual suspected of, or at risk for, being cancerous; detecting methylation status in nucleic acids from a sample from tissue from an individual other than tissue from the individual suspected of, or at risk for, being cancerous; analyzing methylation status in nucleic acids from a sample from tissue from an individual other than tissue from the individual suspected of, or at risk for, being cancerous; assessing methylation status in nucleic acids from a sample from tissue from an individual other than tissue from the individual suspected of, or at risk for, being cancerous; measuring methylation at one or more CpG dinucleotides in a sample but excluding the measuring of methylation at one or more CpG dinucleotides in the sample; assessing methylation at one or more CpG dinucleotides in a sample but excluding the assessing of methylation at one or more CpG dinucleotides in the sample; analyzing methylation at one or more CpG dinucleotides in a sample but excluding the analyzing of methylation at one or more CpG dinucleotides in the sample; detecting methylation at one or more CpG dinucleotides in a sample but excluding the detecting of methylation at one or more CpG dinucleotides in the sample; measuring methylation at one or more CpG dinucleotides in nucleic acids from a sample from tissue from an individual other than tissue from the individual suspected of, or at risk for, being cancerous; detecting methylation at one or more CpG dinucleotides in nucleic acids from a sample from tissue from an individual other than tissue from the individual suspected of, or at risk for, being cancerous; analyzing methylation at one or more CpG dinucleotides in nucleic acids from a sample from tissue from an individual other than tissue from the individual suspected of, or at risk for, being cancerous; assessing methylation at one or more CpG dinucleotides in nucleic acids from a sample from tissue from an individual other than tissue from the individual suspected of, or at risk for, being cancerous; treating an individual for cancer when the individual has been determined to have a methylation status at one or more methylation markers; treating an individual for cancer when the individual has been determined to have methylation at one or more CpG dinucleotides;
wherein any of the aforementioned methods, or any other methods encompassed by the disclosure, may comprise any one or more of the following method steps:
measuring methylation status, wherein the measuring identifies the methylation status of one or markers from nucleic acids in a sample;
measuring methylation status, wherein the measuring identifies the presence of one or more markers from nucleic acids in a sample;
measuring the presence of one or more methylation markers from a sample;
providing DNA from a sample;
providing nucleic acids from a sample;
determining whether one or more methylation markers from nucleic acids from a sample are methylated;
measuring whether one or more methylation markers from nucleic acids from a sample are methylated;
performing a sequencing step on nucleic acids from a sample;
determining a sequence of nucleic acids from a sample;
performing bisulphite conversion on one or more markers;
performing bisulphite conversion on one or more CpG dinucleotides;
hybridizing DNA to an array comprising probes capable of determining methylated versus non-methylated markers;
hybridizing DNA to an array comprising probes capable of determining methylated versus non-methylated CpG dinucleotides;
hybridizing DNA to an array comprising probes capable of discriminating between methylated and non-methylated markers;
hybridizing DNA to an array comprising probes capable of discriminating between methylated and non-methylated CpG dinucleotides;
performing an amplification step on sequence from nucleic acids from a sample;
performing an amplification step on sequence from nucleic acids using methylation-specific primers;
performing amplification of sequence comprising one or more regions suspected of having methylation or in need of determination of a methylation status;
performing PCR on sequence comprising one or more regions suspected of having methylation or in need of determination of a methylation status;
performing a capturing step;
performing a binding step;
performing a purification step;
performing a capturing step comprising binding of polynucleotides comprising one or more methylation markers to binding molecules specific to the one or more methylation markers and collecting complexes thereof;
stratifying the grade of a cancer;
determining the risk of cancer;
determining the risk of recurrence of cancer;
obtaining a sample from an individual;
obtaining DNA from a sample from an individual;
administering a treatment to an individual;
providing DNA from a sample;
determining whether one or more methylation markers from a panel of methylation markers comprises a specific sequence; and/or
obtaining data that identifies whether each one of a group of methylation markers from a panel comprises a specific sequence.

Moreover, in some aspects of the invention, an individual who is administered a therapy or treatment has been subjected to any of the methods and steps described herein.

Described herein are assays that utilise a statistically robust panel of one or more CpGs whose methylation status can be determined to provide a reliable prediction of the presence or development of cancer in an individual. By determining the methylation status of each CpG within the panel of one or more CpGs, a cancer index value may be derived thus enabling stratification of individuals according to their risk of developing endometrial cancer or of having endometrial cancer, with statistically robust sensitivity and specificity. The skilled person would understand that the methylation status of each CpG within a panel of one or more CpGs can be determined by any suitable means in order to thereby derive the cancer index value. Any one method, or a combination of methods, may be used to determine the methylation status of each CpG within a panel of one or more CpGs.

Various exemplary methods for determining the methylation status of each CpG within a panel of one or more CpGs are described herein. For example, in one method a percent methylated reference (PMR) value of a CpG may be determined. In another method the methylation β-values of a CpG may be determined. Different mechanisms may be employed to determine specific values depending on the circumstances, such as PCR-based mechanisms or array-based mechanisms.

### Cancer index values as diagnostic and risk assessment tools

In any of the assays described herein, the assessment of the presence, absence or development of cancer in an individual is based on the cancer index value of the individual at the time of testing.

As explained herein, using panels of the specific CpGs disclosed herein, cancer index values can be established which correspond with cancer negative samples, because they are based on values derived from individuals known to be cancer negative and obtained from tissue samples from an anatomical site other than the endometrium or ovary, such as from the cervix, vagina, buccal area, blood and/or urine, particularly from a liquid-based cytology sample, and more preferably a cervical smear sample. Similarly, using panels of the specific CpGs disclosed herein, cancer index values can be established which correspond with cancer positive samples, because they are based on values derived from an anatomical site other than the ovary or endometrium, as noted above, from tissue samples from individuals known to be cancer positive. A user can then apply these cancer index values to assess the presence, absence or development of cancer in any test individual whose cancer status is required to be tested. As also explained herein, the assays of the invention are capable of being performed with a high degree of statistical accuracy.

As explained herein, the described assays particularly relate to the assessment of the presence, absence or development of endometrial cancer.

A skilled person will readily appreciate that a cancer index value provides a value that indicates a "likelihood" or "risk" or "prediction" of any of the assays of the invention correctly assessing the presence, absence or development of cancer in an individual. This is because the assessment is based upon a correlation between DNA methylation profiles of tissue samples and individual disease status. Nevertheless, as demonstrated by data set out in the Examples and elsewhere herein, the assays of the invention provide such correlations with high statistical accuracy, thus providing the skilled person with a high degree of confidence that the cancer index value which is determined for any test individual whose cancer status is required to be tested will provide an accurate correlation with actual disease status for the individual.

In the context of the present invention, "likelihood", "risk" and "prediction" may be used synonymously with each other.

Any references herein to sequences, genomic sequences and/or genomic coordinates are derived based upon Homo sapiens (human) genome assembly GRCh37 (hg19). The skilled person would understand variations in the nucleotide sequences of any given sequence, particularly DMRs 1 to 308, may exist due to sequencing errors and/or variation between individuals.

The assay of the invention represents a 'prediction' because any cancer index value (WID-EC-Index) derived in accordance with the invention is unlikely to be capable of diagnosing every individual as having or not having cancer with 100% specificity and 100% sensitivity. Rather, depending on the cancer index cutpoint threshold applied by the user for positively predicting the presence of cancer in an individual, the false positive and false negative rate will vary. In other words, the inventors have discovered that the assays of the invention can achieve variable levels of sensitivity and specificity for predicting the presence, absence or development of cancer, as defined by receiver operating characteristics, depending on the cancer index cutpoint threshold chosen and applied by the user. Such sensitivity and specificity can be seen from the data disclosed herein to be achievable at high proportions, demonstrating accurate and statistically-significant discriminatory capability.

Similarly, cancer index values which have been pre-determined to correlate with specific cancer phenotypes, such as the presence or absence of cancer, have been defined with a high level of statistical accuracy as explained further herein.

Assessing the 'development' of cancer in the context of the invention may refer to assessing whether an individual is likely or unlikely to develop cancer. The inventors have shown that the CpGs assayed in order to derive the cancer index value of the assays of the invention are representative of the cells within normal tissue from an anatomical site other than the endometrium or ovary, such as from the cervix, vagina, buccal area, blood and/or urine, particularly from a liquid-based cytology sample, and more preferably a cervical smear sample. Assessing the development of cancer in accordance with the assays of the invention may refer to assessing an increased or decreased likelihood of endometrial cancer development. Assessing the development of cancer in accordance with the assays of the invention may refer to assessing progression or worsening of a pre-existing cancer lesion in an individual. Assessment of the development of cancer in accordance with the assays of the invention may refer to predicting the likelihood of recurrence of cancer.

In any of the assays described herein, the step of assessing the presence or development of cancer in an individual based on a cancer index value may involve the application of a threshold value. Threshold values can provide a risk-based indication of an individual's cancer status, whether that is cancer positive, or cancer negative. Threshold values can also provide a means for identifying whether the cancer index value is intermediate between a cancer positive value and a cancer negative value. As explained herein, the cancer index value may be dynamic and subject to change depending upon genetic and/or environmental factors. Accordingly, the cancer index value may provide a means for assessing and monitoring cancer development. Cancer index values may therefore indicate at least a low risk or a high risk that the individual has a cancer positive status or has a status that is indicative of the development of cancer. If the cancer index value of an individual is determined by the assays of the invention at two or more time points, an increase or decrease in the individual's cancer index value may indicate an increased or decreased risk of the individual having or developing endometrial cancer.

Throughout the disclosure herein the terms "threshold value", "cutpoint", and "cutpoint threshold" are to be considered synonymous and interchangeable.

As explained further herein any assay of the invention is an assay for assessing the presence, absence or development of cancer in an individual. The types of cancer are set out further herein. As explained further herein, the assays of the invention provide means for assessing whether an individual is at risk of having or developing cancer based on specific cutpoint thresholds. Such risk assessments can be provided with a high degree of confidence based on the statistical parameters which characterise the assay. Thus in any of the assays described herein involving cancer index cutpoint thresholds, the cutpoint threshold may be used for risk assessment purposes. Equally, in any of the assays described herein involving cancer index cutpoint thresholds, the cutpoint threshold value may be used to specify whether or not an individual has cancer as a pure diagnostic test. Again, such diagnostic tests can be provided with a high degree of confidence based on the statistical parameters which characterise the assay. Accordingly, in any assay described herein which specifies that a cancer index value for the individual is a specific value or more, or is "about" a specific value or more, the individual may be assessed as having cancer. In any assay described herein which specifies that a cancer index value for the individual is less than a specific value, or is less than "about" a specific value, the individual may be assessed as not having cancer. The term "about" is to be understood as providing a range of +/- 5% of the value.

Accordingly, any assay of the invention is an assay for assessing the presence, absence or development of cancer in an individual, the assay comprising:
a. providing a sample which has been taken from the individual, the sample comprising a population of DNA molecules;
b. determining in the population of DNA molecules in the sample the methylation status of a panel of:
   (i) one or more CpGs comprising the CpG identified in SEQ ID NO 67 wherein the CpG is identified at nucleotide positions 61 to 62 ;
c. deriving a cancer index value based on the methylation status of the one or more CpGs in the panel; and
d. assessing the presence, absence or development of cancer in the individual based on the cancer index value;
   wherein the assay is characterised as having an area under the curve (AUC) of 0.60 or more as determined by receiver operating characteristics (ROC).

Any of the assays of the invention are particularly for assessing the presence or absence of endometrial cancer in an individual.

Such an assay may be performed in accordance with any of the methods disclosed and defined herein.

As explained further herein, any assay of the invention for assessing the presence, absence or development of cancer in an individual may alternatively be referred to as an assay for stratifying an individual in accordance with their cancer status.

Accordingly, any assay of the invention is an assay for stratifying an individual for the presence, absence or development of endometrial cancer in an individual, the assay comprising:
a. providing a sample which has been taken from the individual, the sample comprising a population of DNA molecules;
b. determining in the population of DNA molecules in the sample the methylation status of a panel of:
   (i) one or more CpGs comprising the CpG identified in SEQ ID NO 67 wherein the CpG is identified at nucleotide positions 61 to 62; and/or ;
c. deriving a cancer index value based on the methylation status of the one or more CpGs in the panel; and
d. stratifying the individual for the presence, absence or development of cancer based on the cancer index value;
   wherein the assay is characterised as having an area under the curve (AUC) of 0.60 or more as determined by receiver operating characteristics (ROC).

Such an assay may be performed in accordance with any of the methods disclosed and defined herein.

Accordingly, any assay of the invention is an assay for stratifying an individual for cancer, the assay comprising:
a. providing a sample which has been taken from the individual, the sample comprising a population of DNA molecules;
b. determining in the population of DNA molecules in the sample the methylation status of a panel of:
   (i) one or more CpGs comprising the CpG identified in SEQ ID NO 67 wherein the CpG is identified at nucleotide positions 61 to 62; and/or
c. deriving a cancer index value based on the methylation status of the one or more CpGs in the panel; and
d. stratifying the individual for cancer based on the cancer index value;
wherein the assay is characterised as having an area under the curve (AUC) of 0.60 or more as determined by receiver operating characteristics (ROC).

Such an assay may be performed in accordance with any of the methods disclosed and defined herein.

The cancer index value may be derived by any suitable means. Preferably, the cancer index value may be derived by assessing the methylation status of the panel of:
(i) one or more CpGs comprising the CpG identified in SEQ ID NO 67 wherein the CpG is identified at nucleotide positions 61 to 62; and/or ;
in a sample provided from an individual. The methylation status of the CpGs may be determined by any suitable means. For example, in any of the assays described herein the step of determining the methylation status of each CpG in the panel of one or more CpGs may comprise:
a. performing a sequencing step to determine the sequence of each CpG;
b. hybridising DNA to an array comprising probes capable of discriminating between methylated and non-methylated forms of the CpGs and applying a detection system to the array so as to determine the methylation status of each CpG; and/or
c. performing a PCR step using methylation-specific primers, wherein the methylation status of the CpG is determined by the presence or absence of a PCR product.

The step of determining in the population of DNA molecules in the sample the methylation status of a panel of one or more CpGs may comprises determining a β value of each CpG. Deriving the cancer index value may involve providing a methylation β-value data set comprising the methylation β-values for each CpG in the panel of one or more CpGs. Additionally, or alternatively, the step of determining in the population of DNA molecules in the sample the methylation status of a panel of one or more CpGs may comprises determining a percent methylated reference value for each of the panel of one or more CpGs. Optionally deriving the cancer index value may also involve estimating the fraction of contaminating DNA within the DNA provided from a sample.

DNA may be DNA originating from a particular source organism, tissue or cell type. Preferably the contaminating DNA originates from one or more different cell types to one or more cell types of interest. A cell type of interest may particularly be an epithelial cell. In some aspects of the invention, it may be preferable to estimate the fraction of contaminating DNA after the step of providing a sample which has been take from an individual. The assays described herein may optionally involve estimating a contaminating DNA fraction within DNA in the sample by any suitable means. Preferably, the contaminating DNA fraction for the sample is estimated via any suitable bioinformatics analysis tool. A bioinformatics analysis tool that may be used to estimate a contaminating DNA fraction may be EpIDISH. As described herein, it may be desirable to estimate the fraction of contaminating DNA from the one or more cell types that are different to the one or more cell types of interest because the cancer index value used for predicting the presence or development of cancer in an individual may, in some instances, only be reliably derived from determining the methylation status of a set of CpGs from DNA of a particular cell type of interest. Particularly, methylation status beta-values may differ in the one or more cell types of interest within a sample relative to methylation status beta-values in contaminating DNA from different cell types within the same sample. Thus, the derived cancer index value may in some instances have a decreased predictive power without estimating and controlling for the contaminating DNA fraction within the DNA provided from the sample. In assays of the invention that involve estimating the fraction of contaminating DNA and accordingly controlling for said contaminating DNA, it is preferable to estimate an immune cell DNA fraction within the DNA provided from the sample. In particular assays of the invention, wherein the individual has an immune cell contamination of over 50% *(i.e.* wherein more than 50% of the DNA in the sample is deemed to be derived from immune cells), the assay may preferably involve controlling for the immune cell contamination by deriving the cancer index, in accordance with the invention, solely from the DNA molecules derived from epithelial cells.

Any of the assays described herein comprising a step of deriving a cancer index value based on the methylation status of the one or more CpGs in the panel may further comprise applying an algorithm to the methylation beta-value dataset to obtain the cancer index value. Preferably, in any of the assays described herein, the step of deriving the cancer index value based on the methylation status of the panel of CpGs comprises providing a methylation beta-value data set comprising the methylation beta-values for each CpG in the panel and applying an algorithm to the methylation beta-value data set to obtain the cancer index value.

In any of the assays described herein, the step of deriving the cancer index value based on the methylation status of the one or more CpGs in the panel comprises:
a. providing a methylation β-value data set comprising the methylation β-values for each CpG in the panel;
b. providing a mathematical model capable of generating the cancer index from the methylation β-value data set; and
c. applying the mathematical model to the methylation β-value data set, thereby generating the cancer index.

In any of the assays described herein, the cancer index value may be calculated by any suitable mathematical model such as an algorithm or formula. Preferably, the cancer index value is termed Women's risk Identification for Endometrial Cancer Index (WID-EC-index) and wherein the mathematical model which is applied to the methylation β-value data set to generate the cancer index is calculated by an algorithm according to the following formula: $WID - EC - index = {\sum }_{i = 1}^{n} \frac{{w}_{i} {β}_{i} - μ}{σ}$ wherein:
1. *β*₁, ... , *βₙ* are methylation beta-values (between 0 and 1);
2. *w*₁, ... , *w*₅₀₀ are real valued coefficients;
3. µ and σ are real valued parameters used to scale the index; and
*4. n* refers to the number of CpGs in the set of test CpGs

In any of the assays described herein, the WID-EC-index algorithm applies real valued coefficients inferred by initially training on a dataset (this dataset in the exemplary embodiments of the invention described in the Examples consisted of 144 endometrial cancer cases and 572 controls) to fit a ridge classifier using the R package glmnet with a mixing parameter value of alpha = 0 (ridge penalty) and binomial response type. Ten-fold cross-validation was used internally by the cv.glmnet function in order to determine the optimal value of the regularisation parameter lambda. The beta values from n CpGs for individual υ, ${β}_{1}^{υ} , … , {β}_{n}^{υ}$, are used as inputs to the ridge classifier. The coefficients *w*₁, ... , *wₙ* are obtained from the fitted model. The following quantity was computed for each individual v in the training set: ${x}_{υ} = {\sum }_{i = 1}^{n} {w}_{i} {β}_{i}^{υ}$

Any suitable real valued coefficients may be applied to the WID-EC-Index in any of the assays described herein.

The value of the parameters *µ* and σ are given by the mean and standard deviation of *x_{υ}* in the training dataset respectively.

Thus, any suitable *µ* and σ real valued parameters may be applied to the WID-EC-index in any of the assays described herein. Any suitable training data set may be applied to the assays described herein in order to infer real valued parameters and coefficients that can subsequently be applied to the WID-EC-index formula according to the present invention. Exemplary ways of utilising a training dataset in accordance with the present invention are further described in the '*Statistical analyses for classifier development'* section of the Materials and Methods section of the Examples.

Exemplary *µ* and σ real valued parameters are provided in Table 2 for CpG subsets identified in SEQ ID NOs 1 to 500. These real valued parameters may be applied to any of the assays described herein wherein the real parameters correspond to any one of the sets of CpGs identified in SEQ ID NOs 1 to 500 and set out in the left hand column of Table 2.

**Table 2. Exemplary µ and σ real valued parameters are provided in Table 2 for CpG subsets identified in SEQ ID NOs 1 to 500**

| **SEQ ID NOs:** | **mu** | **sigma** |
|---|---|---|
| 1-50 | 2.880073 | 4.148988 |
| 1-100 | 3.43488 | 4.076555 |
| 1-150 | 3.897919 | 4.129713 |
| 1-200 | 3.861902 | 3.842672 |
| 1-250 | 3.774098 | 4.12993 |
| 1-300 | 3.807151 | 4.17154 |
| 1-350 | 3.662246 | 4.109188 |
| 1-400 | 3.316238 | 3.984151 |
| 1-450 | 3.351052 | 3.982014 |
| 1-500 | 3.667061 | 4.171657 |

Exemplary *w*₁, ... , *wₙ* real value coefficients are provided below for the CpGs identified at positions 61 to 62 in SEQ ID NOs 1 to 500. These real value coefficients may be applied to any of the assays described herein wherein the real parameters correspond to any one of the sets of CpGs identified in SEQ ID NOs 1 to 500 wherein the 500 real value coefficients below in turn correspond to the CpGs in turn identified at nucleotide positions 61 to 62 in SEQ ID NOs 1 to 500 . Accordingly, the listed coefficients are presented below in numerical order corresponding respectively to the CpGs identified in SEQ ID NOs 1 to 5000. Thus the first number below corresponds to SEQ ID NO 1, the second number corresponds to SEQ ID NO 2 etc. The exemplary real value coefficients are as follows:
2.32966, 1.95059, 1.87174, 1.86617, 1.65489, 1.60169, -1.52485, 1.4846, 1.48231, 1.47445, 1.47342, 1.4441, 1.40984, -1.40743, 1.3761, 1.32123, -1.29551, 1.29183, - 1.26448, 1.26166, -1.2578, -1.23771, 1.23418, -1.19877, 1.16649, 1.16451, 1.1421, 1.13892, -1.13096, 1.12541, 1.08222, 1.04719, 1.02739, 1.0262, 1.01259, 0.94512, 0.93919, 0.93148, 0.90943, 0.9025, 0.89635, 0.89152, -0.89049, -0.88852, -0.88615, 0.88339, -0.88238, -0.84763, 0.84381, 0.84281, -0.81762, -0.80659, 0.80114, -0.79765, -0.77473, -0.77145, 0.76203, -0.75583, 0.75558, 0.7419, 0.73368, 0.73031, -0.72253, - 0.71934, -0.7095, 0.70083, 0.69289, -0.68917, -0.68351, -0.67983, 0.67887, 0.67199, - 0.67196, -0.67186, 0.6693, -0.66391, 0.66347, -0.64944, -0.6407, 0.63298, 0.6277, 0.6159, -0.61381, -0.61247, 0.60994, -0.60626, -0.59996, 0.58813, 0.58678, -0.58473, 0.58414, 0.57932, 0.57361, 0.56698, 0.56338, 0.55991, -0.55792, -0.5568, -0.55381, 0.54696, 0.54651, 0.54566, -0.54331, 0.54171, 0.53775, 0.53111, -0.52707, 0.52526, 0.52488, 0.52102, -0.51622, -0.51409, -0.50784, -0.50732, -0.5063, -0.5026, 0.4983, - 0.49476, 0.49368, -0.49317, 0.49183, -0.49079, -0.4825, -0.47419, -0.47231, 0.47057, - 0.46063, -0.45931, -0.4517, -0.45066, 0.45017, 0.44947, 0.44946, 0.44407, 0.44333, 0.44312, -0.44228, 0.4386, -0.43757, 0.43618, -0.43183, 0.43138, 0.42876, -0.42769, - 0.42701, -0.42635, -0.42458, -0.42425, 0.4214, -0.41693, 0.41445, 0.41152, -0.40511, 0.4017, -0.4001, -0.39825, 0.39582, 0.3921, -0.39146, -0.39114, -0.39101, 0.39046, - 0.38991, 0.38989, -0.38837, -0.38587, -0.38464, 0.38453, -0.37816, 0.37631, -0.37551, 0.37536, -0.3738, 0.37293, 0.3729, 0.37046, -0.36971, 0.36901, 0.36684, -0.36451, 0.36449, -0.36442, 0.36383, -0.36291, 0.35913, -0.35596, 0.35594, -0.35422, -0.35344, 0.35168, 0.35008, 0.34932, 0.3479, 0.33951, -0.33902, -0.33693, 0.33589, 0.33518, - 0.33354, -0.32969, -0.32906, 0.32799, -0.32761, -0.32373, 0.32287, -0.32125, - 0.32052, 0.31686, 0.31205, 0.30807, -0.30682, -0.30671, -0.3051, -0.30257, -0.30238, 0.29616, -0.29598, 0.29579, 0.29554, 0.29553, 0.29552, -0.29492, -0.29464, 0.29168, - 0.29124, -0.29108, 0.29078, 0.28992, 0.28904, -0.28898, 0.28857, 0.28584, 0.28496, - 0.28375, 0.28254, -0.28172, 0.28088, 0.28033, 0.28024, 0.27964, 0.27814, 0.27351, - 0.27199, -0.26681, -0.26572, -0.26482, 0.26282, -0.26196, 0.26167, -0.25997, 0.25947, -0.2582, -0.25679, -0.25527, 0.25448, -0.25443, 0.25302, 0.25204, 0.25198, -0.25098, - 0.24987, -0.24811, -0.24735, 0.24589, 0.24566, -0.24389, 0.24355, 0.2433, -0.24253, - 0.24066, -0.23859, 0.2381, 0.23454, -0.23433, 0.23349, -0.2332, -0.23185, -0.23092, 0.22721, 0.22664, -0.22602, 0.22147, 0.22089, 0.21875, 0.21869, 0.21644, -0.2164, - 0.21569, -0.21555, 0.21494, -0.21491, 0.21266, -0.21264, -0.21258, -0.21237, 0.20801, 0.2073, -0.2054, -0.20527, 0.20216, -0.20175, 0.20159, 0.19936, -0.19924, 0.19851, - 0.19521, 0.19502, -0.19436, 0.1942, -0.19284, -0.19253, -0.19086, -0.19076, 0.18983, - 0.18908, -0.18795, 0.18669, 0.18616, 0.18415, 0.18398, -0.18331, 0.18297, 0.1817, 0.18094, -0.17951, -0.17894, 0.17722, -0.17719, 0.1771, 0.17613, -0.17517, -0.1745, - 0.1739, -0.17205, 0.17203, -0.17156, -0.17088, 0.17084, -0.16955, -0.16933, -0.16659, 0.16417, -0.16366, -0.16063, -0.15928, -0.15883, -0.15631, -0.15543, -0.15504, - 0.15367, -0.15211, 0.15155, -0.15136, -0.14875, -0.14763, -0.14623, 0.14084, - 0.14027, 0.13945, -0.13918, -0.13807, 0.13787, 0.13747, -0.13745, -0.1358, -0.1354, 0.13442, 0.13287, -0.13083, -0.13017, -0.12858, -0.12794, 0.1279, -0.12639, 0.12472, 0.12451, -0.12409, -0.12237, 0.12222, -0.12063, 0.1202, -0.1186, -0.11855, -0.11781, 0.1171, 0.11674, 0.11558, -0.11356, -0.11325, -0.11304, 0.11228, -0.11198, -0.11176, - 0.11094, -0.11023, 0.10822, 0.10798, -0.10794, 0.10786, -0.10719, -0.10512, 0.10485, - 0.10472, 0.10422, -0.10366, -0.10352, 0.10349, 0.10115, 0.09742, 0.09554, 0.09475, - 0.09452, 0.09413, 0.09279, 0.09095, -0.09058, -0.08692, 0.08546, 0.08527, 0.08234, 0.08225, 0.08034, -0.07898, -0.07844, 0.07581, 0.07534, -0.07492, -0.07315, -0.06965, -0.06891, -0.06813, -0.06763, 0.06733, -0.06613, -0.06587, 0.06558, -0.0645, -0.06294, 0.06163, -0.06075, -0.06061, 0.05772, 0.05563, -0.05531, -0.05395, -0.05349, - 0.05207, -0.05088, -0.04909, 0.04894, 0.04817, -0.0475, 0.04684, -0.0437, -0.04325, 0.04069, 0.04061, 0.03859, -0.0379, -0.03773, 0.03649, 0.03592, -0.03564, 0.03468, 0.03356, -0.03242, 0.03235, 0.02751, -0.0273, -0.02729, 0.02726, -0.02588, -0.02556, - 0.02538, -0.02536, -0.02408, 0.02189, 0.01846, -0.01608, 0.01572, 0.01462, -0.01254, - 0.0117, 0.01122, -0.01112, -0.01092, -0.00938, -0.00906, -0.00851, -0.00846, -0.00818, -0.00662, -0.00621, -0.00543, 0.00291, 0.00232, 0.00112, -0.00063, -0.00034, and - 0.00021.

The predicting the presence, absence or development of cancer in an individual may particularly involve a threshold cancer index value being applied in order to assess or stratify an individual has having or not having cancer or of having a high or low risk of cancer development.

The assays of the invention may involve a threshold index being applied in order to assess the presence or absence of endometrial cancer in an individual. The assessment may be characterised by receiver operating characteristics, particularly and area under the curve (AUC), sensitivity, and specificity, indicative of the reliability of the threshold being applied in order to assess the presence or absence of endometrial cancer in an individual.

In any of the assays described herein, wherein when the cancer index value for the individual is about -0.201 or more, the individual is assessed as having endometrial cancer or as having a high risk of endometrial cancer development, or wherein when the cancer index value for the individual is less than about -0.201, the individual is assessed as not having endometrial cancer or as having a low risk of endometrial cancer development, preferably wherein the assay comprises determining methylation β-values for each CpG in the panel of one or more CpGs, and more preferably wherein the assessing the presence, absence or development of cancer in an individual is based on the WID-EC-Index. The panel of one or more CpGs used to derive the cancer index value may comprise:
1. at least 50 of the CpGs identified at nucleotide positions 61 to 62 in SEQ ID NOs 1 to 500, and wherein the sensitivity is at least 88% and the specificity is at least 76%;
2. at least 100 of the CpGs identified at nucleotide positions 61 to 62 in SEQ ID NOs 1 to 500, and wherein the sensitivity is at least 88% and specificity is at least 78%;
3. at least 150 of the CpGs identified at nucleotide positions 61 to 62 in SEQ ID NOs 1 to 500, and wherein the sensitivity is at least 88% and specificity is at least 76%;
4. at least 200 of the CpGs identified at nucleotide positions 61 to 62 in SEQ ID NOs 1 to 500, and wherein the sensitivity is at least 88% and specificity is at least 78%;
5. at least 250 of the CpGs identified at nucleotide positions 61 to 62 in SEQ ID NOs 1 to 500, and wherein the sensitivity is at least 88% and specificity is at least 78%;
6. at least 300 of the CpGs identified at nucleotide positions 61 to 62 in SEQ ID NOs 1 to 500, and wherein the sensitivity is at least 89% and specificity is at least 79%;
7. at least 350 of the CpGs identified at nucleotide positions 61 to 62 in SEQ ID NOs 1 to 500, and wherein the sensitivity is at least 88% and specificity is at least 78%;
8. at least 400 of the CpGs identified at nucleotide positions 61 to 62 in SEQ ID NOs 1 to 500, and wherein the sensitivity is at least 90% and specificity is at least 79%;
9. at least 450 of the CpGs identified at nucleotide positions 61 to 62 in SEQ ID NOs 1 to 500, and wherein the sensitivity is at least 89% and specificity is at least 79%; or
10. at least 500 of the CpGs identified at nucleotide positions 61 to 62 in SEQ ID NOs 1 to 500, and wherein the sensitivity is at least 95% and specificity is at least 76%.

In any of the assays described herein, wherein when the cancer index value for the individual is about -0.201 or more, the individual is assessed as having endometrial cancer or as having a high risk of endometrial cancer development, or wherein when the cancer index value for the individual is less than about -0.201, the individual is assessed as not having endometrial cancer or as having a low risk of endometrial cancer development, preferably wherein the assay comprises determining methylation β-values for each CpG in the panel of one or more CpGs, and more preferably wherein the assessing the presence, absence or development of cancer in an individual is based on the WID-EC-Index. The panel of one or more CpGs used to derive the cancer index value may comprise:
a. at least 50 of the CpGs identified at nucleotide positions 61 to 62 in SEQ ID NOs 1 to 500, and wherein the sensitivity is at least 88% and the specificity is at least 76%;
b. at least the CpGs defined by SEQ ID NOs 1 to 50 and identified at nucleotide positions 61 to 62, and wherein the sensitivity is at least 90% and specificity is at least 80%;
c. at least the CpGs defined by SEQ ID NOs 1 to 100 and identified at nucleotide positions 61 to 62, and wherein the sensitivity is at least 92% and specificity is at least 79%; or
d. at least the CpGs defined by SEQ ID NOs 1 to 150 and identified at nucleotide positions 61 to 62, and wherein the sensitivity is at least 93% and specificity is at least 80%.

In any of the assays described herein, wherein when the cancer index value for the individual is about -0.201 or more, the individual is assessed as having endometrial cancer or as having a high risk of endometrial cancer development, or wherein when the cancer index value for the individual is less than about -0.201, the individual is assessed as not having endometrial cancer or as having a low risk of endometrial cancer development, preferably wherein the assay comprises determining methylation β-values for each CpG in the panel of one or more CpGs, and more preferably wherein the assessing the presence, absence or development of cancer in an individual is based on the WID-EC-Index. The panel of one or more CpGs used to derive the cancer index value may comprise:
1. at least the CpGs defined by SEQ ID NOs 1 to 50 and identified at nucleotide positions 61 to 62, and wherein the sensitivity is at least 90% and specificity is at least 80%;
2. at least the CpGs defined by SEQ ID NOs 51 to 100 and identified at nucleotide positions 61 to 62, and wherein the sensitivity is at least 89% and specificity is at least 79%;
3. at least the CpGs defined by SEQ ID NOs 101 to 150 and identified at nucleotide positions 61 to 62, and wherein the sensitivity is at least 92% and specificity is at least 80%;
4. at least the CpGs defined by SEQ ID NOs 151 to 200 and identified at nucleotide positions 61 to 62, and wherein the sensitivity is at least 89% and specificity is at least 71%;
5. at least the CpGs defined by SEQ ID NOs 201 to 250 and identified at nucleotide positions 61 to 62, and wherein the sensitivity is at least 90% and specificity is at least 76%;
6. at least the CpGs defined by SEQ ID NOs 251 to 300 and identified at nucleotide positions 61 to 62, and wherein the sensitivity is at least 89% and specificity is at least 79%;
7. at least the CpGs defined by SEQ ID NOs 301 to 350 and identified at nucleotide positions 61 to 62, and wherein the sensitivity is at least 89% and specificity is at least 80%;
8. at least the CpGs defined by SEQ ID NOs 351 to 400 and identified at nucleotide positions 61 to 62, and wherein the sensitivity is at least 90% and specificity is at least 77%;
9. at least the CpGs defined by SEQ ID NOs 401 to 450 and identified at nucleotide positions 61 to 62, and wherein the sensitivity is at least 88% and specificity is at least 76%;
10. at least the CpGs defined by SEQ ID NOs 451 to 500 and identified at nucleotide positions 61 to 62, and wherein the sensitivity is at least 88% and specificity is at least 76%;
11. at least the CpGs defined by SEQ ID NOs 1 to 50 and identified at nucleotide positions 61 to 62, and wherein the sensitivity is at least 90% and specificity is at least 80%;
12. at least the CpGs defined by SEQ ID NOs 1 to 100 and identified at nucleotide positions 61 to 62, and wherein the sensitivity is at least 92% and specificity is at least 79%;
13. at least the CpGs defined by SEQ ID NOs 1 to 150 and identified at nucleotide positions 61 to 62, and wherein the sensitivity is at least 93% and specificity is at least 80%;
14. at least the CpGs defined by SEQ ID NOs 1 to 200 and identified at nucleotide positions 61 to 62, and wherein the sensitivity is at least 93% and specificity is at least 78%;
15. at least the CpGs defined by SEQ ID NOs 1 to 250 and identified at nucleotide positions 61 to 62, and wherein the sensitivity is at least 95% and specificity is at least 78%;
16. at least the CpGs defined by SEQ ID NOs 1 to 300 and identified at nucleotide positions 61 to 62, and wherein the sensitivity is at least 95% and specificity is at least 77%;
17. at least the CpGs defined by SEQ ID NOs 1 to 350 and identified at nucleotide positions 61 to 62, and wherein the sensitivity is at least 95% and specificity is at least 77%;
18. at least the CpGs defined by SEQ ID NOs 1 to 400 and identified at nucleotide positions 61 to 62, and wherein the sensitivity is at least 95% and specificity is at least 78%;
19. at least the CpGs defined by SEQ ID NOs 1 to 450 and identified at nucleotide positions 61 to 62, and wherein the sensitivity is at least 95% and specificity is at least 78%;
20. at least the CpGs defined by SEQ ID NOs 1 to 100 and identified at nucleotide positions 61 to 62, and wherein the sensitivity is at least 95% and specificity is at least 76%;
21. at least the CpGs defined by SEQ ID NOs 451 to 500 and identified at nucleotide positions 61 to 62, and wherein the sensitivity is at least 88% and specificity is at least 76%;
22. at least the CpGs defined by SEQ ID NOs 401 to 500 and identified at nucleotide positions 61 to 62, and wherein the sensitivity is at least 88% and specificity is at least 78%;
23. at least the CpGs defined by SEQ ID NOs 351 to 500 and identified at nucleotide positions 61 to 62, and wherein the sensitivity is at least 88% and specificity is at least 76%;
24. at least the CpGs defined by SEQ ID NOs 301 to 500 and identified at nucleotide positions 61 to 62, and wherein the sensitivity is at least 88% and specificity is at least 78%;
25. at least the CpGs defined by SEQ ID NOs 251 to 500 and identified at nucleotide positions 61 to 62, and wherein the sensitivity is at least 88% and specificity is at least 78%;
26. at least the CpGs defined by SEQ ID NOs 201 to 500 and identified at nucleotide positions 61 to 62, and wherein the sensitivity is at least 89% and specificity is at least 79%;
27. at least the CpGs defined by SEQ ID NOs 151 to 500 and identified at nucleotide positions 61 to 62, and wherein the sensitivity is at least 88% and specificity is at least 78%;
28. at least the CpGs defined by SEQ ID NOs 101 to 500 and identified at nucleotide positions 61 to 62, and wherein the sensitivity is at least 90% and specificity is at least 79%;
29. at least the CpGs defined by SEQ ID NOs 51 to 500 and identified at nucleotide positions 61 to 62, and wherein the sensitivity is at least 89% and specificity is at least 79%; or
30. at least the CpGs defined by SEQ ID NOs 1 to 500 and identified at nucleotide positions 61 to 62, and wherein the sensitivity is at least 95% and specificity is at least 76%.

In any of the described assays, the methylation status of the one or more CpGs in the panel is preferably determined by a β-value analysis, and the cancer is endometrial cancer. Preferably, the cancer is endometrial cancer.

In any of the assays described herein, wherein when the cancer index value for the individual is about 0.269 or more, the individual is assessed as having endometrial cancer or as having a high risk of endometrial cancer development, or wherein when the cancer index value for the individual is less than about 0.269, the individual is assessed as not having endometrial cancer or as having a low risk of endometrial cancer development, preferably wherein the assay comprises determining methylation β-values for each CpG in the panel of one or more CpGs, and more preferably wherein the assessing the presence, absence or development of cancer in an individual is based on the WID-EC-Index. The panel of one or more CpGs used to derive the cancer index value may comprise:
1. at least 50 of the CpGs identified at nucleotide positions 61 to 62 in SEQ ID NOs 1 to 500, and wherein the sensitivity is at least 75% and the specificity is at least 94%;
2. at least 100 of the CpGs identified at nucleotide positions 61 to 62 in SEQ ID NOs 1 to 500, and wherein the sensitivity is at least 73% and specificity is at least 96%;
3. at least 150 of the CpGs identified at nucleotide positions 61 to 62 in SEQ ID NOs 1 to 500, and wherein the sensitivity is at least 74% and specificity is at least 95%;
4. at least 200 of the CpGs identified at nucleotide positions 61 to 62 in SEQ ID NOs 1 to 500, and wherein the sensitivity is at least 73% and specificity is at least 96%;
5. at least 250 of the CpGs identified at nucleotide positions 61 to 62 in SEQ ID NOs 1 to 500, and wherein the sensitivity is at least 73% and specificity is at least 96%;
6. at least 300 of the CpGs identified at nucleotide positions 61 to 62 in SEQ ID NOs 1 to 500, and wherein the sensitivity is at least 73% and specificity is at least 97%;
7. at least 350 of the CpGs identified at nucleotide positions 61 to 62 in SEQ ID NOs 1 to 500, and wherein the sensitivity is at least 73% and specificity is at least 96%;
8. at least 400 of the CpGs identified at nucleotide positions 61 to 62 in SEQ ID NOs 1 to 500, and wherein the sensitivity is at least 77% and specificity is at least 96%;
9. at least 450 of the CpGs identified at nucleotide positions 61 to 62 in SEQ ID NOs 1 to 500, and wherein the sensitivity is at least 77% and specificity is at least 97%; or
10. at least 500 of the CpGs identified at nucleotide positions 61 to 62 in SEQ ID NOs 1 to 500, and wherein the sensitivity is at least 79% and specificity is at least 96%.

In any of the assays described herein, wherein when the cancer index value for the individual is about 0.269 or more, the individual is assessed as having endometrial cancer or as having a high risk of endometrial cancer development, or wherein when the cancer index value for the individual is less than about 0.269, the individual is assessed as not having endometrial cancer or as having a low risk of endometrial cancer development, preferably wherein the assay comprises determining methylation β-values for each CpG in the panel of one or more CpGs, and more preferably wherein the assessing the presence, absence or development of cancer in an individual is based on the WID-EC-Index. The panel of one or more CpGs used to derive the cancer index value may comprise:
a. at least 50 of the CpGs identified at nucleotide positions 61 to 62 in SEQ ID NOs 1 to 500, and wherein the sensitivity is at least 75% and the specificity is at least 94%;
b. at least the CpGs defined by SEQ ID NOs 1 to 50 and identified at nucleotide positions 61 to 62, and wherein the sensitivity is at least 73% and specificity is at least 98%;
c. at least the CpGs defined by SEQ ID NOs 1 to 100 and identified at nucleotide positions 61 to 62, and wherein the sensitivity is at least 75% and specificity is at least 98%;
d. at least the CpGs defined by SEQ ID NOs 1 to 150 and identified at nucleotide positions 61 to 62, and wherein the sensitivity is at least 79% and specificity is at least 97%.

In any of the assays described herein, wherein when the cancer index value for the individual is about 0.269 or more, the individual is assessed as having endometrial cancer or as having a high risk of endometrial cancer development, or wherein when the cancer index value for the individual is less than about 0.269, the individual is assessed as not having endometrial cancer or as having a low risk of endometrial cancer development, preferably wherein the assay comprises determining methylation β-values for each CpG in the panel of one or more CpGs, and more preferably wherein the assessing the presence, absence or development of cancer in an individual is based on the WID-EC-Index. The panel of one or more CpGs used to derive the cancer index value may comprise:
1. at least the CpGs defined by SEQ ID NOs 1 to 50 and identified at nucleotide positions 61 to 62, and wherein the sensitivity is at least 73% and specificity is at least 98%;
2. at least the CpGs defined by SEQ ID NOs 51 to 100 and identified at nucleotide positions 61 to 62, and wherein the sensitivity is at least 73% and specificity is at least 98%;
3. at least the CpGs defined by SEQ ID NOs 101 to 150 and identified at nucleotide positions 61 to 62, and wherein the sensitivity is at least 74% and specificity is at least 96%;
4. at least the CpGs defined by SEQ ID NOs 151 to 200 and identified at nucleotide positions 61 to 62, and wherein the sensitivity is at least 77% and specificity is at least 95%;
5. at least the CpGs defined by SEQ ID NOs 201 to 250 and identified at nucleotide positions 61 to 62, and wherein the sensitivity is at least 75% and specificity is at least 97%;
6. at least the CpGs defined by SEQ ID NOs 251 to 300 and identified at nucleotide positions 61 to 62, and wherein the sensitivity is at least 71% and specificity is at least 96%;
7. at least the CpGs defined by SEQ ID NOs 301 to 350 and identified at nucleotide positions 61 to 62, and wherein the sensitivity is at least 70% and specificity is at least 97%;
8. at least the CpGs defined by SEQ ID NOs 351 to 400 and identified at nucleotide positions 61 to 62, and wherein the sensitivity is at least 73% and specificity is at least 96%;
9. at least the CpGs defined by SEQ ID NOs 401 to 450 and identified at nucleotide positions 61 to 62, and wherein the sensitivity is at least 71% and specificity is at least 95%;
10. at least the CpGs defined by SEQ ID NOs 451 to 500 and identified at nucleotide positions 61 to 62, and wherein the sensitivity is at least 75% and specificity is at least 94%;
11. at least the CpGs defined by SEQ ID NOs 1 to 50 and identified at nucleotide positions 61 to 62, and wherein the sensitivity is at least 73% and specificity is at least 98%;
12. at least the CpGs defined by SEQ ID NOs 1 to 100 and identified at nucleotide positions 61 to 62, and wherein the sensitivity is at least 75% and specificity is at least 98%;
13. at least the CpGs defined by SEQ ID NOs 1 to 150 and identified at nucleotide positions 61 to 62, and wherein the sensitivity is at least 79% and specificity is at least 97%;
14. at least the CpGs defined by SEQ ID NOs 1 to 200 and identified at nucleotide positions 61 to 62, and wherein the sensitivity is at least 79% and specificity is at least 97%;
15. at least the CpGs defined by SEQ ID NOs 1 to 250 and identified at nucleotide positions 61 to 62, and wherein the sensitivity is at least 79% and specificity is at least 96%;
16. at least the CpGs defined by SEQ ID NOs 1 to 300 and identified at nucleotide positions 61 to 62, and wherein the sensitivity is at least 79% and specificity is at least 96%;
17. at least the CpGs defined by SEQ ID NOs 1 to 350 and identified at nucleotide positions 61 to 62, and wherein the sensitivity is at least 79% and specificity is at least 97%;
18. at least the CpGs defined by SEQ ID NOs 1 to 400 and identified at nucleotide positions 61 to 62, and wherein the sensitivity is at least 79% and specificity is at least 96%;
19. at least the CpGs defined by SEQ ID NOs 1 to 450 and identified at nucleotide positions 61 to 62, and wherein the sensitivity is at least 79% and specificity is at least 97%;
20. at least the CpGs defined by SEQ ID NOs 1 to 500 and identified at nucleotide positions 61 to 62, and wherein the sensitivity is at least 79% and specificity is at least 96%;
21. at least the CpGs defined by SEQ ID NOs 451 to 500 and identified at nucleotide positions 61 to 62, and wherein the sensitivity is at least 75% and specificity is at least 94%;
22. at least the CpGs defined by SEQ ID NOs 401 to 500 and identified at nucleotide positions 61 to 62, and wherein the sensitivity is at least 73% and specificity is at least 96%;
23. at least the CpGs defined by SEQ ID NOs 351 to 500 and identified at nucleotide positions 61 to 62, and wherein the sensitivity is at least 74% and specificity is at least 95%;
24. at least the CpGs defined by SEQ ID NOs 301 to 500 and identified at nucleotide positions 61 to 62, and wherein the sensitivity is at least 73% and specificity is at least 96%;
25. at least the CpGs defined by SEQ ID NOs 251 to 500 and identified at nucleotide positions 61 to 62, and wherein the sensitivity is at least 73% and specificity is at least 96%;
26. at least the CpGs defined by SEQ ID NOs 201 to 500 and identified at nucleotide positions 61 to 62, and wherein the sensitivity is at least 73% and specificity is at least 97%;
27. at least the CpGs defined by SEQ ID NOs 151 to 500 and identified at nucleotide positions 61 to 62, and wherein the sensitivity is at least 73% and specificity is at least 96%;
28. at least the CpGs defined by SEQ ID NOs 101 to 500 and identified at nucleotide positions 61 to 62, and wherein the sensitivity is at least 77% and specificity is at least 96%;
29. at least the CpGs defined by SEQ ID NOs 51 to 500 and identified at nucleotide positions 61 to 62, and wherein the sensitivity is at least 77% and specificity is at least 97%; or
30. at least the CpGs defined by SEQ ID NOs 1 to 500 and identified at nucleotide positions 61 to 62, and wherein the sensitivity is at least 79% and specificity is at least 96%.

In any of the described assays, the methylation status of the one or more CpGs in the panel is preferably determined by a β-value analysis, and the cancer is endometrial cancer. Preferably, the cancer is endometrial cancer.

In any of the assays described herein, wherein when the cancer index value for the individual is about 1.072 or more, the individual is assessed as having endometrial cancer or as having a high risk of endometrial cancer development, or wherein when the cancer index value for the individual is less than about 1.072, the individual is assessed as not having endometrial cancer or as having a low risk of endometrial cancer development, preferably wherein the assay comprises determining methylation β-values for each CpG in the panel of one or more CpGs, and more preferably wherein the assessing the presence, absence or development of cancer in an individual is based on the WID-EC-Index. The panel of one or more CpGs used to derive the cancer index value may comprise:
1. at least 50 of the CpGs identified at nucleotide positions 61 to 62 in SEQ ID NOs 1 to 500, and wherein the sensitivity is at least 58% and the specificity is at least 99%;
2. at least 100 of the CpGs identified at nucleotide positions 61 to 62 in SEQ ID NOs 1 to 500, and wherein the sensitivity is at least 53% and specificity is at least 99%;
3. at least 150 of the CpGs identified at nucleotide positions 61 to 62 in SEQ ID NOs 1 to 500, and wherein the sensitivity is at least 53% and specificity is at least 99%;
4. at least 200 of the CpGs identified at nucleotide positions 61 to 62 in SEQ ID NOs 1 to 500, and wherein the sensitivity is at least 53% and specificity is at least 99%;
5. at least 250 of the CpGs identified at nucleotide positions 61 to 62 in SEQ ID NOs 1 to 500, and wherein the sensitivity is at least 53% and specificity is at least 99%;
6. at least 300 of the CpGs identified at nucleotide positions 61 to 62 in SEQ ID NOs 1 to 500, and wherein the sensitivity is at least 55% and specificity is at least 99%;
7. at least 350 of the CpGs identified at nucleotide positions 61 to 62 in SEQ ID NOs 1 to 500, and wherein the sensitivity is at least 58% and specificity is at least 99%;
8. at least 400 of the CpGs identified at nucleotide positions 61 to 62 in SEQ ID NOs 1 to 500, and wherein the sensitivity is at least 58% and specificity is at least 99%;
9. at least 450 of the CpGs identified at nucleotide positions 61 to 62 in SEQ ID NOs 1 to 500, and wherein the sensitivity is at least 59% and specificity is 100%; or
10. at least 500 of the CpGs identified at nucleotide positions 61 to 62 in SEQ ID NOs 1 to 500, and wherein the sensitivity is at least 64% and specificity is 100%.

In any of the assays described herein, wherein when the cancer index value for the individual is about 1.072 or more, the individual is assessed as having endometrial cancer or as having a high risk of endometrial cancer development, or wherein when the cancer index value for the individual is less than about 1.072, the individual is assessed as not having endometrial cancer or as having a low risk of endometrial cancer development, preferably wherein the assay comprises determining methylation β-values for each CpG in the panel of one or more CpGs, and more preferably wherein the assessing the presence, absence or development of cancer in an individual is based on the WID-EC-Index. The panel of one or more CpGs used to derive the cancer index value may comprise:
a. at least 50 of the CpGs identified at nucleotide positions 61 to 62 in SEQ ID NOs 1 to 500, and wherein the sensitivity is at least 58% and the specificity is at least 99%;
b. at least the CpGs defined by SEQ ID NOs 1 to 50 and identified at nucleotide positions 61 to 62, and wherein the sensitivity is at least 60% and specificity is 100%;
c. at least the CpGs defined by SEQ ID NOs 1 to 100 and identified at nucleotide positions 61 to 62, and wherein the sensitivity is at least 63% and specificity is 100%;
d. at least the CpGs defined by SEQ ID NOs 1 to 150 and identified at nucleotide positions 61 to 62, and wherein the sensitivity is at least 64% and specificity is 100%.

In any of the assays described herein, wherein when the cancer index value for the individual is about 1.072 or more, the individual is assessed as having endometrial cancer or as having a high risk of endometrial cancer development, or wherein when the cancer index value for the individual is less than about 1.072, the individual is assessed as not having endometrial cancer or as having a low risk of endometrial cancer development, preferably wherein the assay comprises determining methylation β-values for each CpG in the panel of one or more CpGs, and more preferably wherein the assessing the presence, absence or development of cancer in an individual is based on the WID-EC-Index. The panel of one or more CpGs used to derive the cancer index value may comprise:
1. at least the CpGs defined by SEQ ID NOs 1 to 50 and identified at nucleotide positions 61 to 62, and wherein the sensitivity is at least 60% and specificity is 100%;
2. at least the CpGs defined by SEQ ID NOs 51 to 100 and identified at nucleotide positions 61 to 62, and wherein the sensitivity is at least 60% and specificity is at least 99%;
3. at least the CpGs defined by SEQ ID NOs 101 to 150 and identified at nucleotide positions 61 to 62, and wherein the sensitivity is at least 58% and specificity is 100%;
4. at least the CpGs defined by SEQ ID NOs 151 to 200 and identified at nucleotide positions 61 to 62, and wherein the sensitivity is at least 60% and specificity is at least 99%;
5. at least the CpGs defined by SEQ ID NOs 201 to 250 and identified at nucleotide positions 61 to 62, and wherein the sensitivity is at least 58% and specificity is at least 99%;
6. at least the CpGs defined by SEQ ID NOs 251 to 300 and identified at nucleotide positions 61 to 62, and wherein the sensitivity is at least 53% and specificity is at least 99%;
7. at least the CpGs defined by SEQ ID NOs 301 to 350 and identified at nucleotide positions 61 to 62, and wherein the sensitivity is at least 52% and specificity is 100%;
8. at least the CpGs defined by SEQ ID NOs 351 to 400 and identified at nucleotide positions 61 to 62, and wherein the sensitivity is at least 56% and specificity is at least 99%;
9. at least the CpGs defined by SEQ ID NOs 401 to 450 and identified at nucleotide positions 61 to 62, and wherein the sensitivity is at least 52% and specificity is at least 99%;
10. at least the CpGs defined by SEQ ID NOs 451 to 500 and identified at nucleotide positions 61 to 62, and wherein the sensitivity is at least 58% and specificity is at least 99%;
11. at least the CpGs defined by SEQ ID NOs 1 to 50 and identified at nucleotide positions 61 to 62, and wherein the sensitivity is at least 60% and specificity is 100%;
12. at least the CpGs defined by SEQ ID NOs 1 to 100 and identified at nucleotide positions 61 to 62, and wherein the sensitivity is at least 63% and specificity is 100%;
13. at least the CpGs defined by SEQ ID NOs 1 to 150 and identified at nucleotide positions 61 to 62, and wherein the sensitivity is at least 64% and specificity is 100%;
14. at least the CpGs defined by SEQ ID NOs 1 to 200 and identified at nucleotide positions 61 to 62, and wherein the sensitivity is at least 64% and specificity is 100%;
15. at least the CpGs defined by SEQ ID NOs 1 to 250 and identified at nucleotide positions 61 to 62, and wherein the sensitivity is at least 64% and specificity is 100%;
16. at least the CpGs defined by SEQ ID NOs 1 to 300 and identified at nucleotide positions 61 to 62, and wherein the sensitivity is at least 64% and specificity is 100%;
17. at least the CpGs defined by SEQ ID NOs 1 to 350 and identified at nucleotide positions 61 to 62, and wherein the sensitivity is at least 64% and specificity is 100%;
18. at least the CpGs defined by SEQ ID NOs 1 to 400 and identified at nucleotide positions 61 to 62, and wherein the sensitivity is at least 63% and specificity is 100%;
19. at least the CpGs defined by SEQ ID NOs 1 to 450 and identified at nucleotide positions 61 to 62, and wherein the sensitivity is at least 64% and specificity is 100%;
20. at least the CpGs defined by SEQ ID NOs 1 to 500 and identified at nucleotide positions 61 to 62, and wherein the sensitivity is at least 64% and specificity is 100%;
21. at least the CpGs defined by SEQ ID NOs 451 to 500 and identified at nucleotide positions 61 to 62, and wherein the sensitivity is at least 58% and specificity is at least 99%;
22. at least the CpGs defined by SEQ ID NOs 401 to 500 and identified at nucleotide positions 61 to 62, and wherein the sensitivity is at least 53% and specificity is at least 99%;
23. at least the CpGs defined by SEQ ID NOs 351 to 500 and identified at nucleotide positions 61 to 62, and wherein the sensitivity is at least 53% and specificity is at least 99%;
24. at least the CpGs defined by SEQ ID NOs 301 to 500 and identified at nucleotide positions 61 to 62, and wherein the sensitivity is at least 53% and specificity is at least 99%;
25. at least the CpGs defined by SEQ ID NOs 251 to 500 and identified at nucleotide positions 61 to 62, and wherein the sensitivity is at least 53% and specificity is at least 99%;
26. at least the CpGs defined by SEQ ID NOs 201 to 500 and identified at nucleotide positions 61 to 62, and wherein the sensitivity is at least 55% and specificity is at least 99%;
27. at least the CpGs defined by SEQ ID NOs 151 to 500 and identified at nucleotide positions 61 to 62, and wherein the sensitivity is at least 58% and specificity is at least 99%;
28. at least the CpGs defined by SEQ ID NOs 101 to 500 and identified at nucleotide positions 61 to 62, and wherein the sensitivity is at least 58% and specificity is at least 99%;
29. at least the CpGs defined by SEQ ID NOs 51 to 500 and identified at nucleotide positions 61 to 62, and wherein the sensitivity is at least 59% and specificity is 100%; or
30. at least the CpGs defined by SEQ ID NOs 1 to 500 and identified at nucleotide positions 61 to 62, and wherein the sensitivity is at least 64% and specificity is 100%.

In any of the described assays, the methylation status of the one or more CpGs in the panel is preferably determined by a β-value analysis, and the cancer is endometrial cancer. Preferably, the cancer is endometrial cancer.

The ROC data set out in Tables 3, 4 and 5 corresponding to each specified panel of SEQ ID NOs: 1 to 500 are derived by determining a cancer index value from said panel.

**Table 3**

| CpG Subset (SEQ ID NO) | AUC (internal validation) | Sensitivity (q = 1.072) | Specificity (q = 1.072) | Sensitivity (q = 0.269) | Specificity (q = 0.269) | Sensitivity (q = -0.201) | Specificity (q = -0.201) |
|---|---|---|---|---|---|---|---|
| 1-50 | 0.95 | 0.6 | 1 | 0.73 | 0.98 | 0.9 | 0.8 |
| 51-100 | 0.94 | 0.6 | 0.99 | 0.73 | 0.98 | 0.89 | 0.79 |
| 101-150 | 0.94 | 0.58 | 1 | 0.74 | 0.96 | 0.92 | 0.8 |
| 151-200 | 0.93 | 0.6 | 0.99 | 0.77 | 0.95 | 0.89 | 0.71 |
| 201-250 | 0.95 | 0.58 | 0.99 | 0.75 | 0.97 | 0.9 | 0.76 |
| 251-300 | 0.93 | 0.53 | 0.99 | 0.71 | 0.96 | 0.89 | 0.79 |
| 301-350 | 0.94 | 0.52 | 1 | 0.7 | 0.97 | 0.89 | 0.8 |
| 351-400 | 0.93 | 0.56 | 0.99 | 0.73 | 0.96 | 0.9 | 0.77 |
| 401-450 | 0.93 | 0.52 | 0.99 | 0.71 | 0.95 | 0.88 | 0.76 |
| 451-500 | 0.92 | 0.58 | 0.99 | 0.75 | 0.94 | 0.88 | 0.76 |

**Table 4**

| CpG Subset (SEQ ID NO) | AUC (internal validation) | Sensitivity (q = 1.072) | Specificity (q = 1.072) | Sensitivity (q = 0.269) | Specificity (q = 0.269) | Sensitivity (q = -0.201) | Specificity (q = -0.201) |
|---|---|---|---|---|---|---|---|
| 1-50 | 0.95 | 0.6 | 1 | 0.73 | 0.98 | 0.9 | 0.8 |
| 1-100 | 0.96 | 0.63 | 1 | 0.75 | 0.98 | 0.92 | 0.79 |
| 1-150 | 0.96 | 0.64 | 1 | 0.79 | 0.97 | 0.93 | 0.8 |
| 1-200 | 0.96 | 0.64 | 1 | 0.79 | 0.97 | 0.93 | 0.78 |
| 1-250 | 0.96 | 0.64 | 1 | 0.79 | 0.96 | 0.95 | 0.78 |
| 1-300 | 0.96 | 0.64 | 1 | 0.79 | 0.96 | 0.95 | 0.77 |
| 1-350 | 0.96 | 0.64 | 1 | 0.79 | 0.97 | 0.95 | 0.77 |
| 1-400 | 0.96 | 0.63 | 1 | 0.79 | 0.96 | 0.95 | 0.78 |
| 1-450 | 0.97 | 0.64 | 1 | 0.79 | 0.96 | 0.95 | 0.78 |
| 1-500 | 0.97 | 0.64 | 1 | 0.79 | 0.96 | 0.95 | 0.76 |

**Table 5**

| CpG subset (SEQ ID NO) | AUC (internal validation) | Sensitivity (q = 1.072) | Specificity (q = 1.072) | Sensitivity (q = 0.269) | Specificity (q = 0.269) | Sensitivity (q = -0.201) | Specificity (q = -0.201) |
|---|---|---|---|---|---|---|---|
| 1-500 | 0.97 | 0.64 | 1 | 0.79 | 0.96 | 0.95 | 0.76 |
| 51-500 | 0.95 | 0.59 | 1 | 0.77 | 0.97 | 0.89 | 0.79 |
| 101-500 | 0.94 | 0.58 | 0.99 | 0.77 | 0.96 | 0.9 | 0.79 |
| 151-500 | 0.94 | 0.58 | 0.99 | 0.73 | 0.96 | 0.88 | 0.78 |
| 201-500 | 0.94 | 0.55 | 0.99 | 0.73 | 0.97 | 0.89 | 0.79 |
| 251-500 | 0.93 | 0.53 | 0.99 | 0.73 | 0.96 | 0.88 | 0.78 |
| 301-500 | 0.93 | 0.53 | 0.99 | 0.73 | 0.96 | 0.88 | 0.78 |
| 351-500 | 0.93 | 0.53 | 0.99 | 0.74 | 0.95 | 0.88 | 0.76 |
| 401-500 | 0.93 | 0.53 | 0.99 | 0.73 | 0.96 | 0.88 | 0.78 |
| 451-500 | 0.92 | 0.58 | 0.99 | 0.75 | 0.94 | 0.88 | 0.76 |

The predicting of the presence, absence, or development of cancer in an individual may particularly involve determining the mean β-value across any panel of one or more CpGs defined herein. A threshold mean β-value may be applied in order to stratify an individual as having or not having cancer, or of having a high or low risk of endometrial cancer development.

In any of the assays described herein, wherein:
a. when the cancer index for the individual is about 0.025 or more the individual is classified as having endometrial cancer or as having a high risk of endometrial cancer development; or
b. when the cancer index for the individual is less than about 0.025 the individual is classified as not having cancer;
preferably wherein the assay has a specificity of 95% or more, more preferably wherein the assay comprises determining mean β-values for each CpG in the panel of one or more CpGs.

In any of the assays described herein, wherein:
a. when the cancer index for the individual is about 0.050 or more the individual is classified as having endometrial cancer or as having a high risk of endometrial cancer development; or
b. when the cancer index for the individual is less than about 0.050 the individual is classified as not having cancer;
preferably wherein the assay has a specificity of 95% or more, more preferably wherein the assay comprises determining mean β-values for each CpG in the panel of one or more CpGs.

In any of the assays described herein, wherein:
a. when the cancer index for the individual is about 0.075 or more the individual is classified as having endometrial cancer or as having a high risk of endometrial cancer development; or
b. when the cancer index for the individual is less than about 0.075 the individual is classified as not having cancer;
preferably wherein the assay has a specificity of 95% or more, more preferably wherein the assay comprises determining mean β-values for each CpG in the panel of one or more CpGs.

In any of the assays described herein, wherein:
a. when the cancer index for the individual is about 0.100 or more the individual is classified as having endometrial cancer or as having a high risk of endometrial cancer development; or
b. when the cancer index for the individual is less than about 0.100 the individual is classified as not having cancer;
preferably wherein the assay has a specificity of 95% or more, more preferably wherein the assay comprises determining mean β-values for each CpG in the panel of one or more CpGs.

In any of the assays described herein, wherein:
a. when the cancer index for the individual is about 0.125 or more the individual is classified as having endometrial cancer or as having a high risk of endometrial cancer development; or
b. when the cancer index for the individual is less than about 0.125 the individual is classified as not having cancer;
preferably wherein the assay has a specificity of 95% or more, more preferably wherein the assay comprises determining mean β-values for each CpG in the panel of one or more CpGs.

In any of the assays described herein, wherein:
a. when the cancer index for the individual is about 0.150 or more the individual is classified as having endometrial cancer or as having a high risk of endometrial cancer development; or
b. when the cancer index for the individual is less than about 0.150 the individual is classified as not having cancer;
preferably wherein the assay has a specificity of 95% or more, more preferably wherein the assay comprises determining mean β-values for each CpG in the panel of one or more CpGs.

In any of the assays described herein, wherein:
a. when the cancer index for the individual is about 0.175 or more the individual is classified as having endometrial cancer or as having a high risk of endometrial cancer development; or
b. when the cancer index for the individual is less than about 0.175 the individual is classified as not having cancer;
preferably wherein the assay has a specificity of 95% or more, more preferably wherein the assay comprises determining mean β-values for each CpG in the panel of one or more CpGs.

In any of the assays described herein, wherein:
a. when the cancer index for the individual is about 0.200 or more the individual is classified as having endometrial cancer or as having a high risk of endometrial cancer development; or
b. when the cancer index for the individual is less than about 0.200 the individual is classified as not having cancer;
preferably wherein the assay has a specificity of 95% or more, more preferably wherein the assay comprises determining mean β-values for each CpG in the panel of one or more CpGs.

In any of the assays described herein, wherein:
a. when the cancer index for the individual is about 0.225 or more the individual is classified as having endometrial cancer or as having a high risk of endometrial cancer development; or
b. when the cancer index for the individual is less than about 0.225 the individual is classified as not having cancer;
preferably wherein the assay has a specificity of 95% or more, more preferably wherein the assay comprises determining mean β-values for each CpG in the panel of one or more CpGs.

In any of the assays described herein, wherein:
a. when the cancer index for the individual is about 0.250 or more the individual is classified as having endometrial cancer or as having a high risk of endometrial cancer development; or
b. when the cancer index for the individual is less than about 0.250 the individual is classified as not having cancer;
preferably wherein the assay has a specificity of 95% or more, more preferably wherein the assay comprises determining mean β-values for each CpG in the panel of one or more CpGs.

In any of the assays described herein, wherein:
a. when the cancer index for the individual is about 0.275 or more the individual is classified as having endometrial cancer or as having a high risk of endometrial cancer development; or
b. when the cancer index for the individual is less than about 0.275 the individual is classified as not having cancer;
preferably wherein the assay has a specificity of 95% or more, more preferably wherein the assay comprises determining mean β-values for each CpG in the panel of one or more CpGs.

In any of the assays described herein, wherein:
a. when the cancer index for the individual is about 0.300 or more the individual is classified as having endometrial cancer or as having a high risk of endometrial cancer development; or
b. when the cancer index for the individual is less than about 0.300 the individual is classified as not having cancer;
preferably wherein the assay has a specificity of 95% or more, more preferably wherein the assay comprises determining mean β-values for each CpG in the panel of one or more CpGs.

In any of the assays described herein, wherein:
a. when the cancer index for the individual is about 0.325 or more the individual is classified as having endometrial cancer or as having a high risk of endometrial cancer development; or
b. when the cancer index for the individual is less than about 0.325 the individual is classified as not having cancer;
preferably wherein the assay has a specificity of 95% or more, more preferably wherein the assay comprises determining mean β-values for each CpG in the panel of one or more CpGs.

In any of the assays described herein, wherein:
a. when the cancer index for the individual is about 0.350 or more the individual is classified as having endometrial cancer or as having a high risk of endometrial cancer development; or
b. when the cancer index for the individual is less than about 0.350 the individual is classified as not having cancer;
preferably wherein the assay has a specificity of 95% or more, more preferably wherein the assay comprises determining mean β-values for each CpG in the panel of one or more CpGs.

In any of the assays described herein, wherein:
a. when the cancer index for the individual is about 0.375 or more the individual is classified as having endometrial cancer or as having a high risk of endometrial cancer development; or
b. when the cancer index for the individual is less than about 0.375 the individual is classified as not having cancer;
preferably wherein the assay has a specificity of 95% or more, more preferably wherein the assay comprises determining mean β-values for each CpG in the panel of one or more CpGs.

In any of the assays described herein, wherein:
a. when the cancer index for the individual is about 0.400 or more the individual is classified as having endometrial cancer or as having a high risk of endometrial cancer development; or
b. when the cancer index for the individual is less than about 0.400 the individual is classified as not having cancer;
preferably wherein the assay has a specificity of 95% or more, more preferably wherein the assay comprises determining mean β-values for each CpG in the panel of one or more CpGs.

In any of the assays described herein, wherein:
a. when the cancer index for the individual is about 0.425 or more the individual is classified as having endometrial cancer or as having a high risk of endometrial cancer development; or
b. when the cancer index for the individual is less than about 0.425 the individual is classified as not having cancer;
preferably wherein the assay has a specificity of 95% or more, more preferably wherein the assay comprises determining mean β-values for each CpG in the panel of one or more CpGs.

In any of the assays described herein, wherein:
a. when the cancer index for the individual is about 0.450 or more the individual is classified as having endometrial cancer or as having a high risk of endometrial cancer development; or
b. when the cancer index for the individual is less than about 0.450 the individual is classified as not having cancer;
preferably wherein the assay has a specificity of 95% or more, more preferably wherein the assay comprises determining mean β-values for each CpG in the panel of one or more CpGs.

In any of the assays described herein, wherein:
a. when the cancer index for the individual is about 0.475 or more the individual is classified as having endometrial cancer or as having a high risk of endometrial cancer development; or
b. when the cancer index for the individual is less than about 0.475 the individual is classified as not having cancer;
preferably wherein the assay has a specificity of 95% or more, more preferably wherein the assay comprises determining mean β-values for each CpG in the panel of one or more CpGs.

In any of the assays described herein, wherein:
a. when the cancer index for the individual is about 0.500 or more the individual is classified as having endometrial cancer or as having a high risk of endometrial cancer development; or
b. when the cancer index for the individual is less than about 0.500 the individual is classified as not having cancer;
preferably wherein the assay has a specificity of 95% or more, more preferably wherein the assay comprises determining mean β-values for each CpG in the panel of one or more CpGs.

In any of the assays described herein, wherein:
a. when the cancer index for the individual is about 0.525 or more the individual is classified as having endometrial cancer or as having a high risk of endometrial cancer development; or
b. when the cancer index for the individual is less than about 0.525 the individual is classified as not having cancer;
preferably wherein the assay has a specificity of 95% or more, more preferably wherein the assay comprises determining mean β-values for each CpG in the panel of one or more CpGs.

In any of the assays described herein, wherein:
a. when the cancer index for the individual is about 0.550 or more the individual is classified as having endometrial cancer or as having a high risk of endometrial cancer development; or
b. when the cancer index for the individual is less than about 0.550 the individual is classified as not having cancer;
preferably wherein the assay has a specificity of 95% or more, more preferably wherein the assay comprises determining mean β-values for each CpG in the panel of one or more CpGs.

In any of the described assays, the methylation status of the one or more CpGs in the panel is preferably determined by a β-value analysis, and the cancer is endometrial cancer.

The ROC data set out in Table 6 corresponding to each of SEQ ID NOs: 501 to 808 are derived by determining a cancer index value from a panel of CpGs in each instance whereby the panel comprises the CpGs denoted by [[CG]].

In any of the assays of the invention described herein, the individual may be stratified according to their cancer index value, and consequently be defined according to their endometrial cancer status and/or endometrial cancer risk. In any of the assays described herein, wherein when the cancer index value for the individual is:
a. less than about -0.660 the individual is assessed as not having endometrial cancer;
b. about -0.660 or more and less than about -0.430 the individual is assessed as having a low risk of endometrial cancer;
c. about -0.430 or more and less than about -0.230 the individual is assessed as having a moderate risk of endometrial cancer;
d. about -0.230 or more the individual is assessed as having a high risk of endometrial cancer;
preferably wherein the assay comprises determining methylation β-values for each CpG in the panel of one or more CpGs.

**Table 6.**

| **SEQ ID NO** | **chr** | **start** | **stop** | **Mean EC cases** | **Mean controls** | **cutoff** | **specificity** | **sensitivity** | **auc** |
|---|---|---|---|---|---|---|---|---|---|
| 501 | chr1 | 2.01E+08 | 2.01E+08 | 0.306 | 0.103 | 0.209 | 0.95 | 0.7 | 0.911 |
| 502 | chr19 | 39260654 | 39261254 | 0.393 | 0.108 | 0.271 | 0.95 | 0.77 | 0.903 |
| 503 | chr2 | 1.27E+08 | 1.27E+08 | 0.292 | 0.05 | 0.123 | 0.95 | 0.73 | 0.899 |
| 504 | chr2 | 1.27E+08 | 1.27E+08 | 0.292 | 0.05 | 0.123 | 0.95 | 0.73 | 0.899 |
| 505 | chr10 | 26506776 | 26507376 | 0.198 | 0.07 | 0.105 | 0.95 | 0.71 | 0.899 |
| 506 | chr10 | 16562170 | 16562770 | 0.149 | 0.038 | 0.056 | 0.95 | 0.66 | 0.897 |
| 507 | chr8 | 70983267 | 70983867 | 0.259 | 0.101 | 0.155 | 0.95 | 0.67 | 0.894 |
| 508 | chr10 | 26222906 | 26223506 | 0.226 | 0.047 | 0.083 | 0.95 | 0.71 | 0.893 |
| 509 | chr11 | 71954682 | 71955282 | 0.309 | 0.124 | 0.168 | 0.95 | 0.62 | 0.892 |
| 510 | chr1 | 2.08E+08 | 2.08E+08 | 0.346 | 0.093 | 0.265 | 0.95 | 0.6 | 0.891 |
| 511 | chr8 | 70983899 | 70984499 | 0.234 | 0.072 | 0.128 | 0.95 | 0.64 | 0.89 |
| 512 | chr2 | 74726333 | 74726933 | 0.243 | 0.06 | 0.127 | 0.95 | 0.64 | 0.888 |
| 513 | chr2 | 74726400 | 74727000 | 0.243 | 0.06 | 0.127 | 0.95 | 0.64 | 0.888 |
| 514 | chr20 | 59827621 | 59828221 | 0.288 | 0.061 | 0.133 | 0.95 | 0.74 | 0.888 |
| 515 | chr8 | 24771166 | 24771766 | 0.202 | 0.067 | 0.125 | 0.95 | 0.64 | 0.888 |
| 516 | chr2 | 74726349 | 74726949 | 0.243 | 0.06 | 0.127 | 0.95 | 0.64 | 0.888 |
| 517 | chr1 | 50886482 | 50887082 | 0.272 | 0.067 | 0.113 | 0.95 | 0.71 | 0.886 |
| 518 | chr1 | 50886649 | 50887249 | 0.272 | 0.067 | 0.113 | 0.95 | 0.71 | 0.886 |
| 519 | chr2 | 467879 | 468479 | 0.239 | 0.075 | 0.143 | 0.95 | 0.64 | 0.885 |
| 520 | chr5 | 1886694 | 1887294 | 0.212 | 0.054 | 0.095 | 0.95 | 0.66 | 0.884 |
| 521 | chr2 | 74726410 | 74727010 | 0.247 | 0.065 | 0.146 | 0.95 | 0.63 | 0.882 |
| 522 | chr6 | 29521120 | 29521720 | 0.239 | 0.045 | 0.098 | 0.95 | 0.68 | 0.881 |
| 523 | chr8 | 23564190 | 23564790 | 0.269 | 0.12 | 0.177 | 0.95 | 0.59 | 0.88 |
| 524 | chr6 | 29521056 | 29521656 | 0.241 | 0.044 | 0.098 | 0.95 | 0.68 | 0.879 |
| 525 | chr6 | 28367271 | 28367871 | 0.193 | 0.029 | 0.039 | 0.95 | 0.74 | 0.878 |
| 526 | chr2 | 1.27E+08 | 1.27E+08 | 0.279 | 0.066 | 0.154 | 0.95 | 0.68 | 0.878 |
| 527 | chr6 | 29521188 | 29521788 | 0.243 | 0.046 | 0.1 | 0.95 | 0.7 | 0.877 |
| 528 | chr6 | 29521206 | 29521806 | 0.254 | 0.058 | 0.124 | 0.95 | 0.67 | 0.875 |
| 529 | chr2 | 74726434 | 74727034 | 0.254 | 0.073 | 0.171 | 0.95 | 0.62 | 0.875 |
| 530 | chr6 | 29521201 | 29521801 | 0.245 | 0.05 | 0.108 | 0.95 | 0.68 | 0.874 |
| 531 | chr6 | 29521199 | 29521799 | 0.245 | 0.05 | 0.108 | 0.95 | 0.68 | 0.874 |
| 532 | chr3 | 1.3E+08 | 1.3E+08 | 0.232 | 0.05 | 0.091 | 0.95 | 0.7 | 0.874 |
| 533 | chr16 | 56696929 | 56697529 | 0.294 | 0.097 | 0.174 | 0.95 | 0.62 | 0.874 |
| 534 | chr6 | 29521302 | 29521902 | 0.251 | 0.059 | 0.123 | 0.95 | 0.67 | 0.873 |
| 535 | chr6 | 29521324 | 29521924 | 0.251 | 0.059 | 0.123 | 0.95 | 0.67 | 0.873 |
| 536 | chr4 | 1399889 | 1400489 | 0.241 | 0.097 | 0.176 | 0.95 | 0.67 | 0.873 |
| 537 | chr17 | 35299300 | 35299900 | 0.327 | 0.119 | 0.257 | 0.95 | 0.63 | 0.873 |
| 538 | chr6 | 29521331 | 29521931 | 0.251 | 0.059 | 0.123 | 0.95 | 0.67 | 0.873 |
| 539 | chr14 | 38724375 | 38724975 | 0.263 | 0.076 | 0.195 | 0.95 | 0.63 | 0.872 |
| 540 | chr14 | 38724348 | 38724948 | 0.263 | 0.076 | 0.195 | 0.95 | 0.63 | 0.872 |
| 541 | chr17 | 58498677 | 58499277 | 0.193 | 0.037 | 0.051 | 0.95 | 0.71 | 0.871 |
| 542 | chr6 | 29760205 | 29760805 | 0.168 | 0.045 | 0.055 | 0.95 | 0.7 | 0.871 |
| 543 | chr8 | 687084 | 687684 | 0.244 | 0.036 | 0.098 | 0.95 | 0.6 | 0.87 |
| 544 | chr10 | 47007902 | 47008502 | 0.186 | 0.036 | 0.071 | 0.95 | 0.67 | 0.87 |
| 545 | chr5 | 1887064 | 1887664 | 0.181 | 0.052 | 0.094 | 0.95 | 0.63 | 0.869 |
| 546 | chr5 | 2750458 | 2751058 | 0.412 | 0.174 | 0.374 | 0.95 | 0.6 | 0.869 |
| 547 | chr17 | 58498765 | 58499365 | 0.263 | 0.084 | 0.104 | 0.95 | 0.71 | 0.869 |
| 548 | chr10 | 1.09E+08 | 1.09E+08 | 0.213 | 0.046 | 0.119 | 0.95 | 0.6 | 0.867 |
| 549 | chr10 | 1.09E+08 | 1.09E+08 | 0.213 | 0.046 | 0.119 | 0.95 | 0.6 | 0.867 |
| 550 | chr18 | 11148469 | 11149069 | 0.251 | 0.092 | 0.168 | 0.95 | 0.59 | 0.867 |
| 551 | chr10 | 8097341 | 8097941 | 0.278 | 0.076 | 0.231 | 0.95 | 0.55 | 0.867 |
| 552 | chr9 | 842333 | 842933 | 0.224 | 0.049 | 0.101 | 0.95 | 0.6 | 0.865 |
| 553 | chr10 | 1.35E+08 | 1.35E+08 | 0.317 | 0.137 | 0.259 | 0.95 | 0.58 | 0.864 |
| 554 | chr10 | 1.35E+08 | 1.35E+08 | 0.317 | 0.137 | 0.259 | 0.95 | 0.58 | 0.864 |
| 555 | chr10 | 22633926 | 22634526 | 0.301 | 0.147 | 0.279 | 0.95 | 0.49 | 0.863 |
| 556 | chr18 | 11148614 | 11149214 | 0.244 | 0.077 | 0.14 | 0.95 | 0.64 | 0.863 |
| 557 | chr2 | 2.29E+08 | 2.29E+08 | 0.212 | 0.052 | 0.093 | 0.95 | 0.63 | 0.863 |
| 558 | chr10 | 1.3E+08 | 1.3E+08 | 0.249 | 0.071 | 0.119 | 0.95 | 0.66 | 0.862 |
| 559 | chr10 | 1.3E+08 | 1.3E+08 | 0.249 | 0.071 | 0.119 | 0.95 | 0.66 | 0.862 |
| 560 | chr10 | 8097389 | 8097989 | 0.323 | 0.098 | 0.311 | 0.95 | 0.55 | 0.861 |
| 561 | chr11 | 636875 | 637475 | 0.204 | 0.04 | 0.073 | 0.95 | 0.66 | 0.861 |
| 562 | chr11 | 636873 | 637473 | 0.204 | 0.04 | 0.073 | 0.95 | 0.66 | 0.861 |
| 563 | chr3 | 1.3E+08 | 1.3E+08 | 0.278 | 0.078 | 0.171 | 0.95 | 0.66 | 0.86 |
| 564 | chr13 | 79169983 | 79170583 | 0.276 | 0.113 | 0.206 | 0.95 | 0.59 | 0.86 |
| 565 | chr13 | 79170003 | 79170603 | 0.276 | 0.113 | 0.206 | 0.95 | 0.59 | 0.86 |
| 566 | chr3 | 1.3E+08 | 1.3E+08 | 0.278 | 0.078 | 0.171 | 0.95 | 0.66 | 0.86 |
| 567 | chr13 | 1.13E+08 | 1.13E+08 | 0.214 | 0.064 | 0.133 | 0.95 | 0.62 | 0.859 |
| 568 | chr16 | 6069101 | 6069701 | 0.254 | 0.112 | 0.233 | 0.95 | 0.49 | 0.859 |
| 569 | chr8 | 65281885 | 65282485 | 0.184 | 0.041 | 0.09 | 0.95 | 0.66 | 0.859 |
| 570 | chr10 | 1.33E+08 | 1.33E+08 | 0.186 | 0.037 | 0.072 | 0.95 | 0.62 | 0.859 |
| 571 | chr12 | 50297493 | 50298093 | 0.248 | 0.075 | 0.129 | 0.95 | 0.71 | 0.859 |
| 572 | chr13 | 1.13E+08 | 1.13E+08 | 0.282 | 0.131 | 0.257 | 0.95 | 0.55 | 0.858 |
| 573 | chr14 | 1.04E+08 | 1.04E+08 | 0.246 | 0.08 | 0.189 | 0.95 | 0.52 | 0.857 |
| 574 | chr21 | 38076742 | 38077342 | 0.24 | 0.107 | 0.175 | 0.95 | 0.59 | 0.857 |
| 575 | chr10 | 26222800 | 26223400 | 0.261 | 0.109 | 0.157 | 0.95 | 0.64 | 0.856 |
| 576 | chr2 | 29033480 | 29034080 | 0.281 | 0.114 | 0.172 | 0.95 | 0.7 | 0.856 |
| 577 | chr20 | 61638274 | 61638874 | 0.164 | 0.038 | 0.059 | 0.95 | 0.66 | 0.856 |
| 578 | chr19 | 44952430 | 44953030 | 0.142 | 0.042 | 0.064 | 0.95 | 0.6 | 0.855 |
| 579 | chr3 | 1.69E+08 | 1.69E+08 | 0.206 | 0.027 | 0.043 | 0.95 | 0.62 | 0.855 |
| 580 | chr5 | 1.15E+08 | 1.15E+08 | 0.216 | 0.099 | 0.178 | 0.95 | 0.52 | 0.855 |
| 581 | chr6 | 29520928 | 29521528 | 0.276 | 0.101 | 0.169 | 0.95 | 0.67 | 0.854 |
| 582 | chr6 | 29520920 | 29521520 | 0.276 | 0.101 | 0.169 | 0.95 | 0.67 | 0.854 |
| 583 | chr6 | 29520972 | 29521572 | 0.271 | 0.097 | 0.162 | 0.95 | 0.67 | 0.854 |
| 584 | chr2 | 45155691 | 45156291 | 0.178 | 0.045 | 0.075 | 0.95 | 0.66 | 0.854 |
| 585 | chr22 | 50623392 | 50623992 | 0.331 | 0.088 | 0.219 | 0.95 | 0.64 | 0.853 |
| 586 | chr22 | 50623387 | 50623987 | 0.331 | 0.088 | 0.219 | 0.95 | 0.64 | 0.853 |
| 587 | chr18 | 56940118 | 56940718 | 0.228 | 0.074 | 0.126 | 0.95 | 0.64 | 0.853 |
| 588 | chr7 | 49812802 | 49813402 | 0.179 | 0.032 | 0.058 | 0.95 | 0.63 | 0.853 |
| 589 | chr1 | 50513570 | 50514170 | 0.226 | 0.08 | 0.147 | 0.95 | 0.59 | 0.852 |
| 590 | chr17 | 42092103 | 42092703 | 0.26 | 0.138 | 0.179 | 0.95 | 0.64 | 0.852 |
| 591 | chr17 | 42092070 | 42092670 | 0.26 | 0.138 | 0.179 | 0.95 | 0.64 | 0.852 |
| 592 | chr6 | 35108639 | 35109239 | 0.256 | 0.057 | 0.198 | 0.95 | 0.56 | 0.851 |
| 593 | chr6 | 35108621 | 35109221 | 0.256 | 0.057 | 0.198 | 0.95 | 0.56 | 0.851 |
| 594 | chr2 | 99439233 | 99439833 | 0.414 | 0.129 | 0.387 | 0.95 | 0.56 | 0.851 |
| 595 | chr18 | 70533998 | 70534598 | 0.237 | 0.085 | 0.165 | 0.95 | 0.64 | 0.851 |
| 596 | chr8 | 24772009 | 24772609 | 0.168 | 0.053 | 0.109 | 0.95 | 0.59 | 0.85 |
| 597 | chr6 | 29760110 | 29760710 | 0.166 | 0.05 | 0.063 | 0.95 | 0.7 | 0.849 |
| 598 | chr20 | 23015632 | 23016232 | 0.31 | 0.125 | 0.253 | 0.95 | 0.53 | 0.849 |
| 599 | chr20 | 23015636 | 23016236 | 0.31 | 0.125 | 0.253 | 0.95 | 0.53 | 0.849 |
| 600 | chr10 | 1.11E+08 | 1.11E+08 | 0.219 | 0.079 | 0.178 | 0.95 | 0.51 | 0.849 |
| 601 | chr4 | 9783098 | 9783698 | 0.288 | 0.112 | 0.208 | 0.95 | 0.56 | 0.849 |
| 602 | chr6 | 29520852 | 29521452 | 0.278 | 0.116 | 0.186 | 0.95 | 0.64 | 0.848 |
| 603 | chr5 | 524203 | 524803 | 0.168 | 0.056 | 0.106 | 0.95 | 0.56 | 0.847 |
| 604 | chr3 | 1.47E+08 | 1.47E+08 | 0.316 | 0.148 | 0.315 | 0.95 | 0.48 | 0.847 |
| 605 | chr1 | 1.76E+08 | 1.76E+08 | 0.225 | 0.091 | 0.16 | 0.95 | 0.58 | 0.847 |
| 606 | chr8 | 35092578 | 35093178 | 0.228 | 0.085 | 0.192 | 0.95 | 0.56 | 0.847 |
| 607 | chr10 | 1.11E+08 | 1.11E+08 | 0.197 | 0.08 | 0.17 | 0.95 | 0.51 | 0.846 |
| 608 | chr4 | 9782892 | 9783492 | 0.315 | 0.154 | 0.245 | 0.95 | 0.55 | 0.845 |
| 609 | chr4 | 9782898 | 9783498 | 0.315 | 0.154 | 0.245 | 0.95 | 0.55 | 0.845 |
| 610 | chr4 | 9782906 | 9783506 | 0.315 | 0.154 | 0.245 | 0.95 | 0.55 | 0.845 |
| 611 | chr4 | 9782896 | 9783496 | 0.315 | 0.154 | 0.245 | 0.95 | 0.55 | 0.845 |
| 612 | chr8 | 1.41E+08 | 1.41E+08 | 0.194 | 0.043 | 0.079 | 0.95 | 0.62 | 0.844 |
| 613 | chr7 | 1.54E+08 | 1.54E+08 | 0.204 | 0.062 | 0.178 | 0.95 | 0.55 | 0.843 |
| 614 | chr7 | 1.54E+08 | 1.54E+08 | 0.204 | 0.062 | 0.178 | 0.95 | 0.55 | 0.843 |
| 615 | chr8 | 31496666 | 31497266 | 0.168 | 0.045 | 0.079 | 0.95 | 0.58 | 0.842 |
| 616 | chr19 | 15343965 | 15344565 | 0.24 | 0.094 | 0.185 | 0.95 | 0.53 | 0.842 |
| 617 | chr2 | 38301456 | 38302056 | 0.369 | 0.1 | 0.323 | 0.95 | 0.53 | 0.841 |
| 618 | chr3 | 50378131 | 50378731 | 0.165 | 0.026 | 0.044 | 0.95 | 0.63 | 0.841 |
| 619 | chr10 | 94834463 | 94835063 | 0.145 | 0.034 | 0.059 | 0.95 | 0.62 | 0.841 |
| 620 | chr4 | 779580 | 780180 | 0.193 | 0.049 | 0.089 | 0.95 | 0.62 | 0.84 |
| 621 | chr1 | 1566387 | 1566987 | 0.356 | 0.183 | 0.261 | 0.95 | 0.63 | 0.839 |
| 622 | chr3 | 2139956 | 2140556 | 0.153 | 0.048 | 0.108 | 0.95 | 0.55 | 0.839 |
| 623 | chr6 | 29894319 | 29894919 | 0.244 | 0.046 | 0.062 | 0.95 | 0.68 | 0.838 |
| 624 | chr8 | 97171601 | 97172201 | 0.166 | 0.048 | 0.086 | 0.95 | 0.58 | 0.838 |
| 625 | chr4 | 1.73E+08 | 1.73E+08 | 0.186 | 0.083 | 0.146 | 0.95 | 0.49 | 0.838 |
| 626 | chr11 | 66188222 | 66188822 | 0.229 | 0.145 | 0.209 | 0.95 | 0.47 | 0.835 |
| 627 | chr19 | 58095359 | 58095959 | 0.197 | 0.048 | 0.077 | 0.95 | 0.64 | 0.835 |
| 628 | chr5 | 40680837 | 40681437 | 0.173 | 0.03 | 0.051 | 0.95 | 0.67 | 0.833 |
| 629 | chr2 | 29033426 | 29034026 | 0.273 | 0.101 | 0.174 | 0.95 | 0.58 | 0.832 |
| 630 | chr7 | 70597621 | 70598221 | 0.188 | 0.075 | 0.148 | 0.95 | 0.59 | 0.832 |
| 631 | chr2 | 45169248 | 45169848 | 0.162 | 0.04 | 0.069 | 0.95 | 0.59 | 0.832 |
| 632 | chr9 | 1.01E+08 | 1.01E+08 | 0.169 | 0.038 | 0.136 | 0.95 | 0.48 | 0.831 |
| 633 | chr6 | 1.23E+08 | 1.23E+08 | 0.165 | 0.051 | 0.103 | 0.95 | 0.56 | 0.831 |
| 634 | chr8 | 98289929 | 98290529 | 0.205 | 0.095 | 0.157 | 0.95 | 0.58 | 0.83 |
| 635 | chr17 | 48546021 | 48546621 | 0.26 | 0.102 | 0.18 | 0.95 | 0.56 | 0.83 |
| 636 | chr19 | 22805884 | 22806484 | 0.324 | 0.142 | 0.298 | 0.95 | 0.51 | 0.83 |
| 637 | chr10 | 44880245 | 44880845 | 0.167 | 0.03 | 0.048 | 0.95 | 0.55 | 0.829 |
| 638 | chr10 | 44880408 | 44881008 | 0.167 | 0.03 | 0.048 | 0.95 | 0.55 | 0.829 |
| 639 | chr7 | 1.57E+08 | 1.57E+08 | 0.273 | 0.072 | 0.154 | 0.95 | 0.59 | 0.829 |
| 640 | chr10 | 44880430 | 44881030 | 0.167 | 0.03 | 0.048 | 0.95 | 0.55 | 0.829 |
| 641 | chr6 | 29894379 | 29894979 | 0.213 | 0.053 | 0.083 | 0.95 | 0.66 | 0.828 |
| 642 | chr18 | 11149170 | 11149770 | 0.281 | 0.098 | 0.188 | 0.95 | 0.52 | 0.828 |
| 643 | chr6 | 29894041 | 29894641 | 0.319 | 0.092 | 0.183 | 0.95 | 0.64 | 0.828 |
| 644 | chr6 | 1.23E+08 | 1.23E+08 | 0.167 | 0.05 | 0.101 | 0.95 | 0.56 | 0.828 |
| 645 | chr10 | 1.19E+08 | 1.19E+08 | 0.18 | 0.025 | 0.034 | 0.95 | 0.66 | 0.828 |
| 646 | chr5 | 60921591 | 60922191 | 0.175 | 0.027 | 0.037 | 0.95 | 0.58 | 0.825 |
| 647 | chr20 | 25062147 | 25062747 | 0.203 | 0.038 | 0.072 | 0.95 | 0.55 | 0.825 |
| 648 | chr2 | 1.21E+08 | 1.21E+08 | 0.548 | 0.441 | 0.552 | 0.95 | 0.49 | 0.824 |
| 649 | chr7 | 95025536 | 95026136 | 0.268 | 0.125 | 0.214 | 0.95 | 0.53 | 0.824 |
| 650 | chr8 | 1.11E+08 | 1.11E+08 | 0.132 | 0.035 | 0.065 | 0.95 | 0.53 | 0.824 |
| 651 | chr20 | 54578784 | 54579384 | 0.236 | 0.11 | 0.185 | 0.95 | 0.49 | 0.824 |
| 652 | chr1 | 34642202 | 34642802 | 0.319 | 0.205 | 0.294 | 0.95 | 0.47 | 0.823 |
| 653 | chr5 | 40681421 | 40682021 | 0.227 | 0.083 | 0.129 | 0.95 | 0.59 | 0.82 |
| 654 | chr3 | 6902545 | 6903145 | 0.186 | 0.06 | 0.138 | 0.95 | 0.52 | 0.82 |
| 655 | chr4 | 1.22E+08 | 1.22E+08 | 0.201 | 0.073 | 0.118 | 0.95 | 0.53 | 0.819 |
| 656 | chr6 | 35108582 | 35109182 | 0.298 | 0.127 | 0.254 | 0.95 | 0.56 | 0.816 |
| 657 | chr19 | 57182818 | 57183418 | 0.225 | 0.085 | 0.18 | 0.95 | 0.48 | 0.816 |
| 658 | chr16 | 31580689 | 31581289 | 0.325 | 0.24 | 0.301 | 0.95 | 0.53 | 0.814 |
| 659 | chr18 | 55094949 | 55095549 | 0.228 | 0.076 | 0.176 | 0.95 | 0.52 | 0.814 |
| 660 | chr11 | 14926704 | 14927304 | 0.404 | 0.242 | 0.411 | 0.95 | 0.47 | 0.814 |
| 661 | chr2 | 1.4E+08 | 1.4E+08 | 0.178 | 0.034 | 0.072 | 0.95 | 0.56 | 0.813 |
| 662 | chr6 | 1.69E+08 | 1.69E+08 | 0.315 | 0.094 | 0.358 | 0.95 | 0.41 | 0.812 |
| 663 | chr2 | 91634983 | 91635583 | 0.335 | 0.146 | 0.313 | 0.95 | 0.44 | 0.811 |
| 664 | chr1 | 1.13E+08 | 1.13E+08 | 0.261 | 0.107 | 0.221 | 0.95 | 0.48 | 0.811 |
| 665 | chr11 | 70508110 | 70508710 | 0.254 | 0.117 | 0.256 | 0.95 | 0.44 | 0.81 |
| 666 | chr1 | 2.37E+08 | 2.37E+08 | 0.261 | 0.111 | 0.202 | 0.95 | 0.55 | 0.809 |
| 667 | chr19 | 52222398 | 52222998 | 0.346 | 0.225 | 0.345 | 0.95 | 0.48 | 0.808 |
| 668 | chr21 | 38077373 | 38077973 | 0.196 | 0.09 | 0.165 | 0.95 | 0.51 | 0.807 |
| 669 | chr2 | 2.03E+08 | 2.03E+08 | 0.166 | 0.057 | 0.134 | 0.95 | 0.47 | 0.807 |
| 670 | chr1 | 2.33E+08 | 2.33E+08 | 0.347 | 0.213 | 0.3 | 0.95 | 0.56 | 0.806 |
| 671 | chr1 | 1.2E+08 | 1.2E+08 | 0.171 | 0.094 | 0.153 | 0.95 | 0.45 | 0.806 |
| 672 | chr11 | 626875 | 627475 | 0.384 | 0.171 | 0.416 | 0.95 | 0.47 | 0.805 |
| 673 | chr11 | 626870 | 627470 | 0.384 | 0.171 | 0.416 | 0.95 | 0.47 | 0.805 |
| 674 | chr19 | 22444293 | 22444893 | 0.234 | 0.126 | 0.223 | 0.95 | 0.48 | 0.803 |
| 675 | chr3 | 42947265 | 42947865 | 0.289 | 0.186 | 0.232 | 0.95 | 0.6 | 0.803 |
| 676 | chr1 | 18434276 | 18434876 | 0.136 | 0.029 | 0.047 | 0.95 | 0.6 | 0.801 |
| 677 | chr13 | 50701319 | 50701919 | 0.231 | 0.063 | 0.162 | 0.95 | 0.55 | 0.799 |
| 678 | chr1 | 3567346 | 3567946 | 0.328 | 0.21 | 0.311 | 0.95 | 0.52 | 0.799 |
| 679 | chr6 | 29760473 | 29761073 | 0.237 | 0.147 | 0.17 | 0.95 | 0.53 | 0.799 |
| 680 | chr1 | 2.37E+08 | 2.37E+08 | 0.359 | 0.228 | 0.314 | 0.95 | 0.56 | 0.799 |
| 681 | chr7 | 98245706 | 98246306 | 0.235 | 0.133 | 0.2 | 0.95 | 0.48 | 0.798 |
| 682 | chr4 | 1.26E+08 | 1.26E+08 | 0.176 | 0.046 | 0.08 | 0.95 | 0.59 | 0.797 |
| 683 | chr21 | 22370138 | 22370738 | 0.142 | 0.043 | 0.089 | 0.95 | 0.45 | 0.796 |
| 684 | chr12 | 75602930 | 75603530 | 0.245 | 0.181 | 0.253 | 0.95 | 0.4 | 0.793 |
| 685 | chr1 | 3567112 | 3567712 | 0.289 | 0.177 | 0.274 | 0.95 | 0.49 | 0.792 |
| 686 | chr11 | 32355024 | 32355624 | 0.447 | 0.355 | 0.451 | 0.95 | 0.49 | 0.792 |
| 687 | chr6 | 1.05E+08 | 1.05E+08 | 0.346 | 0.242 | 0.346 | 0.95 | 0.37 | 0.791 |
| 688 | chr12 | 4918781 | 4919381 | 0.227 | 0.078 | 0.181 | 0.95 | 0.52 | 0.79 |
| 689 | chr12 | 4918838 | 4919438 | 0.227 | 0.078 | 0.181 | 0.95 | 0.52 | 0.79 |
| 690 | chr1 | 2.33E+08 | 2.33E+08 | 0.312 | 0.197 | 0.287 | 0.95 | 0.53 | 0.789 |
| 691 | chr12 | 1.08E+08 | 1.08E+08 | 0.14 | 0.035 | 0.057 | 0.95 | 0.59 | 0.788 |
| 692 | chr2 | 85811132 | 85811732 | 0.307 | 0.19 | 0.292 | 0.95 | 0.49 | 0.788 |
| 693 | chr2 | 1747812 | 1748412 | 0.227 | 0.059 | 0.11 | 0.95 | 0.51 | 0.787 |
| 694 | chr10 | 1.01E+08 | 1.01E+08 | 0.189 | 0.035 | 0.081 | 0.95 | 0.51 | 0.787 |
| 695 | chr2 | 1747832 | 1748432 | 0.227 | 0.059 | 0.11 | 0.95 | 0.51 | 0.787 |
| 696 | chr15 | 35046903 | 35047503 | 0.151 | 0.036 | 0.057 | 0.95 | 0.58 | 0.784 |
| 697 | chr2 | 5835931 | 5836531 | 0.159 | 0.045 | 0.076 | 0.95 | 0.52 | 0.784 |
| 698 | chr6 | 73331773 | 73332373 | 0.231 | 0.045 | 0.076 | 0.95 | 0.47 | 0.783 |
| 699 | chr7 | 1.57E+08 | 1.57E+08 | 0.212 | 0.092 | 0.198 | 0.95 | 0.41 | 0.781 |
| 700 | chr10 | 1.35E+08 | 1.35E+08 | 0.241 | 0.128 | 0.183 | 0.95 | 0.56 | 0.78 |
| 701 | chr20 | 2780962 | 2781562 | 0.137 | 0.046 | 0.063 | 0.95 | 0.58 | 0.777 |
| 702 | chr14 | 52535847 | 52536447 | 0.168 | 0.063 | 0.095 | 0.95 | 0.53 | 0.777 |
| 703 | chr3 | 1.3E+08 | 1.3E+08 | 0.421 | 0.293 | 0.426 | 0.95 | 0.42 | 0.777 |
| 704 | chr14 | 52535875 | 52536475 | 0.168 | 0.063 | 0.095 | 0.95 | 0.53 | 0.777 |
| 705 | chr3 | 238315 | 238915 | 0.133 | 0.032 | 0.074 | 0.95 | 0.45 | 0.777 |
| 706 | chr3 | 1.3E+08 | 1.3E+08 | 0.421 | 0.293 | 0.426 | 0.95 | 0.42 | 0.777 |
| 707 | chr10 | 7449812 | 7450412 | 0.21 | 0.082 | 0.109 | 0.95 | 0.51 | 0.777 |
| 708 | chr18 | 74962067 | 74962667 | 0.204 | 0.05 | 0.099 | 0.95 | 0.53 | 0.776 |
| 709 | chr18 | 74962059 | 74962659 | 0.204 | 0.05 | 0.099 | 0.95 | 0.53 | 0.776 |
| 710 | chr18 | 74962069 | 74962669 | 0.204 | 0.05 | 0.099 | 0.95 | 0.53 | 0.776 |
| 711 | chr1 | 1.49E+08 | 1.49E+08 | 0.359 | 0.184 | 0.392 | 0.95 | 0.44 | 0.775 |
| 712 | chr1 | 1.8E+08 | 1.8E+08 | 0.276 | 0.173 | 0.245 | 0.95 | 0.48 | 0.775 |
| 713 | chr18 | 13641440 | 13642040 | 0.297 | 0.154 | 0.362 | 0.95 | 0.34 | 0.775 |
| 714 | chr10 | 73846957 | 73847557 | 0.15 | 0.037 | 0.049 | 0.95 | 0.58 | 0.771 |
| 715 | chr17 | 1173848 | 1174448 | 0.267 | 0.12 | 0.263 | 0.95 | 0.44 | 0.769 |
| 716 | chr6 | 43612608 | 43613208 | 0.5 | 0.427 | 0.533 | 0.95 | 0.4 | 0.767 |
| 717 | chr7 | 97360952 | 97361552 | 0.196 | 0.107 | 0.171 | 0.95 | 0.44 | 0.762 |
| 718 | chr19 | 57610570 | 57611170 | 0.248 | 0.173 | 0.257 | 0.95 | 0.36 | 0.76 |
| 719 | chr5 | 1.69E+08 | 1.69E+08 | 0.221 | 0.11 | 0.162 | 0.95 | 0.48 | 0.758 |
| 720 | chr12 | 1.27E+08 | 1.27E+08 | 0.218 | 0.09 | 0.211 | 0.95 | 0.4 | 0.757 |
| 721 | chr1 | 36043101 | 36043701 | 0.257 | 0.138 | 0.296 | 0.95 | 0.33 | 0.751 |
| 722 | chr6 | 1.06E+08 | 1.06E+08 | 0.321 | 0.213 | 0.361 | 0.95 | 0.32 | 0.742 |
| 723 | chr19 | 56904645 | 56905245 | 0.126 | 0.043 | 0.056 | 0.95 | 0.49 | 0.738 |
| 724 | chr2 | 2.09E+08 | 2.09E+08 | 0.254 | 0.142 | 0.257 | 0.95 | 0.34 | 0.73 |
| 725 | chr14 | 1.02E+08 | 1.02E+08 | 0.378 | 0.293 | 0.409 | 0.95 | 0.36 | 0.726 |
| 726 | chr8 | 1.45E+08 | 1.45E+08 | 0.358 | 0.235 | 0.46 | 0.95 | 0.26 | 0.726 |
| 727 | chr11 | 20690634 | 20691234 | 0.155 | 0.041 | 0.086 | 0.95 | 0.48 | 0.72 |
| 728 | chr11 | 20690861 | 20691461 | 0.153 | 0.043 | 0.081 | 0.95 | 0.48 | 0.715 |
| 729 | chr6 | 29691700 | 29692300 | 0.255 | 0.181 | 0.257 | 0.95 | 0.33 | 0.713 |
| 730 | chr11 | 20690826 | 20691426 | 0.165 | 0.045 | 0.088 | 0.95 | 0.48 | 0.713 |
| 731 | chr3 | 3840830 | 3841430 | 0.101 | 0.018 | 0.025 | 0.95 | 0.44 | 0.708 |
| 732 | chr2 | 25499746 | 25500346 | 0.37 | 0.263 | 0.459 | 0.95 | 0.25 | 0.707 |
| 733 | chr1 | 1.11E+08 | 1.11E+08 | 0.195 | 0.049 | 0.089 | 0.95 | 0.45 | 0.704 |
| 734 | chr17 | 77721689 | 77722289 | 0.202 | 0.132 | 0.199 | 0.95 | 0.44 | 0.697 |
| 735 | chr3 | 1.25E+08 | 1.25E+08 | 0.188 | 0.113 | 0.329 | 0.95 | 0.15 | 0.697 |
| 736 | chr11 | 1.23E+08 | 1.23E+08 | 0.216 | 0.15 | 0.271 | 0.95 | 0.27 | 0.693 |
| 737 | chr16 | 88700616 | 88701216 | 0.249 | 0.183 | 0.297 | 0.95 | 0.36 | 0.638 |
| 738 | chr19 | 39260654 | 39261253 | 0.393 | 0.108 | 0.271 | 0.95 | 0.77 | 0.903 |
| 739 | chr19 | 39260654 | 39261253 | 0.393 | 0.108 | 0.271 | 0.95 | 0.77 | 0.903 |
| 740 | chr2 | 12741363 6 | 127414235 | 0.292 | 0.05 | 0.123 | 0.95 | 0.73 | 0.899 |
| 741 | chr2 | 12741353 1 | 127414130 | 0.292 | 0.05 | 0.123 | 0.95 | 0.73 | 0.899 |
| 742 | chr10 | 26506776 | 26507375 | 0.198 | 0.07 | 0.105 | 0.95 | 0.71 | 0.899 |
| 743 | chr2 | 12741363 6 | 127414235 | 0.292 | 0.05 | 0.123 | 0.95 | 0.73 | 0.899 |
| 744 | chr2 | 12741353 1 | 127414130 | 0.292 | 0.05 | 0.123 | 0.95 | 0.73 | 0.899 |
| 745 | chr10 | 26506776 | 26507375 | 0.198 | 0.07 | 0.105 | 0.95 | 0.71 | 0.899 |
| 746 | chr8 | 24771026 | 24771625 | 0.209 | 0.059 | 0.119 | 0.95 | 0.67 | 0.895 |
| 747 | chr8 | 24771026 | 24771625 | 0.209 | 0.059 | 0.119 | 0.95 | 0.67 | 0.895 |
| 748 | chr10 | 26222906 | 26223505 | 0.226 | 0.047 | 0.083 | 0.95 | 0.71 | 0.893 |
| 749 | chr10 | 26222906 | 26223505 | 0.226 | 0.047 | 0.083 | 0.95 | 0.71 | 0.893 |
| 750 | chr1 | 20813248 7 | 208133086 | 0.346 | 0.093 | 0.265 | 0.95 | 0.6 | 0.891 |
| 751 | chr1 | 20813248 7 | 208133086 | 0.346 | 0.093 | 0.265 | 0.95 | 0.6 | 0.891 |
| 752 | chr1 | 50886482 | 50887081 | 0.272 | 0.067 | 0.113 | 0.95 | 0.71 | 0.886 |
| 753 | chr1 | 50886649 | 50887248 | 0.272 | 0.067 | 0.113 | 0.95 | 0.71 | 0.886 |
| 754 | chr1 | 50886482 | 50887081 | 0.272 | 0.067 | 0.113 | 0.95 | 0.71 | 0.886 |
| 755 | chr1 | 50886649 | 50887248 | 0.272 | 0.067 | 0.113 | 0.95 | 0.71 | 0.886 |
| 756 | chr6 | 28367271 | 28367870 | 0.193 | 0.029 | 0.039 | 0.95 | 0.74 | 0.878 |
| 757 | chr6 | 28367271 | 28367870 | 0.193 | 0.029 | 0.039 | 0.95 | 0.74 | 0.878 |
| 758 | chr2 | 74726434 | 74727033 | 0.254 | 0.073 | 0.171 | 0.95 | 0.62 | 0.875 |
| 759 | chr2 | 74726434 | 74727033 | 0.254 | 0.073 | 0.171 | 0.95 | 0.62 | 0.875 |
| 760 | chr3 | 12969349 1 | 129694090 | 0.232 | 0.05 | 0.091 | 0.95 | 0.7 | 0.874 |
| 761 | chr3 | 12969349 1 | 129694090 | 0.232 | 0.05 | 0.091 | 0.95 | 0.7 | 0.874 |
| 762 | chr9 | 842333 | 842932 | 0.224 | 0.049 | 0.101 | 0.95 | 0.6 | 0.865 |
| 763 | chr9 | 842333 | 842932 | 0.224 | 0.049 | 0.101 | 0.95 | 0.6 | 0.865 |
| 764 | chr18 | 11148614 | 11149213 | 0.244 | 0.077 | 0.14 | 0.95 | 0.64 | 0.863 |
| 765 | chr20 | 59828228 | 59828827 | 0.216 | 0.045 | 0.082 | 0.95 | 0.67 | 0.863 |
| 766 | chr18 | 11148614 | 11149213 | 0.244 | 0.077 | 0.14 | 0.95 | 0.64 | 0.863 |
| 767 | chr20 | 59828228 | 59828827 | 0.216 | 0.045 | 0.082 | 0.95 | 0.67 | 0.863 |
| 768 | chr6 | 29520919 | 29521918 | 0.261 | 0.086 | 0.146 | 0.95 | 0.68 | 0.862 |
| 769 | chr6 | 29520919 | 29521918 | 0.261 | 0.086 | 0.146 | 0.95 | 0.68 | 0.862 |
| 770 | chr6 | 29520919 | 29521918 | 0.261 | 0.086 | 0.146 | 0.95 | 0.68 | 0.862 |
| 771 | chr6 | 29520919 | 29521918 | 0.261 | 0.086 | 0.146 | 0.95 | 0.68 | 0.862 |
| 772 | chr6 | 29520919 | 29521918 | 0.261 | 0.086 | 0.146 | 0.95 | 0.68 | 0.862 |
| 773 | chr6 | 29520919 | 29521918 | 0.261 | 0.086 | 0.146 | 0.95 | 0.68 | 0.862 |
| 774 | chr6 | 29520919 | 29521918 | 0.261 | 0.086 | 0.146 | 0.95 | 0.68 | 0.862 |
| 775 | chr6 | 29520919 | 29521918 | 0.261 | 0.086 | 0.146 | 0.95 | 0.68 | 0.862 |
| 776 | chr10 | 8097389 | 8097988 | 0.323 | 0.098 | 0.311 | 0.95 | 0.55 | 0.861 |
| 777 | chr10 | 8097389 | 8097988 | 0.323 | 0.098 | 0.311 | 0.95 | 0.55 | 0.861 |
| 778 | chr3 | 12969328 6 | 129693885 | 0.278 | 0.078 | 0.171 | 0.95 | 0.66 | 0.86 |
| 779 | chr3 | 12969328 6 | 129693885 | 0.278 | 0.078 | 0.171 | 0.95 | 0.66 | 0.86 |
| 780 | chr12 | 50297493 | 50298092 | 0.248 | 0.075 | 0.129 | 0.95 | 0.71 | 0.859 |
| 781 | chr12 | 50297493 | 50298092 | 0.248 | 0.075 | 0.129 | 0.95 | 0.71 | 0.859 |
| 782 | chr22 | 50623387 | 50623986 | 0.331 | 0.088 | 0.219 | 0.95 | 0.64 | 0.853 |
| 783 | chr22 | 50623387 | 50623986 | 0.331 | 0.088 | 0.219 | 0.95 | 0.64 | 0.853 |
| 784 | chr6 | 35108639 | 35109238 | 0.256 | 0.057 | 0.198 | 0.95 | 0.56 | 0.851 |
| 785 | chr6 | 35108639 | 35109238 | 0.256 | 0.057 | 0.198 | 0.95 | 0.56 | 0.851 |
| 786 | chr6 | 29760110 | 29760709 | 0.166 | 0.05 | 0.063 | 0.95 | 0.7 | 0.849 |
| 787 | chr6 | 29760110 | 29760709 | 0.166 | 0.05 | 0.063 | 0.95 | 0.7 | 0.849 |
| 788 | chr10 | 10640121 7 | 106401816 | 0.186 | 0.038 | 0.08 | 0.95 | 0.58 | 0.846 |
| 789 | chr10 | 10640121 7 | 106401816 | 0.186 | 0.038 | 0.08 | 0.95 | 0.58 | 0.846 |
| 790 | chr19 | 15343965 | 15344564 | 0.24 | 0.094 | 0.185 | 0.95 | 0.53 | 0.842 |
| 791 | chr19 | 15343965 | 15344564 | 0.24 | 0.094 | 0.185 | 0.95 | 0.53 | 0.842 |
| 792 | chr3 | 50378131 | 50378730 | 0.165 | 0.026 | 0.044 | 0.95 | 0.63 | 0.841 |
| 793 | chr3 | 50378131 | 50378730 | 0.165 | 0.026 | 0.044 | 0.95 | 0.63 | 0.841 |
| 794 | chr8 | 97171601 | 97172200 | 0.166 | 0.048 | 0.086 | 0.95 | 0.58 | 0.838 |
| 795 | chr8 | 97171601 | 97172200 | 0.166 | 0.048 | 0.086 | 0.95 | 0.58 | 0.838 |
| 796 | chr5 | 40680837 | 40681436 | 0.173 | 0.03 | 0.051 | 0.95 | 0.67 | 0.833 |
| 797 | chr5 | 40680837 | 40681436 | 0.173 | 0.03 | 0.051 | 0.95 | 0.67 | 0.833 |
| 798 | chr2 | 29033426 | 29034025 | 0.273 | 0.101 | 0.174 | 0.95 | 0.58 | 0.832 |
| 799 | chr2 | 29033426 | 29034025 | 0.273 | 0.101 | 0.174 | 0.95 | 0.58 | 0.832 |
| 800 | chr9 | 10061630 7 | 100616906 | 0.169 | 0.038 | 0.136 | 0.95 | 0.48 | 0.831 |
| 801 | chr9 | 10061630 7 | 100616906 | 0.169 | 0.038 | 0.136 | 0.95 | 0.48 | 0.831 |
| 802 | chr4 | 107425 | 108024 | 0.331 | 0.211 | 0.293 | 0.95 | 0.55 | 0.826 |
| 803 | chr4 | 107425 | 108024 | 0.331 | 0.211 | 0.293 | 0.95 | 0.55 | 0.826 |
| 804 | chr1 | 23294116 8 | 232941767 | 0.347 | 0.213 | 0.3 | 0.95 | 0.56 | 0.806 |
| 805 | chr1 | 23294116 8 | 232941767 | 0.347 | 0.213 | 0.3 | 0.95 | 0.56 | 0.806 |
| 806 | chr1 | 20084245 6 | 200843056 | 0.306 | 0.103 | 0.209 | 0.95 | 0.7 | 0.911 |
| 807 | chr10 | 26506553 | 26507153 | 0.227 | 0.068 | 0.104 | 0.95 | 0.71 | 0.905 |
| 808 | chr10 | 26506633 | 26507233 | 0.227 | 0.068 | 0.104 | 0.95 | 0.71 | 0.905 |

The predicting of the presence, absence, or development of cancer in an individual may particularly involve determining percent methylated reference for the panel of one or more CpGs. A threshold percent methylated reference value may be applied in order to stratify an individual as having or not having endometrial cancer, or as having a high or low risk of endometrial cancer development.

In any of the assays described herein, the step of determining the methylation status of the one or more CpGs in the panel may comprise determining each CpG within:
1. SEQ ID NO 809 and/or SEQ ID NO 846 and/or SEQ ID NO 883 and wherein when the cancer index value is about 0.282 or more the individual is classified as having endometrial cancer or as having a high risk of endometrial cancer development, and wherein the sensitivity of the assay is at least 85.00%, the specificity of the assay is about 100.00%, and the AUC is about 1.00, preferably wherein the cancer index value is the percent methylated reference for sequence defined by SEQ ID NO 809 and/or SEQ ID NO 846 and/or SEQ ID NO 883;
2. SEQ ID NO 809 and/or SEQ ID NO 846 and/or SEQ ID NO 883 and wherein when the cancer index value is less than about 0.282 the individual is classified as not having endometrial cancer or as having a low risk of endometrial cancer development, and wherein the sensitivity of the assay is at least 85.00%, the specificity of the assay is about 100.00%, and the AUC is about 1.00, preferably wherein the cancer index value is the percent methylated reference for sequence defined by SEQ ID NO 809 and/or SEQ ID NO 846 and/or SEQ ID NO 883;
3. SEQ ID NO 810 and/or SEQ ID NO 847 and/or SEQ ID NO 884 and wherein when the cancer index value is about 0.212 or more the individual is classified as having endometrial cancer or as having a high risk of endometrial cancer development, and wherein the sensitivity of the assay is at least 55.00%, the specificity of the assay is about 100.00%, and the AUC is about 1.00, preferably wherein the cancer index value is the percent methylated reference for sequence defined by SEQ ID NO 810 and/or SEQ ID NO 847 and/or SEQ ID NO 884;
4. SEQ ID NO 810 and/or SEQ ID NO 847 and/or SEQ ID NO 884 and wherein when the cancer index value is less than about 0.212 the individual is classified as not having endometrial cancer or as having a low risk of endometrial cancer development, and wherein the sensitivity of the assay is at least 55.00%, the specificity of the assay is about 100.00%, and the AUC is about 1.00, preferably wherein the cancer index value is the percent methylated reference for sequence defined by SEQ ID NO 810 and/or SEQ ID NO 847 and/or SEQ ID NO 884;
5. SEQ ID NO 811 and/or SEQ ID NO 848 and/or SEQ ID NO 885 and wherein when the cancer index value is about 0.002 or more the individual is classified as having endometrial cancer or as having a high risk of endometrial cancer development, and wherein the sensitivity of the assay is at least 90.00%, the specificity of the assay is about 80.00%, and the AUC is about 0.98, preferably wherein the cancer index value is the percent methylated reference for sequence defined by SEQ ID NO 811 and/or SEQ ID NO 848 and/or SEQ ID NO 885 ;
6. SEQ ID NO 811 and/or SEQ ID NO 848 and/or SEQ ID NO 885 and wherein when the cancer index value is less than about 0.002 the individual is classified as not having endometrial cancer or as having a low risk of endometrial cancer development, and wherein the sensitivity of the assay is at least 90.00%, the specificity of the assay is about 80.00%, and the AUC is about 0.98, preferably wherein the cancer index value is the percent methylated reference for sequence defined by SEQ ID NO 811 and/or SEQ ID NO 848 and/or SEQ ID NO 885;
7. SEQ ID NO 812 and/or SEQ ID NO 849 and/or SEQ ID NO 886 and wherein when the cancer index value is about 0.026 or more the individual is classified as having endometrial cancer or as having a high risk of endometrial cancer development, and wherein the sensitivity of the assay is at least 85.00%, the specificity of the assay is about 90.00%, and the AUC is about 0.98, preferably wherein the cancer index value is the percent methylated reference for sequence defined by SEQ ID NO 812 and/or SEQ ID NO 849 and/or SEQ ID NO 886;
8. SEQ ID NO 812 and/or SEQ ID NO 849 and/or SEQ ID NO 886 815 and wherein when the cancer index value is less than about 0.026 the individual is classified as not having endometrial cancer or as having a low risk of endometrial cancer development, and wherein the sensitivity of the assay is at least 85.00%, the specificity of the assay is about 90.00%, and the AUC is about 0.98, preferably wherein the cancer index value is the percent methylated reference for sequence defined by SEQ ID NO 812 and/or SEQ ID NO 849 and/or SEQ ID NO 886;
9. SEQ ID NO 813 and/or SEQ ID NO 850 and/or SEQ ID NO 887 and wherein when the cancer index value is about 0.003 or more the individual is classified as having endometrial cancer or as having a high risk of endometrial cancer development, and wherein the sensitivity of the assay is at least 85.00%, the specificity of the assay is about 85.00%, and the AUC is about 0.97, preferably wherein the cancer index value is the percent methylated reference for sequence defined by SEQ ID NO 813 and/or SEQ ID NO 850 and/or SEQ ID NO 887;
10. SEQ ID NO 813 and/or SEQ ID NO 850 and/or SEQ ID NO 887 and wherein when the cancer index value is less than about 0.003 the individual is classified as not having endometrial cancer or as having a low risk of endometrial cancer development, and wherein the sensitivity of the assay is at least 85.00%, the specificity of the assay is about 85.00%, and the AUC is about 0.97, preferably wherein the cancer index value is the percent methylated reference for sequence defined by SEQ ID NO 813 and/or SEQ ID NO 850 and/or SEQ ID NO 887;
11. SEQ ID NO 814 and/or SEQ ID NO 851 and/or SEQ ID NO 888 and wherein when the cancer index value is about 0.000 or more the individual is classified as having endometrial cancer or as having a high risk of endometrial cancer development, and wherein the sensitivity of the assay is at least 75.00%, the specificity of the assay is about 85.00%, and the AUC is about 0.96, preferably wherein the cancer index value is the percent methylated reference for sequence defined by SEQ ID NO 814 and/or SEQ ID NO 851 and/or SEQ ID NO 888;
12. SEQ ID NO 814 and/or SEQ ID NO 851 and/or SEQ ID NO 888 and wherein when the cancer index value is less than about 0.000 the individual is classified as not having endometrial cancer or as having a low risk of endometrial cancer development, and wherein the sensitivity of the assay is at least 75.00%, the specificity of the assay is about 85.00%, and the AUC is about 0.96, preferably wherein the cancer index value is the percent methylated reference for sequence defined by SEQ ID NO 814 and/or SEQ ID NO 851 and/or SEQ ID NO 888;
13. SEQ ID NO 815 and/or SEQ ID NO 852 and/or SEQ ID NO 889 and wherein when the cancer index value is about 0.001 or more the individual is classified as having endometrial cancer or as having a high risk of endometrial cancer development, and wherein the sensitivity of the assay is at least 90.00%, the specificity of the assay is about 80.00%, and the AUC is about 0.96, preferably wherein the cancer index value is the percent methylated reference for sequence defined by SEQ ID NO 815 and/or SEQ ID NO 852 and/or SEQ ID NO 889;
14. SEQ ID NO 815 and/or SEQ ID NO 852 and/or SEQ ID NO 889 and wherein when the cancer index value is less than about 0.001 the individual is classified as not having endometrial cancer or as having a low risk of endometrial cancer development, and wherein the sensitivity of the assay is at least 90.00%, the specificity of the assay is about 80.00%, and the AUC is about 0.96, preferably wherein the cancer index value is the percent methylated reference for sequence defined by SEQ ID NO 815 and/or SEQ ID NO 852 and/or SEQ ID NO 889;
15. SEQ ID NO 816 and/or SEQ ID NO 853 and/or SEQ ID NO 890 and wherein when the cancer index value is about 0.025 or more the individual is classified as having endometrial cancer or as having a high risk of endometrial cancer development, and wherein the sensitivity of the assay is at least 90.00%, the specificity of the assay is about 75.00%, and the AUC is about 0.96, preferably wherein the cancer index value is the percent methylated reference for sequence defined by SEQ ID NO 816 and/or SEQ ID NO 853 and/or SEQ ID NO 890;
16. SEQ ID NO 816 and/or SEQ ID NO 853 and/or SEQ ID NO 890 and wherein when the cancer index value is less than about 0.025 the individual is classified as not having endometrial cancer or as having a low risk of endometrial cancer development, and wherein the sensitivity of the assay is at least 90.00%, the specificity of the assay is about 75.00%, and the AUC is about 0.96, preferably wherein the cancer index value is the percent methylated reference for sequence defined by SEQ ID NO 816 and/or SEQ ID NO 853 and/or SEQ ID NO 890;
17. SEQ ID NO 817 and/or SEQ ID NO 854 and/or SEQ ID NO 891 and wherein when the cancer index value is about 0.052 or more the individual is classified as having endometrial cancer or as having a high risk of endometrial cancer development, and wherein the sensitivity of the assay is at least 70.00%, the specificity of the assay is about 85.00%, and the AUC is about 0.95, preferably wherein the cancer index value is the percent methylated reference for sequence defined by SEQ ID NO 817 and/or SEQ ID NO 854 and/or SEQ ID NO 891;
18. SEQ ID NO 817 and/or SEQ ID NO 854 and/or SEQ ID NO 891 and wherein when the cancer index value is less than about 0.052 the individual is classified as not having endometrial cancer or as having a low risk of endometrial cancer development, and wherein the sensitivity of the assay is at least 70.00%, the specificity of the assay is about 85.00%, and the AUC is about 0.95, preferably wherein the cancer index value is the percent methylated reference for sequence defined by SEQ ID NO 817 and/or SEQ ID NO 854 and/or SEQ ID NO 891;
19. SEQ ID NO 818 and/or SEQ ID NO 855 and/or SEQ ID NO 892 and wherein when the cancer index value is about 0.001 or more the individual is classified as having endometrial cancer or as having a high risk of endometrial cancer development, and wherein the sensitivity of the assay is at least 85.00%, the specificity of the assay is about 75.00%, and the AUC is about 0.94, preferably wherein the cancer index value is the percent methylated reference for sequence defined by SEQ ID NO 818 and/or SEQ ID NO 855 and/or SEQ ID NO 892;
20. SEQ ID NO 818 and/or SEQ ID NO 855 and/or SEQ ID NO 892 and wherein when the cancer index value is less than about 0.001 the individual is classified as not having endometrial cancer or as having a low risk of endometrial cancer development, and wherein the sensitivity of the assay is at least 85.00%, the specificity of the assay is about 75.00%, and the AUC is about 0.94, preferably wherein the cancer index value is the percent methylated reference for sequence defined by SEQ ID NO 818 and/or SEQ ID NO 855 and/or SEQ ID NO 892;
21. SEQ ID NO 819 and/or SEQ ID NO 856 and/or SEQ ID NO 893 and wherein when the cancer index value is about 0.470 or more the individual is classified as having endometrial cancer or as having a high risk of endometrial cancer development, and wherein the sensitivity of the assay is at least 90.00%, the specificity of the assay is about 75.00%, and the AUC is about 0.93, preferably wherein the cancer index value is the percent methylated reference for sequence defined by SEQ ID NO 819 and/or SEQ ID NO 856 and/or SEQ ID NO 893;
22. SEQ ID NO 819 and/or SEQ ID NO 856 and/or SEQ ID NO 893 and wherein when the cancer index value is less than about 0.470 the individual is classified as not having endometrial cancer or as having a low risk of endometrial cancer development, and wherein the sensitivity of the assay is at least 90.00%, the specificity of the assay is about 75.00%, and the AUC is about 0.93, preferably wherein the cancer index value is the percent methylated reference for sequence defined by SEQ ID NO 819 and/or SEQ ID NO 856 and/or SEQ ID NO 893;
23. SEQ ID NO 820 and/or SEQ ID NO 857 and/or SEQ ID NO 894 and wherein when the cancer index value is about 0.010 or more the individual is classified as having endometrial cancer or as having a high risk of endometrial cancer development, and wherein the sensitivity of the assay is at least 70.00%, the specificity of the assay is about 80.00%, and the AUC is about 0.92, preferably wherein the cancer index value is the percent methylated reference for sequence defined by SEQ ID NO 820 and/or SEQ ID NO 857 and/or SEQ ID NO 894;
24. SEQ ID NO 820 and/or SEQ ID NO 857 and/or SEQ ID NO 894 and wherein when the cancer index value is less than about 0.010 the individual is classified as not having endometrial cancer or as having a low risk of endometrial cancer development, and wherein the sensitivity of the assay is at least 70.00%, the specificity of the assay is about 80.00%, and the AUC is about 0.92, preferably wherein the cancer index value is the percent methylated reference for sequence defined by SEQ ID NO 820 and/or SEQ ID NO 857 and/or SEQ ID NO 894;
25. SEQ ID NO 821 and/or SEQ ID NO 858 and/or SEQ ID NO 895 and wherein when the cancer index value is about 0.321 or more the individual is classified as having endometrial cancer or as having a high risk of endometrial cancer development, and wherein the sensitivity of the assay is at least 90.00%, the specificity of the assay is about 75.00%, and the AUC is about 0.92, preferably wherein the cancer index value is the percent methylated reference for sequence defined by SEQ ID NO 821 and/or SEQ ID NO 858 and/or SEQ ID NO 895;
26. SEQ ID NO 821 and/or SEQ ID NO 858 and/or SEQ ID NO 895 and wherein when the cancer index value is less than about 0.321 the individual is classified as not having endometrial cancer or as having a low risk of endometrial cancer development, and wherein the sensitivity of the assay is at least 90.00%, the specificity of the assay is about 75.00%, and the AUC is about 0.92, preferably wherein the cancer index value is the percent methylated reference for sequence defined by SEQ ID NO 821 and/or SEQ ID NO 858 and/or SEQ ID NO 895;
27. SEQ ID NO 822 and/or SEQ ID NO 859 and/or SEQ ID NO 896 and wherein when the cancer index value is about 0.067 or more the individual is classified as having endometrial cancer or as having a high risk of endometrial cancer development, and wherein the sensitivity of the assay is at least 85.00%, the specificity of the assay is about 75.00%, and the AUC is about 0.92, preferably wherein the cancer index value is the percent methylated reference for sequence defined by SEQ ID NO 822 and/or SEQ ID NO 859 and/or SEQ ID NO 896;
28. SEQ ID NO 822 and/or SEQ ID NO 859 and/or SEQ ID NO 896 and wherein when the cancer index value is less than about 0.067 the individual is classified as not having endometrial cancer or as having a low risk of endometrial cancer development, and wherein the sensitivity of the assay is at least 85.00%, the specificity of the assay is about 75.00%, and the AUC is about 0.92, preferably wherein the cancer index value is the percent methylated reference for sequence defined by SEQ ID NO 822 and/or SEQ ID NO 859 and/or SEQ ID NO 896;
29. SEQ ID NO 823 and/or SEQ ID NO 860 and/or SEQ ID NO 897 and wherein when the cancer index value is about 0.062 or more the individual is classified as having endometrial cancer or as having a high risk of endometrial cancer development, and wherein the sensitivity of the assay is at least 80.00%, the specificity of the assay is about 75.00%, and the AUC is about 0.91, preferably wherein the cancer index value is the percent methylated reference for sequence defined by SEQ ID NO 823 and/or SEQ ID NO 860 and/or SEQ ID NO 897;
30. SEQ ID NO 823 and/or SEQ ID NO 860 and/or SEQ ID NO 897 and wherein when the cancer index value is less than about 0.062 the individual is classified as not having endometrial cancer or as having a low risk of endometrial cancer development, and wherein the sensitivity of the assay is at least 80.00%, the specificity of the assay is about 75.00%, and the AUC is about 0.91, preferably wherein the cancer index value is the percent methylated reference for sequence defined by SEQ ID NO 823 and/or SEQ ID NO 860 and/or SEQ ID NO 897;
31. SEQ ID NO 824 and/or SEQ ID NO 861 and/or SEQ ID NO 898 and wherein when the cancer index value is about 0.106 or more the individual is classified as having endometrial cancer or as having a high risk of endometrial cancer development, and wherein the sensitivity of the assay is at least 80.00%, the specificity of the assay is about 75.00%, and the AUC is about 0.90, preferably wherein the cancer index value is the percent methylated reference for sequence defined by SEQ ID NO 824 and/or SEQ ID NO 861 and/or SEQ ID NO 898;
32. SEQ ID NO 824 and/or SEQ ID NO 861 and/or SEQ ID NO 898 and wherein when the cancer index value is less than about 0.106 the individual is classified as not having endometrial cancer or as having a low risk of endometrial cancer development, and wherein the sensitivity of the assay is at least 80.00%, the specificity of the assay is about 75.00%, and the AUC is about 0.90, preferably wherein the cancer index value is the percent methylated reference for sequence defined by SEQ ID NO 824 and/or SEQ ID NO 861 and/or SEQ ID NO 898;
33. SEQ ID NO 825 and/or SEQ ID NO 862 and/or SEQ ID NO 899 and wherein when the cancer index value is about 0.354 or more the individual is classified as having endometrial cancer or as having a high risk of endometrial cancer development, and wherein the sensitivity of the assay is at least 80.00%, the specificity of the assay is about 75.00%, and the AUC is about 0.89, preferably wherein the cancer index value is the percent methylated reference for sequence defined by SEQ ID NO 825 and/or SEQ ID NO 862 and/or SEQ ID NO 899;
34. SEQ ID NO 825 and/or SEQ ID NO 862 and/or SEQ ID NO 899 and wherein when the cancer index value is less than about 0.354 the individual is classified as not having endometrial cancer or as having a low risk of endometrial cancer development, and wherein the sensitivity of the assay is at least 80.00%, the specificity of the assay is about 75.00%, and the AUC is about 0.89, preferably wherein the cancer index value is the percent methylated reference for sequence defined by SEQ ID NO 825 and/or SEQ ID NO 862 and/or SEQ ID NO 899;
35. SEQ ID NO 826 and/or SEQ ID NO 863 and/or SEQ ID NO 900 and wherein when the cancer index value is about 0.075 or more the individual is classified as having endometrial cancer or as having a high risk of endometrial cancer development, and wherein the sensitivity of the assay is at least 70.00%, the specificity of the assay is about 75.00%, and the AUC is about 0.89, preferably wherein the cancer index value is the percent methylated reference for sequence defined by SEQ ID NO 826 and/or SEQ ID NO 863 and/or SEQ ID NO 900;
36. SEQ ID NO 826 and/or SEQ ID NO 863 and/or SEQ ID NO 900 and wherein when the cancer index value is less than about 0.075 the individual is classified as not having endometrial cancer or as having a low risk of endometrial cancer development, and wherein the sensitivity of the assay is at least 70.00%, the specificity of the assay is about 75.00%, and the AUC is about 0.89, preferably wherein the cancer index value is the percent methylated reference for sequence defined by SEQ ID NO 826 and/or SEQ ID NO 863 and/or SEQ ID NO 900;
37. SEQ ID NO 827 and/or SEQ ID NO 864 and/or SEQ ID NO 901 and wherein when the cancer index value is about 0.305 or more the individual is classified as having endometrial cancer or as having a high risk of endometrial cancer development, and wherein the sensitivity of the assay is at least 80.00%, the specificity of the assay is about 75.00%, and the AUC is about 0.89, preferably wherein the cancer index value is the percent methylated reference for sequence defined by SEQ ID NO 827 and/or SEQ ID NO 864 and/or SEQ ID NO 901;
38. SEQ ID NO 827 and/or SEQ ID NO 864 and/or SEQ ID NO 901 and wherein when the cancer index value is less than about 0.305 the individual is classified as not having endometrial cancer or as having a low risk of endometrial cancer development, and wherein the sensitivity of the assay is at least 80.00%, the specificity of the assay is about 75.00%, and the AUC is about 0.89, preferably wherein the cancer index value is the percent methylated reference for sequence defined by SEQ ID NO 827 and/or SEQ ID NO 864 and/or SEQ ID NO 901;
39. SEQ ID NO 828 and/or SEQ ID NO 865 and/or SEQ ID NO 902 and wherein when the cancer index value is about 0.110 or more the individual is classified as having endometrial cancer or as having a high risk of endometrial cancer development, and wherein the sensitivity of the assay is at least 80.00%, the specificity of the assay is about 75.00%, and the AUC is about 0.88, preferably wherein the cancer index value is the percent methylated reference for sequence defined by SEQ ID NO 828 and/or SEQ ID NO 865 and/or SEQ ID NO 902;
40. SEQ ID NO 828 and/or SEQ ID NO 865 and/or SEQ ID NO 902 and wherein when the cancer index value is less than about 0.110 the individual is classified as not having endometrial cancer or as having a low risk of endometrial cancer development, and wherein the sensitivity of the assay is at least 80.00%, the specificity of the assay is about 75.00%, and the AUC is about 0.88, preferably wherein the cancer index value is the percent methylated reference for sequence defined by SEQ ID NO 828 and/or SEQ ID NO 865 and/or SEQ ID NO 902;
41. SEQ ID NO 829 and/or SEQ ID NO 866 and/or SEQ ID NO 903 and wherein when the cancer index value is about 0.139 or more the individual is classified as having endometrial cancer or as having a high risk of endometrial cancer development, and wherein the sensitivity of the assay is at least 80.00%, the specificity of the assay is about 75.00%, and the AUC is about 0.88, preferably wherein the cancer index value is the percent methylated reference for sequence defined by SEQ ID NO 829 and/or SEQ ID NO 866 and/or SEQ ID NO 903;
42. SEQ ID NO 829 and/or SEQ ID NO 866 and/or SEQ ID NO 903 and wherein when the cancer index value is less than about 0.139 the individual is classified as not having endometrial cancer or as having a low risk of endometrial cancer development, and wherein the sensitivity of the assay is at least 80.00%, the specificity of the assay is about 75.00%, and the AUC is about 0.88, preferably wherein the cancer index value is the percent methylated reference for sequence defined by SEQ ID NO 829 and/or SEQ ID NO 866 and/or SEQ ID NO 903;
43. SEQ ID NO 830 and/or SEQ ID NO 867 and/or SEQ ID NO 904 and wherein when the cancer index value is about 0.453 or more the individual is classified as having endometrial cancer or as having a high risk of endometrial cancer development, and wherein the sensitivity of the assay is at least 75.00%, the specificity of the assay is about 75.00%, and the AUC is about 0.87, preferably wherein the cancer index value is the percent methylated reference for sequence defined by SEQ ID NO 830 and/or SEQ ID NO 867 and/or SEQ ID NO 904;
44. SEQ ID NO 830 and/or SEQ ID NO 867 and/or SEQ ID NO 904 and wherein when the cancer index value is less than about 0.453 the individual is classified as not having endometrial cancer or as having a low risk of endometrial cancer development, and wherein the sensitivity of the assay is at least 75.00%, the specificity of the assay is about 75.00%, and the AUC is about 0.87, preferably wherein the cancer index value is the percent methylated reference for sequence defined by SEQ ID NO 830 and/or SEQ ID NO 867 and/or SEQ ID NO 904;
45. SEQ ID NO 831 and/or SEQ ID NO 868 and/or SEQ ID NO 905 and wherein when the cancer index value is about 0.511 or more the individual is classified as having endometrial cancer or as having a high risk of endometrial cancer development, and wherein the sensitivity of the assay is at least 90.00%, the specificity of the assay is about 75.00%, and the AUC is about 0.86, preferably wherein the cancer index value is the percent methylated reference for sequence defined by SEQ ID NO 831 and/or SEQ ID NO 868 and/or SEQ ID NO 905 ;
46. SEQ ID NO 831 and/or SEQ ID NO 868 and/or SEQ ID NO 905 and wherein when the cancer index value is less than about 0.511 the individual is classified as not having endometrial cancer or as having a low risk of endometrial cancer development, and wherein the sensitivity of the assay is at least 90.00%, the specificity of the assay is about 75.00%, and the AUC is about 0.86, preferably wherein the cancer index value is the percent methylated reference for sequence defined by SEQ ID NO 831 and/or SEQ ID NO 868 and/or SEQ ID NO 905;
47. SEQ ID NO 832 and/or SEQ ID NO 869 and/or SEQ ID NO 906 and wherein when the cancer index value is about 0.425 or more the individual is classified as having endometrial cancer or as having a high risk of endometrial cancer development, and wherein the sensitivity of the assay is at least 80.00%, the specificity of the assay is about 75.00%, and the AUC is about 0.86, preferably wherein the cancer index value is the percent methylated reference for sequence defined by SEQ ID NO 832 and/or SEQ ID NO 869 and/or SEQ ID NO 906;
48. SEQ ID NO 832 and/or SEQ ID NO 869 and/or SEQ ID NO 906 and wherein when the cancer index value is less than about 0.425 the individual is classified as not having endometrial cancer or as having a low risk of endometrial cancer development, and wherein the sensitivity of the assay is at least 80.00%, the specificity of the assay is about 75.00%, and the AUC is about 0.86, preferably wherein the cancer index value is the percent methylated reference for sequence defined by SEQ ID NO 832 and/or SEQ ID NO 869 and/or SEQ ID NO 906;
49. SEQ ID NO 833 and/or SEQ ID NO 870 and/or SEQ ID NO 907 and wherein when the cancer index value is about 0.636 or more the individual is classified as having endometrial cancer or as having a high risk of endometrial cancer development, and wherein the sensitivity of the assay is at least 75.00%, the specificity of the assay is about 75.00%, and the AUC is about 0.86, preferably wherein the cancer index value is the percent methylated reference for sequence defined by SEQ ID NO 833 and/or SEQ ID NO 870 and/or SEQ ID NO 907;
50. SEQ ID NO 833 and/or SEQ ID NO 870 and/or SEQ ID NO 907 and wherein when the cancer index value is less than about 0.636 the individual is classified as not having endometrial cancer or as having a low risk of endometrial cancer development, and wherein the sensitivity of the assay is at least 75.00%, the specificity of the assay is about 75.00%, and the AUC is about 0.86, preferably wherein the cancer index value is the percent methylated reference for sequence defined by SEQ ID NO 833 and/or SEQ ID NO 870 and/or SEQ ID NO 907;
51. SEQ ID NO 834 and/or SEQ ID NO 871 and/or SEQ ID NO 908 and wherein when the cancer index value is about 0.349 or more the individual is classified as having endometrial cancer or as having a high risk of endometrial cancer development, and wherein the sensitivity of the assay is at least 75.00%, the specificity of the assay is about 75.00%, and the AUC is about 0.86, preferably wherein the cancer index value is the percent methylated reference for sequence defined by SEQ ID NO 833 and/or SEQ ID NO 870 and/or SEQ ID NO 907;
52. SEQ ID NO 833 and/or SEQ ID NO 870 and/or SEQ ID NO 907 and wherein when the cancer index value is less than about 0.349 the individual is classified as not having endometrial cancer or as having a low risk of endometrial cancer development, and wherein the sensitivity of the assay is at least 75.00%, the specificity of the assay is about 75.00%, and the AUC is about 0.86, preferably wherein the cancer index value is the percent methylated reference for sequence defined by SEQ ID NO 833 and/or SEQ ID NO 870 and/or SEQ ID NO 907;
53. SEQ ID NO 834 and/or SEQ ID NO 871 and/or SEQ ID NO 908 and wherein when the cancer index value is about 0.109 or more the individual is classified as having endometrial cancer or as having a high risk of endometrial cancer development, and wherein the sensitivity of the assay is at least 75.00%, the specificity of the assay is about 75.00%, and the AUC is about 0.86, preferably wherein the cancer index value is the percent methylated reference for sequence defined by SEQ ID NO 834 and/or SEQ ID NO 871 and/or SEQ ID NO 908;
54. SEQ ID NO 835 and/or SEQ ID NO 872 and/or SEQ ID NO 909 and wherein when the cancer index value is less than about 0.109 the individual is classified as not having endometrial cancer or as having a low risk of endometrial cancer development, and wherein the sensitivity of the assay is at least 75.00%, the specificity of the assay is about 75.00%, and the AUC is about 0.86, preferably wherein the cancer index value is the percent methylated reference for sequence defined by SEQ ID NO 835 and/or SEQ ID NO 872 and/or SEQ ID NO 909;
55. SEQ ID NO 836 and/or SEQ ID NO 873 and/or SEQ ID NO 910 and wherein when the cancer index value is about 0.106 or more the individual is classified as having endometrial cancer or as having a high risk of endometrial cancer development, and wherein the sensitivity of the assay is at least 75.00%, the specificity of the assay is about 75.00%, and the AUC is about 0.86, preferably wherein the cancer index value is the percent methylated reference for sequence defined by SEQ ID NO 836 and/or SEQ ID NO 873 and/or SEQ ID NO 910;
56. SEQ ID NO 836 and/or SEQ ID NO 873 and/or SEQ ID NO 910 and wherein when the cancer index value is less than about 0.106 the individual is classified as not having endometrial cancer or as having a low risk of endometrial cancer development, and wherein the sensitivity of the assay is at least 75.00%, the specificity of the assay is about 75.00%, and the AUC is about 0.86, preferably wherein the cancer index value is the percent methylated reference for sequence defined by SEQ ID NO 836 and/or SEQ ID NO 873 and/or SEQ ID NO 910;
57. SEQ ID NO 837 and/or SEQ ID NO 874 and/or SEQ ID NO 911 and wherein when the cancer index value is about 0.220 or more the individual is classified as having endometrial cancer or as having a high risk of endometrial cancer development, and wherein the sensitivity of the assay is at least 80.00%, the specificity of the assay is about 75.00%, and the AUC is about 0.85, preferably wherein the cancer index value is the percent methylated reference for sequence defined by SEQ ID NO 837 and/or SEQ ID NO 874 and/or SEQ ID NO 911;
58. SEQ ID NO 837 and/or SEQ ID NO 874 and/or SEQ ID NO 911 and wherein when the cancer index value is less than about 0.220 the individual is classified as not having endometrial cancer or as having a low risk of endometrial cancer development, and wherein the sensitivity of the assay is at least 80.00%, the specificity of the assay is about 75.00%, and the AUC is about 0.85, preferably wherein the cancer index value is the percent methylated reference for sequence defined by SEQ ID NO 837 and/or SEQ ID NO 874 and/or SEQ ID NO 911;
59. SEQ ID NO 838 and/or SEQ ID NO 875 and/or SEQ ID NO 912 and wherein when the cancer index value is about 0.123 or more the individual is classified as having endometrial cancer or as having a high risk of endometrial cancer development, and wherein the sensitivity of the assay is at least 70.00%, the specificity of the assay is about 75.00%, and the AUC is about 0.85, preferably wherein the cancer index value is the percent methylated reference for sequence defined by SEQ ID NO 838 and/or SEQ ID NO 875 and/or SEQ ID NO 912;
60. SEQ ID NO 838 and/or SEQ ID NO 875 and/or SEQ ID NO 912 and wherein when the cancer index value is less than about 0.123 the individual is classified as not having endometrial cancer or as having a low risk of endometrial cancer development, and wherein the sensitivity of the assay is at least 70.00%, the specificity of the assay is about 75.00%, and the AUC is about 0.85, preferably wherein the cancer index value is the percent methylated reference for sequence defined by SEQ ID NO 838 and/or SEQ ID NO 875 and/or SEQ ID NO 912;
61. SEQ ID NO 839 and/or SEQ ID NO 876 and/or SEQ ID NO 913 and wherein when the cancer index value is about 0.146 or more the individual is classified as having endometrial cancer or as having a high risk of endometrial cancer development, and wherein the sensitivity of the assay is at least 80.00%, the specificity of the assay is about 75.00%, and the AUC is about 0.85, preferably wherein the cancer index value is the percent methylated reference for sequence defined by SEQ ID NO 839 and/or SEQ ID NO 876 and/or SEQ ID NO 913;
62. SEQ ID NO 839 and/or SEQ ID NO 876 and/or SEQ ID NO 913 and wherein when the cancer index value is less than about 0.146 the individual is classified as not having endometrial cancer or as having a low risk of endometrial cancer development, and wherein the sensitivity of the assay is at least 80.00%, the specificity of the assay is about 75.00%, and the AUC is about 0.85, preferably wherein the cancer index value is the percent methylated reference for sequence defined by SEQ ID NO 839 and/or SEQ ID NO 876 and/or SEQ ID NO 913;
63. SEQ ID NO 840 and/or SEQ ID NO 877 and/or SEQ ID NO 914 and wherein when the cancer index value is about 0.488 or more the individual is classified as having endometrial cancer or as having a high risk of endometrial cancer development, and wherein the sensitivity of the assay is at least 65.00%, the specificity of the assay is about 75.00%, and the AUC is about 0.83, preferably wherein the cancer index value is the percent methylated reference for sequence defined by SEQ ID NO 840 and/or SEQ ID NO 877 and/or SEQ ID NO 914;
64. SEQ ID NO 840 and/or SEQ ID NO 877 and/or SEQ ID NO 914 and wherein when the cancer index value is less than about 0.488 the individual is classified as not having endometrial cancer or as having a low risk of endometrial cancer development, and wherein the sensitivity of the assay is at least 65.00%, the specificity of the assay is about 75.00%, and the AUC is about 0.83, preferably wherein the cancer index value is the percent methylated reference for sequence defined by SEQ ID NO 840 and/or SEQ ID NO 877 and/or SEQ ID NO 914;
65. SEQ ID NO 841 and/or SEQ ID NO 878 and/or SEQ ID NO 915 and wherein when the cancer index value is about 2.096 or more the individual is classified as having endometrial cancer or as having a high risk of endometrial cancer development, and wherein the sensitivity of the assay is at least 60.00%, the specificity of the assay is about 75.00%, and the AUC is about 0.82, preferably wherein the cancer index value is the percent methylated reference for sequence defined by SEQ ID NO 841 and/or SEQ ID NO 878 and/or SEQ ID NO 915;
66. SEQ ID NO 841 and/or SEQ ID NO 878 and/or SEQ ID NO 915 and wherein when the cancer index value is less than about 2.096 the individual is classified as not having endometrial cancer or as having a low risk of endometrial cancer development, and wherein the sensitivity of the assay is at least 60.00%, the specificity of the assay is about 75.00%, and the AUC is about 0.82, preferably wherein the cancer index value is the percent methylated reference for sequence defined by SEQ ID NO 841 and/or SEQ ID NO 878 and/or SEQ ID NO 915;
67. SEQ ID NO 842 and/or SEQ ID NO 879 and/or SEQ ID NO 916 and wherein when the cancer index value is about 0.803 or more the individual is classified as having endometrial cancer or as having a high risk of endometrial cancer development, and wherein the sensitivity of the assay is at least 70.00%, the specificity of the assay is about 75.00%, and the AUC is about 0.82, preferably wherein the cancer index value is the percent methylated reference for sequence defined by SEQ ID NO 842 and/or SEQ ID NO 879 and/or SEQ ID NO 916;
68. SEQ ID NO 842 and/or SEQ ID NO 879 and/or SEQ ID NO 916 and wherein when the cancer index value is less than about 0.803 the individual is classified as not having endometrial cancer or as having a low risk of endometrial cancer development, and wherein the sensitivity of the assay is at least 70.00%, the specificity of the assay is about 75.00%, and the AUC is about 0.82, preferably wherein the cancer index value is the percent methylated reference for sequence defined by SEQ ID NO 842 and/or SEQ ID NO 879 and/or SEQ ID NO 916;
69. SEQ ID NO 843 and/or SEQ ID NO 880 and/or SEQ ID NO 917 and wherein when the cancer index value is about 1.138 or more the individual is classified as having endometrial cancer or as having a high risk of endometrial cancer development, and wherein the sensitivity of the assay is at least 65.00%, the specificity of the assay is about 75.00%, and the AUC is about 0.80, preferably wherein the cancer index value is the percent methylated reference for sequence defined by SEQ ID NO 843 and/or SEQ ID NO 880 and/or SEQ ID NO 917;
70. SEQ ID NO 843 and/or SEQ ID NO 880 and/or SEQ ID NO 917 and wherein when the cancer index value is less than about 1.138 the individual is classified as not having endometrial cancer or as having a low risk of endometrial cancer development, and wherein the sensitivity of the assay is at least 65.00%, the specificity of the assay is about 75.00%, and the AUC is about 0.80, preferably wherein the cancer index value is the percent methylated reference for sequence defined by SEQ ID NO 843 and/or SEQ ID NO 880 and/or SEQ ID NO 917;
71. SEQ ID NO 844 and/or SEQ ID NO 881 and/or SEQ ID NO 918 and wherein when the cancer index value is about 0.500 or more the individual is classified as having endometrial cancer or as having a high risk of endometrial cancer development, and wherein the sensitivity of the assay is at least 65.00%, the specificity of the assay is about 75.00%, and the AUC is about 0.80, preferably wherein the cancer index value is the percent methylated reference for sequence defined by SEQ ID NO 844 and/or SEQ ID NO 881 and/or SEQ ID NO 918;
72. SEQ ID NO 844 and/or SEQ ID NO 881 and/or SEQ ID NO 918 and wherein when the cancer index value is less than about 0.500 the individual is classified as not having endometrial cancer or as having a low risk of endometrial cancer development, and wherein the sensitivity of the assay is at least 65.00%, the specificity of the assay is about 75.00%, and the AUC is about 0.80, preferably wherein the cancer index value is the percent methylated reference for sequence defined by SEQ ID NO 844 and/or SEQ ID NO 881 and/or SEQ ID NO 918;
73. SEQ ID NO 845 and/or SEQ ID NO 882 and/or SEQ ID NO 919 and wherein when the cancer index value is about 0.162 or more the individual is classified as having endometrial cancer or as having a high risk of endometrial cancer development, and wherein the sensitivity of the assay is at least 65.00%, the specificity of the assay is about 75.00%, and the AUC is about 0.78, preferably wherein the cancer index value is the percent methylated reference for sequence defined by SEQ ID NO 845 and/or SEQ ID NO 882 and/or SEQ ID NO 919; and/or
74. SEQ ID NO 845 and/or SEQ ID NO 882 and/or SEQ ID NO 919 and wherein when the cancer index value is less than about 0.162 the individual is classified as not having endometrial cancer or as having a low risk of endometrial cancer development, and wherein the sensitivity of the assay is at least 65.00%, the specificity of the assay is about 75.00%, and the AUC is about 0.78, preferably wherein the cancer index value is the percent methylated reference for sequence defined by SEQ ID NO 845 and/or SEQ ID NO 882 and/or SEQ ID NO 919.

In any of the described assays, the methylation status of the one or more CpGs in the panel is preferably determined by a percent methylated reference analysis, and the assay is for assessing the presence, absence or development endometrial cancer.

The ROC data set out in Table 11, corresponding to each of SEQ ID NOs: 809 to 919 are derived by determining a cancer index value from a panel of CpGs, wherein the panel in each instance comprises all of the CpGs in the sequence(s) defined by the SEQ ID NO.

The ROC data in Table 13 corresponds to assays of the invention wherein the sample which has been taken from the individual is a self-collected cervico-vaginal sample and wherein the panel of CpGs comprise the CpGs denoted by CG in the amplicons defined by any one of SEQ ID NOs 920, 922 and 924. In such assays of the invention the step of determining the methylation status of the one or more CpGs in the panel comprises determining each CpG within:
a. SEQ ID NO 920 and wherein when the cancer index value is about 0.280 or more the individual is classified as having endometrial cancer or as having a high risk of endometrial cancer development, and wherein the sensitivity of the assay is about 100%, the specificity of the assay is about 100.00%, and the AUC is about 1.00, preferably wherein the cancer index value is the percent methylated reference for sequence defined by SEQ ID NO 920;
b. SEQ ID NO 920 and wherein when the cancer index value is less than about 0.280 the individual is classified as not having endometrial cancer or as having a low risk of endometrial cancer development, and wherein the sensitivity of the assay is about 100%, the specificity of the assay is about 100.00%, and the AUC is about 1.00, preferably wherein the cancer index value is the percent methylated reference for sequence defined by SEQ ID NO 920;
c. SEQ ID NO 922 and wherein when the cancer index value is about 0.000 or more the individual is classified as having endometrial cancer or as having a high risk of endometrial cancer development, and wherein the sensitivity of the assay is about 100%, the specificity of the assay is about 100.00%, and the AUC is about 1.00, preferably wherein the cancer index value is the percent methylated reference for sequence defined by SEQ ID NO 922;
d. SEQ ID NO 922 and wherein when the cancer index value is about 0.000 or more the individual is classified as having endometrial cancer or as having a high risk of endometrial cancer development, and wherein the sensitivity of the assay is about 100%, the specificity of the assay is about 100.00%, and the AUC is about 1.00, preferably wherein the cancer index value is the percent methylated reference for sequence defined by SEQ ID NO 922;
e. SEQ ID NO 924 and wherein when the cancer index value is about 0.000 or more the individual is classified as having endometrial cancer or as having a high risk of endometrial cancer development, and wherein the sensitivity of the assay is about 100%, the specificity of the assay is about 100.00%, and the AUC is about 1.00, preferably wherein the cancer index value is the percent methylated reference for sequence defined by SEQ ID NO 924; or
f. SEQ ID NO 924 and wherein when the cancer index value is about 0.000 or more the individual is classified as having endometrial cancer or as having a high risk of endometrial cancer development, and wherein the sensitivity of the assay is about 100%, the specificity of the assay is about 100.00%, and the AUC is about 1.00, preferably wherein the cancer index value is the percent methylated reference for sequence defined by SEQ ID NO 924.

The ROC data in Table 14 corresponds to assays of the invention wherein the sample which has been taken from the individual is a vaginal swab taken from a woman having post-menopausal bleeding and wherein the panel of CpGs comprise the CpGs denoted by CG in the amplicons defined by SEQ ID NOs SEQ ID NOs 920, 922 and 924. In such assays of the invention the step of determining the methylation status of the one or more CpGs in the panel comprises determining each CpG within:
a. SEQ ID NO 920 and wherein when the cancer index value is about 0.280 or more the individual is classified as having endometrial cancer or as having a high risk of endometrial cancer development, and wherein the sensitivity of the assay is at least 88.00%, the specificity of the assay is about 100.00%, and the AUC is about 1.00, preferably wherein the cancer index value is the percent methylated reference for sequence defined by SEQ ID NO 920;
b. SEQ ID NO 920 and wherein when the cancer index value is less than about 0.280 the individual is classified as not having endometrial cancer or as having a low risk of endometrial cancer development, and wherein the sensitivity of the assay is at least 88.00%, the specificity of the assay is about 100.00%, and the AUC is about 1.00, preferably wherein the cancer index value is the percent methylated reference for sequence defined by SEQ ID NO 920;
c. SEQ ID NO 922 and wherein when the cancer index value is about 0.000 or more the individual is classified as having endometrial cancer or as having a high risk of endometrial cancer development, and wherein the sensitivity of the assay is about 100%, the specificity of the assay is at least 87.00%, and the AUC is about 1.00, preferably wherein the cancer index value is the percent methylated reference for sequence defined by SEQ ID NO 922;
d. SEQ ID NO 922 and wherein when the cancer index value is about 0.000 or more the individual is classified as having endometrial cancer or as having a high risk of endometrial cancer development, and wherein the sensitivity of the assay is about 100%, the specificity of the assay is about 87.00%, and the AUC is about 1.00, preferably wherein the cancer index value is the percent methylated reference for sequence defined by SEQ ID NO 922;
e. SEQ ID NO 924 and wherein when the cancer index value is about 0.000 or more the individual is classified as having endometrial cancer or as having a high risk of endometrial cancer development, and wherein the sensitivity of the assay is about 100%, the specificity of the assay is at least 84.00%, and the AUC is about 1.00, preferably wherein the cancer index value is the percent methylated reference for sequence defined by SEQ ID NO 924; or
f. SEQ ID NO 924 and wherein when the cancer index value is about 0.000 or more the individual is classified as having endometrial cancer or as having a high risk of endometrial cancer development, and wherein the sensitivity of the assay is about 100%, the specificity of the assay is at least 84.00%, and the AUC is about 1.00, preferably wherein the cancer index value is the percent methylated reference for sequence defined by SEQ ID NO 924.

**Table 11**

| **gene_name** | **auc** | **cutoff** | **Specificity** | **Sensitivity** | **SEQ ID NO of forward primer** | **SEQ ID NO of reverse primer** | **SEQ ID NO of probe** | **ML.design.region** | **DNA.strand** | **Chromosome** | **Rxn.Start** | **Rxn.Finish** | **Amplicon Length** |
|---|---|---|---|---|---|---|---|---|---|---|---|---|---|
| ZSCAN12 | 1 | 0.282 | 1 | 0.85 | 809 | 846 | 883 | chr6:28367271-28367870 | negative | 6 | 28368198 | 28368134 | 65 |
| HCG4 | 1 | 0.212 | 1 | 0.55 | 810 | 847 | 884 | chr6:29760110-29760709 | positive | 6 | 29760385 | 29760319 | 67 |
| GYPC | 0.98 | 0.002 | 0.8 | 0.9 | 811 | 848 | 885 | chr2:127413531-127414130 | positive | 2 | 127413902 | 127413797 | 106 |
| RASSF1 | 0.98 | 0.026 | 0.9 | 0.85 | 812 | 849 | 886 | chr3:50378131-50378730 | positive | 3 | 50378379 | 50378305 | 75 |
| GYPC | 0.97 | 0.003 | 0.85 | 0.85 | 813 | 850 | 887 | chr2:127413636-127414235 | negative | 2 | 127414543 | 127414457 | 87 |
| TRH | 0.96 | 0 | 0.85 | 0.75 | 814 | 851 | 888 | chr3:129693491-129694090 | negative | 3 | 129694467 | 129694379 | 89 |
| PTGER4 | 0.96 | 0.001 | 0.8 | 0.9 | 815 | 852 | 889 | chr5:40680837-40681436 | positive | 5 | 40681173 | 40681071 | 103 |
| FAIM2 | 0.96 | 0.025 | 0.75 | 0.9 | 816 | 853 | 890 | chr12:50297493-50298092 | positive | 12 | 50297790 | 50297727 | 64 |
| DMRTA2 | 0.95 | 0.052 | 0.85 | 0.7 | 817 | 854 | 891 | chr1:50886482-50887081 | negative | 1 | 50887427 | 50887344 | 84 |
| NA | 0.94 | 0.001 | 0.75 | 0.85 | 818 | 855 | 892 | chr6:29520919-29521918 | chrm6_EC neg ii | 6 | 29522458 | 29522374 | 85 |
| TRABD | 0.93 | 0.47 | 0.75 | 0.9 | 819 | 856 | 893 | chr22:50623387-50623986 | positive | 22 | 50623686 | 50623618 | 69 |
| TCP11 | 0.92 | 0.01 | 0.8 | 0.7 | 820 | 857 | 894 | chr6:35108639-35109238 | negative | 6 | 35109589 | 35109491 | 99 |
| GATA3 | 0.92 | 0.321 | 0.75 | 0.9 | 821 | 858 | 895 | chr10:8097389-8097988 | positive | 10 | 8097681 | 8097600 | 82 |
| NA | 0.92 | 0.067 | 0.75 | 0.85 | 822 | 859 | 896 | chr6:29520919-29521918 | chrm6_EC pos ii | 6 | 29521423 | 29521337 | 87 |
| SPDYA | 0.91 | 0.062 | 0.75 | 0.8 | 823 | 860 | 897 | chr2:29033426-29034025 | positive | 2 | 29033782 | 29033710 | 73 |
| NA | 0.9 | 0.106 | 0.75 | 0.8 | 824 | 861 | 898 | chr6:29520919-29521918 | chrm6_EC neg iii | 6 | 29522384 | 29522287 | 98 |
| KIAA1383 | 0.89 | 0.354 | 0.75 | 0.8 | 825 | 862 | 899 | chr1:232941168-232941767 | negative | 1 | 232942100 | 232942032 | 69 |
| CDH4 | 0.89 | 0.075 | 0.75 | 0.7 | 826 | 863 | 900 | chr20:59828228-59828827 | positive | 20 | 59828556 | 59828486 | 71 |
| EPHX3 | 0.89 | 0.305 | 0.75 | 0.8 | 827 | 864 | 901 | chr19:15343965-15344564 | positive | 19 | 15344316 | 15344240 | 77 |
| NEFM | 0.88 | 0.11 | 0.75 | 0.8 | 828 | 865 | 902 | chr8:24771026-24771625 | positive | 8 | 24771260 | 24771188 | 73 |
| SORCS3 | 0.88 | 0.139 | 0.75 | 0.8 | 829 | 866 | 903 | chr10:106401217-106401816 | positive | 10 | 106401574 | 106401479 | 96 |
| ZNF718 | 0.87 | 0.453 | 0.75 | 0.75 | 830 | 867 | 904 | chr4:107425-108024 | negative | 4 | 108401 | 108313 | 89 |
| TRH | 0.86 | 0.511 | 0.75 | 0.9 | 831 | 868 | 905 | chr3:129693286-129693885 | negative | 3 | 129694220 | 129694143 | 78 |
| DMRT1 | 0.86 | 0.425 | 0.75 | 0.8 | 832 | 869 | 906 | chr9:842333-842932 | negative | 9 | 843293 | 843222 | 72 |
| MYO3A | 0.86 | 0.636 | 0.75 | 0.75 | 833 | 870 | 907 | chr10:26222906-26223505 | negative | 10 | 26223879 | 26223805 | 75 |
| NA | 0.86 | 0.349 | 0.75 | 0.75 | 834 | 871 | 908 | chr19:39260654-39261253 | negative | 19 | 39261614 | 39261550 | 65 |
| FAM38B | 0.86 | 0.109 | 0.75 | 0.75 | 835 | 872 | 909 | chr18:11148614-11149213 | negative | 18 | 11149533 | 11149441 | 93 |
| NA | 0.86 | 0.106 | 0.75 | 0.75 | 836 | 873 | 910 | chr6:29520919-29521918 | chrm6_EC pos iv | 6 | 29521637 | 29521559 | 79 |
| DMRTA2 | 0.85 | 0.22 | 0.75 | 0.8 | 837 | 874 | 911 | chr1:50886649-50887248 | positive | 1 | 50886963 | 50886879 | 85 |
| LBX2 | 0.85 | 0.123 | 0.75 | 0.7 | 838 | 875 | 912 | chr2:74726434-74727033 | negative | 2 | 74727346 | 74727247 | 100 |
| NA | 0.85 | 0.146 | 0.75 | 0.8 | 839 | 876 | 913 | chr6:29520919-29521918 | chrm6_EC pos i | 6 | 29521300 | 29521219 | 82 |
| NA | 0.83 | 0.488 | 0.75 | 0.65 | 840 | 877 | 914 | chr6:29520919-29521918 | chrm6_EC neg iv | 6 | 29522298 | 29522214 | 85 |
| NA | 0.82 | 2.096 | 0.75 | 0.6 | 841 | 878 | 915 | chr1:208132487-208133086 | positive | 1 | 208132848 | 208132750 | 99 |
| GDF6 | 0.82 | 0.803 | 0.75 | 0.7 | 842 | 879 | 916 | chr8:97171601-97172200 | negative | 8 | 97172546 | 97172462 | 85 |
| GAD2 | 0.8 | 1.138 | 0.75 | 0.65 | 843 | 880 | 917 | chr10:26506776-26507375 | negative | 10 | 26507753 | 26507674 | 80 |
| NA | 0.8 | 0.5 | 0.75 | 0.65 | 844 | 881 | 918 | chr6:29520919-29521918 | chrm6_EC pos iii | 6 | 29521539 | 29521450 | 90 |
| FOXE1 | 0.78 | 0.162 | 0.75 | 0.65 | 845 | 882 | 919 | chr9:100616307-100616906 | positive | 9 | 100616638 | 100616565 | 74 |

**Table 13**

| rxn_name | gene_name | auc | mean_EC_cases | mean_controls | cutoff | specificity | sensitivity | Amplicon SEQ ID NO |
|---|---|---|---|---|---|---|---|---|
| ML_cg25060829_ii | ZSCAN 12 | 1 | 3.82 | 0 | 0.28 | 1 | 1 | 920 |
| ML_cg15768103_ii | GYPC | 1 | 4.26 | 0 | 0 | 1 | 1 | 922 |
| ML_cg15975865_i | GYPC | 1 | 4.92 | 0 | 0 | 1 | 1 | 924 |

**Table 14**

| rxn_name | gene_name | auc | mean_EC_cases | mean_controls | cutoff | specificity | sensitivity | Amplicon SEQ ID NO |
|---|---|---|---|---|---|---|---|---|
| ML_cg25060829_ii | ZSCAN12 | 0.94 | 6.46 | 0 | 0.28 | 1 | 0.88 | 920 |
| ML_cg15768103_ii | GYPC | 1 | 6.01 | 0.01 | 0 | 0.87 | 1 | 922 |
| ML_cg15975865_i | GYPC | 1 | 5.2 | 0.02 | 0 | 0.84 | 1 | 924 |

### Relationship between cancer index value and determining methylation status of CpGs

In view of the observations described herein (see Examples), the inventors derived a cancer index based on an analysis of methylation status (DNAme; as described above) for use in assays for assessing the presence or development of cancer in an individual.

As explained herein, the described assays particularly relate to the assessment of assessing the presence, absence or development of endometrial cancer.

Any of the assays described herein involve deriving a cancer index value based on the methylation of status of a panel of one or more CpGs assayed in a sample provided from an individual, as described and defined herein.

The cancer index value may be derived by any suitable means.

The inventors have identified specific CpGs, as described and defined herein, which may be used to form a panel of CpGs whose methylation status is determined in order to establish cancer index values in accordance with the assays described and defined herein. Using these panels the inventors have demonstrated that it is possible to derive a cancer index value which correlates with and is indicative of normal tissue, i.e. endometrial tissue which is cancer negative. Accordingly, cancer can be assessed to be absent in the individual. Using these panels the inventors have demonstrated that it is possible to derive a cancer index value which correlates with and is indicative of cancer tissue, i.e. endometrial tissue which is cancer positive. Accordingly, cancer can be assessed to be present in the individual. As explained herein, the inventors have shown that using panels of the CpGs that have been identified cancer can be assessed to be present in the individual. As explained herein, the inventors have shown that using panels of the CpGs that have been identified it can be shown that the DNA methylation profile of epithelial cells from normal tissue such as from the cervix, vagina, buccal area, or from blood and/or urine, particularly from a liquid-based cytology sample, and more preferably a cervical smear sample, as indicated by the cancer index value, is dynamic and subject to change on a continuum from indicating cancer negative to cancer positive tissue. In particular, the cancer index value described herein acts as a surrogate for indicating whether the endometrial tissue of an individual is cancer negative or cancer positive to a high degree of statistical accuracy. As such, using panels of the CpGs that have been identified it is possible to establish a cancer index value scale that can be used to assess the presence, absence or development of cancer in an individual.

As described herein, the inventors have used certain methods for determining the methylation status of specific CpGs in the population of DNA molecules in the sample. For example, in one method a percent methylated reference (PMR) value of a CpG may be determined. In another method the methylation β-values of a CpG may be determined. Different mechanisms may be employed to determine specific values depending on the circumstances, such as PCR-based mechanisms or array-based mechanisms.

As will be apparent to a skilled person, in the assays of the invention the steps of determining the methylation status of specific CpGs in the population of DNA molecules in the sample are not limited to any one specific methodology. As the skilled person will appreciate, because the cancer index value is based on the methylation status of CpGs, and since the methylation status of CpGs can be represented by values which may be specific to a specific methodology, e.g. percent methylated reference (PMR) value or methylation β-value, then the range of cancer index values which define cancer negative and cancer positive samples may be dependent upon the methodology used to determine the methylation status of CpGs. Nevertheless, a user may readily reproduce and implement the assays of the invention using any suitable methodology for determining the methylation status of CpGs, provided that the same methodology is used consistently. Moreover, the user can readily establish, *de novo,* cancer index values which define cancer negative and cancer positive samples by determining the methylation status of CpGs in panels constituting the specific CpGs disclosed herein from known cancer negative and cancer positive patient samples. Once such cancer index values are established using the CpGs identified herein, a user may use these values as a basis for assessing the presence, absence or development of cancer in any test individual whose cancer status is to be determined. Accordingly, cancer index values according to the present invention are not limited to specific methods of determination of methylation status of CpGs. On the contrary, the skilled person will appreciate that cancer index values can be established which reflect the intrinsic capabilities of the CpGs identified herein to correlate methylation status with cancer disease status.

Accordingly, the cancer index value may be derived by assessing the methylation status of the one or more CpGs in the panel in a sample provided from an individual by any suitable means.

The step of determining the methylation status of each CpG in the panel of one or more CpGs may be achieved by determining a percent methylated reference (PMR) value of each one of the one or more CpGs. The step of determining the methylation status of each CpG in the panel of one or more CpGs may be achieved by determining the methylation β-value of each one of the one or more CpGs.

In any of the assays described herein, the methylation status of the CpGs may be determined by any suitable means. For example, in any of the assays described herein the step of determining the methylation status of each CpG in the panel of one or more CpGs may comprise:
a. performing a sequencing step to determine the sequence of each CpG;
b. hybridising DNA to an array comprising probes capable of discriminating between methylated and non-methylated forms of the CpGs and applying a detection system to the array so as to determine the methylation status of each CpG; and/or
c. performing a PCR step using methylation-specific primers, wherein the methylation status of the CpG is determined by the presence or absence of a PCR product.

The step of determining the methylation status of each CpG in the panel of one or more CpGs may comprise a conversion step in order to distinguish methylated CpG dinucleotides relative to non-methylated CpG dinucleotides. The conversion step may comprise e.g. bisulfite conversion or TAPS (TET-assisted pyridine borane sequencing) conversion of the DNA in a sample that is to be applied to any one or more of a. to c. above. TAPS may particularly involve the steps of oxidising 5-methylcytosine bases (5mC) to 5-carboxylcytosine bases (5caC), preferably by ten-eleven translocation (TET), and/or oxidising 5-hydroxymethylcytosine bases (5hmC) to 5-carboxylcytosine bases (5caC), preferably by ten-eleven translocation (TET); followed by reducing 5-carboxylcytosine bases (5caC) to dihydrouracil bases (DHU), optionally with pyridine borane.

The step of determining the methylation status of each CpG in the panel of one or more CpGs may additionally, or alternatively, comprise the use of TempO-seq (templated Olig-sequencing). The oligoniclueotides in the context of TempO-seq may or may not be designed such that they hybridise with methylated CpG dinucleotides following a prior conversion as described herein.

The step of determining the methylation status of each CpG in the panel of one or more CpGs may comprise the contacting the DNA in the sample with one or more methylation sensitive restriction endonucleases that cleave methylated and/or unmethylated forms of their restriction sites, and preferably the contacting of the DNA is prior to performing any one of a. to c. above. In assays of the invention wherein methylation sensitive restriction enzymes are used, one or more control reactions are performed. Preferably, the one or more control reactions involve interrogation of known loci that contain (i) no restriction endonuclease sites; (ii) a restriction site that is methylated; (iii) a restriction site that is unmethylated.

Using any of the methods for determining the methylation status of each CpG in the panel of one or more CpGs, the proportion of methylated and unmethylated CpGs at any given locus may be determined, thereby enabling generation of a cancer index value.

Preferably, the step of determining in the population of DNA molecules in the sample the methylation status of a panel of one or more CpGs further comprises determining a β value of each CpG. Deriving the cancer index value may involve providing a methylation β-value data set comprising the methylation β-values for each CpG in the panel of one or more CpGs.

### Assessment of methylation status of CpGs

Methylation of DNA is a recognised form of epigenetic modification which has the capability of altering the expression of genes and other elements such as microRNAs. In cancer development and progression, methylation may have the effect of *e.g.* silencing tumor suppressor genes and/or increasing the expression of oncogenes. Other forms of dysregulation may occur as a result of methylation. Methylation of DNA occurs at discrete loci which are predominately dinucleotides consisting of a CpG motif, but may also occur at CHH motifs (where H is A, C, or T). During methylation, a methyl group is added to the fifth carbon of cytosine bases to create methylcytosine.

Methylation can occur throughout the genome and is not limited to regions with respect to an expressed sequence such as a gene. Methylation typically, but not always, occurs in a promoter or other regulatory region of an expressed sequence such as enhancer elements. Most typically, the methylation status of CpGs is clustered in CpG islands, for example CpG islands present in the regulatory regions of genes, especially in their promoter regions.

Typically, an assessment of DNA methylation status involves analysing the presence or absence of methyl groups in DNA, for example methyl groups on the 5 position of one or more cytosine nucleotides. Preferably, the methylation status of one or more cytosine nucleotides present as a CpG dinucleotide (where C stands for Cytosine, G for Guanine and p for the phosphate group linking the two) is assessed.

A variety of techniques are available for the identification and assessment of CpG methylation status, as will be outlined briefly below. The assays described herein encompass any suitable technique for the determination of CpG methylation status.

Methyl groups are lost from a starting DNA molecule during conventional *in vitro* handling steps such as PCR. To avoid this, techniques for the detection of methyl groups commonly involve the preliminary treatment of DNA prior to subsequent processing, in a way that preserves the methylation status information of the original DNA molecule. Such preliminary techniques involve three main categories of processing, *i.e.* bisulphite modification, restriction enzyme digestion and affinity-based analysis. Products of these techniques can then be coupled with sequencing or array-based platforms for subsequent identification or qualitative assessment of CpG methylation status.

Techniques involving bisulphite modification of DNA have become the most common assays for detection and assessment of methylation status of CpG dinucleotides. Treatment of DNA with bisulphite, e.g. sodium bisulphite, converts cytosine bases to uracil bases, but has no effect on 5-methylcytosines. Thus, the presence of a cytosine in bisulphite-treated DNA is indicative of the presence of a cytosine base which was previously methylated in the starting DNA molecule. Such cytosine bases can be detected by a variety of techniques. For example, primers specific for unmethylated versus methylated DNA can be generated and used for PCR-based identification of methylated CpG dinucleotides. DNA may be amplified, either before or after bisulphite conversion. A separation/capture step may be performed, e.g. using binding molecules such as complementary oligonucleotide sequences. Standard and next-generation DNA sequencing protocols can also be used.

In other approaches, methylation-sensitive enzymes can be employed which digest or cut only in the presence of methylated DNA. Analysis of resulting fragments is commonly carried out using mircroarrays.

Affinity-based techniques exploit binding interactions to capture fragments of methylated DNA for the purposes of enrichment. Binding molecules such as anti-5-methylcytosine antibodies are commonly employed prior to subsequent processing steps such as PCR and sequencing.

Olkhov-Mitsel and Bapat (2012) provide a comprehensive review of techniques available for the identification and assessment of biomarkers involving methylcytosine.

For the purposes of assessing the methylation status of the CpG-based biomarkers characterised and described herein, any suitable assay can be employed.

Assays described herein may comprise determining methylation status of CpGs by bisulphite converting the DNA. Preferred assays involve bisulphite treatment of DNA, including amplification of the identified CpG loci for methylation specific PCR and/or sequencing and/or assessment of the methylation status of target loci using methylation-discriminatory microarrays.

Amplification of CpG loci can be achieved by a variety of approaches. Preferably, CpG loci are amplified using PCR. A variety of PCR-based approaches may be used. For example, methylation-specific primers may be hybridized to DNA containing the CpG sequence of interest. Such primers may be designed to anneal to a sequence derived from either a methylated or non-methylated CpG locus. Following annealing, a PCR reaction is performed and the presence of a subsequent PCR product indicates the presence of an annealed CpG of identifiable sequence. In such assays, DNA is bisulphite converted prior to amplification. Such techniques are commonly referred to as methylation specific PCR (MSP).

In other techniques, PCR primers may anneal to the CpG sequence of interest independently of the methylation status, and further processing steps may be used to determine the status of the CpG. Assays are designed so that the CpG site(s) are located between primer annealing sites. This assay scheme is used in techniques such as bisulphite genomic sequencing, COBRA, Ms-SNuPE. In such assay, DNA can be bisulphite converted before or after amplification.

Small-scale PCR approaches may be used. Such approaches commonly involve mass partitioning of samples (e.g. digital PCR). These techniques offer robust accuracy and sensitivity in the context of a highly miniaturised system (pico-liter sized droplets), ideal for the subsequent handling of small quantities of DNA obtainable from the potentially small volume of cellular material present in biological samples, particularly urine samples. A variety of such small-scale PCR techniques are widely available. For example, microdroplet-based PCR instruments are available from a variety of suppliers, including RainDance Technologies, Inc. (Billerica, MA; http://raindancetech.com/) and Bio-Rad, Inc. (http://www.bio-rad.com/). Microarray platforms may also be used to carry out small-scale PCR. Such platforms may include microfluidic network-based arrays e.g. available from Fluidigm Corp. (www.fluidigm.com).

Following amplification of CpG loci, amplified PCR products may be coupled to subsequent analytical platforms in order to determine the methylation status of the CpGs of interest. For example, the PCR products may be directly sequenced to determine the presence or absence of a methylcytosine at the target CpG or analysed by array-based techniques.

Any suitable sequencing techniques may be employed to determine the sequence of target DNA. In the assays of the present invention the use of high-throughput, so-called "second generation", "third generation" and "next generation" techniques to sequence bisulphite-treated DNA can be used.

In second generation techniques, large numbers of DNA molecules are sequenced in parallel. Typically, tens of thousands of molecules are anchored to a given location at high density and sequences are determined in a process dependent upon DNA synthesis. Reactions generally consist of successive reagent delivery and washing steps, *e.g.* to allow the incorporation of reversible labelled terminator bases, and scanning steps to determine the order of base incorporation. Array-based systems of this type are available commercially e.g. from Illumina, Inc. (San Diego, CA; http://www.illumina.com/).

Third generation techniques are typically defined by the absence of a requirement to halt the sequencing process between detection steps and can therefore be viewed as real-time systems. For example, the base-specific release of hydrogen ions, which occurs during the incorporation process, can be detected in the context of microwell systems (e.g. see the Ion Torrent system available from Life Technologies; http://www.lifetechnologies.com/). Similarly, in pyrosequencing the base-specific release of pyrophosphate (PPi) is detected and analysed. In nanopore technologies, DNA molecules are passed through or positioned next to nanopores, and the identities of individual bases are determined following movement of the DNA molecule relative to the nanopore. Systems of this type are available commercially e.g. from Oxford Nanopore (https://www.nanoporetech.com/). In an alternative assay, a DNA polymerase enzyme is confined in a "zero-mode waveguide" and the identity of incorporated bases are determined with florescence detection of gamma-labeled phosphonucleotides (see *e.g.* Pacific Biosciences; http://www.pacificbiosciences.com/).

In other assays sequencing steps may be omitted. For example, amplified PCR products may be applied directly to hybridization arrays based on the principle of the annealing of two complementary nucleic acid strands to form a double-stranded molecule. Hybridization arrays may be designed to include probes which are able to hybridize to amplification products of a CpG and allow discrimination between methylated and non-methylated loci. For example, probes may be designed which are able to selectively hybridize to an CpG locus containing thymine, indicating the generation of uracil following bisulphite conversion of an unmethylated cytosine in the starting template DNA. Conversely, probes may be designed which are able to selectively hybridize to a CpG locus containing cytosine, indicating the absence of uracil conversion following bisulphite treatment. This corresponds with a methylated CpG locus in the starting template DNA.

Following the application of a suitable detection system to the array, computer-based analytical techniques can be used to determine the methylation status of a CpG. Detection systems may include, *e.g.* the addition of fluorescent molecules following a methylation status-specific probe extension reaction. Such techniques allow CpG status determination without the specific need for the sequencing of CpG amplification products. Such array-based discriminatory probes may be termed methylation-specific probes.

Any suitable methylation-discriminatory microarrays may be employed to assess the methylation status of the CpGs described herein. One particular methylation-discriminatory microarray system is provided by Illumina, Inc. (San Diego, CA; http://www.illumina.com/). In particular, the Infinium MethylationEPIC BeadChip array and the Infinium HumanMethylation450 BeadChip array systems may be used to assess the methylation status of CpGs for predicting cancer development as described herein. Such a system exploits the chemical modifications made to DNA following bisulphite treatment of the starting DNA molecule. Briefly, the array comprises beads to which are coupled oligonucleotide probes specific for DNA sequences corresponding to the unmethylated form of a CpG, as well as separate beads to which are coupled oligonucleotide probes specific for DNA sequences corresponding to the methylated form of an CpG. Candidate DNA molecules are applied to the array and selectively hybridize, under appropriate conditions, to the oligonucleotide probe corresponding to the relevant epigenetic form. Thus, a DNA molecule derived from a CpG which was methylated in the corresponding genomic DNA will selectively attach to the bead comprising the methylation-specific oligonucleotide probe, but will fail to attach to the bead comprising the non-methylation-specific oligonucleotide probe. Single-base extension of only the hybridized probes incorporates a labeled ddNTP, which is subsequently stained with a fluorescence reagent and imaged. The methylation status of the CpG is determined by calculating the ratio of the fluorescent signal derived from the methylated and unmethylated sites.

Infinium HumanMethylation450 BeadChip array systems can be used to interrogate CpGs in the assays described herein. Alternative or customised arrays could, however, be employed to interrogate the cancer-specific CpG biomarkers defined herein, provided that they comprise means for interrogating all CpG for a given assay, as defined herein.

Techniques involving combinations of the above-described assays may also be used. For example, DNA containing CpG sequences of interest may be hybridized to microarrays and then subjected to DNA sequencing to determine the status of the CpG as described above.

In the assays described above, sequences corresponding to CpG loci may also be subjected to an enrichment process if desired. DNA containing CpG sequences of interest may be captured by binding molecules such as oligonucleotide probes complementary to the CpG target sequence of interest. Sequences corresponding to CpG loci may be captured before or after bisulphite conversion or before or after amplification. Probes may be designed to be complementary to bisulphite converted DNA. Captured DNA may then be subjected to further processing steps to determine the status of the CpG, such as DNA sequencing steps.

Capture/separation steps may be custom designed. Alternatively a variety of such techniques are available commercially, e.g. the SureSelect target enrichment system available from Agilent Technologies (http://www.agilent.com/home). In this system biotinylated "bait" or "probe" sequences (e.g. RNA) complementary to the DNA containing CpG sequences of interest are hybridized to sample nucleic acids. Streptavidin-coated magnetic beads are then used to capture sequences of interest hybridized to bait sequences. Unbound fractions are discarded. Bait sequences are then removed (e.g. by digestion of RNA) thus providing an enriched pool of CpG target sequences separated from non-CpG sequences. Template DNA may be subjected to bisulphite conversion and target loci amplified by small-scale PCR such as microdroplet PCR using primers which are independent of the methylation status of the CpG. Following amplification, samples may be subjected to a capture step to enrich for PCR products containing the target CpG, *e.g.* captured and purified using magnetic beads, as described above. Following capture, a standard PCR reaction is carried out to incorporate DNA sequencing barcodes into CpG-containing amplicons. PCR products are again purified and then subjected to DNA sequencing and analysis to determine the presence or absence of a methylcytosine at the target genomic CpG.

CpG biomarker loci defined herein by SEQ ID NOs 1 to 500 correspond to Illumina^{®} identifiers (IlmnID) known in the art. These CpG loci identifiers refer to individual CpG sites used in the commercially available Illumina^{®} Infinium Methylation EPIC BeadChip kit and Illumina^{®} Infinium Human Methylation450 BeadChip kit. The identity of each CpG site represented by each CpG loci identifier is publicly available from the Illumina, Inc. website under reference to the CpG sites used in the Infinium Methylation EPIC BeadChip kit and the Infinium Human Methylation450 BeadChip kit.

To complement evolving public databases to provide accurate CpG loci identifiers and strand orientation, Illumina^{®} has developed a method to consistently designate CpG loci based on the actual or contextual sequence of each individual CpG locus. To unambiguously refer to CpG loci in any species, Illumina^{®} has developed a consistent and deterministic CpG loci database to ensure uniformity in the reporting of methylation data. The Illumina^{®} method takes advantage of sequences flanking a CpG locus to generate a unique CpG locus cluster ID. This number is based on sequence information only and is unaffected by genome version. Illumina's standardized nomenclature also parallels the TOP/BOT strand nomenclature (which indicates the strand orientation) commonly used for single nucleotide polymorphism (SNP) designation.

Illumina^{®} Identifiers for the Infinium MethylationEPIC BeadChip and Infinium Human Methylation450 BeadChip system are also available from public repositories such as Gene Expression Omnibus (GEO) (http://www.ncbi.nlm.nih.gov/geo/).

By assessing the methylation status of a CpG it is meant that a determination is made as to whether a given CpG is methylated or unmethylated. In addition, it is meant that a determination is made as to the degree to which a given CpG site is methylated across a population of CpG loci in a sample.

CpG methylation status may be measured indirectly using a detection system such as fluorescence. A methylation-discriminatory microarray may be used. When calculating the degree of methylation of a given CpG, the Illumina^{®} definition of beta-values may be used. The Illumina^{®} methylation beta-value of a specific CpG site is calculated from the intensity of the methylated (M) and unmethylated (U) alleles, as the ratio of fluorescent signals β=Max(M,0)/[Max(M,0)+Max(U,0)+100]. On this scale, 0<β<1, β values of 1 or close to 1 indicate 100% methylation whereas values of 0 or close to 0 indicate 0% methylation.

The methylation status of any one or more CpGs of the CpGs defined defined by SEQ ID NOs: 1 to 500 or identified in SEQ ID NOs: 501 to 808 may be assessed by any suitable technique. As explained in more detail in the Examples below, one particular exemplary technique which the inventors have used is a methylation discriminatory array, such as an Illumina InfiniumMethylation EPIC BeadChip. These assays utilise probes directed to methylated and unmethylated CpGs at a given locus.

Another exemplary technique which the inventors have used to determine the methylation status of any one or more CpGs is a fluorescence-based PCR technique referred to as MethyLight. These assays utilise forward and reverse PCR primers specific for sequences encompassing any one or more of the 500 CpGs defined according to SEQ ID NOS: 1 to 500 or identified in SEQ ID NOs: 501 to 808. The methylation status of one or more of the CpGs defined by SEQ ID NOs: 1 to 500 or identified in SEQ ID NOs: 501 to 808 may therefore be determined by MethyLight analysis. The detectable probes are typically designed such that they hybridise only to methylated forms of the one or more CpGs to be assayed in view of the bisulfite conversion step within a typical MethyLight protocol.

### Bioinformatic tools and statistical metrics for CpG-based assays

Software programs which aid in the *in silico* analysis of bisulphite converted DNA sequences and in primer design for the purposes of methylation-specific analyses are generally available and have been described previously.

In risk models for predicting cancer, a receiver-operating-characteristic (ROC) curve analysis is often used, in which the area under the curve (AUC) is assessed. Each point on the ROC curve shows the effect of a rule for turning a risk/likelihood estimate into a prediction of the presence, absence or development of cancer in an individual. The AUC measures how well the model discriminates between case subjects and control subjects. An ROC curve that corresponds to a random classification of case subjects and control subjects is a straight line with an AUC of 50%. An ROC curve that corresponds to perfect classification has an AUC of 100%.

In any of the methods described herein, the 95% confidence interval for the ROC AUC may be between 0.60 and 1.

In any of the methods described herein, the interval may be defined as a range having as an upper limit any number between 0.60 and 1. The upper limit number may be 0.60, 0.61, 0.62, 0.63, 0.64, 0.65, 0.66, 0.67, 0.68, 0.69, 0.70, 0.71, 0.72, 0.73, 0.74, 0.75, 0.76, 0.77, 0.78, 0.79, 0.80, 0.81, 0.82, 0.83, 0.84, 0.85, 0.86, 0.87, 0.88, 0.89, 0.90, 0.91, 0.92, 0.93, 0.94, 0.95, 0.96, 0.97, 0.98, 0.99 or 1.00.

In any of the methods described herein, the interval may be defined as a range having as a lower limit any number between 0.60 and 1. The lower limit number may be 0.60, 0.61, 0.62, 0.63, 0.64, 0.65, 0.66, 0.67, 0.68, 0.69, 0.70, 0.71, 0.72, 0.73, 0.74, 0.75, 0.76, 0.77, 0.78, 0.79, 0.80, 0.81, 0.82, 0.83, 0.84, 0.85, 0.86, 0.87, 0.88, 0.89, 0.90, 0.91, 0.92, 0.93, 0.94, 0.95, 0.96, 0.97, 0.98, 0.99 or 1.00.

In any of the methods described herein, the interval range may comprise any of the above lower limit numbers combined with any of the above upper limit numbers as appropriate.

Preferably, the upper limit number is 1. Thus, the 95% confidence ROC AUC interval may be defined as a range having an upper limit of 1 and as a lower limit any number between 0.60 and 1. The lower limit number may be 0.60, 0.61, 0.62, 0.63, 0.64, 0.65, 0.66, 0.67, 0.68, 0.69, 0.70, 0.71, 0.72, 0.73, 0.74, 0.75, 0.76, 0.77, 0.78, 0.79, 0.80, 0.81, 0.82, 0.83, 0.84, 0.85, 0.86, 0.87, 0.88, 0.89, 0.90, 0.91, 0.92, 0.93, 0.94, 0.95, 0.96, 0.97, 0.98, 0.99 or 1.00.

The upper limit number may be 0.99. Thus, the 95% confidence ROC AUC interval may be defined as a range having an upper limit of 0.99 and as a lower limit any number between 0.60 and 0.99. The lower limit number may be 0.60, 0.61, 0.62, 0.63, 0.64, 0.65, 0.66, 0.67, 0.68, 0.69, 0.70, 0.71, 0.72, 0.73, 0.74, 0.75, 0.76, 0.77, 0.78, 0.79, 0.80, 0.81, 0.82, 0.83, 0.84, 0.85, 0.86, 0.87, 0.88, 0.89, 0.90, 0.91, 0.92, 0.93, 0.94, 0.95, 0.96, 0.97, 0.98 or 0.99.

The upper limit number may be 0.98. Thus, the 95% confidence ROC AUC interval may be defined as a range having an upper limit of 0.98 and as a lower limit any number between 0.60 and 0.98. The lower limit number may be 0.60, 0.61, 0.62, 0.63, 0.64, 0.65, 0.66, 0.67, 0.68, 0.69, 0.70, 0.71, 0.72, 0.73, 0.74, 0.75, 0.76, 0.77, 0.78, 0.79, 0.80, 0.81, 0.82, 0.83, 0.84, 0.85, 0.86, 0.87, 0.88, 0.89, 0.90, 0.91, 0.92, 0.93, 0.94, 0.95, 0.96, 0.97 or 0.98.

The upper limit number may be 0.97. Thus, the 95% confidence ROC AUC interval may be defined as a range having an upper limit of 0.97 and as a lower limit any number between 0.60 and 0.97. The lower limit number may be 0.60, 0.61, 0.62, 0.63, 0.64, 0.65, 0.66, 0.67, 0.68, 0.69, 0.70, 0.71, 0.72, 0.73, 0.74, 0.75, 0.76, 0.77, 0.78, 0.79, 0.80, 0.81, 0.82, 0.83, 0.84, 0.85, 0.86, 0.87, 0.88, 0.89, 0.90, 0.91, 0.92, 0.93, 0.94, 0.95, 0.96 or 0.97.

The upper limit number may be 0.96. Thus, the 95% confidence ROC AUC interval may be defined as a range having an upper limit of 0.96 and as a lower limit any number between 0.60 and 0.96. The lower limit number may be 0.60, 0.61, 0.62, 0.63, 0.64, 0.65, 0.66, 0.67, 0.68, 0.69, 0.70, 0.71, 0.72, 0.73, 0.74, 0.75, 0.76, 0.77, 0.78, 0.79, 0.80, 0.81, 0.82, 0.83, 0.84, 0.85, 0.86, 0.87, 0.88, 0.89, 0.90, 0.91, 0.92, 0.93, 0.94, 0.95 or 0.96.

The upper limit number may be 0.95. Thus, the 95% confidence ROC AUC interval may be defined as a range having an upper limit of 0.95 and as a lower limit any number between 0.60 and 0.95. The lower limit number may be 0.60, 0.61, 0.62, 0.63, 0.64, 0.65, 0.66, 0.67, 0.68, 0.69, 0.70, 0.71, 0.72, 0.73, 0.74, 0.75, 0.76, 0.77, 0.78, 0.79, 0.80, 0.81, 0.82, 0.83, 0.84, 0.85, 0.86, 0.87, 0.88, 0.89, 0.90, 0.91, 0.92, 0.93, 0.94 or 0.95.

The upper limit number may be 0.94. Thus, the 95% confidence ROC AUC interval may be defined as a range having an upper limit of 0.94 and as a lower limit any number between 0.60 and 0.94. The lower limit number may be 0.60, 0.61, 0.62, 0.63, 0.64, 0.65, 0.66, 0.67, 0.68, 0.69, 0.70, 0.71, 0.72, 0.73, 0.74, 0.75, 0.76, 0.77, 0.78, 0.79, 0.80, 0.81, 0.82, 0.83, 0.84, 0.85, 0.86, 0.87, 0.88, 0.89, 0.90, 0.91, 0.92, 0.93 or 0.94.

The upper limit number may be 0.93. Thus, the 95% confidence ROC AUC interval may be defined as a range having an upper limit of 0.93 and as a lower limit any number between 0.60 and 0.93. The lower limit number may be 0.60, 0.61, 0.62, 0.63, 0.64, 0.65, 0.66, 0.67, 0.68, 0.69, 0.70, 0.71, 0.72, 0.73, 0.74, 0.75, 0.76, 0.77, 0.78, 0.79, 0.80, 0.81, 0.82, 0.83, 0.84, 0.85, 0.86, 0.87, 0.88, 0.89, 0.90, 0.91, 0.92 or 0.93.

The upper limit number may be 0.92. Thus, the 95% confidence ROC AUC interval may be defined as a range having an upper limit of 0.92 and as a lower limit any number between 0.60 and 0.92. The lower limit number may be 0.60, 0.61, 0.62, 0.63, 0.64, 0.65, 0.66, 0.67, 0.68, 0.69, 0.70, 0.71, 0.72, 0.73, 0.74, 0.75, 0.76, 0.77, 0.78, 0.79, 0.80, 0.81, 0.82, 0.83, 0.84, 0.85, 0.86, 0.87, 0.88, 0.89, 0.90, 0.91 or 0.92.

The upper limit number may be 0.91. Thus, the 95% confidence ROC AUC interval may be defined as a range having an upper limit of 0.91 and as a lower limit any number between 0.60 and 0.91. The lower limit number may be 0.60, 0.61, 0.62, 0.63, 0.64, 0.65, 0.66, 0.67, 0.68, 0.69, 0.70, 0.71, 0.72, 0.73, 0.74, 0.75, 0.76, 0.77, 0.78, 0.79, 0.80, 0.81, 0.82, 0.83, 0.84, 0.85, 0.86, 0.87, 0.88, 0.89, 0.90 or 0.91.

The upper limit number may be 0.90. Thus, the 95% confidence ROC AUC interval may be defined as a range having an upper limit of 0.90 and as a lower limit any number between 0.60 and 0.90. The lower limit number may be 0.60, 0.61, 0.62, 0.63, 0.64, 0.65, 0.66, 0.67, 0.68, 0.69, 0.70, 0.71, 0.72, 0.73, 0.74, 0.75, 0.76, 0.77, 0.78, 0.79, 0.80, 0.81, 0.82, 0.83, 0.84, 0.85, 0.86, 0.87, 0.88, 0.89 or 0.90.

The upper limit number may be 0.89. Thus, the 95% confidence ROC AUC interval may be defined as a range having an upper limit of 0.89 and as a lower limit any number between 0.60 and 0.89. The lower limit number may be 0.60, 0.61, 0.62, 0.63, 0.64, 0.65, 0.66, 0.67, 0.68, 0.69, 0.70, 0.71, 0.72, 0.73, 0.74, 0.75, 0.76, 0.77, 0.78, 0.79, 0.80, 0.81, 0.82, 0.83, 0.84, 0.85, 0.86, 0.87, 0.88 or 0.89.

The upper limit number may be 0.88. Thus, the 95% confidence ROC AUC interval may be defined as a range having an upper limit of 0.88 and as a lower limit any number between 0.60 and 0.88. The lower limit number may be 0.60, 0.61, 0.62, 0.63, 0.64, 0.65, 0.66, 0.67, 0.68, 0.69, 0.70, 0.71, 0.72, 0.73, 0.74, 0.75, 0.76, 0.77, 0.78, 0.79, 0.80, 0.81, 0.82, 0.83, 0.84, 0.85, 0.86, 0.87 or 0.88.

The upper limit number may be 0.87. Thus, the 95% confidence ROC AUC interval may be defined as a range having an upper limit of 0.87 and as a lower limit any number between 0.60 and 0.87. The lower limit number may be 0.60, 0.61, 0.62, 0.63, 0.64, 0.65, 0.66, 0.67, 0.68, 0.69, 0.70, 0.71, 0.72, 0.73, 0.74, 0.75, 0.76, 0.77, 0.78, 0.79, 0.80, 0.81, 0.82, 0.83, 0.84, 0.85, 0.86 or 0.87.

The upper limit number may be 0.86. Thus, the 95% confidence ROC AUC interval may be defined as a range having an upper limit of 0.86 and as a lower limit any number between 0.60 and 0.86. The lower limit number may be 0.60, 0.61, 0.62, 0.63, 0.64, 0.65, 0.66, 0.67, 0.68, 0.69, 0.70, 0.71, 0.72, 0.73, 0.74, 0.75, 0.76, 0.77, 0.78, 0.79, 0.80, 0.81, 0.82, 0.83, 0.84, 0.85 or 0.86.

The upper limit number may be 0.85. Thus, the 95% confidence ROC AUC interval may be defined as a range having an upper limit of 0.85 and as a lower limit any number between 0.60 and 0.85. The lower limit number may be 0.60, 0.61, 0.62, 0.63, 0.64, 0.65, 0.66, 0.67, 0.68, 0.69, 0.70, 0.71, 0.72, 0.73, 0.74, 0.75, 0.76, 0.77, 0.78, 0.79, 0.80, 0.81, 0.82, 0.83, 0.84 or 0.85.

The upper limit number may be 0.84. Thus, the 95% confidence ROC AUC interval may be defined as a range having an upper limit of 0.84 and as a lower limit any number between 0.60 and 0.84. The lower limit number may be 0.60, 0.61, 0.62, 0.63, 0.64, 0.65, 0.66, 0.67, 0.68, 0.69, 0.70, 0.71, 0.72, 0.73, 0.74, 0.75, 0.76, 0.77, 0.78, 0.79, 0.80, 0.81, 0.82, 0.83 or 0.84.

The upper limit number may be 0.83. Thus, the 95% confidence ROC AUC interval may be defined as a range having an upper limit of 0.83 and as a lower limit any number between 0.60 and 0.83. The lower limit number may be 0.60, 0.61, 0.62, 0.63, 0.64, 0.65, 0.66, 0.67, 0.68, 0.69, 0.70, 0.71, 0.72, 0.73, 0.74, 0.75, 0.76, 0.77, 0.78, 0.79, 0.80, 0.81, 0.82 or 0.83.

The upper limit number may be 0.82. Thus, the 95% confidence ROC AUC interval may be defined as a range having an upper limit of 0.82 and as a lower limit any number between 0.60 and 0.82. The lower limit number may be 0.60, 0.61, 0.62, 0.63, 0.64, 0.65, 0.66, 0.67, 0.68, 0.69, 0.70, 0.71, 0.72, 0.73, 0.74, 0.75, 0.76, 0.77, 0.78, 0.79, 0.80, 0.81 or 0.82.

The upper limit number may be 0.81. Thus, the 95% confidence ROC AUC interval may be defined as a range having an upper limit of 0.81 and as a lower limit any number between 0.60 and 0.81. The lower limit number may be 0.60, 0.61, 0.62, 0.63, 0.64, 0.65, 0.66, 0.67, 0.68, 0.69, 0.70, 0.71, 0.72, 0.73, 0.74, 0.75, 0.76, 0.77, 0.78, 0.79, 0.80 or 0.81.

The upper limit number may be 0.80. Thus, the 95% confidence ROC AUC interval may be defined as a range having an upper limit of 0.80 and as a lower limit any number between 0.60 and 0.80. The lower limit number may be 0.60, 0.61, 0.62, 0.63, 0.64, 0.65, 0.66, 0.67, 0.68, 0.69, 0.70, 0.71, 0.72, 0.73, 0.74, 0.75, 0.76, 0.77, 0.78, 0.79 or 0.80.

The upper limit number may be 0.79. Thus, the 95% confidence ROC AUC interval may be defined as a range having an upper limit of 0.79 and as a lower limit any number between 0.60 and 0.79. The lower limit number may be 0.60, 0.61, 0.62, 0.63, 0.64, 0.65, 0.66, 0.67, 0.68, 0.69, 0.70, 0.71, 0.72, 0.73, 0.74, 0.75, 0.76, 0.77, 0.78 or 0.79.

The upper limit number may be 0.78. Thus, the 95% confidence ROC AUC interval may be defined as a range having an upper limit of 0.78 and as a lower limit any number between 0.60 and 0.78. The lower limit number may be 0.60, 0.61, 0.62, 0.63, 0.64, 0.65, 0.66, 0.67, 0.68, 0.69, 0.70, 0.71, 0.72, 0.73, 0.74, 0.75, 0.76, 0.77 or 0.78.

The upper limit number may be 0.77. Thus, the 95% confidence ROC AUC interval may be defined as a range having an upper limit of 0.77 and as a lower limit any number between 0.60 and 0.77. The lower limit number may be 0.60, 0.61, 0.62, 0.63, 0.64, 0.65, 0.66, 0.67, 0.68, 0.69, 0.70, 0.71, 0.72, 0.73, 0.74, 0.75, 0.76 or 0.77.

The upper limit number may be 0.76. Thus, the 95% confidence ROC AUC interval may be defined as a range having an upper limit of 0.76 and as a lower limit any number between 0.60 and 0.76. The lower limit number may be 0.60, 0.61, 0.62, 0.63, 0.64, 0.65, 0.66, 0.67, 0.68, 0.69, 0.70, 0.71, 0.72, 0.73, 0.74, 0.75 or 0.76.

The upper limit number may be 0.75. Thus, the 95% confidence ROC AUC interval may be defined as a range having an upper limit of 0.75 and as a lower limit any number between 0.60 and 0.75. The lower limit number may be 0.60, 0.61, 0.62, 0.63, 0.64, 0.65, 0.66, 0.67, 0.68, 0.69, 0.70, 0.71, 0.72, 0.73, 0.74 or 0.75.

The upper limit number may be 0.74. Thus, the 95% confidence ROC AUC interval may be defined as a range having an upper limit of 0.74 and as a lower limit any number between 0.60 and 0.74. The lower limit number may be 0.60, 0.61, 0.62, 0.63, 0.64, 0.65, 0.66, 0.67, 0.68, 0.69, 0.70, 0.71, 0.72, 0.73 or 0.74.

The upper limit number may be 0.73. Thus, the 95% confidence ROC AUC interval may be defined as a range having an upper limit of 0.73 and as a lower limit any number between 0.60 and 0.73. The lower limit number may be 0.60, 0.61, 0.62, 0.63, 0.64, 0.65, 0.66, 0.67, 0.68, 0.69, 0.70, 0.71, 0.72 or 0.73.

The upper limit number may be 0.72. Thus, the 95% confidence ROC AUC interval may be defined as a range having an upper limit of 0.72 and as a lower limit any number between 0.60 and 0.72. The lower limit number may be 0.60, 0.61, 0.62, 0.63, 0.64, 0.65, 0.66, 0.67, 0.68, 0.69, 0.70, 0.71 or 0.72.

The upper limit number may be 0.71. Thus, the 95% confidence ROC AUC interval may be defined as a range having an upper limit of 0.71 and as a lower limit any number between 0.60 and 0.71. The lower limit number may be 0.60, 0.61, 0.62, 0.63, 0.64, 0.65, 0.66, 0.67, 0.68, 0.69, 0.70 or 0.71.

The upper limit number may be 0.70. Thus, the 95% confidence ROC AUC interval may be defined as a range having an upper limit of 0.70 and as a lower limit any number between 0.60 and 0.70. The lower limit number may be 0.60, 0.61, 0.62, 0.63, 0.64, 0.65, 0.66, 0.67, 0.68, 0.69 or 0.70.

### Methods of treatment and diagnosis

Disclosed but not claimed are methods of treatment.

The term "treatment" as used herein is intended to refer to any intervention or procedure performed on an individual, including a surgical intervention or a pharmacological intervention such as the administration of a compound or drug. Any such treatment may be performed for therapeutic purposes or for preventative or prophylactic purposes.

One or more treatment steps following a positive classification of endometrial cancer may be performed, based on any of the methods described herein. Said treatments may be considered "therapeutic" treatments.

One or more treatment steps following a negative classification of cancer or prediction of an individual being at risk of endometrial cancer development may be performed, based on any of the methods described herein. Said treatments may be considered "risk prevention", "preventative" or "prophylactic" treatments.

One or more treatment steps following a negative classification of cancer or prediction of an individual being at risk of cancer development may be performed based on any of the methods described herein, in an individual that harbours one or more mutations that predispose the individual to an increased risk of developing cancer.

Disclosed is a method of treating a cancer patient comprising administering chemotherapy, radiation, immunotherapy or any cancer therapy described herein to the patient determined to have a cancer index value which indicates that the patient has is positive for cancer based on any of the assays described herein.

Disclosed is a method of treating and/or preventing cancer in an individual, the method comprising:
a. assessing the cancer status of the individual by assessing the presence, absence or development of cancer in the individual by performing any one of the assays described herein;
b. administering one or more therapeutic or preventative treatments to the individual based on the assessment,

preferably wherein the cancer is endometrial and/or ovarian cancer, most
preferably endometrial cancer.

Disclosed is a method of treating and/or preventing cancer
in an individual, the method comprising:
a. assessing the cancer status of the individual by assessing the presence, absence or development of cancer in the individual comprising:
   i. providing a sample which has been taken from the individual, the sample comprising a population of DNA molecules;
   ii. determining in the population of DNA molecules in the sample the methylation status of a panel of:
      1. one or more CpGs comprising the CpG identified in SEQ ID NO 67 wherein the CpG is identified at nucleotide positions 61 to 62
   iii. deriving a cancer index value based on the methylation status of the one or more CpGs in the panel; and
   iv. assessing the presence, absence or development of cancer in the individual based on the cancer index value, wherein the assay is characterised as having an area under the curve (AUC) of 0.90 or more as determined by receiver operating characteristics (ROC);
b. administering one or more therapeutic treatments to the individual based on the assessment,

preferably wherein the cancer in endometrial and/or ovarian cancer, most
preferably endometrial cancer.

In any of the methods of treatment, the step of predicting the presence or development of cancer, preferably wherein the cancer in endometrial and/or ovarian cancer, in an individual may involve deriving a cancer index value.

In any of the methods of treatment, the step of predicting the presence or development of cancer in an individual may involve the use of any one of the arrays described herein.

In any of the methods of treatment, the step of stratifying the individual may involve applying any one of the thresholds according to any one of the assays of the invention described herein.

The step of administering one or more treatments may comprise different treatment steps depending on the stratification of an individual on the basis of their cancer status or their risk of having cancer or on the basis of risk of cancer development, particularly endometrial and/or ovarian cancer, most preferably endometrial cancer. Particularly the amount of an invasiveness of the treatments administered may vary dependent on the stratification of an individual on the basis of their cancer status or their risk of having cancer or on the basis of their risk of cancer development. The treatments administered to the individual may comprise any treatments considered suitable by a person skilled in the art.

For example, when the individual is assessed as not having endometrial cancer or as having a low risk of endometrial cancer development, and wherein the cancer index value is about -0.660 or more and less than about -0.430, and preferably wherein the assay comprises determining methylation β-values for each CpG in the panel of one or more CpGs, the individual is subjected to one or more treatments according to their cancer index value, the one or more treatments may comprise any of:
a. a transvaginal ultrasound to assess endometrium and ovaries;
b. a repeat assay according to any one of the assays of the invention, preferably wherein the repeat assay is performed about two years after the previous assay

When the individual is assessed as having endometrial cancer or as having a moderate risk of Endometrial cancer development, and wherein the cancer index value is about -0.430 or more and less than about -0.230, and preferably wherein the assay comprises determining methylation β-values for each CpG in the panel of one or more CpGs, the individual is subjected to one or more treatments according to their cancer index value, the one or more treatments may comprise any of:
a. a transvaginal ultrasound to assess endometrium and ovaries;
b. intensified screening, preferably wherein the intensified screening comprises one or more of:
   ix. a colposcopy;
   x. a HPV test;
   xi. a cervical cytology test;
   xii. a test for CA125, preferably wherein the test is repeated six-monthly;
   xiii. a test for cell-free tumour DNA methylation in plasma/serum, preferably wherein the test is repeated annually;
   xiv. a test for cell-free tumour DNA methylation in vaginal fluid, preferably wherein the test is repeated annually
   xv. a pelvic MRI scan, preferably wherein the individual being subjected to the pelvic MRI scan is post-menopausal, and preferably wherein the scan is repeated annually;
   xvi. a repeat assay according to any one of the assays described herein, preferably wherein the repeat assay is performed about one year after the previous assay
c. administration of one or more of progestogens, particularly wherein the progestogens are delivered locally or systemically, Aspirin, Metformin, aromatase-inhibitors, weight-loss regimen

In any of the methods of treatment described herein, when the colposcopy, HPV and cytology tests are negative, the intensified screening may further comprise a hysteroscopy and endocervical and endometrial biopsy. More preferably, when the transvaginal ultrasound and intensified screening are both negative:
a. the transvaginal ultrasound, the test for CA125, the test for cell-free tumour DNA methylation in plasma/serum, and the test for cell-free tumour DNA methylation in vaginal fluid may be repeated about six months after the previous assay; and
b. the colposcopy, the HPV test, and the cervical cytology test, may be repeated about one year after the previous assay.

When the individual is assessed as having endometrial cancer or as having a high risk of endometrial cancer development, and wherein the cancer index value is about - 0.230 or more, and preferably wherein the assay comprises determining methylation (3-values for each CpG in the panel of one or more CpGs, and the individual is subjected to one or more treatments according to their cancer index value, the one or more treatments may comprise any of:
a. a transvaginal ultrasound to assess endometrium and ovaries;
b. intensified screening, preferably wherein the intensified screening comprises one or more of:
   i. a colposcopy;
   ii. a HPV test;
   iii. a cervical cytology test;
   iv. a test for CA125, preferably wherein the test is repeated six-monthly;
   v. a test for cell-free tumour DNA methylation in plasma/serum, preferably wherein the test is repeated annually;
   vi. a test for cell-free tumour DNA methylation in vaginal fluid, preferably wherein the test is repeated annually
   vii. a pelvic MRI scan, preferably wherein the individual being subjected to the pelvic MRI scan is post-menopausal, and preferably wherein the scan is repeated annually;
   viii. a repeat assay according to any of the assays described herein, preferably wherein the repeat assay is performed about one year after the previous assay;
c. administration of one or more of progestogens, particularly wherein the progestogens are delivered locally or systemically, Aspirin, Metformin, aromatase-inhibitors, weight-loss regimen;
d. a total hysterectomy and bilateral salpingo-oophorectomy

In any of the methods of treatment described herein, when the colposcopy, HPV and cytology tests are negative, the intensified screening may further comprise a hysteroscopy and endocervical and endometrial biopsy. More preferably, when the transvaginal ultrasound and intensified screening are both negative:
a. the transvaginal ultrasound, the test for CA125, the test for cell-free tumour DNA methylation in plasma/serum, the test for cell-free tumour DNA methylation in vaginal fluid, the colposcopy, the HPV test, and the cervical cytology test may be repeated about six months after the previous assay; and
b. the pelvic MRI scan may be repeated about one year after the previous assay.

In any of the assays described herein, wherein tests are performed as part of intensified screening the tests may be repeated at any suitable interval. A test for CA125 may be performed three-monthly, six-monthly, annually or about once every two, three or four years. A test for cell-free tumour DNA methylation in plasma/serum may be performed three-monthly, six-monthly, annually or about once every two, three or four years. A test for cell-free tumour DNS methylation in vaginal fluid may be performed three-monthly, six-monthly, annually or about once every two, three or four years. A pelvic MRI scan may be performed three-monthly, six-monthly, annually or about once every two, three or four years.

Wherein the individual is assessed as having a moderate to high risk of having endometrial cancer or a moderate to high risk of endometrial cancer development, the one or more treatments may comprise administration of one or more of progestogens, particularly wherein the progestogens are delivered locally or systemically, Aspirin, Metformin, aromatase-inhibitors, and a weight-loss regimen.

Other exemplary treatments comprise one or more surgical procedures, one or more chemotherapeutic agents, one or more cytotoxic chemotherapeutic agents one or more radiotherapeutic agents, one or more immunotherapeutic agents, one or more biological therapeutics, one or more anti-hormonal treatments or any combination of the above following a positive diagnosis of cancer.

In any of the methods of treatment described herein, the individual may particularly be administered treatments recited in Table 9. Four sub-groups defined by ranges of cancer index values are specified in Table 9 as corresponding to preferred clinical actions, comprising intensified screening, administration of therapeutics and surgery.

Cancer treatments may be administered to an individual harbouring cancer or at risk of cancer development, in an amount sufficient to prevent, treat, cure, alleviate or partially arrest cancer or one or more of its symptoms. Such treatments may result in a decrease in severity, and/or decreased cancer index value, of cancer symptoms, or an increase in frequency or duration of symptom-free periods. A treatment amount adequate to accomplish this is defined as "therapeutically effective amount". Effective amounts for a given purpose will depend on the severity of cancer and/or the individual's cancer index value as well as the weight and general state of the individual. As used herein, the term *"individual"* includes any human, preferably wherein the human is a woman. As used herein, *"treatment"* is to be considered synonymous with *"therapeutic agent".*

The following therapeutic agents may be administered to an individual based on their cancer risk alone or in combination with any other treatment described herein. The therapeutic agent may be directly attached, for example by chemical conjugation, to an antibody. Methods of conjugating agents or labels to an antibody are known in the art. For example, carbodiimide conjugation (Bauminger & Wilchek (1980) Methods Enzymol. 70, 151-159) may be used to conjugate a variety of agents, including doxorubicin, to antibodies or peptides. The water-soluble carbodiimide, 1-ethyl-3-(3-dimethylaminopropyl) carbodiimide (EDC) is particularly useful for conjugating a functional moiety to a binding moiety. Other methods for conjugating a moiety to antibodies can also be used. For example, sodium periodate oxidation followed by reductive alkylation of appropriate reactants can be used, as can glutaraldehyde crosslinking. However, it is recognised that, regardless of which method of producing a conjugate is selected, a determination must be made that the antibody maintains its targeting ability and that the functional moiety maintains its relevant function.

A cytotoxic moiety may be directly and/or indirectly cytotoxic. By "directly cytotoxic" it is meant that the moiety is one which on its own is cytotoxic. By "indirectly cytotoxic" it is meant that the moiety is one which, although is not itself cytotoxic, can induce cytotoxicity, for example by its action on a further molecule or by further action on it. The cytotoxic moiety may be cytotoxic only when intracellular and is preferably not cytotoxic when extracellular.

Cytotoxic chemotherapeutic agents are well known in the art. Cytotoxic chemotherapeutic agents, such as anticancer agents, include: alkylating agents including nitrogen mustards such as mechlorethamine (HN2), cyclophosphamide, ifosfamide, melphalan (L-sarcolysin) and chlorambucil; ethylenimines and methylmelamines such as hexamethylmelamine, thiotepa; alkyl sulphonates such as busulfan; nitrosoureas such as carmustine (BCNU), lomustine (CCNU), semustine (methyl-CCNU) and streptozocin (streptozotocin); and triazenes such as decarbazine (DTIC; dimethyltriazenoimidazole-carboxamide); Antimetabolites including folic acid analogues such as methotrexate (amethopterin); pyrimidine analogues such as fluorouracil (5-fluorouracil; 5-FU), floxuridine (fluorodeoxyuridine; FUdR) and cytarabine (cytosine arabinoside); and purine analogues and related inhibitors such as mercaptopurine (6-mercaptopurine; 6-MP), thioguanine (6-thioguanine; TG) and pentostatin (2'-deoxycoformycin). Natural Products including vinca alkaloids such as vinblastine (VLB) and vincristine; epipodophyllotoxins such as etoposide and teniposide; antibiotics such as dactinomycin (actinomycin D), daunorubicin (daunomycin; rubidomycin), doxorubicin, bleomycin, plicamycin (mithramycin) and mitomycin (mitomycin C); enzymes such as L-asparaginase; and biological response modifiers such as interferon alphenomes. Miscellaneous agents including platinum coordination complexes such as cisplatin (cis-DDP) and carboplatin; anthracenedione such as mitoxantrone and anthracycline; substituted urea such as hydroxyurea; methyl hydrazine derivative such as procarbazine (N-methylhydrazine, MIH); and adrenocortical suppressant such as mitotane (o,p'-DDD) and aminoglutethimide; taxol and analogues/derivatives; and hormone agonists/antagonists such as flutamide and tamoxifen.

A cytotoxic chemotherapeutic agent may be a cytotoxic peptide or polypeptide moiety which leads to cell death. Cytotoxic peptide and polypeptide moieties are well known in the art and include, for example, ricin, abrin, Pseudomonas exotoxin, tissue factor and the like. Methods for linking them to targeting moieties such as antibodies are also known in the art. Other ribosome inactivating proteins are described as cytotoxic agents in WO 96/06641. Pseudomonas exotoxin may also be used as the cytotoxic polypeptide. Certain cytokines, such as TNFα and IL-2, may also be useful as cytotoxic agents.

Certain radioactive atoms may also be cytotoxic if delivered in sufficient doses. Radiotherapeutic agents may comprise a radioactive atom which, in use, delivers a sufficient quantity of radioactivity to the target site so as to be cytotoxic. Suitable radioactive atoms include phosphorus-32, iodine-125, iodine-131, indium-111, rhenium-186, rhenium-188 or yttrium-90, or any other isotope which emits enough energy to destroy neighbouring cells, organelles or nucleic acid. Preferably, the isotopes and density of radioactive atoms are such that a dose of more than 4000 cGy (preferably at least 6000, 8000 or 10000 cGy) is delivered to the target site and, preferably, to the cells at the target site and their organelles, particularly the nucleus.

The radioactive atom may be attached to an antibody, antigen-binding fragment, variant, fusion or derivative thereof in known ways. For example, EDTA or another chelating agent may be attached to the binding moiety and used to attach 111In or 90Y. Tyrosine residues may be directly labelled with 1251 or 1311.

A cytotoxic chemotherapeutic agent may be a suitable indirectly-cytotoxic polypeptide. In a particularly preferred embodiment, the indirectly cytotoxic polypeptide is a polypeptide which has enzymatic activity and can convert a non-toxic and/or relatively non-toxic prodrug into a cytotoxic drug. With antibodies, this type of system is often referred to as ADEPT (Antibody-Directed Enzyme Prodrug Therapy). The system requires that the antibody locates the enzymatic portion to the desired site in the body of the patient and after allowing time for the enzyme to localise at the site, administering a prodrug which is a substrate for the enzyme, the end product of the catalysis being a cytotoxic compound. The object of the approach is to maximise the concentration of drug at the desired site and to minimise the concentration of drug in normal tissues. In a preferred embodiment, the cytotoxic moiety is capable of converting a non-cytotoxic prodrug into a cytotoxic drug.

In any of the methods of treatment described herein, the one or more treatments that the individual is subjected to may be repeated on one or more occasions. The one or more treatments may be repeated at regular intervals. The repetitive nature of the treatment administration may depend on the particular treatment being administered. Some treatments may require repetitive administration at greater frequency than others. The skilled person would be aware of the frequency of administration required for therapies known in the art. The one or more treatments may be repeated weekly, two weekly, three weekly, four weekly, monthly, three monthly, six monthly, yearly, two yearly, three yearly, four yearly, or five yearly.

In any of the methods described herein, when the individual is assessed as having a cancer index value of less than about -0.660, and preferably wherein the assay comprises determining methylation β-values for each CpG in the panel of one or more CpGs, no treatment is administered to the individual.

### Methods of monitoring

Disclosed but not claimed are methods of monitoring the presence, or risk of the presence or development of cancer in an individual.

*"Monitoring"* may refer to longitudinal assessment of an individual's cancer status, risk of harbouring cancer or risk of cancer development. This longitudinal assessment may be carried out according to any of the assays described herein. This longitudinal assessment may involve performance of any of the assays described herein to predict the presence or development of cancer in an individual at more than one time point over the course of an undetermined time window. The time window may be any period of time whilst the individual is still living. The time window may persist for the lifetime of the individual. The time window may persist until the individual's cancer status, risk of harbouring cancer or risk of cancer development falls below a certain level. The level may be a particular cancer index value.

Disclosed is a method of monitoring for the presence, absence or development of cancer, particularly endometrial and/or ovarian cancer, most preferably endometrial cancer, in an individual, the method comprising:
a. assessing the presence or absence of endometrial cancer in an individual or assessing cancer development in an individual to establish a cancer status for the individual by performing any one of the assays of the invention described herein at a first time point;
b. assessing the presence or absence of cancer in the individual or assessing cancer development in the individual to establish a cancer status for the individual by performing any one of the assays of the invention described herein at one or more further time points, preferably wherein the cancer status of the individual in steps a and b are assessed using the same assay; and
c. monitoring any change in the cancer status of the individual between time points.

Disclosed is a method of monitoring for the presence, absence or development of cancer, particularly endometrial and/or ovarian cancer, in an individual, the method comprising:
a. assessing the presence or absence of endometrial cancer in an individual or assessing cancer development in an individual to establish a cancer status for the individual by performing an assay at a first time point, comprising:
   1. providing a sample which has been taken from the individual, the sample comprising a population of DNA molecules;
   2. determining in the population of DNA molecules in the sample the methylation status of a panel of:
      a. one or more CpGs comprising the CpG identified in SEQ ID NO 67 wherein the CpG is identified at nucleotide positions 61 to 62 ;
   3. deriving a cancer index value based on the methylation status of the one or more CpGs in the panel; and
   4. assessing the presence, absence or development of cancer in the individual based on the cancer index value, wherein the assay is characterised as having an area under the curve (AUC) of 0.90 or more as determined by receiver operating characteristics (ROC);
b. assessing the presence or absence of cancer in the individual or assessing cancer development in the individual to establish a cancer status for the individual by performing the assay of steps a(1) to a(4) or by performing any one of the assays of the invention described herein at one or more further time points; and
c. monitoring any change in the cancer status of the individual between time points.

In any of the methods of monitoring described herein, the steps of assessing the presence, absence or development of cancer in an individual based on a cancer index value may involve the application of threshold values. Threshold values can provide an indication of an individual's cancer status, risk of having cancer or an individual's risk of cancer development. For example, cancer index values may indicate the presence or absence of cancer, or a high or low risk of harbouring or developing cancer. In any of the methods of monitoring, the step of predicting the presence, absence or development of cancer in an individual involves deriving a cancer index value.

Disclosed is a method of measuring methylation in a patient at multiple time points comprising (a) assessing the presence, absence or development of cancer in an individual by performing any one of the assays of the invention described herein at a first time point; (b) assessing the presence, absence or development of cancer in the individual by performing any one of the assays of the invention described herein at one or more further time points, and (c) detecting differential methylation status between (a) and (b).

In any of the methods of monitoring described herein, the individual may already harbour cancer, particularly endometrial and/or ovarian cancer, most preferably endometrial cancer. The individual may not have cancer. The individual may not harbour cancer. The individual may not harbour cancer but may harbour one or more genetic mutations that predispose the individual to an increased risk of cancer development e.g. the individual may have Lynch syndrome, and therefore harbour mutations in one or more mutations of the *MLH1, MSH2, MSH6, PMS2,* or *EPCAM* genes. Other mutations may include any mutations in the art that are considered to pre-dispose individuals to cancer. In any of the methods of monitoring described herein, the individual may not harbour cancer but may harbour one or more genetic mutations that pre-dispose the individual to cancer, and this individual may be subjected to any of the methods of monitoring described herein in order to determine their risk of having cancer or of developing cancer. For example, in any of the methods described herein, the individual does not harbour cancer and harbours one or more mutations that predispose the individual to an increased risk of developing cancer, particularly endometrial and/or ovarian cancer, and wherein one or more treatments are administered to the individual in accordance with any of the methods of treatment described herein as a method of prophylaxis. In any of the methods described herein, the individual does not harbour cancer and harbours one or more mutations that predispose the individual to an increased risk of developing cancer, and wherein one or more treatments are administered to the individual in accordance with any of the methods of treatment described herein as a method of prophylaxis, and wherein the one or more treatments administered to the individual comprises one or more doses of progestogens, particularly wherein the progestogens are delivered locally or systemically, Aspirin, Metformin, aromatase-inhibitors, and/or a weight-loss regimen.

In any of the methods of monitoring described herein, depending on the risk of the presence or development of cancer in the individual, one or more treatments are administered to the individual according to any one of the methods of treatment described herein, or wherein the cancer index value of the individual is less than about -0.660 no treatment is administered to the individual. Different treatments may be administered depending on the stratification of an individual on the basis of their cancer status, risk of harbouring cancer or on the basis of their risk of cancer development. The method may further comprise administration of one or more treatments according to the methods of treatment described herein.

The cancer index value may change between any two or more time points. For this reason, longitudinal monitoring of an individual's cancer index value could be of particular benefit to the assessment of, for example, cancer progression, prevention of recurrence of cancer, cancer treatment efficacy, or cancer efficacy.

In any of the methods of monitoring described herein, the one or more further time points may be any suitable time point. Preferably the one or more further time points may of suitable distance apart for sufficiently frequent screening in order to predict any particularly early onset cases of presence or development of cancer in an individual. Preferably the one or more further time points may be of suitable distance apart for assessing the efficacy of one or more treatments. Preferably the one or more further time points may be of suitable distance apart for predicting whether an individual remains free of cancer after a successful course of treatment. The one or more further time points may be about monthly, about two monthly, about three monthly, about four monthly, about five monthly, about six monthly, about seven monthly, about eight monthly, about nine monthly, about ten monthly, about eleven monthly, about yearly, about two yearly, or more than two yearly.

In any of the methods of monitoring described herein, changes may be made to the one or more treatments wherein a positive or negative responses to the one or more treatments are observed. Treatments may be changed in accordance with the methods of treatments described herein. Treatments may particularly be changed if the individual's cancer status or risk stratification, based on their cancer index value, changes.

In any of the methods of monitoring, the step of predicting the presence or development of cancer in an individual may involve the use of any one of the arrays described herein.

### Biological samples

The assays described herein are preferably performed on samples comprising epithelial cells, particularly tissue obtained from an anatomical site other than the endometrium or ovary. The sample may particularly be derived from the cervix, the vagina, the buccal area, blood and/or urine. The sample is preferably a cervical liquid-based cytology sample, and more preferably a cervical smear sample.

Preferably, any one of the assays described herein for assessing the presence, absence or development of cancer in an individual comprises providing a sample which has been taken from the individual. Preferably the individual is a woman.

In any of the assays described herein, the assay may or may not encompass the step of obtaining the sample from the individual. In assays which do not encompass the step of obtaining the sample from the individual, a sample which has previously been obtained from the individual is provided.

The sample may be provided directly from the individual for analysis or may be derived from stored material, e.g. frozen, preserved, fixed or cryopreserved material.

In any of the assays described herein, the sample may be self-collected or collected by any suitable medical professional.

Any of the assays described herein, the sample may comprise cells. The sample may comprise genetic material such as DNA and/or RNA.

Any of the assays described herein may involve providing a biological sample from the patient as the source of patient DNA for methylation analysis.

Any of the assays described herein may involve obtaining patient DNA from a biological sample which has previously been obtained from the patient.

Any of the assays described herein may involve obtaining a biological sample from the patient as the source of patient DNA for methylation analysis. The sample may be self-collected or collected by any suitable medical professional. Procedures for obtaining a biological sample include biopsy.

In any of the assays described herein, the sample may be obtained from a woman having abnormal vaginal bleeding, such as postmenopausal bleeding. Women with post-menopausal bleeding in particular should be assessed by any one of the assays described herein.

Methods for sample isolation and for the subsequent extraction and isolation of DNA from such cell or tissue samples in preparation for assessing DNA methylation, are well known to those skilled in the art. In the context of the assays or methods described herein, the entirety of a sample may be used, or alternatively cells may be concentrated or cell types may be fractionated in order to only apply subsets of one or more cell types to the present assays or methods. Any suitable methods of concentration or fractionation may be used.

In any one of the assays described and defined herein the sample from the individual, or the sample which has been taken from the individual, may derive from a tissue which is different from the tissue which harbours the tumour, if a tumour is present in the individual. Accordingly, in any one of the assays described and defined herein the sample from the individual, or the sample which has been taken from the individual, may not comprise nucleic acid, including DNA, which derives from the tumour, i.e. tumour-specific nucleic acid, including tumour-specific DNA. Thus, consistent with the data set out in the examples and the disclosures herein, methylation profiles derived from DNA molecules in the sample are used as surrogate markers for tumour-specific nucleic acid, including tumour-specific DNA, which exists at an anatomical site in the body of the individual which is remote from the anatomical site from which the sample is derived. A skilled person would able to identify the absence of tumour-specific DNA in any given population of sample-specific DNA molecules by routine means, such as determining the absence of known genetic mutations which are characteristic of the particular cancer, by e.g. sequence based screening. However, the performance of any one of the assays described and defined herein does not require any assessment to verify the absence of tumour-specific DNA in any given population of DNA molecules.

### Types of cancers

The methods described herein may be applied to any endometrial cancer. Preferably, the methods described herein may be applied to endometrial cancer and/or ovarian endometrial cancer, particularly endometrial cancer. The methods described herein are most preferably applied to endometrial cancer.

The cancer may be a primary cancer lesion. The cancer may be a secondary cancer lesion. The cancer may be a metastatic lesion.

In assays described herein, wherein the assays is for assessing the presence, absence or development of endometrial cancer, the endometrial cancer may preferably be endometriod cancer, uterine carcinosarcoma, squamous cell carcinoma, small cell carcinoma, transitional carcinoma, serous carcinoma, clear-cell carcinoma, mucinous adenocarcinoma, undifferentiated carcinoma, dedifferentiated carcinoma or serous adenocarcinoma. Any of the assays described herein may additionally, or alternatively, be for assessing the presence, absence or development of ovarian cancer.

In assays described herein, wherein the assays is for assessing the presence, absence or development of ovarian cancer cancer, the ovarian cancer cancer may preferably be serous carcinoma, mucinous carcinoma, endometrioid carcinoma, clear cell carcinoma, low malignant potential (LMP) tumor, borderline epithelial ovarian cancer, teratoma, dysgerminoma, endodermal sinus tumor, Choriocarcinoma, granulosa-theca tumor, Sertoli-Leydig tumor, granulosa cell tumor, small cell carcinoma of the ovary or primary peritoneal carcinoma.

### Arrays and kits

Disclosed but not claimed are claims and kits.

Disclosed are arrays capable of discriminating between methylated and non-methylated forms of CpGs as defined herein; the arrays may comprise oligonucleotide probes specific for methylated forms of CpGs as defined herein and oligonucleotide probes specific for non-methylated forms of CpGs as defined herein.

In any of the arrays described herein, the array may comprise oligonucleotide probes specific for a methylated form of each CpG in a CpG panel and oligonucleotide probes specific for a non-methylated form of each CpG in the panel; wherein the panel consists of at least 50 CpGs selected from the CpGs identified in SEQ ID NOs 1 to 808.

The panel may consist of at least 50 CpGs selected from the CpGs identified in SEQ ID NOs 1 to 808, preferably wherein the CpGs comprise the CpGs identified in SEQ ID NOs 1 to 50.

The panel may consist of at least 100 CpGs selected from the CpGs identified in SEQ ID NOs 1 to 808, preferably wherein the CpGs comprise the CpGs identified in SEQ ID NOs 1 to 100.

The panel may consist of at least 150 CpGs selected from the CpGs identified in SEQ ID NOs 1 to 808, preferably wherein the CpGs comprise the CpGs identified in SEQ ID NOs 1 to 150.

The panel may consist of at least 200 CpGs selected from the CpGs identified in SEQ ID NOs 1 to 808, preferably wherein the CpGs comprise the CpGs identified in SEQ ID NOs 1 to 200.

The panel may consist of at least 250 CpGs selected from the CpGs identified in SEQ ID NOs 1 to 808, preferably wherein the CpGs comprise the CpGs identified in SEQ ID NOs 1 to 250.

The panel may consist of at least 300 CpGs selected from the CpGs identified in SEQ ID NOs 1 to 808, preferably wherein the CpGs comprise the CpGs identified in SEQ ID NOs 1 to 300.

The panel may consist of at least 350 CpGs selected from the CpGs identified in SEQ ID NOs 1 to 808, preferably wherein the CpGs comprise the CpGs identified in SEQ ID NOs 1 to 350.

The panel may consist of at least 400 CpGs selected from the CpGs identified in SEQ ID NOs 1 to 808, preferably wherein the CpGs comprise the CpGs identified in SEQ ID NOs 1 to 400.

The panel may consist of at least 450 CpGs selected from the CpGs identified in SEQ ID NOs 1 to 808, preferably wherein the CpGs comprise the CpGs identified in SEQ ID NOs 1 to 450.

The panel may consist of at least 500 CpGs selected from the CpGs identified in SEQ ID NOs 1 to 808, preferably wherein the CpGs are the CpGs identified in SEQ ID NOs 1 to 500.

The panel may consist of all CpGs identified in SEQ ID NOs 1 to 808.

In some embodiments the array is not an Infinium MethylationEPIC BeadChip array or an Illumina Infinium HumanMethylation450 BeadChip array.

Separately or additionally, in some embodiments the number of CpG-specific oligonucleotide probes of the array is 482,000 or less, 480,000 or less, 450,000 or less, 440,000 or less, 430,000 or less, 420,000 or less, 410,000 or less, or 400,000 or less, 375,000 or less, 350,000 or less, 325,000 or less, 300,000 or less, 275,000 or less, 250,000 or less, 225,000 or less, 200,000 or less, 175,000 or less, 150,000 or less, 125,000 or less, 100,000 or less, 75,000 or less, 50,000 or less, 45,000 or less, 40,000 or less, 35,000 or less, 30,000 or less, 25,000 or less, 20,000 or less, 15,000 or less, 10,000 or less, 5,000 or less, 4,000 or less, 3,000 or less or 2,000 or less.

The CpG panel may comprise any set of CpGs defined in the assays of the invention described herein.

The arrays may comprise one or more oligonucleotides comprising any set of CpGs defined in the assays of the invention, wherein the one or more oligonucleotides are hybridized to corresponding oligonucleotide probes of the array.

Disclosed is a process for making a hybridized array described herein, comprising contacting an array with a group of oligonucleotides comprising any set of CpGs defined in the assays of the invention.

Any of the arrays as defined herein may be comprised in a kit. The kit may comprise any array as defined herein together with instructions for use.

Disclosed is the use of any of the arrays as defined herein in any of the assays for determining the methylation status of CpGs for the purposes of predicting the presence or development of cancer in an individual.

The following Examples serve to illustrate but not to limit the invention.

### Examples

In the Examples described herein, WID-EC-Index is a cancer index value wherein the index value has been determined by assaying in a population of DNA molecules derived from a given sample from an individual the methylation status of a panel of CpGs selected from the CpGs defined by SEQ ID NOs: 1 to 500.

In some instances within the Examples, all CpGs defined by SEQ ID NOs: 1 to 500 have been included in the panel which has been assayed to obtain a cancer index value. In addition, specific sub-selections of CpGs from among the 500 CpGs defined by SEQ ID NOs: 1 to 500 have been included in the panel which has been assayed to obtain a cancer index value. In these instances, the cancer index value's ability to discriminate between cancer positive and cancer negative women is described, wherein discriminatory ability of the index is characterised by AUC and received operating characteristics.

The inventors, along with other scientists, have shown in the past that DNA methylation in vaginal fluid or cervical smears may be able to identify women with endometrial cancer. All of these studies were relatively small and did not validate the findings in a large data set. In addition, none of these studies focused specifically on high risk endometrial cancers and, most importantly, none assessed cohort-based samples from women prior to the diagnosis of the disease.

DNA methylation changes can be used as both, (i) a surrogate readout for factors which drive cancer formation and thereby predict cancer risk and (ii) a diagnostic tool which indicates the presence of a cancer. The bulk of the DNA extracted from a cervical smear sample contains: (i) DNA from normal cells (e.g. hormone-sensitive cervical epithelial cells whose DNA methylation is likely to capture long-term effects triggered by unopposed estrogen - the core risk factor for endometrial cancer) that provides the *cancer risk component* of the signature and (ii) DNA from cell-detritus draining from the endometrial cavity, the quantity of which is likely increased with respect to endometrial cancers, which provides the *diagnostic component* of the signature.

Here, the inventors have developed and validated a DNA methylation signature in cervical smear samples which is capable of both diagnosing and predicting the risk of developing endometrial cancer.

### Materials and Methods

### Study design and epidemiological data acquisition

The study was conducted as part of a multi-centre study involving several recruitment sites in 5 European countries (i.e. the UK, Czech Republic, Italy, Norway and Germany). Participants were aged >18 years. Prior to taking part, each prospective study volunteer was given a Participant Information Sheet as well as a Consent Form and the rationale for the study was explained. Additional resources, including an explanatory video and further online resources, were also made available. Women diagnosed with breast or ovarian cancer (case) or a non-malignant benign gynaecological condition (control) were approached during outpatient hospital clinics, while women recruited as *BRCA* mutation carriers or healthy volunteers from the general population (control) were approached via outreach campaigns, public engagement, and as part of cervical screening programmes. After signing an informed consent, participants completed an epidemiological questionnaire as well as a feedback form after their participation. The study itself is a sub-study of the FORECEE (4C) Programme, which has ethical approval from the UK Health Research Authority (REC 14/LO/1633) and other contributing centres.

The epidemiological survey was administered via the Qualtrics application on dedicated iPads. The survey contained questions relating to health habits, relevant risk factors, and also made enquiries as to historical health habits, as well as obtaining a thorough medical and obstetric history. Cervical samples were collected at appropriate clinical venues by trained staff and the cervical smears were carried out by a small group of research midwives or physicians with a view to establishing standard practice. Buccal samples were collected using Copan 4N6FLOQ Swabs, Thermofisher Scientific.

Biological samples were given an anonymous Participant ID Number which was assigned to the person's name in a securely stored link file. Following sample taking, an email survey was sent to each participant, enabling them to feedback with respect to the recruitment process. Women with a current diagnosis of grade 3 and/or stage IB or above malignant endometrial cancer and recruited prior to receiving any systemic treatment (chemo- or antihormonal or Herceptin, etc.) or surgery or radiotherapy were eligible as endometrial cancer cases. For the FORECEE Discovery set controls were initially matched one-to-one with cases based on menopausal status, age (5 year age ranges where possible), and recruitment centre/country. However, due to an imbalance in recruitment of cases and controls at some centres, a number of cases were matched on age and menopausal status alone. Cancer histological data was collected post-recruitment either by clinicians directly involved in the diagnosis/treatment of the cancer cases or by a nominated data manager with access to the in-house hospital systems.

### Cervical Smear Sample Collection

Cervical smears were taken at collaborating hospitals and recruitment centres using the ThinPrep system (Hologic Inc., cat #70098-002). Cervical cells were sampled from the cervix using a cervix brush (Rovers Medical Devices, cat #70671-001) which was rotated 5 times through 360 degrees whilst in contact with the cervix to maximise cell sampling. The brush was removed from the vagina and immersed in a ThinPrep vial containing Preserve-cyt fluid and then pushed against the bottom of the vial 10 times to facilitate release of the cells from the brush into the solution. The sample vial was sealed and stored locally at room temperature. Buccal cells were collected using two Copan 4N6FLOQ Buccal Swabs (Copan Medical Diagnostics, cat #4504C) by firmly brushing the swab head 5-6 times against the buccal mucosa of each cheek. The swabs were re-capped and left to dry out at room temperature within the sampling tube which contains a drying desiccant. 2.5 ml of venous whole blood was collected in PAX gene blood DNA tubes (BD Biosciences #761165) and stored locally at 4°C. All samples were shipped to UCL at ambient temperature.

The vaginal-swab endometrial cancer set (55 controls and 8 endometrial cancers) consists of swabs from women presenting at UCLH for postmenopausal bleeding.

### Self-collected samples

Two endometrial cancer cases and three controls matched by age provided a cervico-vaginal sample using the self-collection device Evalyn Brush at the outpatient clinic after a brief explanation by medical staff. The Evalyn Brush was rotated 5 times through 360 degrees once introduced in the vagina as indicated per fabricant protocol to maximize cell sampling. Samples were stored using ThinPrep.

### Sample processing and DNA extraction

When preparing for sample storage in the laboratory, cervical smear samples were poured into 50 ml Falcon tubes and left to sediment at room temperature for 2 hours. 1 mL wide bore tips were then used to transfer the enriched cellular sediment into a 2 mL vial. The cervical sediments were washed twice with PBS, lysed, and stored temporarily at - 20°C ahead of extraction.

### DNA methylation array analysis

Cervical DNA was normalised to 25 ng/ul and 500 ng total DNA was bisulfite modified using the EZ-96 DNA Methylation-Lightning kit (Zymo Research Corp, cat #D5047) on the Hamilton Star Liquid handling platform. 8 ul of modified DNA was subjected to methylation analysis on the Illumina InfiniumMethylation EPIC BeadChip (Illumina, CA, USA) at UCL Genomics according to the manufacturer's standard protocol.

### Methylation analysis

All methylation microarray data were processed through the same standardised pipeline. Raw data was loaded using the R package minfi. Any samples with median methylated and unmethylated intensities < 9.5 were removed. Any probes with a detection p-value >0.01 were regarded as failed. Any samples with >10% failed probes, and any probes with >10% failure rate were removed from the dataset. Beta values from failed probes (approximately 0.001% of the dataset) were imputed using the impute.knn function as part of the impute R package.

Non-CpG probes (2,932), SNP-related probes as identified by Zhou et.al. (82,108), and chrY probes were removed from the dataset. An additional 6,102 previously identified probes that followed a trimodal methylation pattern characteristic of an underlying SNP were removed.

Background intensity correction and dye bias correction was performed using the minfi single sample preprocessNoob function. Probe bias correction was performed using the beta mixture quantile normalisation (BMIQ) algorithm.

The fraction of immune cell contamination, and the relative proportions of different immune cell subtypes in each sample, were estimated using the EpiDISH algorithm using the epithelial, fibroblast and immune cell reference dataset. The top 1,000 most variable probes (ranked by standard deviation) were used in a principal component analysis. Statistical tests were performed in order to identify any anomalous associations between plate, sentrix position, date of array processing, date of DNA creation, study centre, immune contamination fraction, age, type (case versus control) and the top ten principal components. Finally, two-thirds of the discovery dataset was randomly selected for use as the training dataset and the remaining third was allocated to the internal validation dataset. This split was carried out once, and the same training and validation sets were used in all subsequent analyses.

### Methylight reaction design

Two ranked lists of CpGs were generated. The first was ranked according to the epithelial delta-beta estimates (the estimated difference in methylation between cases and controls in cervical epithelial cells). The second was ranked according to p-values (derived from a linear model comparing cases to controls after adjustment for immune cell proportion and age). For each CpG we identified any contiguous CpGs within +/-500bp. The inventors computed and plotted the mean methylation in cases and controls across all CpGs within this 1000bp region. Upon visual inspection of the top 50 CpGs (in both ranked lists) we identified a number of candidate regions according to the following criteria:
1. Regions where the methylation level was close to zero in healthy controls.
2. Regions where the variability within controls was relatively low.
3. Regions where the methylation level was elevated in cases.
4. Regions that included two or more CpG sites

Cervical smear samples from 20 healthy controls (age range 34 to 75, 11 post-menopause and 9 pre-menopause) and 20 women with endometrial cancer (age range from 34 to 75, 11 post-menopause and 9 pre-menopause) were analysed

### Statistical analyses for classifier development

Contamination by immune cells presented a challenge with respect to the identification of differentially methylated positions (DMPs) as differential methylation that occurred solely in epithelial cells was diminished in samples with high IC and vice versa. In order to overcome this, the inventors linearly regressed the beta values on IC for each CpG site, the linear models being fitted to cases and controls separately. The intercept points at IC = 0 were used as estimates of mean beta values in cases and controls in a pure epithelial cell population. The difference between these intercept points provided a delta-beta estimate in epithelial cells. The difference between intercept points at IC = 1 provided immune cell delta-beta estimates. A list of ranked CpGs was produced according to delta-beta estimates in epithelial cells.

The R package glmnet was used to train classifiers with a mixing parameter value of alpha = 0 (ridge penalty) and alpha = 1 (lasso penalty) with binomial response type. Data from the training dataset were used to fit the classifiers. A ranked list of CpGs was generated by taking the CpG with the largest epithelial delta-beta, followed by the CpG with the largest immune delta-beta, followed by the next largest epithelial delta-beta and so forth (any duplicates were removed). The top n CpGs from the list of ranked CpGs were used as inputs to the classifier. Ten-fold cross-validation was used inside the training set by the cv.glmnet function in order to determine the optimal value of the regularisation parameter lambda. The AUC was used as a metric of classifier performance which was evaluated on the internal validation dataset as a function of n, the number of CpGs used as inputs during training. The maximum value of n was 30,000.

The optimal classifier was selected based on the highest AUC obtained in the internal validation dataset. Once the optimal number of inputs was determined, the training and internal validation datasets were combined and the classifier was refitted using the entire discovery dataset with alpha and lambda fixed to their optimal values. This finalised classifier was then applied to the external validation dataset and the corresponding AUC was computed.

Denoting the top n CpGs as *x*₁, ... *, xₙ* and the regression coefficients from the trained classifier as *w*₁, ... , *wₙ* then $WID - EC - index = {\sum }_{i = 1}^{n} \left({w}_{i}{x}_{i}-μ\right) / σ$ where *µ* and *σ* are defined as the mean and standard deviation of the quantity ${\sum }_{i = 1}^{n} {w}_{i} {x}_{i}$ in the training dataset (that is, the index is scaled to have zero mean and unit standard deviation in the training dataset).

### Data availability

DNAme data has been deposited in the European Genome-phenome Archive (EGA), which is hosted by the EBI and the CRG, under accession number EGASXXXXXXXXXXX.

### Example:

The inventors performed an epigenome-wide DNAme analysis in cervical smear samples from women who were subsequently diagnosed with endometrial cancer, and in matched controls, and established the WID-EC-index (Women's risk IDentification for Endometrial Cancer index) which the inventors further validated in an independent set of cervical samples.

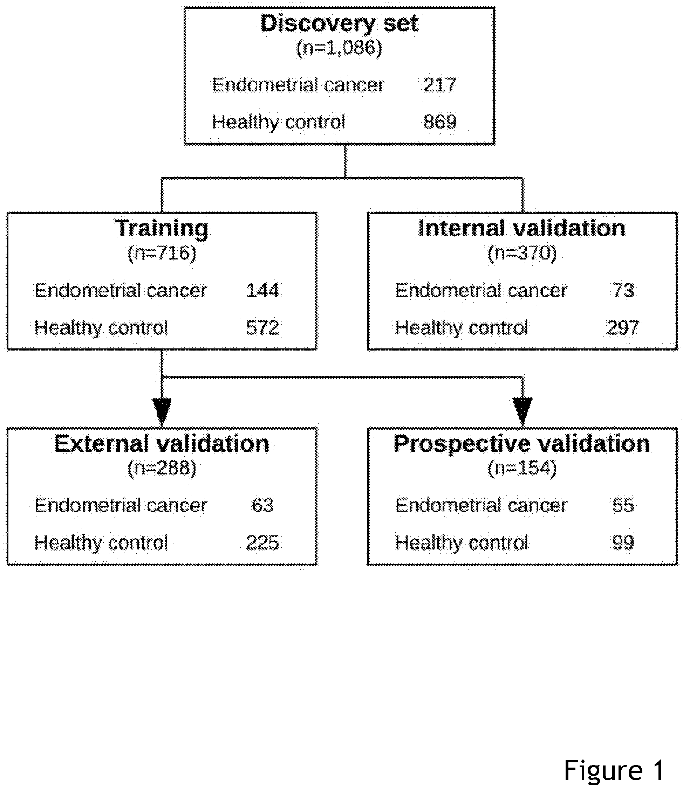

For the Discovery Set (Figure 1), the inventors collected samples from 217 women at the time of endometrial cancer diagnosis with at least one poor prognostic feature (i.e. grade 3 or clear cell or serous histology or >50% myometrial invasion) from 15 European centres (either at the time before an endometrial biopsy was taken for diagnostic purpose or before commencing a hysterectomy), and 869 women without a cancer (593 from the general population and 276 from women attending hospital for benign women-specific conditions) (Table 8; samples from a greater proportion of younger women were deliberately used in the Discovery set in order to develop a risk predictor applicable also to younger women; the external validation set was composed of age-matched cases and controls). Epigenome-wide DNAme was analysed using an Illumina Infinium EPIC bead chip array which encompasses over 850,000 CpG sites.

### Sample heterogeneity and differential methylation

The inventors assessed the number of CpGs which were significantly differentially methylated between cancer cases and controls (Figure 6A); after Bonferonni multiple test adjustment, 116,658 CpGs showed significant differential methylation. Previously the inventors found that methylation differences may vary due to immune cell type composition in cases compared to controls. Hence the inventors assessed the level of cell type heterogeneity in each cervical smear sample using HEpiDISH, an algorithm that infers the relative proportion of epithelial cells, fibroblasts, and seven subtypes of immune cells in each sample. The cell-type distributions were broadly similar between cancer cases and controls (although there were small but significant differences in some immune cell subtypes; Figure 6B).

### Development of discriminatory index

In order to derive a diagnostic methylation signature, termed the WID-EC-index, the inventors used ridge and lasso regression to classify individuals as cases or controls. Classifiers were trained on two thirds of the discovery dataset (572 cancer-free controls, 144 endometrial cancer cases) and the remaining one third was used as an internal validation set (297 controls, 73 cases) with the intention of evaluating their performance as a function of the number of CpGs used to construct the index. The area under the receiver operator characteristic curve (AUC) was used as a measure of predictive performance. CpGs were ranked according to their epithelial delta-beta.

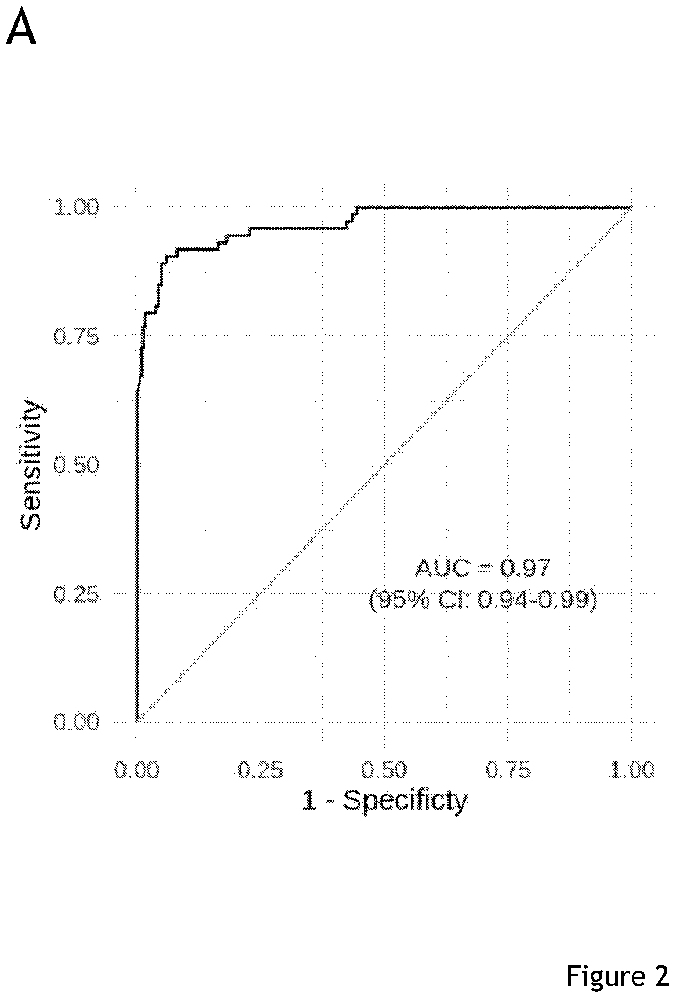

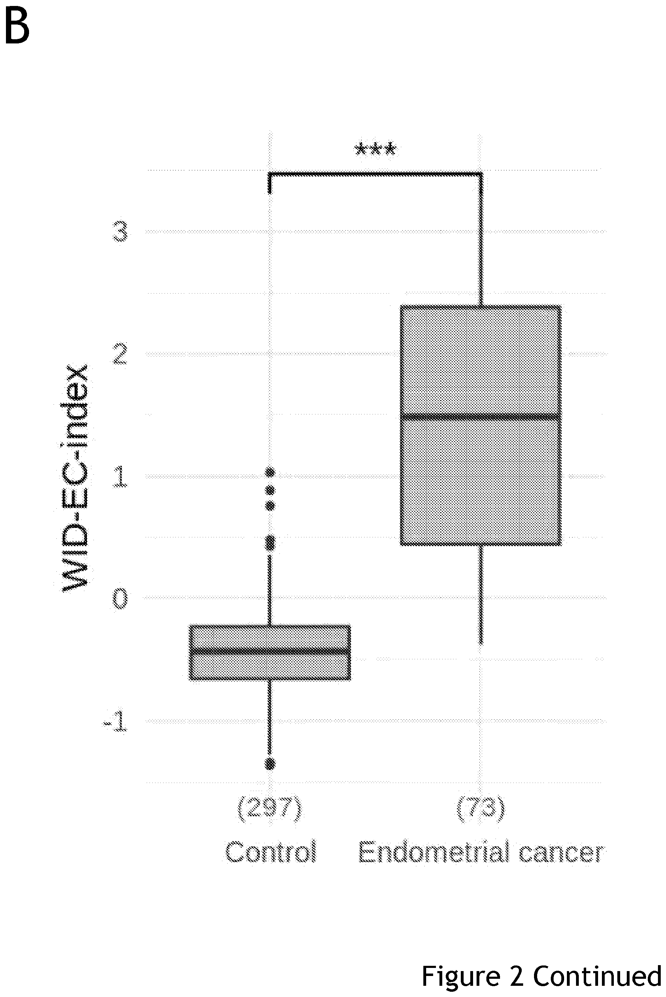

Predictive performance was evaluated as a function of the number of CpGs used to train the classifier using the internal validation dataset and optimal performance of 0.97 (Figure 2; 95% CI: 0.94-0.99) was achieved using 500 CpGs with ridge regression (Figure 6C). Discriminatory performance was broadly independent of immune cell proportion (Figure 6D). In samples with an immune cell proportion ≤0.5 the AUC was 0.98 (95% CI: 0.97-1.00), and in those with a proportion >0.5 the AUC was 0.95 (95% CI: 0.91-0.99). The WID-EC-index was slightly associated with IC fraction in controls (linear regression coefficients of 0.29, p<0.001), with a negative trend in cancer cases (linear regression coefficient of -0.28, p=0.6).

### External validation

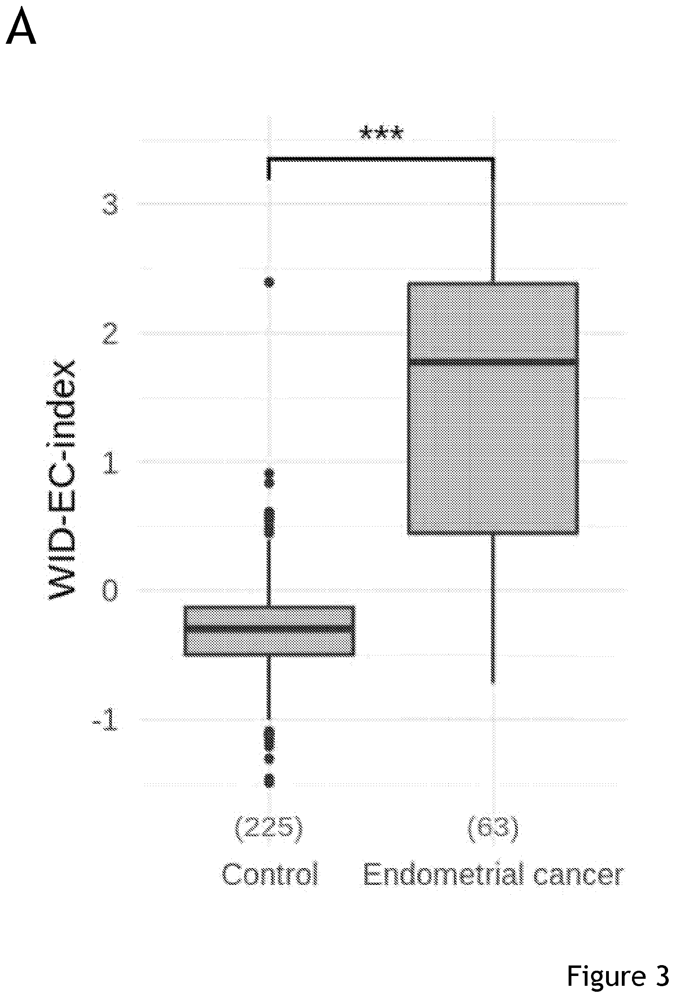

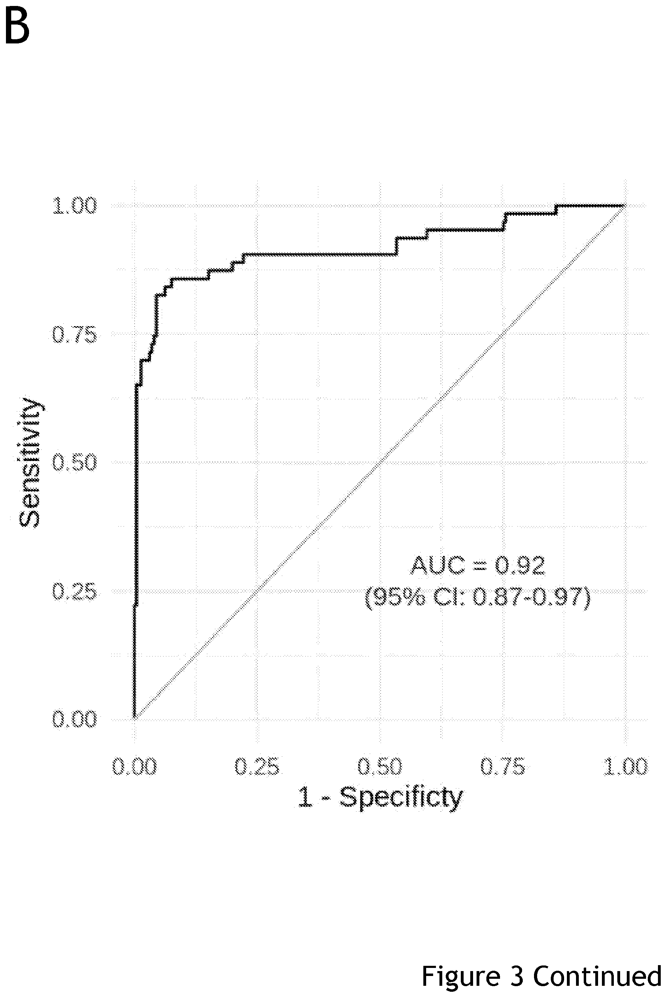

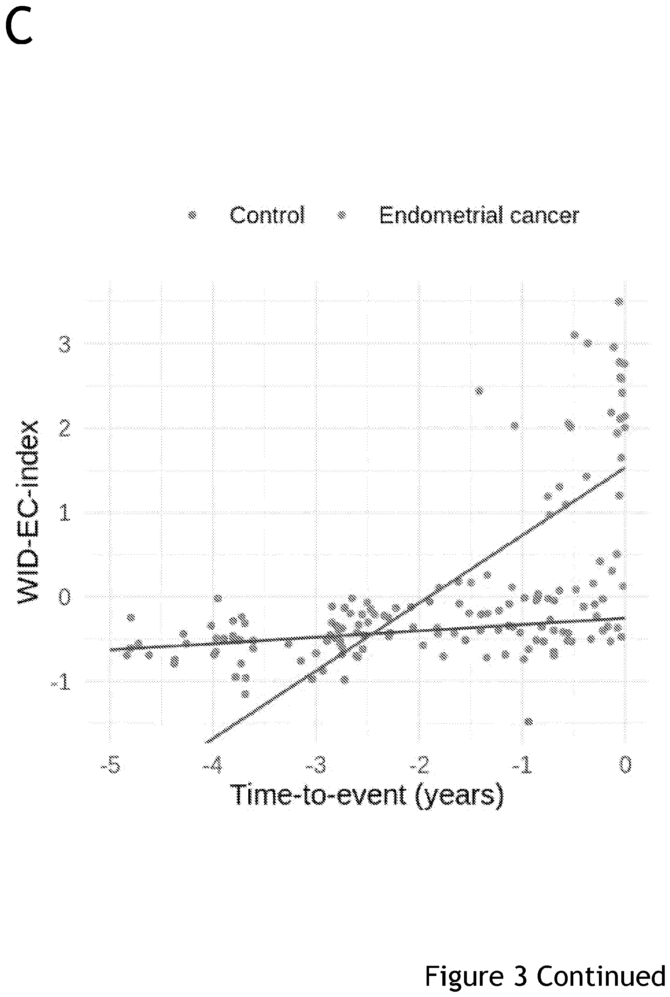

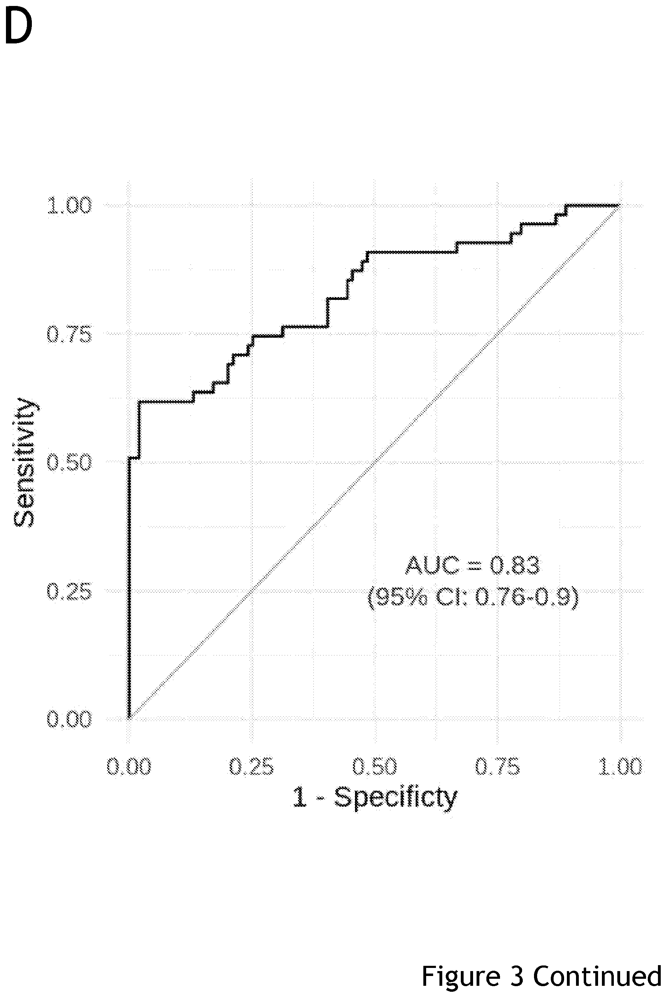

A separate independent external validation dataset consisting of 63 endometrial cancer cases and 225 controls was used to validate the index performance (Figure 1). The WID-EC-index was computed for each woman (Figure 3A) resulting in an AUC of 0.92 (95% CI: 0.87-0.97) and 0.93 (95% CI: 0.88-0.99) and 0.89 (95% CI: 0.80-0.98) for IC ≤0.5 and >0.5, respectively. Odds ratios corresponding to quartiles defined on the internal validation set are displayed for the external validation set in Table 7.

In order to assess whether the WID-EC-index is able to predict endometrial cancer risk, the inventors analysed samples which have been collected as part of the routine cervical screening program in Stockholm from healthy women who subsequently developed endometrial cancer (cases; n=55; average time between sample collection and cancer diagnosis was 304 days) and from women who did not (controls, n=99). The inventors observed an AUC of 0.83 (95% CI: 0.76-0.90) overall with 0.82 (95% CI: 0.72-0.93) and 0.85 (95% CI: 0.75-0.95) for IC ≤0.5 and >0.5, respectively.

### Sample degradation in the prospective set

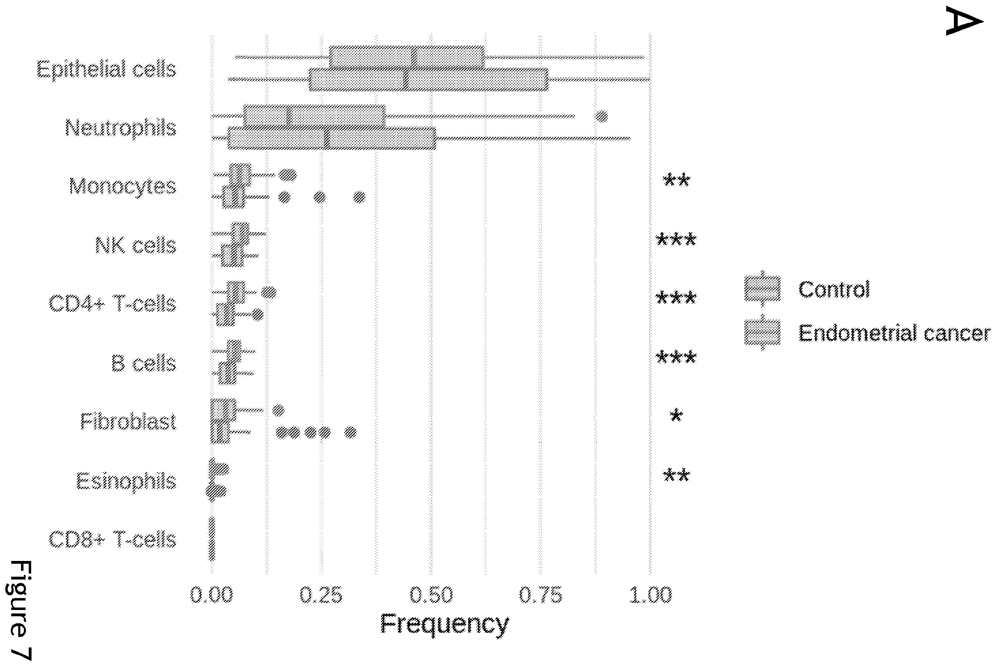

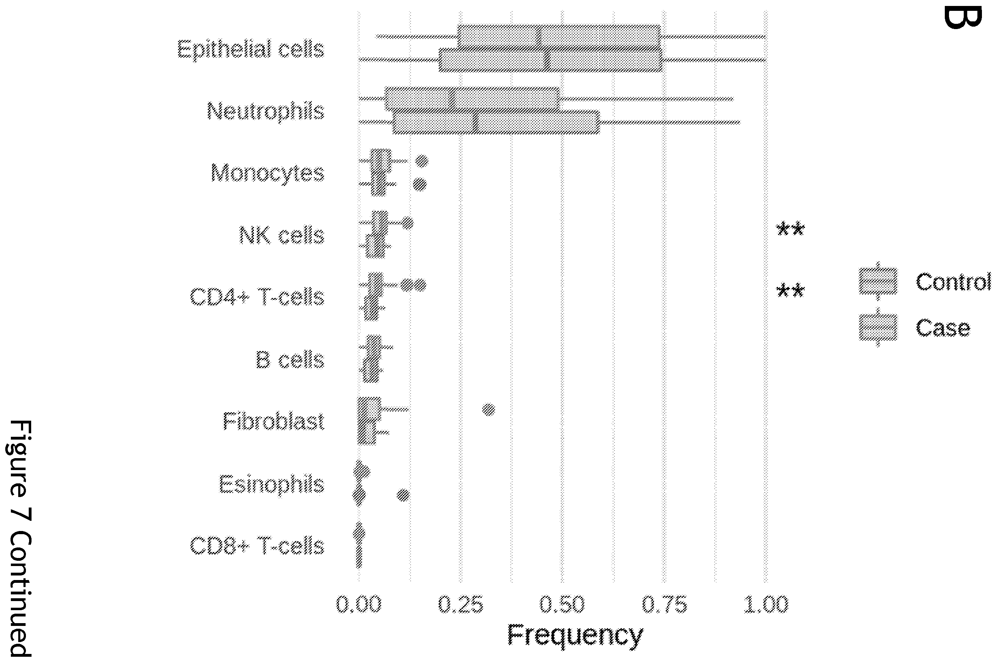

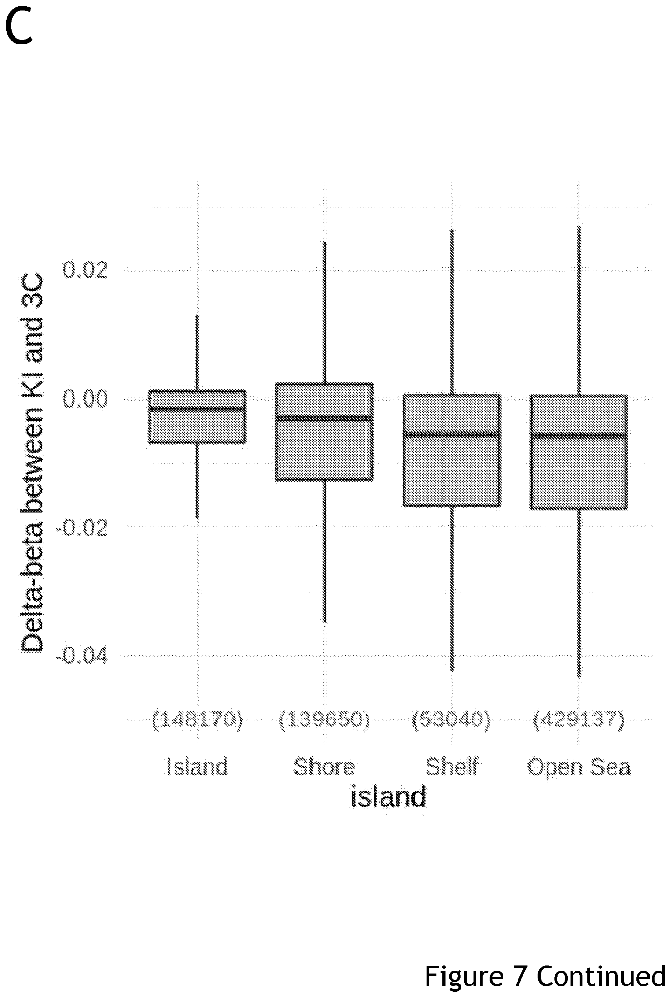

The cell-type composition of these three datasets was broadly similar to the discovery dataset used to develop the index and did not show any significant differences between cases and controls (Figure 7A,B). However, the inventors observed a systematic loss of methylation in cancer free controls from the prospective set in comparison to the discovery set, a loss that predominantly occurred at CpG-sparse "Open Sea" and "Shore" regions of the genome (Figure 7C). The inventors hypothesise that these changes may be due to storage related degradation due to long term biobank storage (the median storage time was 95 days and ranged from 15 to 1,001 days).

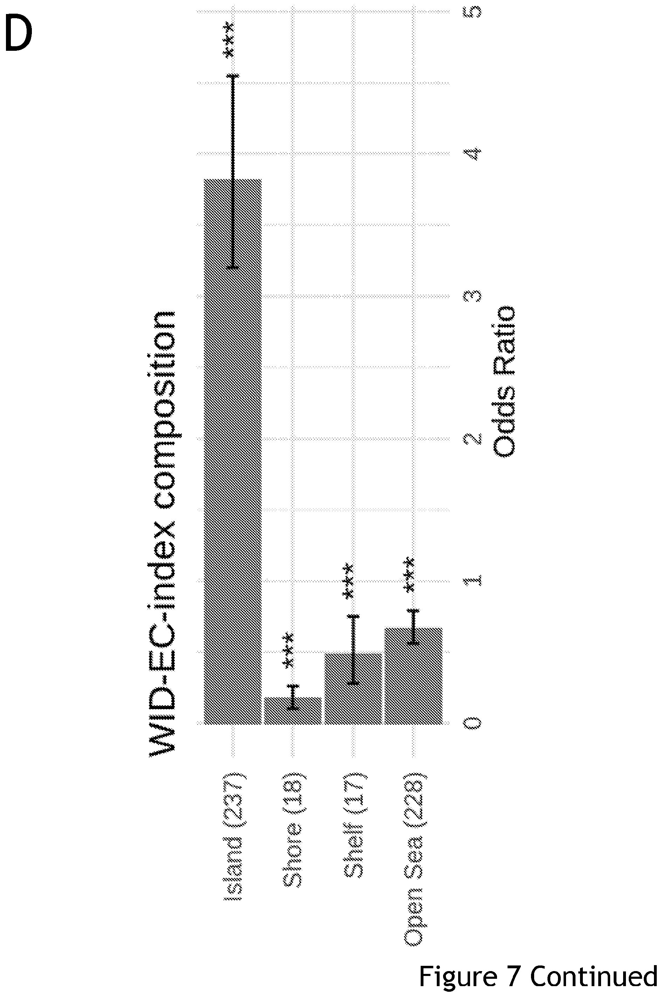

The WID-EC-index is highly enriched for CpG-dense CpG Islands and depleted for Open Sea CpG regions relative to the overall illumina EPIC array (Figure 7D). The inventors decomposed the index into two subcomponents consisting of only CpGs from Islands (237 CpGs) and Open Sea regions (228 CpGs). The CpG island subcomponent offered superior performance in the prospective samples (AUC: 0.87, 95% CI: 0.81-0.94; Figure 7E) whereas the Open Sea subcomponent suffered from a substantial corruption of the discriminatory signal (Figure 7F), suggesting that the overall performance of WID-EC-index was diminished by the degradation of Open Sea CpG regions. In the external validation set the Island and Open Sea components generated AUC values of 0.92 and 0.53 respectively, indicating that the discriminatory signal is largely driven by the CpG island component (Figure 7G,H).

### Association with epidemiological, clinical and technical factors

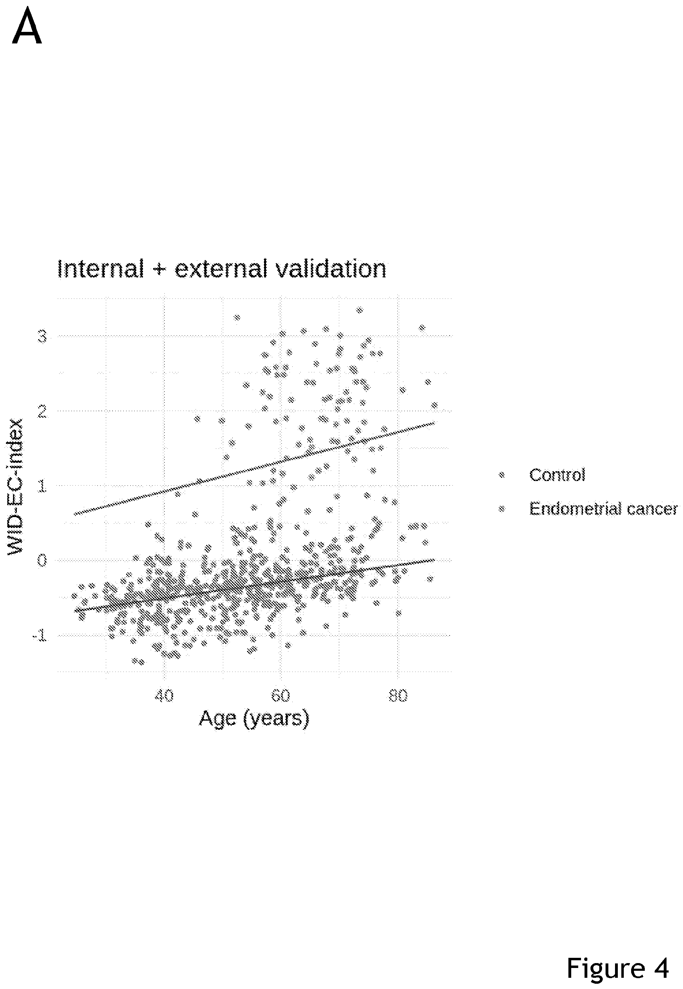

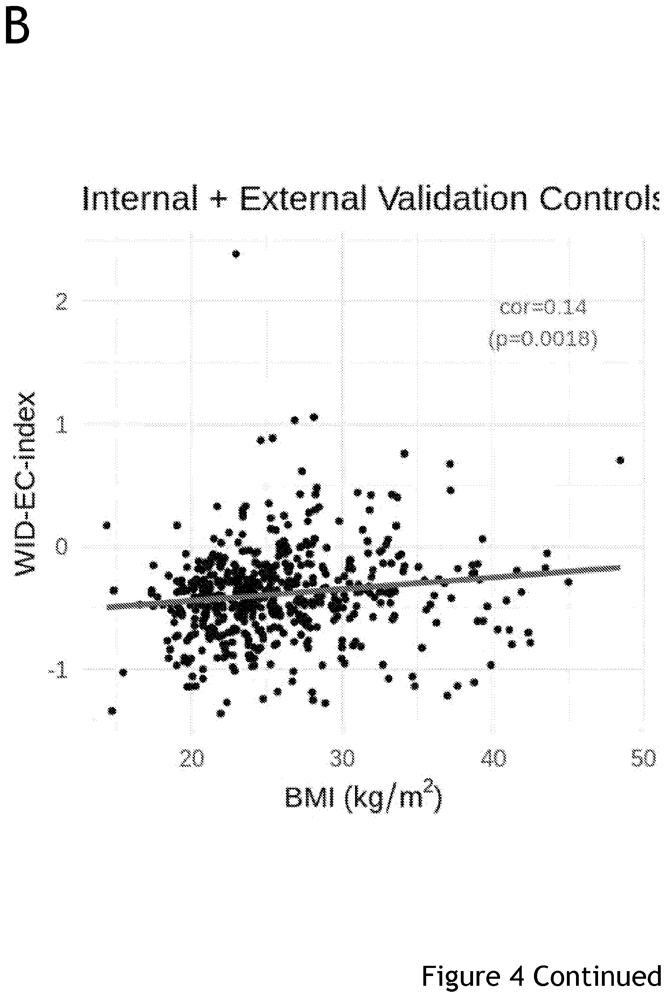

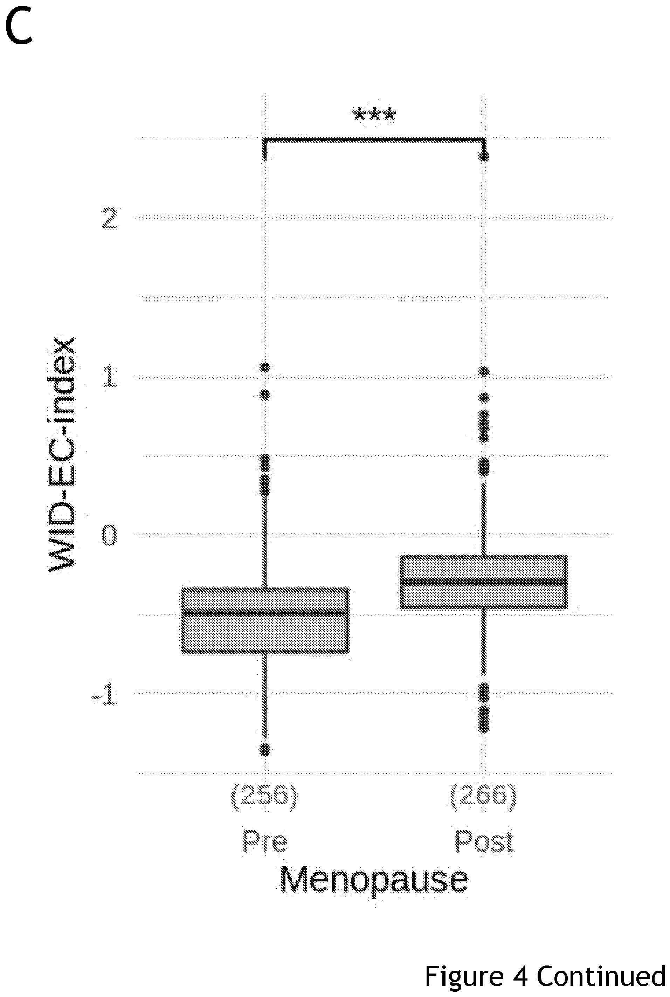

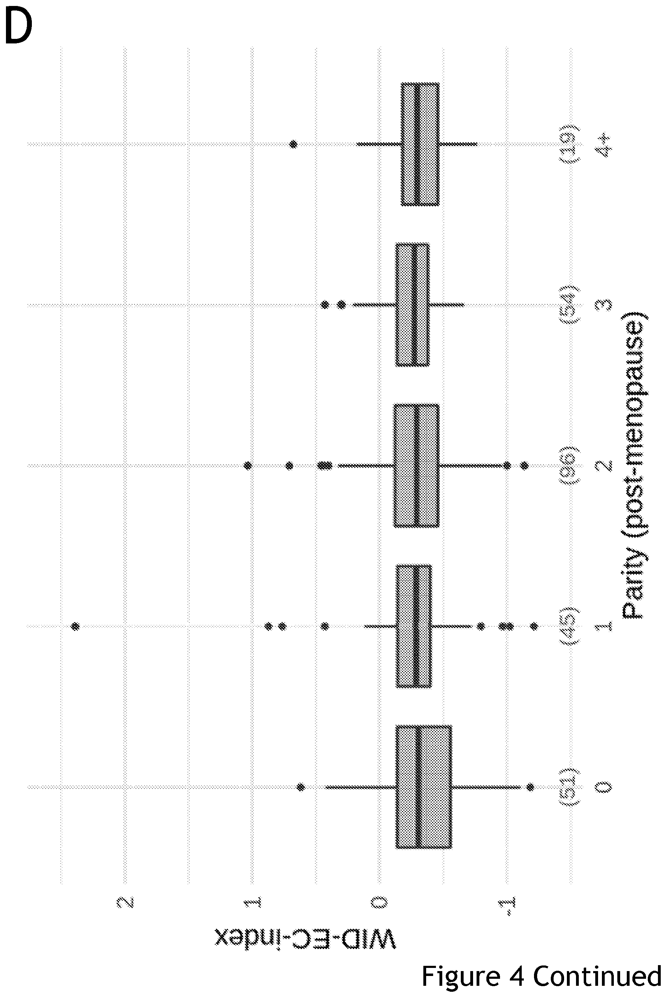

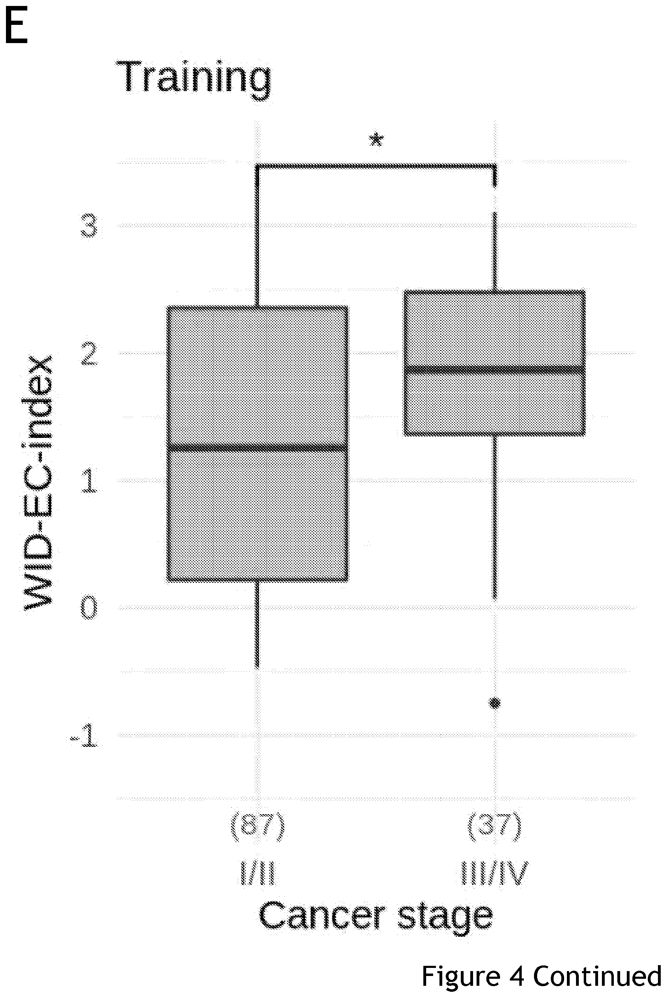

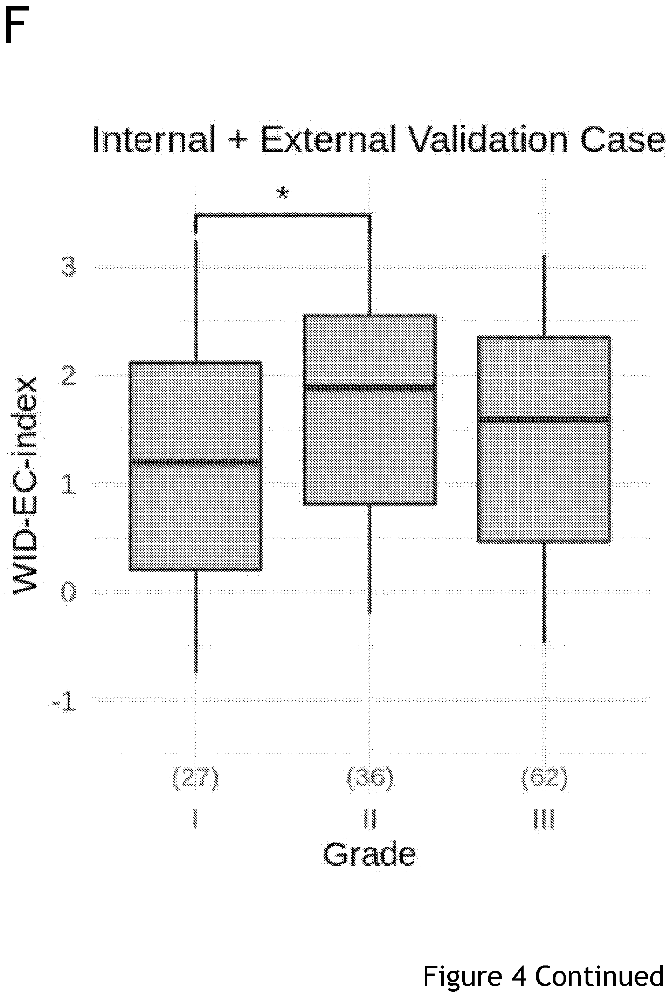

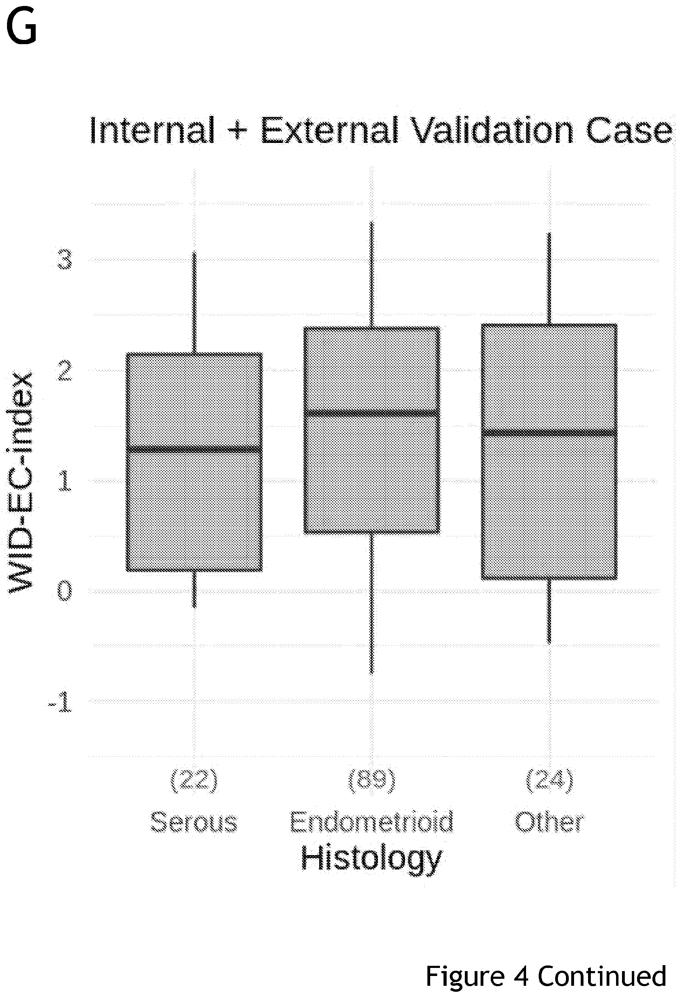

The inventors investigated the relationship between the WID-EC-index and various epidemiological and clinical variables in the internal and external validation sets. A statistically significant association was found between the WID-EC-index and age in controls (correlation coefficient=0.37, p<10⁻¹⁶; Figure 4A). A similar trend was observed in cancer cases (correlation coefficient=0.16, p=0.06). A significant correlation of 0.14 (p<0.01) was observed between the index and BMI in controls. The WID-EC-index was significantly elevated in post-menopausal controls (Figure 4C), reflecting the association with age. No significant association was observed with parity in post-menopausal controls (Figure 4D). The index was significantly elevated in stage III/IV cancers (Figure 4E) and in grade II cancers compared to grade I cancers (Figure 4F). No association was observed with histological subtypes (Figure 4G).

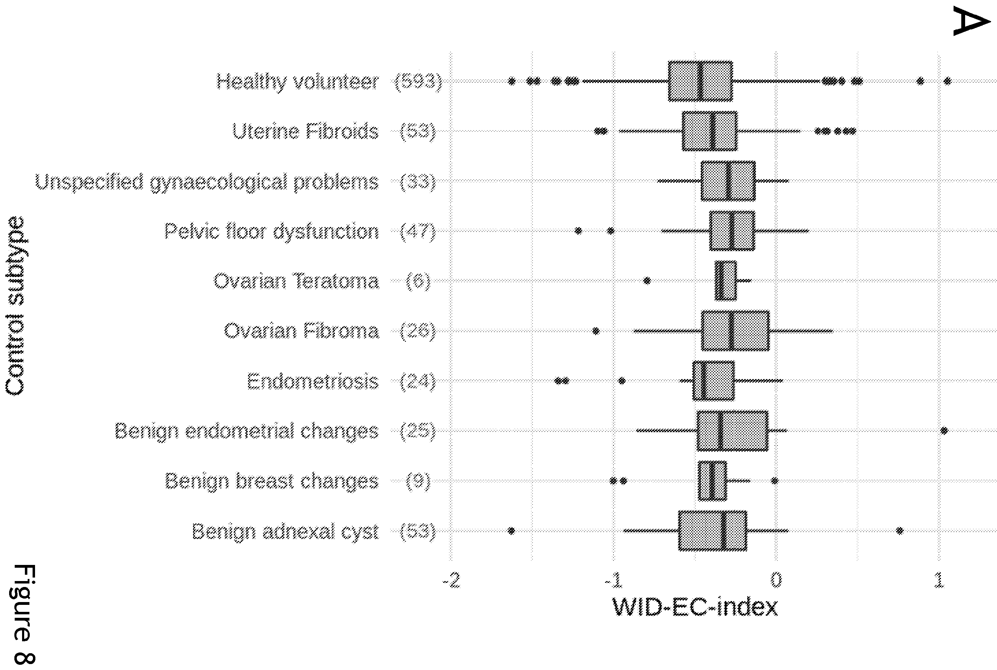

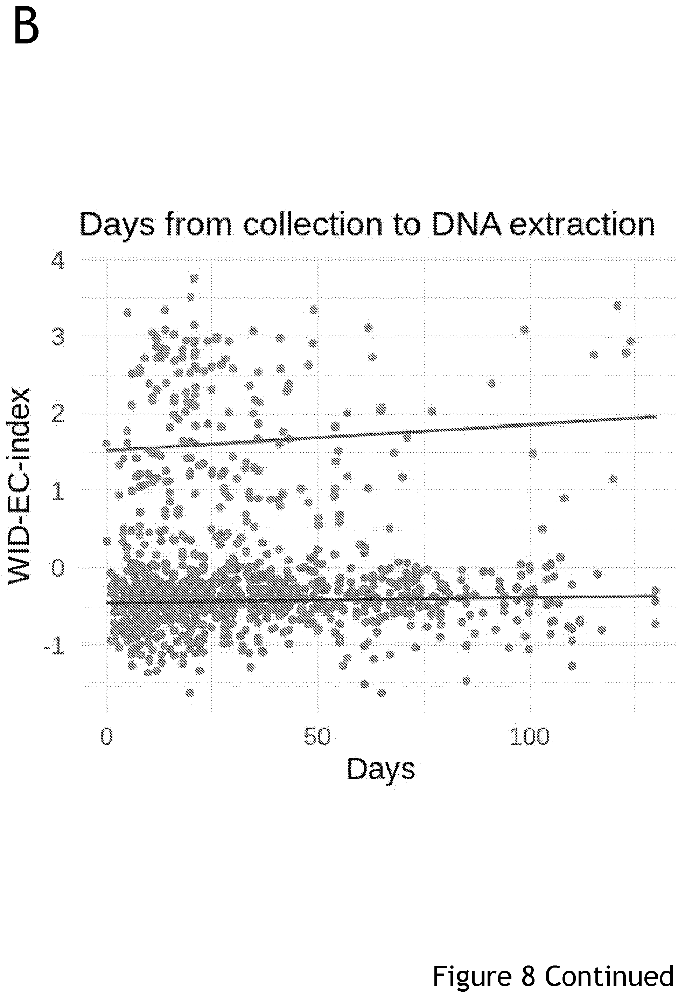

The inventors investigated whether there was any association between the WID-EC-index and various technical parameters including date of sample processing, plate number (samples were processed on 96 sample plates) and sentrix position but no significant associations were found. The inventors compared the 593 control samples from healthy volunteers to 276 control samples taken from women presenting with benign women-specific conditions but did not find any significant differences (Figure 8A). The inventors also observed no significant dependence on the time from sample collection to DNA extraction (Figure 8B).

### Inferred proportion of tumour DNA

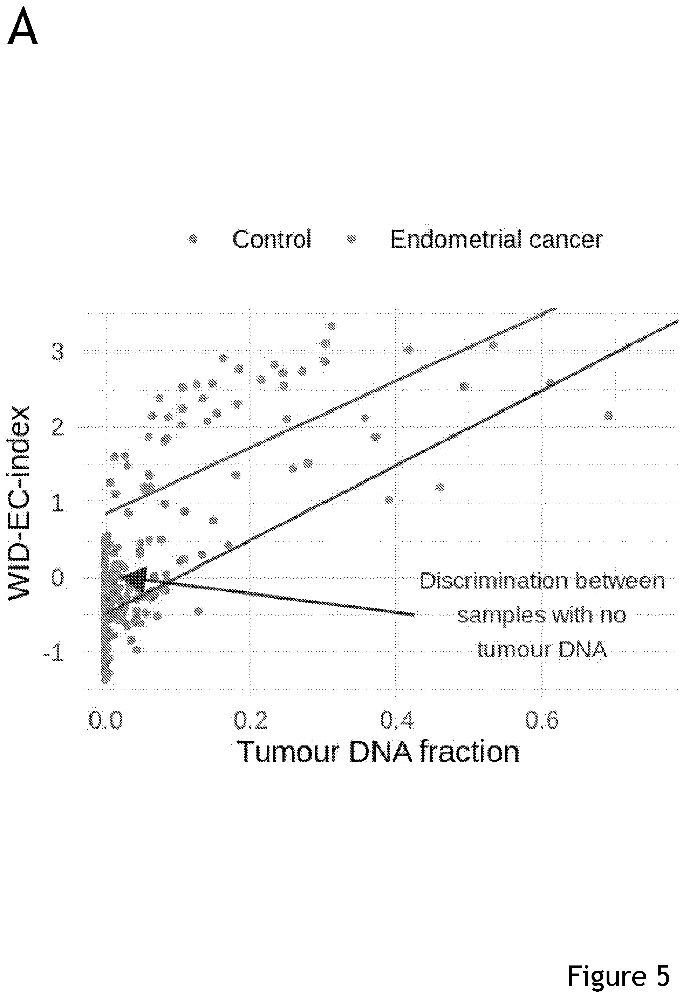

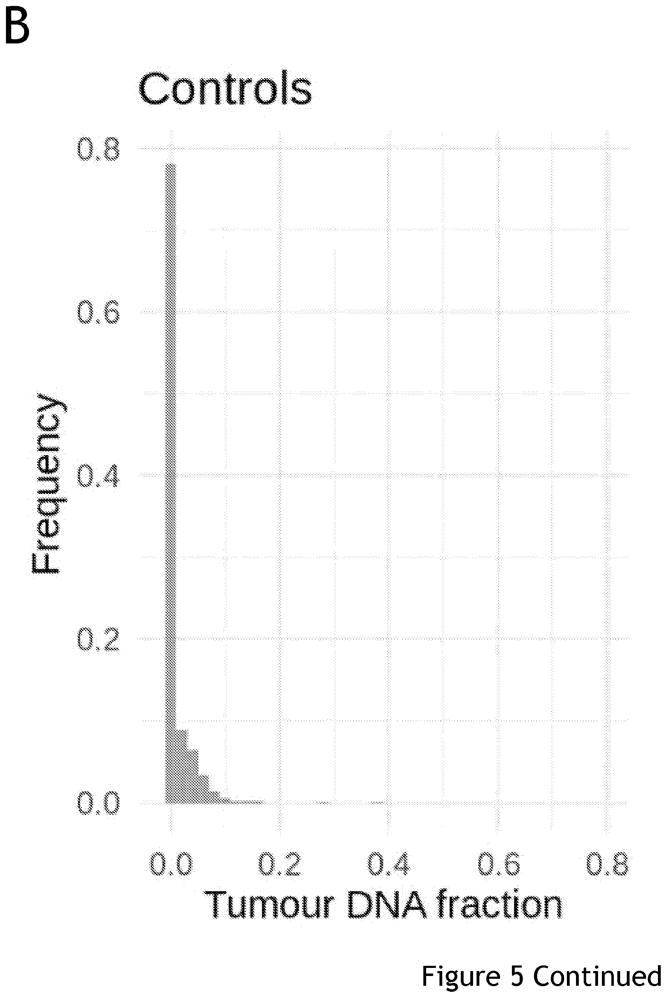

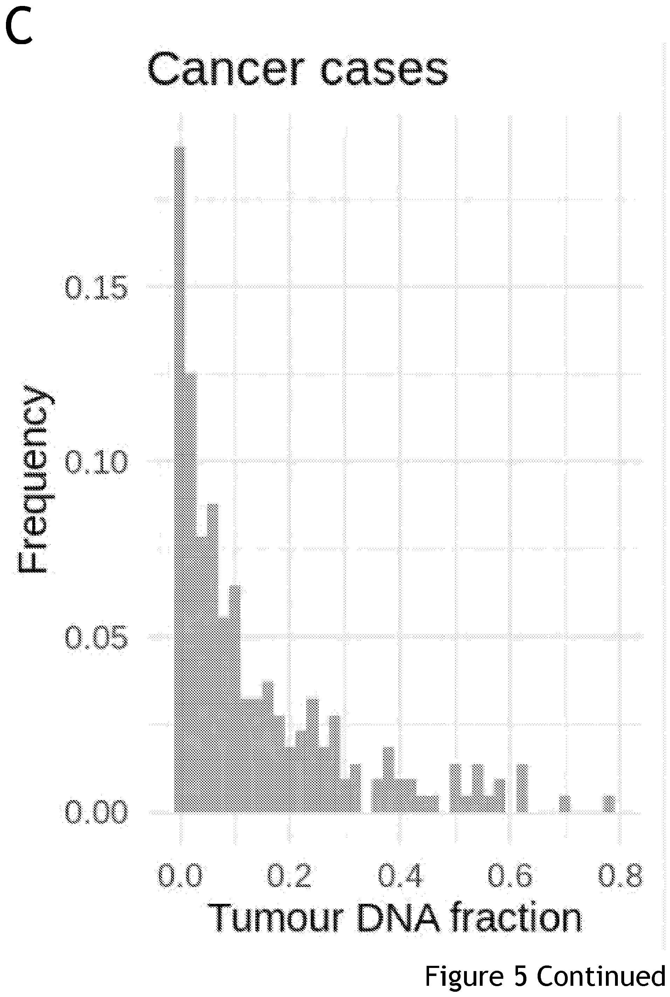

Due to the anatomical proximity between the cancer (i.e. endometrium) and the area from which the inventors have taken the sample (i.e. cervix) the inventors investigated whether the discriminatory signal is driven by tumour DNA draining from the uterus to the cervix or whether the signal is a generic risk signal retained in cervical epithelial cells. The inventors used 11 epithelial, 7 fibroblast, 42 immune cell, and 9 endometrial cancer tissue samples to develop a new reference panel for use with the EpiDISH algorithm (see Methods). For each sample the inventors obtained estimates of the proportion of DNA from each of the four cell types. The inventors observed that the proportion of tumour DNA in cases is substantially higher compared to controls (Figure 5A) for which the proportion of tumour DNA is close to zero. The inventors found that 43% of cancer cases provided cervical smear samples that consisted of >10% tumour DNA. Although the WID-EC index strongly increases with the proportion of tumour DNA (correlation coefficient 0.70, p<10⁻¹⁶) it is also dramatically higher in those cases which have no tumour DNA present (Figure 5B) indicating that the discriminatory signal does not depend on the presence of tumour DNA.

### Cancer index values and clinical actions

Four sub-groups defined by ranges of cancer index values are specified in Table 9 as corresponding to preferred clinical actions, comprising intensified screening, administration of therapeutics and surgery. The subgroups are quartiles based on control samples from the internal validation set. That is, these values of the index split the control samples into four equally sized groups. Odds ratio values are calculated by comparing the number of cases and controls in a given quartile to the first quartile (which is taken as a reference). Odds ratio values are determined for endometrial cancer risk, ovarian cancer risk and CIN3 risk. For example, a woman in the fourth quartile is roughly 25 times more likely to have endometrial cancer than a woman in the first quartile.

### Discussion

Endometrial cancer is the most common gynaecological cancer and amongst those cancers with the most rapidly increasing incidence rates.

Here the inventors have established for the first time a test - the WID-EC-index - which based on a DNA methylation signature in cervical smear samples is able to identify women both with endometrial cancer and at risk for endometrial cancer with a very high sensitivity and specificity.

The WID-EC-index is able to identify 70% of women with an endometrial cancer with a specificity of 99% and 90% of women with an endometrial cancer with a specificity of 78%. Even more importantly the inventors demonstrated that based on a general population cervical screening cohort the inventors identified 50% of women who developed endometrial cancer subsequent to cancer diagnosis with a specificity of 100%. The inventors expect that the sensitivity (at similar specificity) could be even higher in this cohort setting because long-term storage (i.e. several years) of smear samples within the fluid which is used for liquid based cytology (e.g. Preservcyt) at -25C had a strong impact on DNA methylation (unpublished) with the least impact on CpG islands (which largely contribute to the diagnostic component of the WID-EC-index) and the strongest impact on open sea CpGs (i.e. CpGs which contribute to the risk predictive component of the index).

The inventors propose that - once validated in a prospective setting - that women amongst the top 10^{th} percentile should undergo an endometrial biopsy which for the far majority of cases can easily be done in an outpatient setting and - using for instance a pipelle - is easy tolerated by the patient.

Before validating the WID-EC-index in the general population it needs to be prospectively validated in particular in women at high risk for endometrial cancer including women with high BMI (currently 38% of all endometrial cancers are attributable to high BMI with increasing tendency) or Lynch syndrome. Lynch syndrome is an autosomal dominant inherited predisposition to cancer, caused by the mutation of DNA mismatch repair and associated with a 33-60% lifetime risk of developing endometrial cancer. Endometrial cancers in these women appear to be characterised by a higher proportion of advanced stage than in the general population and more aggressive histologic types (clear cell carcinomas, papillary serous carcinoma and carcino-sarcoma) and endometrial cancer occurs at a younger age in these women. It needs to be demonstrated whether the WID-EC-index test will allow these women - ideally based on self-collected samples - to personalise risk-reducing measures.

**Table 7A**

| **Quantile** | **Control** | **Cancer** | **OR (unadjusted)** | **OR (adjusted)** |
|---|---|---|---|---|
| (-1.36, -0.66) | 35 | 1 | 1.00 (reference) | 1.00 (reference) |
| (-0.66, -0.43) | 45 | 1 | 0.78 (0.02,31.16) | 0.61 (0.01, 20.2) |
| (-0.43, -0.23) | 77 | 4 | 1.65 (0.22,46.2) | 0.66 (0.05, 16.74) |
| (-0.23, 1.03) | 68 | 57 | 25.5 (5.27,612.01) | 13.66 (2.54, 254.56) |

**Table 7B**

| Quantile | Control | Cancer | OR (unadjusted) | OR (adjusted) |
|---|---|---|---|---|
| (-1.36, -0.66) | 20 | 3 | 1.00 (reference) | 1.00 (reference) |
| (-0.66, -0.43) | 32 | 3 | 0.63 (0.1,3.99) | 0.63 (0.11, 3.74) |
| (-0.43, -0.23) | 22 | 8 | 2.33 (0.57,12.47) | 3.64 (0.83, 20.4) |
| (-0.23, 1.03) | 25 | 41 | 10.26 (3.09,49.01) | 13.47 (3.4, 71.78) |

**Table 7C. Prospective samples odds ratio table**

| **Quantile** | **Control** | **Cancer** | **OR (unadjusted)** | **OR (adjusted)** |
|---|---|---|---|---|
| (-1.36, -0.66) | 20 | 3 | 1.00 (reference) | 1.00 (reference) |
| (-0.66, -0.43) | 32 | 3 | 0.63 (0.1,3.99) | 0.63 (0.11, 3.74) |
| (-0.43, -0.23) | 22 | 8 | 2.33 (0.57,12.47) | 3.64 (0.83, 20.4) |
| (-0.23, 1.03) | 25 | 41 | 10.26 (3.09,49.01) | 13.47 (3.4, 71.78) |

**Table 7D. Ovarian cancer odds ratio table**

| **Quantile** | **Control** | **Cancer** | **OR (unadjusted)** | **OR (adjusted)** |
|---|---|---|---|---|
| (-1.36, -0.66) | 75 | 17 | 1.00 (reference) | 1.00 (reference) |
| (-0.66, -0.43) | 74 | 35 | 2.07 (1.08,4.11) | 1.61 (0.8, 3.3) |
| (-0.43, -0.23) | 74 | 61 | 3.6 (1.95,6.91) | 1.97 (0.97, 4.07) |
| (-0.23, 1.03) | 74 | 129 | 7.59 (4.25,14.23) | 3.42 (1.71, 7.04) |

**Table 8A. Internal validation**

| Factor | Group | Control | Cancer | OR | p-value |
|---|---|---|---|---|---|
| Age (years) | <52 | 443 (56%) | 16 (8%) | 1.00 (reference) | - |
| | 52-64 | 222 (28%) | 66 (32%) | 8.15 (4.72,14.91) | <10^-15 |
| | >64 | 132 (17%) | 125 (60%) | 25.86 (15.24,46.76) | <10^-Inf |
| Age at menarche (years) | <12 | 124 (16%) | 37 (18%) | 1.27 (0.79,2.02) | 0.32 |
| | Dec-13 | 418 (53%) | 108 (53%) | 1.1 (0.77,1.57) | 0.6 |
| | >13 | 251 (32%) | 59 (29%) | 1.00 (reference) | - |
| Parous | Yes | 598 (75%) | 165 (80%) | 1.00 (reference) | - |
| | No | 199 (25%) | 41 (20%) | 0.75 (0.51,1.08) | 0.13 |
| Age at birth of first live child (years) | <21 | 34 (6%) | 17 (10%) | 1.00 (reference) | - |
| | 21-27 | 202 (34%) | 92 (56%) | 0.91 (0.49,1.75) | 0.77 |
| | >27 | 352 (60%) | 55 (34%) | 0.31 (0.16,0.61) | <10^-3 |
| Menopausal status | Pre | 411 (52%) | 19 (9%) | 1.00 (reference) | - |
| | Post | 386 (48%) | 188 (91%) | 10.44 (6.54,17.63) | <10^-Inf |
| Age at menopause (years) | <46 | 359 (49%) | 25 (14%) | 0.09 (0.06,0.15) | <10^-Inf |
| | 46-52 | 267 (36%) | 69 (39%) | 0.35 (0.23,0.51) | <10^-7 |
| | >52 | 110 (15%) | 82 (47%) | 1.00 (reference) | - |
| BMI (kg/m2) | <25 | 409 (51%) | 57 (28%) | 1.00 (reference) | - |
| | 25-30 | 238 (30%) | 50 (24%) | 1.51 (1,2.28) | 0.05 |
| | >30 | 150 (19%) | 100 (48%) | 4.77 (3.29,6.97) | <10^-Inf |
| Oral contraceptive use (pre menopausal only) | No | 194 (68%) | 109 (76%) | 1.00 (reference) | - |
| | Yes | 89 (31%) | 29 (20%) | 0.58 (0.36,0.93) | 0.02 |
| Hormone replacement therapy (post menopausal only) | No | 279 (98%) | 143 (99%) | 1.00 (reference) | - |
| | Yes | 7 (2%) | 1 (1%) | 0.31 (0.01,1.83) | 0.23 |
| Smoking | No | 685 (86%) | 193 (94%) | 1.00 (reference) | - |
| | Yes | 98 (12%) | 12 (6%) | 0.44 (0.22,0.79) | 0.005 |
| First degree relatives with endometrial cancer | 0 | 778 (98%) | 200 (97%) | 1.00 (reference) | - |
| | 1+ | 19 (2%) | 7 (3%) | 1.45 (0.55,3.38) | 0.42 |

**Table 8A. External validation**

| Factor | Group | Control | Cancer | OR | p-value |
|---|---|---|---|---|---|
| Age (years) | <52 | 91 (40%) | 3 (5%) | 1.00 (reference) | - |
| | 52-64 | 85 (38%) | 25 (40%) | 8.48 (2.82,38.04) | <10^-4 |
| | >64 | 49 (22%) | 35 (56%) | 20.41 (6.86,91.26) | <10^-10 |
| Age at menarche (years) | <12 | 34 (15%) | 11 (18%) | 1.3 (0.53,3.04) | 0.56 |
| | Dec-13 | 119 (53%) | 33 (53%) | 1.11 (0.58,2.15) | 0.76 |
| | >13 | 72 (32%) | 18 (29%) | 1.00 (reference) | - |
| Parous | Yes | 166 (74%) | 48 (76%) | 1.00 (reference) | - |
| | No | 59 (26%) | 15 (24%) | 0.88 (0.45,1.67) | 0.71 |
| Age at birth of first live child (years) | <21 | 16 (10%) | 8 (17%) | 1.00 (reference) | - |
| | 21-27 | 61 (38%) | 24 (51%) | 0.78 (0.3,2.18) | 0.63 |
| | >27 | 84 (52%) | 15 (32%) | 0.36 (0.13,1.03) | 0.06 |
| Menopausal status | Pre | 79 (35%) | 6 (10%) | 1.00 (reference) | - |
| | Post | 146 (65%) | 57 (90%) | 5.01 (2.22,13.61) | <10^-4 |
| Age at menopause (years) | <46 | 82 (37%) | 5 (8%) | 0.11 (0.03,0.27) | <10^-6 |
| | 46-52 | 91 (41%) | 26 (44%) | 0.48 (0.25,0.92) | 0.03 |
| | >52 | 47 (21%) | 28 (47%) | 1.00 (reference) | - |
| BMI (kg/m2) | <25 | 117 (52%) | 17 (27%) | 1.00 (reference) | - |
| | 25-30 | 57 (25%) | 11 (17%) | 1.33 (0.57,3.02) | 0.5 |
| | >30 | 51 (23%) | 35 (56%) | 4.67 (2.42,9.3) | <10^-5 |
| Oral contraceptive use (pre menopausal only) | No | 26 (38%) | 5 (31%) | 1.00 (reference) | - |
| | Yes | 42 (62%) | 11 (69%) | 1.34 (0.43,4.78) | 0.63 |
| Hormone replacement therapy (post menopausal only) | No | 64 (93%) | 16 (100%) | 1.00 (reference) | - |
| | Yes | 5 (7%) | 0 (0%) | | |
| Smoking | No | 190 (85%) | 59 (95%) | 1.00 (reference) | - |
| | Yes | 34 (15%) | 3 (5%) | 0.3 (0.07,0.87) | 0.02 |
| First degree relatives with endometrial cancer | 0 | 219 (97%) | 60 (95%) | 1.00 (reference) | - |
| | 1+ | 6 (3%) | 3 (5%) | 1.86 (0.36,7.53) | 0.42 |

**Table 8B. Internal validation**

| Factor | Group | Set |
|---|---|---|
| Age (years) | <52 | 16 (8%) |
| | 52-64 | 66 (32%) |
| | >64 | 125 (60%) |
| Menopausal status | Pre | 19 (9%) |
| | Post | 188 (91%) |
| Stage | T1 | 125 (60%) |
| | T2 | 14 (7%) |
| | T3 | 44 (21%) |
| | T4 | 15 (7%) |
| Grade | I | 45 (22%) |
| | II | 49 (24%) |
| | III | 94 (45%) |
| Histology | Endometrioid | 132 (64%) |
| | Serous | 37 (18%) |
| | Other | 38 (18%) |

**Table 8B. External validation**

| Factor | Group | Set |
|---|---|---|
| Age (years) | <52 | 3 (5%) |
| | 52-64 | 25 (40%) |
| | >64 | 35 (56%) |
| Menopausal status | Pre | 6 (10%) |
| | Post | 57 (90%) |
| Stage | T1 | 45 (71%) |
| | T2 | 3 (5%) |
| | T3 | 9 (14%) |
| | T4 | 4 (6%) |
| Grade | I | 10 (16%) |
| | II | 14 (22%) |
| | III | 31 (49%) |
| Histology | Endometrioid | 40 (63%) |
| | Serous | 10 (16%) |
| | Other | 13 (21%) |

**Table 8B. Prospective validation**

| Factor | Group | |
|---|---|---|
| Age (years) | <52 | 17 (31%) |
| | 52-64 | 18 (33%) |
| | >64 | 20 (36%) |
| Stage | I | 34 (62%) |
| | II | 5 (9%) |
| | III | 6 (11%) |
| | IV | 5 (9%) |

**Table 9.**

| **WID-EC ranges** | **Endometrial cancer risk** | **Ovarian cancer risk** | **CIN3 risk** | **Clinical Action** |
|---|---|---|---|---|
| **(-1.36, -0.66)** | 1 | 1 | 1 | no action required |
| **(-0.66, -0.43)** | 0.78 (0.02,31.16) | 2.07 (1.08,4.11) | 1.41 (0.21,37.61) | transvaginal ultrasound to assess endometrium and ovaries - if normal then no clinical action required |
| **(-0.43, -0.23)** | 1.65 (0.22,46.2) | 3.6 (1.95,6.91) | 23.53 (4.93,561.42) | Immediate colposcopy and HPV and cytology to assess cervix - if cervix normal perform hysteroscopy and endocervical and endometrial biopsy. CA125 and MRI pelvis to assess presence of ovarian cancer. In case all these tests negative then 6-monthly CA125, cell-free DNAme and transvaginal scan and yearly colpscopy and endometrial biopsy. |
| **(-0.23, 1.03)** | 25.5 (5.27,612.01) | 7.59 (4.25,14.23) | 2696.02 (391.72,4.5e15) | Immediate colposcopy and HPV and cytology to assess cervix and hysteroscopy and endocervical and endometrial biopsy. CA125 and MRI pelvis to assess presence of ovarian cancer. In case all these tests are negative then consider prophylactic total hysterectomy and bilateral salpingoophorectomy in case family planning finished; alternatively 6-monthly CA125 and cell-free DNAme and colposcopy/HPV/cytology and yearly MRI pelvis |

## Claims

1. An assay for assessing the presence, absence or development of endometrial cancer in an individual, the assay comprising:
a. providing a sample which has been taken from the individual, the sample comprising a population of DNA molecules;
b. determining in the population of DNA molecules in the sample the methylation status of a panel of one or more CpGs comprising the CpG identified in SEQ ID NO 67 wherein the CpG is identified at nucleotide positions 61 to 62;
c. deriving a cancer index value based on the methylation status of the one or more CpGs in the panel; and
d. assessing the presence, absence or development of endometrial cancer based on the cancer index value;
wherein the assay is characterised as having an area under the curve (AUC) of 0.60 or more as determined by receiver operating characteristics (ROC).

2. An assay according to claim 1, wherein:
i) the panel of one or more CpGs comprises at least 50 CpGs selected from the CpGs identified at nucleotide positions 61 to 62 in SEQ ID NOs 1 to 500, preferably wherein the assay is characterised as having an AUC of at least 0.90, and optionally wherein the panel of one or more CpGs comprises at least the CpGs identified in SEQ ID NOs 1 to 50 and identified at nucleotide positions 61 to 62, preferably wherein the assay is characterised as having an AUC of at least 0.95; or
ii) the panel of one or more CpGs comprises at least 100 CpGs selected from the CpGs identified at nucleotide positions 61 to 62 in SEQ ID NOs 1 to 500, preferably wherein the assay is characterised as having an AUC of at least 0.93, and optionally wherein the panel of one or more CpGs comprises at least the CpGs identified in SEQ ID NOs 1 to 100 and identified at nucleotide positions 61 to 62, preferably wherein the assay is characterised as having an AUC of at least 0.96; or
iii) the panel of one or more CpGs comprises at least 150 CpGs selected from the CpGs identified at nucleotide positions 61 to 62 in SEQ ID NOs 1 to 500, preferably wherein the assay is characterised as having an AUC of at least 0.93, and optionally wherein the panel of one or more CpGs comprises at least the CpGs identified in SEQ ID NOs 1 to 150 and identified at nucleotide positions 61 to 62, preferably wherein the assay is characterised as having an AUC of at least 0.96; or
iv) the panel of one or more CpGs comprises at least 200 CpGs selected from the CpGs identified at nucleotide positions 61 to 62 in SEQ ID NOs 1 to 500, preferably wherein the assay is characterised as having an AUC of at least 0.93, and optionally wherein the panel of one or more CpGs comprises at least the CpGs identified in SEQ ID NOs 1 to 200 and identified at nucleotide positions 61 to 62, preferably wherein the assay is characterised as having an AUC of at least 0.96; or
v) the panel of one or more CpGs comprises at least the 500 CpGs identified at nucleotide positions 61 to 62 in SEQ ID NOs 1 to 500, and further wherein the assay is characterised as having an AUC of at least 0.97.

3. An assay according to any one of claims 1 or 2, wherein the step of determining in the population of DNA molecules in the sample the methylation status of the one or more CpGs in the panel comprises determining a β value of each CpG, and optionally wherein the step of deriving the cancer index value based on the methylation status of the one or more CpGs in the panel comprises:
a. providing a methylation β-value data set comprising the methylation β-values for each CpG in the panel;
b. providing a mathematical model capable of generating the cancer index from the methylation β-value data set; and
c. applying the mathematical model to the methylation β-value data set, thereby generating the cancer index, and further optionally
wherein the cancer index value is a WID-EC-Index cancer index value , and wherein the mathematical model which is applied to the methylation β-value data set to generate the cancer index is an algorithm according to the following formula: $WID - EC - index = {\sum }_{i = 1}^{n} \frac{{w}_{i} {β}_{i} - μ}{σ}$ wherein:
a. *β*₁, ... , *βₙ* are methylation beta-values (between 0 and 1);
b. *w*₁, ... , *w*₅₀₀ are real valued coefficients;
c. µ and σ are real valued parameters used to scale the index; and
d. *n* refers to the number of CpGs in the panel of one or more CpGs.

4. An assay according to any one of claims 1 to 3, wherein when the cancer index value for the individual is:
i) about -0.201 or more, the individual is assessed as having endometrial cancer or as having a high risk of endometrial cancer development, or wherein when the cancer index value for the individual is less than about -0.201, the individual is assessed as not having endometrial cancer or as having a low risk of endometrial cancer development, preferably wherein:
a. the panel of one or more CpGs comprises at least 50 of the CpGs identified at nucleotide positions 61 to 62 in SEQ ID NOs 1 to 500, and wherein the sensitivity is at least 88% and the specificity is at least 76%;
b. the panel of one or more CpGs comprises at least the CpGs defined by SEQ ID NOs 1 to 50 and identified at nucleotide positions 61 to 62, and wherein the sensitivity is at least 90% and specificity is at least 80%;
c. the panel of one or more CpGs comprises at least the CpGs defined by SEQ ID NOs 1 to 100 and identified at nucleotide positions 61 to 62, and wherein the sensitivity is at least 92% and specificity is at least 79%; or
d. the panel of one or more CpGs comprises at least the CpGs defined by SEQ ID NOs 1 to 150 and identified at nucleotide positions 61 to 62, and wherein the sensitivity is at least 93% and specificity is at least 80%;
preferably wherein the assay comprises determining methylation β-values for each CpG in the panel of one or more CpGs; or
ii) about 0.269 or more, the individual is assessed as having endometrial cancer, or as having a high risk of endometrial cancer development, or wherein when the cancer index value for the individual is less than about 0.269, the individual is assessed as not having endometrial cancer or as having a low risk of endometrial cancer development, preferably wherein:
a. the panel of one or more CpGs comprises at least 50 of the CpGs identified at nucleotide positions 61 to 62 in SEQ ID NOs 1 to 500, and wherein the sensitivity is at least 75% and the specificity is at least 94%;
b. the panel of one or more CpGs comprises at least the CpGs defined by SEQ ID NOs 1 to 50 and identified at nucleotide positions 61 to 62, and wherein the sensitivity is at least 73% and specificity is at least 98%;
c. the panel of one or more CpGs comprises at least the CpGs defined by SEQ ID NOs 1 to 100 and identified at nucleotide positions 61 to 62, and wherein the sensitivity is at least 75% and specificity is at least 98%; or
d. the panel of one or more CpGs comprises at least the CpGs defined by SEQ ID NOs 1 to 150 and identified at nucleotide positions 61 to 62, and wherein the sensitivity is at least 79% and specificity is at least 97%;
preferably wherein the assay comprises determining methylation β-values for each CpG in the panel of one or more CpGs; or
iii) about 1.072 or more, the individual is assessed as having endometrial cancer or as having a high risk of endometrial cancer development, or wherein when the cancer index value for the individual is less than about 1.072, the individual is assessed as not having endometrial cancer or as having a low risk of endometrial cancer development, preferably wherein:
a. the panel of one or more CpGs comprises at least 50 of the CpGs identified at nucleotide positions 61 to 62 in SEQ ID NOs 1 to 500, and wherein the sensitivity is at least 58% and the specificity is at least 99%;
b. the panel of one or more CpGs comprises at least the CpGs defined by SEQ ID NOs 1 to 50 and identified at nucleotide positions 61 to 62, and wherein the sensitivity is at least 60% and specificity is 100%;
c. the panel of one or more CpGs comprises at least the CpGs defined by SEQ ID NOs 1 to 100 and identified at nucleotide positions 61 to 62, and wherein the sensitivity is at least 63% and specificity is 100%; or
d. the panel of one or more CpGs comprises at least the CpGs defined by SEQ ID NOs 1 to 150 and identified at nucleotide positions 61 to 62, and wherein the sensitivity is at least 64% and specificity is 100%;
preferably wherein the assay comprises determining methylation β-values for each CpG in the panel of one or more CpGs.

5. An assay according to any one of claims 1 to 4, wherein when the cancer index value for the individual is:
a. less than about -0.660 the individual is assessed as not having endometrial cancer;
b. about -0.660 or more and less than about -0.430 the individual is assessed as having a low risk of endometrial cancer development;
c. about -0.430 or more and less than about -0.230 the individual is assessed as having a moderate risk of endometrial cancer development; or
d. about -0.230 or more the individual is assessed as having a high risk of endometrial cancer development;
preferably wherein the assay comprises determining methylation β-values for each CpG in the panel of one or more CpGs.

6. An assay according to claim 1 or according to any one of the preceding claims, wherein the step of determining the methylation status of a panel of one or more CpGs comprises determining the methylation status of one or more CpGs denoted by CG identified in a panel of one or more DMRs defined by SEQ ID NOs 501 to 808, optionally wherein the panel of one or more CpGs comprises two or more CpGs denoted by CG identified in the panel of DMR(s), three or more CpGs denoted by CG identified in the panel of DMR(s), four or more CpGs denoted by CG identified in the panel of DMR(s), or all CpGs denoted by CG identified in the DMR(s) defined by SEQ ID NOs 501 to 808, and optionally wherein:
I) the step of determining the methylation status of a panel of the one or more CpGs comprises determining the methylation status of five or more, six or more, seven or more, eight or more, or nine or more, or all of the CpGs denoted by CG within any one or more of the DMRs defined by SEQ ID NOs 501 to 808; and/or
II) the step of determining the methylation status of a panel of one or more CpGs comprises determining the methylation status of two or more, three or more, four or more, five or more, six or more, seven or more, eight or more, or nine or more, or all of the CpGs denoted by CG within:
a. any combination of two, three, four, five, six, seven, eight, or nine or more of DMRs 1 to 308;
b. any combination of ten, twenty, thirty, forty, fifty, sixty, seventy, eighty, or ninety or more of DMRs 1 to 308;
c. all 237 of DMRs 1 to 308;
d. one DMR defined by SEQ ID NO: 501, two DMRs defined by SEQ ID NOs: 501 to 502, three DMRs defined by SEQ ID NOs: 501 to 503, four DMRs defined by SEQ ID NOs: 501 to 504, five DMRs defined by SEQ ID NOs: 501 to 505, six DMRs defined by SEQ ID NOs: 501 to 506, seven DMRs defined by SEQ ID NOs: 501 to 507, eight DMRs defined by SEQ ID NOs: 501 to 508, or nine DMRs defined by SEQ ID NOs: 501 to 509;
e. one DMR defined by SEQ ID NOs 922, 924, or 920, preferably within all of the DMRs defined by SEQ ID NOs 922, 924 and 920;
f. any combination of:
i. one or more DMRs defined by SEQ ID NOs: 525, 756 and 757, preferably within all of SEQ ID NOs 525, 756 and 757;
ii. one or more DMRs defined by SEQ ID NOs: 503, 504, 526, 740, 741, 743 and 744, preferably within all of SEQ ID NOs: 503, 504, 526, 740, 741, 743 and 744;
iii. one or more DMRs defined by SEQ ID NOs: 525, 756, 757, 503, 504, 526, 740, 741, 743 and 744, preferably within all of SEQ ID NOs: 525, 756, 757, 503, 504, 526, 740, 741, 743 and 744; or
g. ten DMRs defined by SEQ ID NOs: 501 to 510, twenty DMRs defined by SEQ ID NOs: 501 to 520, thirty DMRs defined by SEQ ID NOs: 501 to 530, forty DMRs defined by SEQ ID NOs: 501 to 540, fifty DMRs defined by SEQ ID NOs: 501 to 550, sixty DMRs defined by SEQ ID NOs: 501 to 560, seventy DMRs defined by SEQ ID NOs: 501 to 570, eighty DMRs defined by SEQ ID NOs: 501 to 580, or ninety DMRs defined by SEQ ID NOs: 501 to 590; or
h. ten DMRs defined by SEQ ID NOs: 501 to 510, SEQ ID NOs: 511 to 520, SEQ ID NOs: 521 to 530, SEQ ID NOs: 531 to 540, SEQ ID NOs: 541 to 550, SEQ ID NOs: 551 to 560, SEQ ID NOs: 561 to 570, SEQ ID NOs: 571 to 580, SEQ ID NOs: 581 to 590, SEQ ID NOs: 591 to 600, SEQ ID NOs: 601 to 610, SEQ ID NOs: 611 to 620, SEQ ID NOs: 621 to 630, SEQ ID NOs: 631 to 640, SEQ ID NOs: 641 to 650, SEQ ID NOs: 651 to 660, SEQ ID NOs: 661 to 670, SEQ ID NOs: 671 to 680, SEQ ID NOs: 681 to 690, SEQ ID NOs: 691 to 700, SEQ ID NOs: 701 to 710, SEQ ID NOs: 711 to 720, SEQ ID NOs: 721 to 730, SEQ ID NOs: 731 to 740, SEQ ID NOs: 741 to 750; SEQ ID NOs: 751 to 760; SEQ ID NOs: 761 to 770; SEQ ID NOs: 771 to 780; SEQ ID NOs: 781 to 790; SEQ ID NOs: 791 to 800 or SEQ ID NOs: 801 to 808.

7. An assay according to claim 6,
I) wherein the step of determining the methylation status of a panel of one or more CpGs comprises determining the methylation status of one or more CpGs within any one or more DMRs selected from the group of DMRs consisting of DMRs 1 to 308 as defined by SEQ ID NOs 501 to 808, including:
a. one or more CpGs within DMR 1 as defined by SEQ ID NO: 501 and denoted by CG, preferably wherein the assay is characterised as having an ROC AUC of at least 0.911, and more preferably wherein the panel of one or more CpGs comprises at least the CpGs denoted by [[CG]];
b. one or more CpGs within DMR 2 as defined by SEQ ID NO: 502 and denoted by CG, preferably wherein the assay is characterised as having an ROC AUC of at least 0.903, and more preferably wherein the panel of one or more CpGs comprises at least the CpGs denoted by [[CG]];
c. one or more CpGs within DMR 3 as defined by SEQ ID NO: 503 and denoted by CG, preferably wherein the assay is characterised as having an ROC AUC of at least 0.899, and more preferably wherein the panel of one or more CpGs comprises at least the CpGs denoted by [[CG]];
d. one or more CpGs within DMR 4 as defined by SEQ ID NO: 504 and denoted by CG, preferably wherein the assay is characterised as having an ROC AUC of at least 0.899, and more preferably wherein the panel of one or more CpGs comprises at least the CpGs denoted by [[CG]];
e. one or more CpGs within DMR 5 as defined by SEQ ID NO: 505 and denoted by CG, preferably wherein the assay is characterised as having an ROC AUC of at least 0.899, and more preferably wherein the panel of one or more CpGs comprises at least the CpGs denoted by [[CG]];
f. one or more CpGs within DMR 6 as defined by SEQ ID NO: 506 and denoted by CG, preferably wherein the assay is characterised as having an ROC AUC of at least 0.897, and more preferably wherein the panel of one or more CpGs comprises at least the CpGs denoted by [[CG]];
g. one or more CpGs within DMR 7 as defined by SEQ ID NO: 507 and denoted by CG, preferably wherein the assay is characterised as having an ROC AUC of at least 0.894, and more preferably wherein the panel of one or more CpGs comprises at least the CpGs denoted by [[CG]];
h. one or more CpGs within DMR 8 as defined by SEQ ID NO: 508 and denoted by CG, preferably wherein the assay is characterised as having an ROC AUC of at least 0.894, and more preferably wherein the panel of one or more CpGs comprises at least the CpGs denoted by [[CG]];
i. one or more CpGs within DMR 9 as defined by SEQ ID NO: 509 and denoted by CG, preferably wherein the assay is characterised as having an ROC AUC of at least 0.892, and more preferably wherein the panel of one or more CpGs comprises at least the CpGs denoted by [[CG]]; or
j. one or more CpGs within DMR 10 as defined by SEQ ID NO: 510 and denoted by CG, preferably wherein the assay is characterised as having an ROC AUC of at least 0.891, and more preferably wherein the panel of one or more CpGs comprises at least the CpGs denoted by [[CG]]; and/or
II) wherein:
a. when the cancer index for the individual is about 0.025 or more the individual is classified as having endometrial cancer or as having a high risk of endometrial cancer development; or
b. when the cancer index for the individual is less than about 0.025 the individual is classified as not having endometrial cancer;
preferably wherein the assay has a specificity of 95% or more, more preferably wherein the assay comprises determining mean β-values for each CpG in the panel of one or more CpGs; or
III) wherein:
a. CpGs denoted by CG whose cancer index value is determined are located within at least DMR 1 defined by SEQ ID NO: 501, and wherein when the cancer index for the individual is about 0.209 or more the individual is classified as having endometrial cancer or as having a high risk of endometrial cancer development, and wherein the sensitivity of the assay is at least 70.00% and the specificity of the assay is at least 95.00%, preferably wherein the panel of one or more CpGs comprises at least three CpGs from DMR 1, and more preferably wherein the cancer index value is the mean β-value for the CpGs denoted by [[CG]] in SEQ ID NO: 501;
b. CpGs denoted by CG whose cancer index value is determined are located within at least DMR 1 defined by SEQ ID NO: 501, and wherein when the cancer index for the individual is less than about 0.209 the individual is classified as not having endometrial cancer or as having a low risk of endometrial cancer development, and wherein the sensitivity of the assay is at least 70.00% and the specificity of the assay is at least 95.00%, preferably wherein the panel of one or more CpGs comprises at least three CpGs from DMR 1, and more preferably wherein the cancer index value is the mean β-value for the CpGs denoted by [[CG]] in SEQ ID NO: 501;
c. CpGs denoted by CG whose cancer index value is determined are located within at least DMR 2 defined by SEQ ID NO: 502, and wherein when the cancer index for the individual is about 0.271 or more the individual is classified as having endometrial cancer or as having a high risk of endometrial cancer development, and wherein the sensitivity of the assay is at least 77.00% and the specificity of the assay is at least 95.00%, preferably wherein the panel of one or more CpGs comprises at least one CpG from DMR 2, and more preferably wherein the cancer index value is the mean β-value for the CpGs denoted by [[CG]] in SEQ ID NO: 502;
d. CpGs denoted by CG whose cancer index value is determined are located within at least DMR 2 defined by SEQ ID NO: 502, and wherein when the cancer index for the individual is less than about 0.271 the individual is classified as not having endometrial cancer or as having a low risk of endometrial cancer development, and wherein the sensitivity of the assay is at least 77.00% and the specificity of the assay is at least 95.00%, preferably wherein the panel of one or more CpGs comprises at least one CpGs from DMR 2, and more preferably wherein the cancer index value is the mean β-value for the CpGs denoted by [[CG]] in SEQ ID NO: 502;
e. CpGs denoted by CG whose cancer index value is determined are located within at least DMR 3 defined by SEQ ID NO: 503, and wherein when the cancer index for the individual is about 0.123 or more the individual is classified as having endometrial cancer or as having a high risk of endometrial cancer development, and wherein the sensitivity of the assay is at least 73.00% and the specificity of the assay is at least 95.00%, preferably wherein the panel of one or more CpGs comprises at least three CpGs from DMR 3, and more preferably wherein the cancer index value is the mean β-value for the CpGs denoted by [[CG]] in SEQ ID NO: 503;
f. CpGs denoted by CG whose cancer index value is determined are located within at least DMR 3 defined by SEQ ID NO: 503, and wherein when the cancer index for the individual is less than about 0.123 the individual is classified as not having endometrial cancer or as having a low risk of endometrial cancer development, and wherein the sensitivity of the assay is at least 73.00% and the specificity of the assay is at least 95.00%, preferably wherein the panel of one or more CpGs comprises at least three CpGs from DMR 3, and more preferably wherein the cancer index value is the mean β-value for the CpGs denoted by [[CG]] in SEQ ID NO: 503;
g. CpGs denoted by CG whose cancer index value is determined are located within at least DMR 4 defined by SEQ ID NO: 504, and wherein when the cancer index for the individual is about 0.123 or more the individual is classified as having endometrial cancer or as having a high risk of endometrial cancer development, and wherein the sensitivity of the assay is at least 73.00% and the specificity of the assay is at least 95.00%, preferably wherein the panel of one or more CpGs comprises at least three CpGs from DMR 4, and more preferably wherein the cancer index value is the mean β-value for the CpGs denoted by [[CG]] in SEQ ID NO: 504;
h. CpGs denoted by CG whose cancer index value is determined are located within at least DMR 4 defined by SEQ ID NO: 504, and wherein when the cancer index for the individual is less than about 0.123 the individual is classified as not having endometrial cancer or as having a low risk of endometrial cancer development, and wherein the sensitivity of the assay is at least 73.00% and the specificity of the assay is at least 95.00%, preferably wherein the panel of one or more CpGs comprises at least three CpGs from DMR 4, and more preferably wherein the cancer index value is the mean β-value for the CpGs denoted by [[CG]] in SEQ ID NO: 504;
i. CpGs denoted by CG whose cancer index value is determined are located within at least DMR 5 defined by SEQ ID NO: 505, and wherein when the cancer index for the individual is about 0.105 or more the individual is classified as having endometrial cancer or as having a high risk of endometrial cancer development, and wherein the sensitivity of the assay is at least 71.00% and the specificity of the assay is at least 95.00%, preferably wherein the panel of one or more CpGs comprises at least five CpGs from DMR 5, and more preferably wherein the cancer index value is the mean β-value for the CpGs denoted by [[CG]] in SEQ ID NO: 505; or
j. CpGs denoted by CG whose cancer index value is determined are located within at least DMR 5 defined by SEQ ID NO: 505, and wherein when the cancer index for the individual is less than about 0.105 the individual is classified as not having endometrial cancer or as having a low risk of endometrial cancer development, and wherein the sensitivity of the assay is at least 71.00% and the specificity of the assay is at least 95.00%, preferably wherein the panel of one or more CpGs comprises at least five CpGs from DMR 5, and more preferably wherein the cancer index value is the mean β-value for the CpGs denoted by [[CG]] in SEQ ID NO: 505.

8. An assay according to claim 1 or any one of the preceding claims, wherein the step of determining the methylation status of the one or more CpGs in the panel further comprises or additionally comprises determining the methylation status of each CpG within one or more sequence identified by SEQ ID NOs 809 to 919, and optionally wherein the step of determining the methylation status of the one or more CpGs in the panel comprises determining each CpG within:
a. SEQ ID NO 809 and/or SEQ ID NO 846 and/or SEQ ID NO 883 and wherein when the cancer index value is about 0.282 or more the individual is classified as having endometrial cancer or as having a high risk of endometrial cancer development, and wherein the sensitivity of the assay is at least 85.00%, the specificity of the assay is about 100.00%, and the AUC is about 1.00, preferably wherein the cancer index value is the percent methylated reference for sequence defined by SEQ ID NO 809 and/or SEQ ID NO 846 and/or SEQ ID NO 883;
b. SEQ ID NO 809 and/or SEQ ID NO 846 and/or SEQ ID NO 883 and wherein when the cancer index value is less than about 0.282 the individual is classified as not having endometrial cancer or as having a low risk of endometrial cancer development, and wherein the sensitivity of the assay is at least 85.00%, the specificity of the assay is about 100.00%, and the AUC is about 1.00, preferably wherein the cancer index value is the percent methylated reference for sequence defined by SEQ ID NO 809 and/or SEQ ID NO 846 and/or SEQ ID NO 883;
c. SEQ ID NO 810 and/or SEQ ID NO 847 and/or SEQ ID NO 884 and wherein when the cancer index value is about 0.212 or more the individual is classified as having endometrial cancer or as having a high risk of endometrial cancer development, and wherein the sensitivity of the assay is at least 55.00%, the specificity of the assay is about 100.00%, and the AUC is about 1.00, preferably wherein the cancer index value is the percent methylated reference for sequence defined by SEQ ID NO 810 and/or SEQ ID NO 847 and/or SEQ ID NO 884;
d. SEQ ID NO 810 and/or SEQ ID NO 847 and/or SEQ ID NO 884 and wherein when the cancer index value is less than about 0.212 the individual is classified as not having endometrial cancer or as having a low risk of endometrial cancer development, and wherein the sensitivity of the assay is at least 55.00%, the specificity of the assay is about 100.00%, and the AUC is about 1.00, preferably wherein the cancer index value is the percent methylated reference for sequence defined by SEQ ID NO 810 and/or SEQ ID NO 847 and/or SEQ ID NO 884;
e. SEQ ID NO 811 and/or SEQ ID NO 848 and/or SEQ ID NO 885 and wherein when the cancer index value is about 0.002 or more the individual is classified as having endometrial cancer or as having a high risk of endometrial cancer development, and wherein the sensitivity of the assay is at least 90.00%, the specificity of the assay is about 80.00%, and the AUC is about 0.98, preferably wherein the cancer index value is the percent methylated reference for sequence defined by SEQ ID NO 811 and/or SEQ ID NO 848 and/or SEQ ID NO 885;
f. SEQ ID NO 811 and/or SEQ ID NO 848 and/or SEQ ID NO 885 and wherein when the cancer index value is less than about 0.002 the individual is classified as not having endometrial cancer or as having a low risk of endometrial cancer development, and wherein the sensitivity of the assay is at least 90.00%, the specificity of the assay is about 80.00%, and the AUC is about 0.98, preferably wherein the cancer index value is the percent methylated reference for sequence defined by SEQ ID NO 811 and/or SEQ ID NO 848 and/or SEQ ID NO 885;
g. SEQ ID NO 812 and/or SEQ ID NO 849 and/or SEQ ID NO 886 and wherein when the cancer index value is about 0.026 or more the individual is classified as having endometrial cancer or as having a high risk of endometrial cancer development, and wherein the sensitivity of the assay is at least 85.00%, the specificity of the assay is about 90.00%, and the AUC is about 0.98, preferably wherein the cancer index value is the percent methylated reference for sequence defined by SEQ ID NO 812 and/or SEQ ID NO 849 and/or SEQ ID NO 886;
h. SEQ ID NO 812 and/or SEQ ID NO 849 and/or SEQ ID NO 886 and wherein when the cancer index value is less than about 0.026 the individual is classified as not having endometrial cancer or as having a low risk of endometrial cancer development, and wherein the sensitivity of the assay is at least 85.00%, the specificity of the assay is about 90.00%, and the AUC is about 0.98, preferably wherein the cancer index value is the percent methylated reference for sequence defined by SEQ ID NO 812 and/or SEQ ID NO 849 and/or SEQ ID NO 886;
i. SEQ ID NO 813 and/or SEQ ID NO 850 and/or SEQ ID NO 887 and wherein when the cancer index value is about 0.003 or more the individual is classified as having endometrial cancer or as having a high risk of endometrial cancer development, and wherein the sensitivity of the assay is at least 85.00%, the specificity of the assay is about 85.00%, and the AUC is about 0.97, preferably wherein the cancer index value is the percent methylated reference for sequence defined by SEQ ID NO 813 and/or SEQ ID NO 850 and/or SEQ ID NO 887; and/or
j. SEQ ID NO 813 and/or SEQ ID NO 850 and/or SEQ ID NO 887 and wherein when the cancer index value is less than about 0.003 the individual is classified as not having endometrial cancer or as having a low risk of endometrial cancer development, and wherein the sensitivity of the assay is at least 85.00%, the specificity of the assay is about 85.00%, and the AUC is about 0.97, preferably wherein the cancer index value is the percent methylated reference for sequence defined by SEQ ID NO 813 and/or SEQ ID NO 850 and/or SEQ ID NO 887;

9. An assay according to any one of claims 1 to 8, wherein:
I) the step of determining in the population of DNA molecules in the sample the methylation status of each CpG in the panel of one or more CpGs comprises:
a. performing a sequencing step to determine the sequence of each CpG;
b. hybridising DNA to an array comprising probes capable of discriminating between methylated and non-methylated forms of the CpGs and applying a detection system to the array so as to determine the methylation status of each CpG; and/or
c. performing a PCR step using methylation-specific primers, wherein the methylation status of the CpG is determined by the presence or absence of a PCR product; and/or
II) the step of determining the methylation status of each CpG in the panel of one or more CpGs comprises:
a. bisulphite converting the DNA; or
b. performing the steps of oxidising 5-methylcytosine bases (5mC) to 5-carboxylcytosine bases (5caC), preferably by ten-eleven translocation (TET), and/or oxidising 5-hydroxymethylcytosine bases (5hmC) to 5-carboxylcytosine bases (5caC), preferably by ten-eleven translocation (TET); followed by reducing 5-carboxylcytosine bases (5caC) to dihydrouracil bases (DHU), optionally with pyridine borane.

10. An assay according to any one of the preceding claims, wherein the endometrial cancer is an endometroid cancer, uterine carcinosarcoma, squamous cell carcinoma, small cell carcinoma, transitional carcinoma, serous carcinoma, clear-cell carcinoma, mucinous adenocarcinoma, undifferentiated carcinoma, dedifferentiated carcinoma or serous adenocarcinoma.

## Patentansprüche

1. Assay zum Beurteilen des Vorliegens, des Nichtvorliegens oder der Entwicklung von Gebärmutterschleimhautkrebs bei einem Individuum, wobei der Assay umfasst:
a. Bereitstellen einer Probe, die dem Individuum entnommen wurde, wobei die Probe eine Population von DNA-Molekülen umfasst;
b. Bestimmen des Methylierungsstatus eines Panels von einem oder mehreren CpGs in der Population von DNA-Molekülen in der Probe, die das in SEQ ID NO 67 identifizierte CpG umfasst, wobei das CpG an den Nukleotidpositionen 61 bis 62 identifiziert wird;
c. Ableiten eines Krebsindexwerts auf Grundlage des Methylierungsstatus des einen oder der mehreren CpGs in dem Panel und
d. Beurteilen des Vorliegens, des Nichtvorliegens oder der Entwicklung von Gebärmutterschleimhautkrebs auf Grundlage des Krebsindexwerts;
wobei der Assay **dadurch gekennzeichnet ist, dass** er eine Fläche unter der Kurve (Area Under the Curve, AUC) von 0,60 oder mehr aufweist, wie durch die Operationscharakteristik eines Beobachters (Receiver Operating Characteristics, ROC) bestimmt wurde.

2. Assay nach Anspruch 1, wobei:
i) das Panel aus einem oder mehreren CpGs wenigstens 50 CpGs umfasst, die aus den an den Nukleotidpositionen 61 bis 62 in den SEQ ID NOs 1 bis 500 identifizierten CpGs ausgewählt sind, wobei der Assay vorzugsweise durch das Aufweisen einer AUC von wenigstens 0,90 gekennzeichnet ist, und wobei das Panel aus einem oder mehreren CpGs optional wenigstens die in den SEQ ID NOs 1 bis 50 identifizierten und an den Nukleotidpositionen 61 bis 62 identifizierten CpGs umfasst, wobei der Assay vorzugsweise durch das Aufweisen einer AUC von wenigstens 0,95 gekennzeichnet ist; oder
ii) das Panel aus einem oder mehreren CpGs wenigstens 100 CpGs umfasst, die aus den an den Nukleotidpositionen 61 bis 62 in den SEQ ID NOs 1 bis 500 identifizierten CpGs ausgewählt sind, wobei der Assay vorzugsweise durch das Aufweisen einer AUC von wenigstens 0,93 gekennzeichnet ist, und wobei das Panel aus einem oder mehreren CpGs optional wenigstens die in den SEQ ID NOs 1 bis 100 identifizierten und an den Nukleotidpositionen 61 bis 62 identifizierten CpGs umfasst, wobei der Assay vorzugsweise durch das Aufweisen einer AUC von wenigstens 0,96 gekennzeichnet ist; oder
iii) das Panel aus einem oder mehreren CpGs wenigstens 150 CpGs umfasst, die aus den an den Nukleotidpositionen 61 bis 62 in den SEQ ID NOs 1 bis 500 identifizierten CpGs ausgewählt sind, wobei der Assay vorzugsweise durch das Aufweisen einer AUC von wenigstens 0,93 gekennzeichnet ist, und wobei das Panel aus einem oder mehreren CpGs optional wenigstens die in den SEQ ID NOs 1 bis 150 identifizierten und an den Nukleotidpositionen 61 bis 62 identifizierten CpGs umfasst, wobei der Assay vorzugsweise durch das Aufweisen einer AUC von wenigstens 0,96 gekennzeichnet ist; oder
iv) das Panel aus einem oder mehreren CpGs wenigstens 200 CpGs umfasst, die aus den an den Nukleotidpositionen 61 bis 62 in den SEQ ID NOs 1 bis 500 identifizierten CpGs ausgewählt sind, wobei der Assay vorzugsweise durch das Aufweisen einer AUC von wenigstens 0,93 gekennzeichnet ist, und wobei das Panel aus einem oder mehreren CpGs optional wenigstens die in den SEQ ID NOs 1 bis 200 identifizierten und an den Nukleotidpositionen 61 bis 62 identifizierten CpGs umfasst, wobei der Assay vorzugsweise durch das Aufweisen einer AUC von wenigstens 0,96 gekennzeichnet ist; oder
v) das Panel aus einem oder mehreren CpGs wenigstens die in den SEQ ID NOs 1 bis 500 an den Nukleotidpositionen 61 bis 62 identifizierten 500 CpGs umfasst, und wobei der Assay ferner durch das Aufweisen einer AUC von wenigstens 0,97 gekennzeichnet ist.

3. Assay nach einem der Ansprüche 1 oder 2, wobei der Schritt des Bestimmens des Methylierungsstatus des einen oder der mehreren CpGs in dem Panel in der Population von DNA-Molekülen in der Probe das Bestimmen eines β-Werts für jedes CpG umfasst, und optional der Schritt des Ableitens des Krebsindexwerts auf Grundlage des Methylierungsstatus des einen oder der mehreren CpGs in dem Panel umfasst:
a. Bereitstellen eines Methylierungs-β-Wertdatensatzes, der die Methylierungs-β-Werte für jedes CpG in dem Panel umfasst;
b. Bereitstellen eines mathematischen Modells, das in der Lage ist, den Krebsindex aus dem Methylierungs-β-Wertdatensatz zu erzeugen; und
c. Anwenden des mathematischen Modells auf den Methylierungs-β-Wertdatensatz, wodurch der Krebsindex erzeugt wird, und ferner optional
wobei der Krebsindexwert ein WID-EC-Index-Krebsindexwert ist und wobei das mathematische Modell, das auf den Methylierungs-β-Wertdatensatz angewendet wird, um den Krebsindex zu erzeugen, ein Algorithmus gemäß der folgenden Formel ist: $WID - EC - Index = {\sum }_{i = 1}^{n} \frac{{w}_{i} {β}_{i} - μ}{σ}$ wobei:
a. β₁, ..., βₙ sind Methylierungs-Beta-Werte (zwischen 0 und 1) sind;
b. w₁, ... w₅₀₀ reellwertige Koeffizienten sind;
c. µ und σ reellwertige Parameter sind, die für die Skalierung des Indexes verwendet werden; und
d. *n* sich auf die Anzahl der CpGs in dem Panel aus einem oder mehreren CpGs bezieht.

4. Assay nach einem der Ansprüche 1 bis 3, wobei, wenn der Krebsindexwert für das Individuum ist:
i) etwa -0,201 oder mehr, das Individuum als Gebärmutterschleimhautkrebs aufweisend oder als ein hohes Risiko für die Entwicklung von Gebärmutterschleimhautkrebs aufweisend beurteilt wird, oder wobei, wenn der Krebsindexwert für das Individuum weniger als etwa -0,201 ist, das Individuum als keinen Gebärmutterschleimhautkrebs aufweisend oder ein geringes Risiko für die Entwicklung von Gebärmutterschleimhautkrebs aufweisend beurteilt wird, vorzugsweise wobei:
a. das Panel aus einem oder mehreren CpGs wenigstens 50 der CpGs umfasst, die an den Nukleotidpositionen 61 bis 62 in den SEQ ID NOs 1 bis 500 identifiziert wurden, und wobei die Sensitivität wenigstens 88 % ist und die Spezifität wenigstens 76 % ist;
b. das Panel aus einem oder mehreren CpGs wenigstens die CpGs umfasst, die durch die SEQ ID NOs 1 bis 50 definiert und an den Nukleotidpositionen 61 bis 62 identifiziert wurden, und wobei die Sensitivität wenigstens 90 % ist und die Spezifität wenigstens 80 % ist;
c. das Panel aus einem oder mehreren CpGs wenigstens die CpGs umfasst, die durch die SEQ ID NOs 1 bis 100 definiert und an den Nukleotidpositionen 61 bis 62 identifiziert wurden, und wobei die Sensitivität wenigstens 92 % ist und die Spezifität wenigstens 79 % ist; oder
d. das Panel aus einem oder mehreren CpGs wenigstens die CpGs umfasst, die durch die SEQ ID NOs 1 bis 150 definiert und an den Nukleotidpositionen 61 bis 62 identifiziert wurden, und wobei die Sensitivität wenigstens 93 % ist und die Spezifität wenigstens 80 % ist;
vorzugsweise, wobei der Assay das Bestimmen von Methylierungs-β-Werten für jedes CpG in dem Panel aus einem oder mehreren CpGs umfasst; oder
ii) etwa 0,269 oder mehr, das Individuum als Gebärmutterschleimhautkrebs aufweisend oder als ein hohes Risiko für die Entwicklung von Gebärmutterschleimhautkrebs aufweisend beurteilt wird, oder wobei, wenn der Krebsindexwert für das Individuum weniger als etwa 0,269 ist, das Individuum als keinen Gebärmutterschleimhautkrebs aufweisend oder ein geringes Risiko für die Entwicklung von Gebärmutterschleimhautkrebs aufweisend beurteilt wird, vorzugsweise wobei:
a. das Panel aus einem oder mehreren CpGs wenigstens 50 der CpGs umfasst, die an den Nukleotidpositionen 61 bis 62 in den SEQ ID NOs 1 bis 500 identifiziert wurden, und wobei die Sensitivität wenigstens 75 % ist und die Spezifität wenigstens 94 % ist;
b. das Panel aus einem oder mehreren CpGs wenigstens die CpGs umfasst, die durch die SEQ ID NOs 1 bis 50 definiert und an den Nukleotidpositionen 61 bis 62 identifiziert wurden, und wobei die Sensitivität wenigstens 73 % ist und die Spezifität wenigstens 98 % ist;
c. das Panel aus einem oder mehreren CpGs wenigstens die CpGs umfasst, die durch die SEQ ID NOs 1 bis 100 definiert und an den Nukleotidpositionen 61 bis 62 identifiziert wurden, und wobei die Sensitivität wenigstens 75 % ist und die Spezifität wenigstens 98 % ist; oder
d. das Panel aus einem oder mehreren CpGs wenigstens die CpGs umfasst, die durch die SEQ ID NOs 1 bis 150 definiert und an den Nukleotidpositionen 61 bis 62 identifiziert wurden, und wobei die Sensitivität wenigstens 79 % ist und die Spezifität wenigstens 97 % ist;
vorzugsweise, wobei der Assay das Bestimmen von Methylierungs-β-Werten für jedes CpG in dem Panel aus einem oder mehreren CpGs umfasst; oder
iii) etwa 1,072 oder mehr, das Individuum als Gebärmutterschleimhautkrebs aufweisend oder als ein hohes Risiko für die Entwicklung von Gebärmutterschleimhautkrebs aufweisend beurteilt wird, oder wobei, wenn der Krebsindexwert für das Individuum weniger als etwa 1,072 ist, das Individuum als keinen Gebärmutterschleimhautkrebs aufweisend oder ein geringes Risiko für die Entwicklung von Gebärmutterschleimhautkrebs aufweisend beurteilt wird, vorzugsweise wobei:
a. das Panel aus einem oder mehreren CpGs wenigstens 50 der CpGs umfasst, die an den Nukleotidpositionen 61 bis 62 in den SEQ ID NOs 1 bis 500 identifiziert wurden, und wobei die Sensitivität wenigstens 58 % ist und die Spezifität wenigstens 99 % ist;
b. das Panel aus einem oder mehreren CpGs wenigstens die CpGs umfasst, die durch die SEQ ID NOs 1 bis 50 definiert und an den Nukleotidpositionen 61 bis 62 identifiziert wurden, und wobei die Sensitivität wenigstens 60 % ist und die Spezifität 100 % ist;
c. das Panel aus einem oder mehreren CpGs wenigstens die CpGs umfasst, die durch die SEQ ID NOs 1 bis 100 definiert und an den Nukleotidpositionen 61 bis 62 identifiziert wurden, und wobei die Sensitivität wenigstens 63 % ist und die Spezifität 100 % ist; oder
d. das Panel aus einem oder mehreren CpGs wenigstens die CpGs umfasst, die durch die SEQ ID NOs 1 bis 150 definiert und an den Nukleotidpositionen 61 bis 62 identifiziert wurden, und wobei die Sensitivität wenigstens 64 % ist und die Spezifität 100 % ist;
vorzugsweise, wobei der Assay das Bestimmen von Methylierungs-β-Werten für jedes CpG in dem Panel aus einem oder mehreren CpGs umfasst.

5. Assay nach einem der Ansprüche 1 bis 4, wobei, wenn der Krebsindexwert für das Individuum ist:
a. unter etwa -0,660, wird das Individuum als keinen Gebärmutterschleimhautkrebs aufweisend beurteilt;
b. etwa -0,660 oder mehr und unter etwa -0,430, wird das Individuum als ein niedriges Risiko für die Entwicklung von Gebärmutterschleimhautkrebs aufweisend beurteilt;
c. etwa -0,430 oder mehr und unter etwa -0,230, wird das Individuum als ein mäßiges Risiko für die Entwicklung von Gebärmutterschleimhautkrebs aufweisend beurteilt; oder
d. etwa -0,230 oder mehr, wird das Individuum als ein hohes Risiko für die Entwicklung von Gebärmutterschleimhautkrebs aufweisend beurteilt;
vorzugsweise, wobei der Assay das Bestimmen von Methylierungs-β-Werten für jedes CpG in dem Panel aus einem oder mehreren CpGs umfasst.

6. Assay nach Anspruch 1 oder nach einem der vorstehenden Ansprüche, wobei der Schritt des Bestimmens des Methylierungsstatus eines Panels aus einem oder mehreren CpGs das Bestimmen des Methylierungsstatus eines oder mehrerer mit CG bezeichneter CpGs umfasst, die in einem Panel aus einem oder mehreren DMRs identifiziert wurden, die durch die SEQ ID NOs 501 bis 808 definiert sind, optional wobei das Panel aus einem oder mehreren CpGs zwei oder mehr mit CG bezeichnete CpGs, die in dem Panel von DMR(s) identifiziert wurden, drei oder mehr mit CG bezeichnete CpGs, die in dem Panel von DMR(s) identifiziert wurden, vier oder mehr mit CG bezeichnete CpGs, die in dem Panel von DMR(s) identifiziert wurden, oder alle mit CG bezeichneten CpGs, die in den durch SEQ ID NOs 501 bis 808 definierten DMR(s) identifiziert wurden, umfasst, und optional wobei
I) der Schritt des Bestimmens des Methylierungsstatus eines Penals aus einem oder mehreren CpGs das Bestimmen des Methylierungsstatus von fünf oder mehr, sechs oder mehr, sieben oder mehr, acht oder mehr oder neun oder mehr oder aller CpGs, die mit CG bezeichnet sind, innerhalb eines oder mehrerer der durch die SEQ ID NOs 501 bis 808 definierten DMRs umfasst; und/oder
II) der Schritt zum Bestimmen des Methylierungsstatus eines Panels aus einem oder mehreren CpGs das Bestimmen des Methylierungsstatus von zwei oder mehr, drei oder mehr, vier oder mehr, fünf oder mehr, sechs oder mehr, sieben oder mehr, acht oder mehr oder neun oder mehr oder allen CpGs, die mit CG bezeichnet sind, innerhalb umfasst von:
a. Einer beliebigen Kombination aus zwei, drei, vier, fünf, sechs, sieben, acht oder neun oder mehr der DMRs 1 bis 308;
b. einer beliebige Kombination aus zehn, zwanzig, dreißig, vierzig, fünfzig, sechzig, siebzig, achtzig oder neunzig oder mehr der DMRs 1 bis 308;
c. aller 237 DMRs von 1 bis 308;
d. eines DMR, definiert durch SEQ ID NO 501, zwei DMRs, definiert durch die SEQ ID NOs: 501 bis 502, drei DMRs, definiert durch die SEQ ID NOs 501 bis 503, vier DMRs, definiert durch die SEQ ID NOs 501 bis 504, fünf DMRs, definiert durch die SEQ ID NOs 501 bis 505, sechs DMRs, definiert durch die SEQ ID NOs 501 bis 506, sieben DMRs, definiert durch die SEQ ID NOs: 501 bis 507, acht DMRs, definiert durch die SEQ ID NOs 501 bis 508, oder neun DMRs, definiert durch die SEQ ID NOs 501 bis 509;
e. eines DMR, definiert die SEQ ID NOs 922, 924 oder 920, vorzugsweise innerhalb aller DMRs, definiert durch die SEQ ID NOs 922, 924 und 920;
f. einer beliebigen Kombination aus:
i. einem oder mehreren DMRs, definiert durch die SEQ ID NOs 525, 756 und 757, vorzugsweise innerhalb aller SEQ ID NOs 525, 756 und 757;
ii. einem oder mehreren DMRs, definiert durch die SEQ ID NOs 503, 504, 526, 740, 741, 743 und 744, vorzugsweise innerhalb aller SEQ ID NOs: 503, 504, 526, 740, 741, 743 und 744;
iii. einem oder mehreren DMRs, definiert durch die SEQ ID NOs 525, 756, 757, 503, 504, 526, 740, 741, 743 und 744, vorzugsweise innerhalb aller SEQ ID NOs 525, 756, 757, 503, 504, 526, 740, 741, 743 und 744; oder
g zehn DMRs, definiert durch die SEQ ID NOs 501 bis 510, zwanzig DMRs, definiert durch die SEQ ID NOs 501 bis 520, dreißig DMRs, definiert durch die SEQ ID NOs: 501 bis 530, vierzig DMRs, definiert durch die SEQ ID NOs: 501 bis 540, fünfzig DMRs, definiert durch die SEQ ID NOs: 501 bis 550, sechzig DMRs, definiert durch die SEQ ID NOs 501 bis 560, siebzig DMRs, definiert durch die SEQ ID NOs: 501 bis 570, achtzig DMRs, definiert durch die SEQ ID NOs: 501 bis 580 oder neunzig DMRs, definiert durch die SEQ ID NOs 501 bis 590; oder
h zehn DMRs, definiert durch die SEQ ID NOs 501 bis 510, die SEQ ID NOs 511 bis 520, die SEQ ID NOs: 521 bis 530, SEQ ID NOs 531 bis 540, SEQ ID Nos 541 bis 550, SEQ ID NOs: 551 bis 560, SEQ ID NOs 561 bis 570, SEQ ID NOs 571 bis 580, SEQ ID NOs 581 bis 590, SEQ ID NOs 591 bis 600, SEQ ID NOs: 601 bis 610, SEQ ID NOs 611 bis 620, SEQ ID NOs: 621 bis 630, SEQ ID NOs: 631 bis 640, SEQ ID NOs 641 bis 650, SEQ ID NOs 651 bis 660, SEQ ID NOs 661 bis 670, SEQ ID NOs: 671 bis 680, SEQ ID NOs: 681 bis 690, SEQ ID NOs 691 bis 700, SEQ ID NOs: 701 bis 710, SEQ ID NOs: 711 bis 720, SEQ ID NOs 721 bis 730, SEQ ID NOs: 731 bis 740, SEQ ID NOs 741 bis 750; SEQ ID NOs: 751 bis 760; SEQ ID NOs: 761 bis 770; SEQ ID NOs: 771 bis 780; SEQ-ID-NOs 781 bis 790; SEQ-ID-NOs 791 bis 800 oder SEQ-ID-NOs: 801 bis 808.

7. Assay nach Anspruch 6,
I) wobei der Schritt des Bestimmens des Methylierungsstatus eines Panels aus einem oder mehreren CpGs das Bestimmen des Methylierungsstatus von einem oder mehreren CpGs innerhalb eines oder mehrerer DMRs umfasst, die aus der Gruppe von DMRs ausgewählt sind, bestehend aus den DMRs 1 bis 308, wie durch die SEQ ID NOs 501 bis 808 definiert, einschließlich:
a. eines oder mehrerer CpGs innerhalb von DMR 1, wie durch SEQ ID NO 501 definiert und mit CG bezeichnet, wobei der Assay vorzugsweise durch eine ROC AUC von wenigstens 0,911 gekennzeichnet ist und wobei das Panel aus einem oder mehreren CpGs stärker bevorzugt wenigstens die mit [[CG]] bezeichneten CpGs umfasst;
b eines oder mehrerer CpGs innerhalb von DMR 2, wie durch SEQ ID NO: 502 definiert und mit CG bezeichnet, wobei der Assay vorzugsweise durch eine ROC AUC von wenigstens 0,903 gekennzeichnet ist und wobei das Panel aus einem oder mehreren CpGs stärker bevorzugt wenigstens die mit [[CG]] bezeichneten CpGs umfasst;
c. eines oder mehrerer CpGs innerhalb von DMR 3, wie durch SEQ ID NO 503 definiert und mit CG bezeichnet, wobei der Assay vorzugsweise durch eine ROC AUC von wenigstens 0,899 gekennzeichnet ist und wobei das Panel aus einem oder mehreren CpGs stärker bevorzugt wenigstens die mit [[CG]] bezeichneten CpGs umfasst;
d. eines oder mehrerer CpGs innerhalb von DMR 4, wie durch SEQ ID NO: 504 definiert und mit CG bezeichnet, wobei der Assay vorzugsweise durch eine ROC AUC von wenigstens 0,899 gekennzeichnet ist und wobei das Panel aus einem oder mehreren CpGs stärker bevorzugt wenigstens die mit [[CG]] bezeichneten CpGs umfasst;
e. eines oder mehrerer CpGs innerhalb von DMR 5, wie durch SEQ ID NO 505 definiert und mit CG bezeichnet, wobei der Assay vorzugsweise durch eine ROC AUC von wenigstens 0,899 gekennzeichnet ist und wobei das Panel aus einem oder mehreren CpGs stärker bevorzugt wenigstens die mit [[CG]] bezeichneten CpGs umfasst;
f. eines oder mehrerer CpGs innerhalb von DMR 6, wie durch SEQ ID NO: 506 definiert und mit CG bezeichnet, wobei der Assay vorzugsweise durch eine ROC AUC von wenigstens 0,897 gekennzeichnet ist und wobei das Panel aus einem oder mehreren CpGs stärker bevorzugt wenigstens die mit [[CG]] bezeichneten CpGs umfasst;
g. eines oder mehrerer CpGs innerhalb von DMR 7, wie durch SEQ ID NO 507 definiert und mit CG bezeichnet, wobei der Assay vorzugsweise durch eine ROC AUC von wenigstens 0,894 gekennzeichnet ist und wobei das Panel aus einem oder mehreren CpGs stärker bevorzugt wenigstens die mit [[CG]] bezeichneten CpGs umfasst;
h. eines oder mehrerer CpGs innerhalb von DMR 8, wie durch SEQ ID NO 508 definiert und mit CG bezeichnet, wobei der Assay vorzugsweise durch eine ROC AUC von wenigstens 0,894 gekennzeichnet ist und wobei das Panel aus einem oder mehreren CpGs stärker bevorzugt wenigstens die mit [[CG]] bezeichneten CpGs umfasst;
i. eines oder mehrerer CpGs innerhalb von DMR 9, wie durch SEQ ID NO 509 definiert und mit CG bezeichnet, wobei der Assay vorzugsweise durch eine ROC AUC von wenigstens 0,892 gekennzeichnet ist und wobei das Panel aus einem oder mehreren CpGs stärker bevorzugt wenigstens die mit [[CG]] bezeichneten CpGs umfasst; oder
j. eines oder mehrerer CpGs innerhalb von DMR 10, wie durch SEQ ID NO 510 definiert und mit CG bezeichnet, wobei der Assay vorzugsweise durch eine ROC AUC von wenigstens 0,891 gekennzeichnet ist und wobei das Panel aus einem oder mehreren CpGs stärker bevorzugt wenigstens die mit [[CG]] bezeichneten CpGs umfasst; oder
II) wobei,
a. wenn der Krebsindex für das Individuum bei etwa 0,025 oder mehr liegt, das Individuum als Gebärmutterschleimhautkrebs aufweisend oder als ein hohes Risiko für die Entwicklung von Gebärmutterschleimhautkrebs aufweisend klassifiziert wird; oder
b. wenn der Krebsindex für das Individuum unter etwa 0,025 liegt, das Individuum als keinen Gebärmutterschleimhautkrebs aufweisend klassifiziert wird;
vorzugsweise, wobei der Assay eine Spezifität von 95 % oder mehr aufweist, stärker bevorzugt, wobei der Assay das Bestimmen der mittleren β-Werte für jedes CpG in dem Panel aus einem oder mehreren CpGs umfasst; oder
III) wobei:
a. die mit CG bezeichneten CpGs, deren Krebsindexwert bestimmt wird, sich innerhalb wenigstens eines durch SEQ ID NO 501 definierten DMR1 befinden, und wobei, wenn der Krebsindex für das Individuum etwa 0,209 oder mehr beträgt, das Individuum als Gebärmutterschleimhautkrebs aufweisend oder als ein hohes Risiko für die Entwicklung von Gebärmutterschleimhautkrebs aufweisend klassifiziert wird, und wobei die Sensitivität des Assays wenigstens 70,00 % ist und die Spezifität des Assays wenigstens 95,00 % beträgt, vorzugsweise wobei das Panel aus einem oder mehreren CpGs wenigstens drei CpGs aus DMR 1 umfasst, und stärker bevorzugt wobei der Krebsindexwert der mittlere β-Wert für die in SEQ ID NO 501 mit [[CG]] bezeichneten CpGs ist;
b. die mit CG bezeichneten CpGs, deren Krebsindexwert bestimmt wurde, sich innerhalb wenigstens eines DMR 1 befinden, das durch SEQ ID NO: 501 definiert ist, und wobei, wenn der Krebsindex für das Individuum weniger als etwa 0,209 beträgt, das Individuum als keinen Gebärmutterschleimhautkrebs aufweisend oder als ein niedriges Risiko für die Entwicklung von Gebärmutterschleimhautkrebs aufweisend klassifiziert wird, und wobei die Sensitivität des Assays wenigstens 70,00 % und die Spezifität des Assays wenigstens 95,00 % beträgt, vorzugsweise wobei das Panel aus einem oder mehreren CpGs wenigstens drei CpGs aus DMR 1 umfasst, und stärker bevorzugt wobei der Krebsindexwert der mittlere β-Wert für die in SEQ ID NO: 501 mit [[CG]] bezeichneten CpGs ist;
c. die mit CG bezeichneten CpGs, deren Krebsindexwert bestimmt wurde, sich innerhalb wenigstens eines DMR 2 befinden, das durch SEQ ID NO: 502 definiert ist, und wobei, wenn der Krebsindex für das Individuum weniger als etwa 0,271 oder mehr beträgt, das Individuum als Gebärmutterschleimhautkrebs aufweisend oder als ein hohes Risiko für die Entwicklung von Gebärmutterschleimhautkrebs aufweisend klassifiziert wird, und wobei die Sensitivität des Assays wenigstens 77,00 % und die Spezifität des Assays wenigstens 95,00 % beträgt, vorzugsweise wobei das Panel aus einem oder mehreren CpGs wenigstens ein CpG aus DMR 2 umfasst, und stärker bevorzugt wobei der Krebsindexwert der mittlere β-Wert für die in SEQ ID NO: 502 mit [[CG]] bezeichneten CpGs ist;
d. die mit CG bezeichneten CpGs, deren Krebsindexwert bestimmt wurde, sich innerhalb wenigstens eines DMR 2 befinden, das durch SEQ ID NO: 502 definiert ist, und wobei, wenn der Krebsindex für das Individuum weniger als etwa 0,271 beträgt, das Individuum als keinen Gebärmutterschleimhautkrebs aufweisend oder als ein niedriges Risiko für die Entwicklung von Gebärmutterschleimhautkrebs aufweisend klassifiziert wird, und wobei die Sensitivität des Assays wenigstens 77,00 % und die Spezifität des Assays wenigstens 95,00 % beträgt, vorzugsweise wobei das Panel aus einem oder mehreren CpGs wenigstens ein CpG aus DMR 2 umfasst, und stärker bevorzugt wobei der Krebsindexwert der mittlere β-Wert für die in SEQ ID NO: 502 mit [[CG]] bezeichneten CpGs ist;
e. die mit CG bezeichneten CpGs, deren Krebsindexwert bestimmt wird, sich innerhalb wenigstens eines durch SEQ ID NO 503 definierten DMR 3 befinden, und wobei, wenn der Krebsindex für das Individuum etwa 0,123 oder mehr beträgt, das Individuum als Gebärmutterschleimhautkrebs aufweisend oder als ein hohes Risiko für die Entwicklung von Gebärmutterschleimhautkrebs aufweisend klassifiziert wird, und wobei die Sensitivität des Assays wenigstens 73,00 % ist und die Spezifität des Assays wenigstens 95,00 % beträgt, vorzugsweise wobei das Panel aus einem oder mehreren CpGs wenigstens drei CpGs aus DMR 3 umfasst, und stärker bevorzugt wobei der Krebsindexwert der mittlere β-Wert für die in SEQ ID NO 503 mit [[CG]] bezeichneten CpGs ist;
f. die mit CG bezeichneten CpGs, deren Krebsindexwert bestimmt wird, sich innerhalb wenigstens eines durch SEQ ID NO 503 definierten DMR 3 befinden, und wobei, wenn der Krebsindex für das Individuum weniger als etwa 0,123 beträgt, das Individuum als keinen Gebärmutterschleimhautkrebs aufweisend oder als ein niedriges Risiko für die Entwicklung von Gebärmutterschleimhautkrebs aufweisend klassifiziert wird, und wobei die Sensitivität des Assays wenigstens 73,00 % ist und die Spezifität des Assays wenigstens 95,00 % beträgt, vorzugsweise wobei das Panel aus einem oder mehreren CpGs wenigstens drei CpGs aus DMR 3 umfasst, und stärker bevorzugt wobei der Krebsindexwert der mittlere β-Wert für die in SEQ ID NO: 503 mit [[CG]] bezeichneten CpGs ist;
g. die mit CG bezeichneten CpGs, deren Krebsindexwert bestimmt wird, sich innerhalb wenigstens eines durch SEQ ID NO 504 definierten DMR 4 befinden, und wobei, wenn der Krebsindex für das Individuum etwa 0,123 oder mehr beträgt, das Individuum als Gebärmutterschleimhautkrebs aufweisend oder als ein hohes Risiko für die Entwicklung von Gebärmutterschleimhautkrebs aufweisend klassifiziert wird, und wobei die Sensitivität des Assays wenigstens 73,00 % ist und die Spezifität des Assays wenigstens 95,00 % beträgt, vorzugsweise wobei das Panel aus einem oder mehreren CpGs wenigstens drei CpGs aus DMR 4 umfasst, und stärker bevorzugt wobei der Krebsindexwert der mittlere β-Wert für die in SEQ ID NO 504 mit [[CG]] bezeichneten CpGs ist;
h. die mit CG bezeichneten CpGs, deren Krebsindexwert bestimmt wurde, sich innerhalb wenigstens eines DMR 4 befinden, das durch SEQ ID NO: 504 definiert ist, und wobei, wenn der Krebsindex für das Individuum weniger als etwa 0,123 beträgt, das Individuum als keinen Gebärmutterschleimhautkrebs aufweisend oder als ein niedriges Risiko für die Entwicklung von Gebärmutterschleimhautkrebs aufweisend klassifiziert wird, und wobei die Sensitivität des Assays wenigstens 73,00 % und die Spezifität des Assays wenigstens 95,00 % beträgt, vorzugsweise wobei das Panel aus einem oder mehreren CpGs wenigstens drei CpGs aus DMR 4 umfasst, und stärker bevorzugt wobei der Krebsindexwert der mittlere β-Wert für die in SEQ ID NO: 504 mit [[CG]] bezeichneten CpGs ist;
i. die mit CG bezeichneten CpGs, deren Krebsindexwert bestimmt wird, sich innerhalb wenigstens eines DMR 5 befinden, das durch SEQ ID NO 505 definiert ist, und wobei, wenn der Krebsindex für das Individuum etwa 0,105 oder mehr beträgt, das Individuum als Gebärmutterschleimhautkrebs aufweisend oder als ein hohes Risiko für die Entwicklung von Gebärmutterschleimhautkrebs aufweisend klassifiziert wird, und wobei die Sensitivität des Assays wenigstens 71,00 % und die Spezifität des Assays wenigstens 95,00 % beträgt, vorzugsweise wobei das Panel aus einem oder mehreren CpGs wenigstens fünf CpGs aus DMR 5 umfasst, und stärker bevorzugt wobei der Krebsindexwert der mittlere β-Wert für die in SEQ ID NO: 505 mit [[CG]] bezeichneten CpGs ist; oder
j. die mit CG bezeichneten CpGs, deren Krebsindexwert bestimmt wird, sich innerhalb wenigstens eines DMR 5 befinden, das durch SEQ ID NO 505 definiert ist, und wobei, wenn der Krebsindex für das Individuum weniger als etwa 0,105 beträgt, das Individuum als keinen Gebärmutterschleimhautkrebs aufweisend oder als ein niedriges Risiko für die Entwicklung von Gebärmutterschleimhautkrebs aufweisend klassifiziert wird, und wobei die Sensitivität des Assays wenigstens 71,00 % und die Spezifität des Assays wenigstens 95,00 % beträgt, vorzugsweise wobei das Panel aus einem oder mehreren CpGs wenigstens fünf CpGs aus DMR 5 umfasst, und stärker bevorzugt wobei der Krebsindexwert der mittlere β-Wert für die in SEQ ID NO: 505 mit [[CG]] bezeichneten CpGs ist.

8. Assay nach Anspruch 1 oder einem der vorangehenden Ansprüche, wobei der Schritt des Bestimmens des Methylierungsstatus des einen oder der mehreren CpGs in dem Panel ferner oder zusätzlich das Bestimmen des Methylierungsstatus jedes CpG innerhalb einer oder mehrerer durch die SEQ ID NOs 809 bis 919 identifizierter Sequenzen umfasst, und wobei der Schritt des Bestimmens des Methylierungsstatus des einen oder der mehreren CpGs in dem Panel optional das Bestimmen jedes CpG innerhalb umfasst von:
a. SEQ ID NO 809 und/oder SEQ ID NO 846 und/oder SEQ ID NO 883, und wobei, wenn der Krebsindex etwa 0,282 oder mehr beträgt, das Individuum als Gebärmutterschleimhautkrebs aufweisend oder als ein hohes Risiko für die Entwicklung von Gebärmutterschleimhautkrebs aufweisend klassifiziert wird, und wobei die Sensitivität des Assays wenigstens 85,00 % beträgt, die Spezifität des Assays bei etwa 100,00 % liegt und die AUC bei etwa 1,00 liegt, vorzugsweise wobei der Krebsindexwert der methylierte Prozentbezug für die durch SEQ ID NO 809 und/oder SEQ ID NO 846 und/oder SEQ ID NO 883 definierten Sequenz ist,
b. SEQ ID NO 809 und/oder SEQ ID NO 846 und/oder SEQ ID NO 883, und wobei, wenn der Krebsindex weniger als 0,282 beträgt, das Individuum als keinen Gebärmutterschleimhautkrebs aufweisend oder als ein niedriges Risiko für die Entwicklung von Gebärmutterschleimhautkrebs aufweisend klassifiziert wird, und wobei die Sensitivität des Assays wenigstens 85,00 % beträgt, die Spezifität des Assays bei etwa 100,00 % liegt und die AUC bei etwa 1,00 liegt, vorzugsweise wobei der Krebsindexwert der methylierte Prozentbezug für die durch SEQ ID NO 809 und/oder SEQ ID NO 846 und/oder SEQ ID NO 883 definierten Sequenz ist;
c. SEQ ID NO 810 und/oder SEQ ID NO 847 und/oder SEQ ID NO 884, und wobei, wenn der Krebsindex etwa 0,212 oder mehr beträgt, das Individuum als Gebärmutterschleimhautkrebs aufweisend oder als ein hohes Risiko für die Entwicklung von Gebärmutterschleimhautkrebs aufweisend klassifiziert wird, und wobei die Sensitivität des Assays wenigstens 55,00 % beträgt, die Spezifität des Assays bei etwa 100,00 % liegt und die AUC bei etwa 1,00 liegt, vorzugsweise wobei der Krebsindexwert der methylierte Prozentbezug für die durch SEQ ID NO 810 und/oder SEQ ID NO 847 und/oder SEQ ID NO 884 definierten Sequenz ist;
d. SEQ ID NO 810 und/oder SEQ ID NO 847 und/oder SEQ ID NO 884, und wobei, wenn der Krebsindex weniger als etwa 0,212 beträgt, das Individuum als keinen Gebärmutterschleimhautkrebs aufweisend oder als ein niedriges Risiko für die Entwicklung von Gebärmutterschleimhautkrebs aufweisend klassifiziert wird, und wobei die Sensitivität des Assays wenigstens 55,00 % beträgt, die Spezifität des Assays bei etwa 100,00 % liegt und die AUC bei etwa 1,00 liegt, vorzugsweise wobei der Krebsindexwert der methylierte Prozentbezug für die durch SEQ ID NO 810 und/oder SEQ ID NO 847 und/oder SEQ ID NO 884 definierten Sequenz ist;
e. SEQ ID NO 811 und/oder SEQ ID NO 848 und/oder SEQ ID NO 885, und wobei, wenn der Krebsindex etwa 0,002 oder mehr beträgt, das Individuum als Gebärmutterschleimhautkrebs aufweisend oder als ein hohes Risiko für die Entwicklung von Gebärmutterschleimhautkrebs aufweisend klassifiziert wird, und wobei die Sensitivität des Assays wenigstens 90,00 % beträgt, die Spezifität des Assays bei etwa 80,00 % liegt und die AUC bei etwa 0,98 liegt, vorzugsweise wobei der Krebsindexwert der methylierte Prozentbezug für die durch SEQ ID NO 811 und/oder SEQ ID NO 848 und/oder SEQ ID NO 885 definierten Sequenz ist;
f. SEQ ID NO 811 und/oder SEQ ID NO 848 und/oder SEQ ID NO 885, und wobei, wenn der Krebsindex weniger als 0,002 beträgt, das Individuum als keinen Gebärmutterschleimhautkrebs aufweisend oder als ein niedriges Risiko für die Entwicklung von Gebärmutterschleimhautkrebs aufweisend klassifiziert wird, und wobei die Sensitivität des Assays wenigstens 85,00 % beträgt, die Spezifität des Assays bei etwa 90,00 % liegt und die AUC bei etwa 0,98 liegt, vorzugsweise wobei der Krebsindexwert der methylierte Prozentbezug für die durch SEQ ID NO 811 und/oder SEQ ID NO 848 und/oder SEQ ID NO 885 definierten Sequenz ist;
g. SEQ ID NO 812 und/oder SEQ ID NO 849 und/oder SEQ ID NO 886, und wobei, wenn der Krebsindex etwa 0,026 oder mehr beträgt, das Individuum als Gebärmutterschleimhautkrebs aufweisend oder als ein hohes Risiko für die Entwicklung von Gebärmutterschleimhautkrebs aufweisend klassifiziert wird, und wobei die Sensitivität des Assays wenigstens 85,00 % beträgt, die Spezifität des Assays bei etwa 90,00 % liegt und die AUC bei etwa 0,98 liegt, vorzugsweise wobei der Krebsindexwert der methylierte Prozentbezug für die durch SEQ ID NO 812 und/oder SEQ ID NO 849 und/oder SEQ ID NO 886 definierten Sequenz ist;
h. SEQ ID NO 812 und/oder SEQ ID NO 849 und/oder SEQ ID NO 886, und wobei, wenn der Krebsindex weniger als etwa 0,026 beträgt, das Individuum als keinen Gebärmutterschleimhautkrebs aufweisend oder als ein niedriges Risiko für die Entwicklung von Gebärmutterschleimhautkrebs aufweisend klassifiziert wird, und wobei die Sensitivität des Assays wenigstens 85,00 % beträgt, die Spezifität des Assays bei etwa 90,00 % liegt und die AUC bei etwa 0,98 liegt, vorzugsweise wobei der Krebsindexwert der methylierte Prozentbezug für die durch SEQ ID NO 812 und/oder SEQ ID NO 849 und/oder SEQ ID NO 886 definierten Sequenz ist;
i. SEQ ID NO 813 und/oder SEQ ID NO 850 und/oder SEQ ID NO 887, und wobei, wenn der Krebsindex etwa 0,003 oder mehr beträgt, das Individuum als Gebärmutterschleimhautkrebs aufweisend oder als ein hohes Risiko für die Entwicklung von Gebärmutterschleimhautkrebs aufweisend klassifiziert wird, und wobei die Sensitivität des Assays wenigstens 85,00 % beträgt, die Spezifität des Assays bei etwa 85,00 % liegt und die AUC bei etwa 0,97 liegt, vorzugsweise wobei der Krebsindexwert der methylierte Prozentbezug für die durch SEQ ID NO 813 und/oder SEQ ID NO 850 und/oder SEQ ID NO 887 definierten Sequenz ist; und/oder
j. SEQ ID NO 813 und/oder SEQ ID NO 850 und/oder SEQ ID NO 887, und wobei, wenn der Krebsindex weniger als etwa 0,003 beträgt, das Individuum als keinen Gebärmutterschleimhautkrebs aufweisend oder als ein niedriges Risiko für die Entwicklung von Gebärmutterschleimhautkrebs aufweisend klassifiziert wird, und wobei die Sensitivität des Assays wenigstens 85,00 % beträgt, die Spezifität des Assays bei etwa 85,00 % liegt und die AUC bei etwa 0,97 liegt, vorzugsweise wobei der Krebsindexwert der methylierte Prozentbezug für die durch SEQ ID NO 813 und/oder SEQ ID NO 850 und/oder SEQ ID NO 887 definierten Sequenz ist;

9. Assay nach einem der Ansprüche 1 bis 8, wobei
I) der Schritt des Bestimmens in der Population von DNA-Molekülen in der Probe des Methylierungsstatus jedes CpG in dem Panel aus einem oder mehreren CpGs umfasst:
a. Durchführen eines Sequenzierungsschritts zum Bestimmen der Sequenz jedes CpG;
b. Hybridisieren von DNA an ein Array, das Sonden umfasst, die in der Lage sind, um zwischen methylierten und nicht methylierten Formen der CpGs zu unterscheiden, und Anwenden eines Nachweissystems auf das Array, um den Methylierungsstatus jedes CpGs zu bestimmen; und/oder
c. Durchführen eines PCR-Schritts unter Verwendung methylierungsspezifischer Primer, wobei der Methylierungsstatus des CpG durch das Vorliegen oder Nichtvorliegen eines PCR-Produkts bestimmt wird; und/oder
II) der Schritt des Bestimmens des Methylierungsstatus jedes CpG in dem Panel aus einem oder mehreren CpGs umfasst
a. Bisulfitumwandeln der DNA; oder
b. Durchführen der Schritte des Oxidierens von 5-Methylcytosin-Basen (5mC) zu 5-Carboxylcytosin-Basen (5caC), vorzugsweise durch eine Ten-Eleven-Translokation (TET), und/oder des Oxidierens von 5-Hydroxymethylcytosin-Basen (5hmC) zu 5-Carboxylcytosinbasen (5caC), vorzugsweise durch Ten-Eleven-Translokation (TET); gefolgt durch Reduzieren von 5-Carboxylcytosinbasen (5caC) zu Dihydrouracilbasen (DHU), optional mit Pyridinboran.

10. Assay nach einem der vorangehenden Ansprüche, wobei der Gebärmutterschleimhautkrebs ein endometroider Krebs, ein Uterus-Karzinosarkom, ein Plattenepithelkarzinom, ein kleinzelliges Karzinom, ein Übergangszellkarzinom, ein seröses Karzinom, ein klarzelliges Karzinom, ein muzinöses Adenokarzinom, ein undifferenziertes Karzinom, ein dedifferenziertes Karzinom oder ein seröses Adenokarzinom ist.

## Revendications

1. Dosage permettant d'évaluer la présence, l'absence ou le développement d'un cancer de l'endomètre chez un individu, le dosage comprenant :
a. la fourniture d'un échantillon prélevé chez l'individu, l'échantillon comprenant une population de molécules d'ADN,
b. la détermination, au sein de la population de molécules d'ADN de l'échantillon, du statut de méthylation d'un panel d'un ou plusieurs CpG comprenant le CpG identifié dans la SEQ ID N° 67, le CpG étant identifié aux positions nucléotidiques 61 et 62,
c. le calcul de la valeur d'un indice de cancer en fonction du statut de méthylation du ou des CpG du panel, et
d. l'évaluation de la présence, de l'absence ou du développement d'un cancer de l'endomètre en fonction de la valeur de l'indice de cancer ;
le dosage étant **caractérisé par** une aire sous la courbe (AUC, area under the curve) supérieure ou égale à 0,60, d'après une détermination par la courbe caractéristique de la fonction d'efficacité du récepteur (ROC, receiver operating characteristic).

2. Dosage selon la revendication 1, dans lequel :
i) le panel d'un ou plusieurs CpG comprend au moins 50 CpG choisis parmi les CpG identifiés aux positions nucléotidiques 61 et 62 dans les SEQ ID N° 1 à 500, le dosage étant de préférence **caractérisé par le fait qu'**il présente une AUC d'au moins 0,90, et le panel d'un ou plusieurs CpG comprenant éventuellement au moins les CpG identifiés dans les SEQ ID N° 1 à 50 et identifiés aux positions nucléotidiques 61 et 62, le dosage étant de préférence **caractérisé par le fait qu'**il présente une AUC d'au moins 0,95, ou
ii) le panel d'un ou plusieurs CpG comprend au moins 100 CpG choisis parmi les CpG identifiés aux positions nucléotidiques 61 et 62 dans les SEQ ID N° 1 à 500, le dosage étant de préférence **caractérisé par le fait qu'**il présente une AUC d'au moins 0,93, et le panel d'un ou plusieurs CpG comprenant éventuellement au moins les CpG identifiés dans les SEQ ID N° 1 à 100 et identifiés aux positions nucléotidiques 61 et 62, le dosage étant de préférence **caractérisé par le fait qu'**il présente une AUC d'au moins 0,96, ou
iii) le panel d'un ou plusieurs CpG comprend au moins 150 CpG choisis parmi les CpG identifiés aux positions nucléotidiques 61 et 62 dans les SEQ ID N° 1 à 500, le dosage étant de préférence **caractérisé par le fait qu'**il présente une AUC d'au moins 0,93, et le panel d'un ou plusieurs CpG comprenant éventuellement au moins les CpG identifiés dans les SEQ ID N° 1 à 150 et identifiés aux positions nucléotidiques 61 et 62, le dosage étant de préférence **caractérisé par le fait qu'**il présente une AUC d'au moins 0,96, ou
iv) le panel d'un ou plusieurs CpG comprend au moins 200 CpG choisis parmi les CpG identifiés aux positions nucléotidiques 61 et 62 dans les SEQ ID N° 1 à 500, le dosage étant de préférence **caractérisé par le fait qu'**il présente une AUC d'au moins 0,93, et le panel d'un ou plusieurs CpG comprenant éventuellement au moins les CpG identifiés dans les SEQ ID N° 1 à 200 et identifiés aux positions nucléotidiques 61 et 62, le dosage étant de préférence **caractérisé par le fait qu'**il présente une AUC d'au moins 0,96, ou
v) le panel d'un ou plusieurs CpG comprend au moins les 500 CpG identifiés aux positions nucléotidiques 61 et 62 dans les SEQ ID N° 1 à 500, le dosage **se caractérisant en outre par le fait qu'**il présente une AUC d'au moins 0,97.

3. Dosage selon l'une quelconque des revendications 1 et 2, dans lequel l'étape de détermination, au sein de la population de molécules d'ADN de l'échantillon, du statut de méthylation du ou des CpG du panel comprend la détermination d'une valeur β pour chaque CpG, l'étape de calcul de la valeur de l'indice de cancer en fonction du statut de méthylation du ou des CpG du panel comprenant éventuellement :
a. la fourniture d'un ensemble de données de valeurs β de méthylation comprenant les valeurs β de méthylation de chaque CpG du panel,
b. la fourniture d'un modèle mathématique capable de générer l'indice de cancer à partir de l'ensemble de données de valeurs β de méthylation, et
c. l'application du modèle mathématique à l'ensemble de données de valeurs β de méthylation, générant ainsi l'indice de cancer ;
la valeur de l'indice de cancer étant éventuellement en outre la valeur d'un indice de cancer WID-EC-Index (Indice d'identification du risque de cancer chez les femmes - Cancer de l'endomètre), et le modèle mathématique appliqué à l'ensemble de données de valeurs β de méthylation pour générer l'indice de cancer étant un algorithme selon la formule suivante : $WID - EC - index = {\sum }_{i = 1}^{n} \frac{{w}_{i} {β}_{i} - μ}{σ}$ où :
a. *β₁, ..., βₙ* sont des valeurs bêta de méthylation (entre 0 et 1),
b. *w₁,* ..., *w₅₀₀* sont des coefficients à valeurs réelles,
c. µ et δ sont des paramètres à valeurs réelles utilisés pour mettre l'indice à l'échelle, et
d. *n* désigne le nombre de CpG du panel d'un ou plusieurs CpG.

4. Dosage selon l'une quelconque des revendications 1 à 3, dans lequel, lorsque la valeur de l'indice de cancer relatif à l'individu est :
i) supérieure ou égale à environ -0,201, l'individu est considéré comme atteint d'un cancer de l'endomètre ou comme présentant un risque élevé de développer un cancer de l'endomètre, ou lorsque la valeur de l'indice de cancer de l'individu est inférieure à environ - 0,201, l'individu est considéré comme non atteint d'un cancer de l'endomètre ou comme présentant un faible risque de développer un cancer de l'endomètre, étant entendu de préférence que :
a. le panel d'un ou plusieurs CpG comprend au moins 50 des CpG identifiés aux positions nucléotidiques 61 et 62 dans les SEQ ID N° 1 à 500, et la sensibilité est d'au moins 88 % et la spécificité d'au moins 76 %,
b. le panel d'un ou plusieurs CpG comprend au moins les CpG définis par les SEQ ID N° 1 à 50 et identifiés aux positions nucléotidiques 61 et 62, et la sensibilité est d'au moins 90 % et la spécificité d'au moins 80 %,
c. le panel d'un ou plusieurs CpG comprend au moins les CpG définis par les SEQ ID N° 1 à 100 et identifiés aux positions nucléotidiques 61 et 62, et la sensibilité est d'au moins 92 % et la spécificité d'au moins 79 %, ou
d. le panel d'un ou plusieurs CpG comprend au moins les CpG définis par les SEQ ID N° 1 à 150 et identifiés aux positions nucléotidiques 61 et 62, et la sensibilité est d'au moins 93 % et la spécificité d'au moins 80 %,
le dosage comprenant de préférence la détermination des valeurs β de méthylation pour chaque CpG du panel d'un ou plusieurs CpG ; ou
ii) supérieure ou égale à environ 0,269, l'individu est considéré comme atteint d'un cancer de l'endomètre ou comme présentant un risque élevé de développer un cancer de l'endomètre, ou lorsque la valeur de l'indice de cancer de l'individu est inférieure à environ 0,269, l'individu est considéré comme non atteint d'un cancer de l'endomètre ou comme présentant un faible risque de développer un cancer de l'endomètre, étant entendu de préférence que :
a. le panel d'un ou plusieurs CpG comprend au moins 50 des CpG identifiés aux positions nucléotidiques 61 et 62 dans les SEQ ID N° 1 à 500, et la sensibilité est d'au moins 75 % et la spécificité d'au moins 94 %,
b. le panel d'un ou plusieurs CpG comprend au moins les CpG définis par les SEQ ID N° 1 à 50 et identifiés aux positions nucléotidiques 61 et 62, et la sensibilité est d'au moins 73 % et la spécificité d'au moins 98 %,
c. le panel d'un ou plusieurs CpG comprend au moins les CpG définis par les SEQ ID N° 1 à 100 et identifiés aux positions nucléotidiques 61 et 62, et la sensibilité est d'au moins 75 % et la spécificité d'au moins 98 %, ou
d. le panel d'un ou plusieurs CpG comprend au moins les CpG définis par les SEQ ID N° 1 à 150 et identifiés aux positions nucléotidiques 61 et 62, et la sensibilité est d'au moins 79 % et la spécificité d'au moins 97 %,
le dosage comprenant de préférence la détermination des valeurs β de méthylation pour chaque CpG du panel d'un ou plusieurs CpG, ou
iii) supérieure ou égale à environ 1,072, l'individu est considéré comme atteint d'un cancer de l'endomètre ou comme présentant un risque élevé de développer un cancer de l'endomètre, ou lorsque la valeur de l'indice de cancer de l'individu est inférieure à environ 1,072, l'individu est considéré comme non atteint d'un cancer de l'endomètre ou comme présentant un faible risque de développer un cancer de l'endomètre, étant entendu de préférence que :
a. le panel d'un ou plusieurs CpG comprend au moins 50 des CpG identifiés aux positions nucléotidiques 61 et 62 dans les SEQ ID N° 1 à 500, et la sensibilité est d'au moins 58 % et la spécificité d'au moins 99 %,
b. le panel d'un ou plusieurs CpG comprend au moins les CpG définis par les SEQ ID N° 1 à 50 et identifiés aux positions nucléotidiques 61 et 62, et la sensibilité est d'au moins 60 % et la spécificité est de 100 %,
c. le panel d'un ou plusieurs CpG comprend au moins les CpG définis par les SEQ ID N° 1 à 100 et identifiés aux positions nucléotidiques 61 et 62, et la sensibilité est d'au moins 63 % et la spécificité est de 100 %, ou
d. le panel d'un ou plusieurs CpG comprend au moins les CpG définis par les SEQ ID N° 1 à 150 et identifiés aux positions nucléotidiques 61 et 62, et la sensibilité est d'au moins 64 % et la spécificité est de 100 %,
le dosage comprenant de préférence la détermination des valeurs β de méthylation pour chaque CpG du panel d'un ou plusieurs CpG.

5. Dosage selon l'une quelconque des revendications 1 à 4, dans lequel, lorsque la valeur de l'indice de cancer relatif à l'individu est :
a. inférieure à environ -0,660, l'individu est considéré comme non atteint d'un cancer de l'endomètre,
b. égale ou supérieure à environ -0,660 et inférieure à environ -0,430, l'individu est considéré comme présentant un faible risque de développer un cancer de l'endomètre,
c. égale ou supérieure à environ -0,430 et inférieure à environ -0,230, l'individu est considéré comme présentant un risque modéré de développer un cancer de l'endomètre, ou
d. égale ou supérieure à environ -0,230, l'individu est considéré comme présentant un risque élevé de développer un cancer de l'endomètre,
le dosage comprenant de préférence la détermination des valeurs β de méthylation pour chaque CpG du panel d'un ou plusieurs CpG.

6. Dosage selon la revendication 1 ou selon l'une quelconque des revendications précédentes, dans lequel l'étape de détermination du statut de méthylation d'un panel d'un ou plusieurs CpG comprend la détermination du statut de méthylation d'un ou plusieurs CpG désignés par CG identifiés dans un panel d'une ou plusieurs DMR définies par les SEQ ID N° 501 à 808, le panel d'un ou plusieurs CpG comprenant éventuellement au moins deux CpG désignés par CG identifiés dans le panel de DMR, au moins trois CpG désignés par CG identifiés dans le panel de DMR, au moins quatre CpG désignés par CG identifiés dans le panel de DMR, ou la totalité des CpG désignés par CG identifiés dans la ou les DMR définies par les SEQ ID N° 501 à 808, étant entendu éventuellement que :
I) l'étape de détermination du statut de méthylation d'un panel du ou des CpG comprend la détermination du statut de méthylation d'au moins cinq, au moins six, au moins sept, au moins huit ou au moins neuf, ou de la totalité des CpG désignés par CG dans une ou plusieurs des DMR définies par les SEQ ID N° 501 à 808, et/ou
II) l'étape de détermination du statut de méthylation d'un panel d'un ou plusieurs CpG comprend la détermination du statut de méthylation d'au moins deux, au moins trois, au moins quatre, au moins cinq, au moins six, au moins sept, au moins huit ou au moins neuf, ou de la totalité des CpG désignés par CG dans :
a. une quelconque combinaison d'au moins deux, trois, quatre, cinq, six, sept, huit ou neuf des DMR 1 à 308,
b. une quelconque combinaison d'au moins dix, vingt, trente, quarante, cinquante, soixante, soixante-dix, quatre-vingts ou quatre-vingt-dix des DMR 1 à 308,
c. la totalité des 237 DMR 1 à 308,
d. une DMR définie par la SEQ ID N° : 501, deux DMR définies par les SEQ ID N° : 501 et 502, trois DMR définies par les SEQ ID N° : 501 à 503, quatre DMR définies par les SEQ ID N° : 501 à 504, cinq DMR définies par les SEQ ID N° : 501 à 505, six DMR définies par les SEQ ID N° : 501 à 506, sept DMR définies par les SEQ ID N° : 501 à 507, huit DMR définies par les SEQ ID N° : 501 à 508 ou neuf DMR définies par les SEQ ID N° : 501 à 509,
e. une DMR définie par les SEQ ID N° 922, 924 et 920, de préférence toutes les DMR définies par les SEQ ID N° 922, 924 et 920,
f. une quelconque combinaison de :
i. une ou plusieurs DMR définies par les SEQ ID N° 525, 756 et 757, de préférence toutes les DMR définies par les SEQ ID N° 525, 756 et 757,
ii. une ou plusieurs DMR définies par les SEQ ID N° : 503, 504, 526, 740, 741, 743 et 744, de préférence toutes les DMR définies par les SEQ ID N° : 503, 504, 526, 740, 741, 743 et 744,
iii. une ou plusieurs DMR définies par les SEQ ID N° : 525, 756, 757, 503, 504, 526, 740, 741, 743 et 744, de préférence toutes les DMR définies par les SEQ ID N° : 525, 756, 757, 503, 504, 526, 740, 741, 743 et 744, ou
g. dix DMR définies par les SEQ ID N° : 501 à 510, vingt DMR définies par les SEQ ID N° : 501 à 520, trente DMR définies par les SEQ ID N° : 501 à 530, quarante DMR définies par les SEQ ID N° : 501 à 540, cinquante DMR définies par les SEQ ID N° : 501 à 550, soixante DMR définies par les SEQ ID N° : 501 à 560, soixante-dix DMR définies par les SEQ ID N° : 501 à 570, quatre-vingts DMR définies par les SEQ ID N° : 501 à 580 ou quatre-vingt-dix DMR définies par les SEQ ID N° : 501 à 590, ou
h. dix DMR définies par les SEQ ID N° : 501 à 510, les SEQ ID N° : 511 à 520, les SEQ ID N° : 521 à 530, les SEQ ID N° : 531 à 540, les SEQ ID N° : 541 à 550, les SEQ ID N° : 551 à 560, les SEQ ID N° : 561 à 570, les SEQ ID N° : 571 à 580, les SEQ ID N° : 581 à 590, les SEQ ID N° : 591 à 600, les SEQ ID N° : 601 à 610, les SEQ ID N° : 611 à 620, les SEQ ID N° : 621 à 630, les SEQ ID N° : 631 à 640, les SEQ ID N° : 641 à 650, les SEQ ID N° : 651 à 660, les SEQ ID N° : 661 à 670, les SEQ ID N° : 671 à 680, les SEQ ID N° : 681 à 690, les SEQ ID N° : 691 à 700, les SEQ ID N° : 701 à 710, les SEQ ID N° : 711 à 720, les SEQ ID N° : 721 à 730, les SEQ ID N° : 731 à 740, les SEQ ID N° : 741 à 750, les SEQ ID N° : 751 à 760, les SEQ ID N° : 761 à 770, les SEQ ID N° : 771 à 780, les SEQ ID N° : 781 à 790, les SEQ ID N° : 791 à 800 ou les SEQ ID N° : 801 à 808.

7. Dosage selon la revendication 6,
I) dans lequel l'étape de détermination du statut de méthylation d'un panel d'un ou plusieurs CpG comprend la détermination du statut de méthylation d'un ou plusieurs CpG dans une ou plusieurs DMR choisies dans le groupe de DMR constitué des DMR 1 à 308 définies par les SEQ ID N° 501 à 808, dont :
a. un ou plusieurs CpG de la DMR 1 définie par la SEQ ID N° : 501 et désignés par CG, le dosage se caractérisant de préférence par une aire sous la courbe ROC d'au moins 0,911, et le panel d'un ou plusieurs CpG comprenant plus préférablement au moins les CpG désignés par [[CG]],
b. un ou plusieurs CpG de la DMR 2 définie par la SEQ ID N° : 502 et désignés par CG, le dosage se caractérisant de préférence par une aire sous la courbe ROC d'au moins 0,903, et le panel d'un ou plusieurs CpG comprenant plus préférablement au moins les CpG désignés par [[CG]],
c. un ou plusieurs CpG de la DMR 3 définie par la SEQ ID N° : 503 et désignés par CG, le dosage se caractérisant de préférence par une aire sous la courbe ROC d'au moins 0,899, et le panel d'un ou plusieurs CpG comprenant plus préférablement au moins les CpG désignés par [[CG]],
d. un ou plusieurs CpG de la DMR 4 définie par la SEQ ID N° : 504 et désignés par CG, le dosage se caractérisant de préférence par une aire sous la courbe ROC d'au moins 0,899, et le panel d'un ou plusieurs CpG comprenant plus préférablement au moins les CpG désignés par [[CG]],
e. un ou plusieurs CpG de la DMR 5 définie par la SEQ ID N° : 505 et désignés par CG, le dosage se caractérisant de préférence par une aire sous la courbe ROC d'au moins 0,899, et le panel d'un ou plusieurs CpG comprenant plus préférablement au moins les CpG désignés par [[CG]],
f. un ou plusieurs CpG de la DMR 6 définie par la SEQ ID N° : 506 et désignés par CG, le dosage se caractérisant de préférence par une aire sous la courbe ROC d'au moins 0,897, et le panel d'un ou plusieurs CpG comprenant plus préférablement au moins les CpG désignés par [[CG]],
g. un ou plusieurs CpG de la DMR 7 définie par la SEQ ID N° : 507 et désignés par CG, le dosage se caractérisant de préférence par une aire sous la courbe ROC d'au moins 0,894, et le panel d'un ou plusieurs CpG comprenant plus préférablement au moins les CpG désignés par [[CG]],
h. un ou plusieurs CpG de la DMR 8 définie par la SEQ ID N° : 508 et désignés par CG, le dosage se caractérisant de préférence par une aire sous la courbe ROC d'au moins 0,894, et le panel d'un ou plusieurs CpG comprenant plus préférablement au moins les CpG désignés par [[CG]],
i. un ou plusieurs CpG de la DMR 9 définie par la SEQ ID N° : 509 et désignés par CG, le dosage se caractérisant de préférence par une aire sous la courbe ROC d'au moins 0,892, et le panel d'un ou plusieurs CpG comprenant plus préférablement au moins les CpG désignés par [[CG]],ou
j. un ou plusieurs CpG de la DMR 10 définie par la SEQ ID N° : 510 et désignés par CG, le dosage se caractérisant de préférence par une aire sous la courbe ROC d'au moins 0,891, et le panel d'un ou plusieurs CpG comprenant plus préférablement au moins les CpG désignés par [[CG]] ; et/ou
II) dans lequel
a. lorsque l'indice de cancer relatif à l'individu est supérieur ou égal à environ 0,025, l'individu est classé comme atteint d'un cancer de l'endomètre ou comme présentant un risque élevé de développer un cancer de l'endomètre, ou
b. lorsque l'indice de cancer relatif à l'individu est inférieur à environ 0,025, l'individu est classé comme non atteint d'un cancer de l'endomètre,
le dosage présentant de préférence une spécificité d'au moins 95 %, et le dosage comprenant plus préférablement la détermination de la moyenne des valeurs β de méthylation pour chaque CpG du panel d'un ou plusieurs CpG ; ou
III) dans lequel :
a. des CpG désignés par CG dont la valeur de l'indice de cancer est déterminée sont situés au moins dans la DMR 1 définie par la SEQ ID N° : 501, et lorsque l'indice de cancer de l'individu est supérieur ou égal à environ 0,209, l'individu est classé comme atteint d'un cancer de l'endomètre ou présentant un risque élevé de développer un cancer de l'endomètre, la sensibilité du dosage étant d'au moins 70,00 % et sa spécificité d'au moins 95,00 %, le panel d'un ou plusieurs CpG comprenant préférablement au moins trois CpG issus de la DMR 1, et la valeur de l'indice de cancer étant plus préférablement la moyenne des valeurs β des CpG désignés par [[CG]] dans la SEQ ID N° : 501,
b. des CpG désignés par CG dont la valeur de l'indice de cancer est déterminée sont situés au moins dans la DMR 1 définie par la SEQ ID N° : 501, et lorsque l'indice de cancer de l'individu est inférieur à environ 0,209, l'individu est classé comme n'étant pas atteint d'un cancer de l'endomètre ou présentant un faible risque de développer un cancer de l'endomètre, la sensibilité du dosage étant d'au moins 70,00 % et sa spécificité d'au moins 95,00 %, le panel d'un ou plusieurs CpG comprenant préférablement au moins trois CpG issus de la DMR 1, et la valeur de l'indice de cancer étant plus préférablement la moyenne des valeurs β des CpG désignés par [[CG]] dans la SEQ ID N° : 501,
c. des CpG désignés par CG dont la valeur de l'indice de cancer est déterminée sont situés au moins dans la DMR 2 définie par la SEQ ID N° : 502, et lorsque l'indice de cancer de l'individu est supérieur ou égal à environ 0,271, l'individu est classé comme atteint d'un cancer de l'endomètre ou présentant un risque élevé de développer un cancer de l'endomètre, la sensibilité du dosage étant d'au moins 77,00 % et sa spécificité d'au moins 95,00 %, le panel d'un ou plusieurs CpG comprenant préférablement au moins un CpG issu de la DMR 2, et la valeur de l'indice de cancer étant plus préférablement la moyenne des valeurs β des CpG désignés par [[CG]] dans la SEQ ID N° : 502,
d. des CpG désignés par CG dont la valeur de l'indice de cancer est déterminée sont situés au moins dans la DMR 2 définie par la SEQ ID N° : 502, et lorsque l'indice de cancer de l'individu est inférieur à environ 0,271, l'individu est classé comme n'étant pas atteint d'un cancer de l'endomètre ou présentant un faible risque de développer un cancer de l'endomètre, la sensibilité du dosage étant d'au moins 77,00 % et sa spécificité d'au moins 95,00 %, le panel d'un ou plusieurs CpG comprenant préférablement au moins un CpG issu de la DMR 2, et la valeur de l'indice de cancer étant plus préférablement la moyenne des valeurs β des CpG désignés par [[CG]] dans la SEQ ID N° : 502,
e. des CpG désignés par CG dont la valeur de l'indice de cancer est déterminée sont situés au moins dans la DMR 3 définie par la SEQ ID N° : 503, et lorsque l'indice de cancer de l'individu est supérieur ou égal à environ 0,123, l'individu est classé comme atteint d'un cancer de l'endomètre ou présentant un risque élevé de développer un cancer de l'endomètre, la sensibilité du dosage étant d'au moins 73,00 % et sa spécificité d'au moins 95,00 %, le panel d'un ou plusieurs CpG comprenant préférablement au moins trois CpG issus de la DMR 3, et la valeur de l'indice de cancer étant plus préférablement la moyenne des valeurs β des CpG désignés par [[CG]] dans la SEQ ID N° : 503,
f. des CpG désignés par CG dont la valeur de l'indice de cancer est déterminée sont situés au moins dans la DMR 3 définie par la SEQ ID N° : 503, et lorsque l'indice de cancer de l'individu est inférieur à environ 0,123, l'individu est classé comme n'étant pas atteint d'un cancer de l'endomètre ou présentant un faible risque de développer un cancer de l'endomètre, la sensibilité du dosage étant d'au moins 73,00 % et sa spécificité d'au moins 95,00 %, le panel d'un ou plusieurs CpG comprenant préférablement au moins trois CpG issus de la DMR 3, et la valeur de l'indice de cancer étant plus préférablement la moyenne des valeurs β des CpG désignés par [[CG]] dans la SEQ ID N° : 503,
g. des CpG désignés par CG dont la valeur de l'indice de cancer est déterminée sont situés au moins dans la DMR 4 définie par la SEQ ID N° : 504, et lorsque l'indice de cancer de l'individu est supérieur ou égal à environ 0,123, l'individu est classé comme atteint d'un cancer de l'endomètre ou présentant un risque élevé de développer un cancer de l'endomètre, la sensibilité du dosage étant d'au moins 73,00 % et sa spécificité d'au moins 95,00 %, le panel d'un ou plusieurs CpG comprenant préférablement au moins trois CpG issus de la DMR 4, et la valeur de l'indice de cancer étant plus préférablement la moyenne des valeurs β des CpG désignés par [[CG]] dans la SEQ ID N° : 504,
h. des CpG désignés par CG dont la valeur de l'indice de cancer est déterminée sont situés au moins dans la DMR 4 définie par la SEQ ID N° : 504, et lorsque l'indice de cancer de l'individu est inférieur à environ 0,123, l'individu est classé comme n'étant pas atteint d'un cancer de l'endomètre ou présentant un faible risque de développer un cancer de l'endomètre, la sensibilité du dosage étant d'au moins 73,00 % et sa spécificité d'au moins 95,00 %, le panel d'un ou plusieurs CpG comprenant préférablement au moins trois CpG issus de la DMR 4, et la valeur de l'indice de cancer étant plus préférablement la moyenne des valeurs β des CpG désignés par [[CG]] dans la SEQ ID N° : 504,
i. des CpG désignés par CG dont la valeur de l'indice de cancer est déterminée sont situés au moins dans la DMR 5 définie par la SEQ ID N° : 505, et lorsque l'indice de cancer de l'individu est supérieur ou égal à environ 0,105, l'individu est classé comme atteint d'un cancer de l'endomètre ou présentant un risque élevé de développer un cancer de l'endomètre, la sensibilité du dosage étant d'au moins 71,00 % et sa spécificité d'au moins 95,00 %, le panel d'un ou plusieurs CpG comprenant préférablement au moins cinq CpG issus de la DMR 5, et la valeur de l'indice de cancer étant plus préférablement la moyenne des valeurs β des CpG désignés par [[CG]] dans la SEQ ID N° : 505, ou
j. des CpG désignés par CG dont la valeur de l'indice de cancer est déterminée sont situés au moins dans la DMR 5 définie par la SEQ ID N° : 505, et lorsque l'indice de cancer de l'individu est inférieur à environ 0,105, l'individu est classé comme n'étant pas atteint d'un cancer de l'endomètre ou présentant un faible risque de développer un cancer de l'endomètre, la sensibilité du dosage étant d'au moins 71,00 % et sa spécificité d'au moins 95,00 %, le panel d'un ou plusieurs CpG comprenant préférablement au moins cinq CpG issus de la DMR 5, et la valeur de l'indice de cancer étant plus préférablement la moyenne des valeurs β des CpG désignés par [[CG]] dans la SEQ ID N° : 505.

8. Dosage selon la revendication 1 ou l'une quelconque des revendications précédentes, dans lequel l'étape de détermination du statut de méthylation du ou des CpG du panel comprend en outre ou comprend également la détermination du statut de méthylation de chaque CpG dans une ou plusieurs séquences identifiées par les SEQ ID N° 809 à 919, et l'étape de détermination du statut de méthylation du ou des CpG du panel comprend éventuellement la détermination de chaque CpG dans :
a. les SEQ ID N° 809 et/ou SEQ ID N° 846 et/ou SEQ ID N° 883, et lorsque la valeur de l'indice de cancer est supérieure ou égale à environ 0,282, l'individu est classé comme atteint d'un cancer de l'endomètre ou comme présentant un risque élevé de développer un cancer de l'endomètre, la sensibilité du dosage étant d'au moins 85,00 % et sa spécificité étant d'environ 100,00 %, et l'AUC étant d'environ 1,00, la valeur de l'indice de cancer étant de préférence le pourcentage de référence méthylée pour la séquence définie par les SEQ ID N° 809 et/ou SEQ ID N° 846 et/ou SEQ ID N° 883,
b. les SEQ ID N° 809 et/ou SEQ ID N° 846 et/ou SEQ ID N° 883, et lorsque la valeur de l'indice de cancer est inférieure à environ 0,282, l'individu est classé comme n'étant pas atteint d'un cancer de l'endomètre ou comme présentant un faible risque de développer un cancer de l'endomètre, la sensibilité du dosage étant d'au moins 85,00 % et sa spécificité étant d'environ 100,00 %, et l'AUC étant d'environ 1,00, la valeur de l'indice de cancer étant de préférence le pourcentage de référence méthylée pour la séquence définie par les SEQ ID N° 809 et/ou SEQ ID N° 846 et/ou SEQ ID N° 883,
c. les SEQ ID N° 810 et/ou SEQ ID N° 847 et/ou SEQ ID N° 884, et lorsque la valeur de l'indice de cancer est supérieure ou égale à environ 0,212, l'individu est classé comme atteint d'un cancer de l'endomètre ou comme présentant un risque élevé de développer un cancer de l'endomètre, la sensibilité du dosage étant d'au moins 55,00 % et sa spécificité étant d'environ 100,00 %, et l'AUC étant d'environ 1,00, la valeur de l'indice de cancer étant de préférence le pourcentage de référence méthylée pour la séquence définie par les SEQ ID N° 810 et/ou SEQ ID N° 847 et/ou SEQ ID N° 884,
d. les SEQ ID N° 810 et/ou SEQ ID N° 847 et/ou SEQ ID N° 884, et lorsque la valeur de l'indice de cancer est inférieure à environ 0,212, l'individu est classé comme n'étant pas atteint d'un cancer de l'endomètre ou comme présentant un faible risque de développer un cancer de l'endomètre, la sensibilité du dosage étant d'au moins 55,00 % et sa spécificité étant d'environ 100,00 %, et l'AUC étant d'environ 1,00, la valeur de l'indice de cancer étant de préférence le pourcentage de référence méthylée pour la séquence définie par les SEQ ID N° 810 et/ou SEQ ID N° 847 et/ou SEQ ID N° 884,
e. les SEQ ID N° 811 et/ou SEQ ID N° 848 et/ou SEQ ID N° 885, et lorsque la valeur de l'indice de cancer est supérieure ou égale à environ 0,002, l'individu est classé comme atteint d'un cancer de l'endomètre ou comme présentant un risque élevé de développer un cancer de l'endomètre, la sensibilité du dosage étant d'au moins 90,00 % et sa spécificité étant d'environ 80,00 %, et l'AUC étant d'environ 0,98, la valeur de l'indice de cancer étant de préférence le pourcentage de référence méthylée pour la séquence définie par les SEQ ID N° 811 et/ou SEQ ID N° 848 et/ou SEQ ID N° 885,
f. les SEQ ID N° 811 et/ou SEQ ID N° 848 et/ou SEQ ID N° 885, et lorsque la valeur de l'indice de cancer est inférieure à environ 0,002, l'individu est classé comme n'étant pas atteint d'un cancer de l'endomètre ou comme présentant un faible risque de développer un cancer de l'endomètre, la sensibilité du dosage étant d'au moins 90,00 % et sa spécificité étant d'environ 80,00 %, et l'AUC étant d'environ 0,98, la valeur de l'indice de cancer étant de préférence le pourcentage de référence méthylée pour la séquence définie par les SEQ ID N° 811 et/ou SEQ ID N° 848 et/ou SEQ ID N° 885,
g. les SEQ ID N° 812 et/ou SEQ ID N° 849 et/ou SEQ ID N° 886, et lorsque la valeur de l'indice de cancer est supérieure ou égale à environ 0,026, l'individu est classé comme atteint d'un cancer de l'endomètre ou comme présentant un risque élevé de développer un cancer de l'endomètre, la sensibilité du dosage étant d'au moins 85,00 % et sa spécificité étant d'environ 90,00 %, et l'AUC étant d'environ 0,98, la valeur de l'indice de cancer étant de préférence le pourcentage de référence méthylée pour la séquence définie par les SEQ ID N° 812 et/ou SEQ ID N° 849 et/ou SEQ ID N° 886,
h. les SEQ ID N° 812 et/ou SEQ ID N° 849 et/ou SEQ ID N° 886, et lorsque la valeur de l'indice de cancer est inférieure à environ 0,026, l'individu est classé comme n'étant pas atteint d'un cancer de l'endomètre ou comme présentant un faible risque de développer un cancer de l'endomètre, la sensibilité du dosage étant d'au moins 85,00 % et sa spécificité étant d'environ 90,00 %, et l'AUC étant d'environ 0,98, la valeur de l'indice de cancer étant de préférence le pourcentage de référence méthylée pour la séquence définie par les SEQ ID N° 812 et/ou SEQ ID N° 849 et/ou SEQ ID N° 886,
i. les SEQ ID N° 813 et/ou SEQ ID N° 850 et/ou SEQ ID N° 887, et lorsque la valeur de l'indice de cancer est supérieure ou égale à environ 0,003, l'individu est classé comme atteint d'un cancer de l'endomètre ou comme présentant un risque élevé de développer un cancer de l'endomètre, la sensibilité du dosage étant d'au moins 85,00 % et sa spécificité étant d'environ 85,00 %, et l'AUC étant d'environ 0,97, la valeur de l'indice de cancer étant de préférence le pourcentage de référence méthylée pour la séquence définie par les SEQ ID N° 813 et/ou SEQ ID N° 850 et/ou SEQ ID N° 887,
j. les SEQ ID N° 813 et/ou SEQ ID N° 850 et/ou SEQ ID N° 887, et lorsque la valeur de l'indice de cancer est inférieure à environ 0,003, l'individu est classé comme n'étant pas atteint d'un cancer de l'endomètre ou comme présentant un faible risque de développer un cancer de l'endomètre, la sensibilité du dosage étant d'au moins 85,00 % et sa spécificité étant d'environ 85,00 %, et l'AUC étant d'environ 0,97, la valeur de l'indice de cancer étant de préférence le pourcentage de référence méthylée pour la séquence définie par les SEQ ID N° 813 et/ou SEQ ID N° 850 et/ou SEQ ID N° 887.

9. Dosage selon l'une quelconque des revendications 1 à 8, dans lequel :
I) l'étape de détermination, au sein de la population de molécules d'ADN de l'échantillon, du statut de méthylation de chaque CpG du panel d'un ou plusieurs CpG comprend :
a. la réalisation d'une étape de séquençage pour déterminer la séquence de chaque CpG,
b. l'hybridation de l'ADN sur un ensemble comprenant des sondes capables de distinguer les formes méthylées et non méthylées des CpG, et l'application d'un système de détection à l'ensemble afin de déterminer le statut de méthylation de chaque CpG, et/ou
c. la réalisation d'une étape de PCR au moyen d'amorces spécifiques à la méthylation, le statut de méthylation du CpG étant déterminé par la présence ou l'absence d'un produit de PCR, et/ou
II) l'étape de détermination du statut de méthylation de chaque CpG du panel d'un ou plusieurs CpG comprend :
a. la conversion de l'ADN par le bisulfite, ou
b. la réalisation des étapes d'oxydation des bases 5-méthylcytosine (5mC) en bases 5-carboxylcytosine (5caC), de préférence par translocation dix-onze (TET, ten-eleven translocation) et/ou d'oxydation des bases 5-hydroxyméthylcytosine (5hmC) en bases 5-carboxylcytosine (5caC), de préférence par translocation dix-onze (TET) ; puis la réduction des bases 5-carboxylcytosine (5caC) en bases dihydro-uracile (DHU), éventuellement au moyen de pyridine borane.

10. Dosage selon l'une quelconque des revendications précédentes, dans lequel le cancer de l'endomètre est un cancer endométrioïde, un carcinosarcome utérin, un carcinome épidermoïde, un carcinome à petites cellules, un carcinome de transition, un carcinome séreux, un carcinome à cellules claires, un adénocarcinome mucineux, un carcinome indifférencié, un carcinome dédifférencié ou un adénocarcinome séreux.
